# EUROPEAN PATENT APPLICATION

(11) **EP 4 768 468 A2**
(43) Date of publication of application: **01.07.2026**
(21) Application number: 26177219.8
(22) Date of filing: 26.10.2017
(51) Int. Cl.: C07C 255/24

(54) **LIPID NANOPARTICLE MRNA VACCINES**

(30) Priority: 26.10.2016 WO PCT/EP2016/075843; 27.10.2016 WO PCT/EP2016/075929; 09.06.2017 WO PCT/EP2017/064066
(62) Divisional of application: 17798129.7
(71) Applicant: CureVac SE, 72076 Tübingen (DE); Acuitas Therapeutics Inc., Vancouver, British Columbia V6L 2A1 (CA)
(72) Inventor: BAUMHOF, Patrick, 72076 Tübingen (DE); FOTIN-MLECZEK, Mariola, 72076 Tübingen (DE); HEIDENREICH, Regina, 72076 Tubingen (DE); HOPE, Michael J., Vancouver British Columbia V6R 2K2 (CA); JASNY, Edith, 72076 Tubingen (DE); LAZZARO, Sandra, 72076 Tübingen (DE); LIN, Paulo Jia Ching, Vancouver British Columbia, V5S 1YA (CA); LUTZ, Johannes, 72076 Tübingen (DE); MUI, Barbara, Vancouver British Columbia, V5T 2G3 (CA); PETSCH, Benjamin, 72076 Tübingen (DE); RAUCH, Susanne, 72076 Tübingen (DE); SCHWENDT, Kim Ellen, 72076 Tübingen (DE); TAM, Ying, V6S 1C3 Vancouver British Columbia (DE)
(74) Representative: Pinsent Masons LLP

(57) **Abstract**

The invention relates to mRNA comprising lipid nanoparticles and their medical uses. The lipid nanoparticles of the present invention comprise a cationic lipid according to formula (I), (II) or (III) and/or a PEG lipid according to formula (IV), as well as an mRNA compound comprising an mRNA sequence encoding an antigenic peptide or protein. The invention further relates to the use of said lipid nanoparticles as vaccines or medicaments, in particular with respect to influenza or rabies vaccination.

## Description

### Background of the Invention

The present invention relates to mRNA comprising lipid nanoparticles useful as mRNA-based vaccines. Additionally, the present invention relates to a composition comprising the mRNA comprising lipid nanoparticles and the use of the mRNA comprising lipid nanoparticles or the composition for the preparation of a pharmaceutical composition, especially a vaccine, e.g. for use in the prophylaxis or treatment of infectious diseases, tumour or cancer diseases, allergies or autoimmune diseases. The present invention further describes a method of treatment or prophylaxis of the afore-mentioned diseases.

Gene therapy and genetic vaccination belong to the most promising and quickly developing methods of modern medicine. They may provide highly specific and individual options for therapy of a large variety of diseases.

Genetic vaccination allows evoking a desired immune response to selected antigens, such as characteristic components of bacterial surfaces, viral particles, tumour antigens or the like. Generally, vaccination is one of the pivotal achievements of modern medicine. However, effective vaccines are currently available only for a limited number of diseases. Accordingly, infections that are not preventable by vaccination still affect millions of people every year.

Commonly, vaccines may be subdivided into "first", "second" and "third" generation vaccines. "First generation" vaccines are, typically, whole-organism vaccines. They are based on either live and attenuated or killed pathogens, e.g. viruses, bacteria or the like. The major drawback of live and attenuated vaccines is the risk for a reversion to life-threatening variants. Thus, although attenuated, such pathogens may still intrinsically bear unpredictable risks. Killed pathogens may not be as effective as desired for generating a specific immune response. In order to minimize these risks, "second generation" vaccines were developed. These are, typically, subunit vaccines, consisting of defined antigens or recombinant protein components which are derived from pathogens.

Genetic vaccines, i.e. vaccines for genetic vaccination, are usually understood as "third generation" vaccines. They are typically composed of genetically engineered nucleic acid molecules which allow expression of peptide or protein (antigen) fragments characteristic for a pathogen or a tumor antigen in vivo. Genetic vaccines are expressed upon administration to a patient after uptake by target cells. Expression of the administered nucleic acids results in production of the encoded proteins. In the event these proteins are recognized as foreign by the patient's immune system, an immune response is triggered.

DNA as well as RNA may be used as nucleic acid molecules for administration in the context of genetic vaccination. DNA is known to be relatively stable and easy to handle. However, the use of DNA bears the risk of undesired insertion of the administered DNA-fragments into the patient's genome potentially resulting mutagenic events such as in loss of function of the impaired genes. As a further risk, the undesired generation of anti-DNA antibodies has emerged. Another drawback is the limited expression level of the encoded peptide or protein that is achievable upon DNA administration because the DNA must enter the nucleus in order to be transcribed before the resulting mRNA can be translated. Among other reasons, the expression level of the administered DNA will be dependent on the presence of specific transcription factors which regulate DNA transcription. In the absence of such factors, DNA transcription will not yield satisfying amounts of RNA. As a result, the level of translated peptide or protein obtained is limited.

By using RNA instead of DNA for genetic vaccination, the risk of undesired genomic integration and generation of anti-DNA antibodies is minimized or avoided. However, RNA is considered to be a rather unstable molecular species which may readily be degraded by ubiquitous RNAses.

mRNA vaccines comprising antigen-encoding mRNA complexed to protamine are already described in the prior art (e.g. Petsch et al., Nat Biotechnol. 2012 Dec;30(12):1210-6., Schnee et al., PLoS Negl Trop Dis. 2016 Jun 23;10(6):e0004746., EP1083232, WO2010/037539, WO2012/116811, WO2012/116810, and WO2015/024665). Also WO2016/176330 describes lipid nanoparticle compositions comprising nucleoside-modified RNA encoding different antigens.

Even if a lot of progress was made in the last years there is still a need in the art for providing an efficient method for mRNA vaccination, which allows eliciting an adaptive immune response, wherein the administration is not severely impaired by early degradation of the antigen or by an inefficient translation of the mRNA due to inefficient release of the mRNA in the cell. Furthermore, there is an urgent need to decrease the dose of mRNA vaccines to decrease potential safety concerns and to make the vaccines affordable for the third world.

There are many challenges associated with the delivery of nucleic acids to effect a desired response in a biological system. Nucleic acid based therapeutics, such as vaccines, have enormous potential but there remains a need for more effective delivery of nucleic acids to appropriate sites within a cell or organism in order to realize this potential.

However, two problems currently face the use of oligonucleotides in therapeutic contexts. First, free RNAs are susceptible to nuclease digestion in plasma. Second, free RNAs have limited ability to gain access to the intracellular compartment where the relevant translation machinery resides. Lipid nanoparticles formed from cationic lipids with other lipid components, such as neutral lipids, cholesterol, PEG, PEGylated lipids, and oligonucleotides have been used to block degradation of the RNAs in plasma and facilitate the cellular uptake of the oligonucleotides.

There remains a need for improved cationic lipids and lipid nanoparticles for the delivery of oligonucleotides. Preferably, these lipid nanoparticles would provide optimal drug:lipid ratios, protect the nucleic acid from degradation and clearance in serum, be suitable for systemic or local delivery, and provide intracellular delivery of the nucleic acid. In addition, these lipid-nucleic acid particles should be well-tolerated and provide an adequate therapeutic index, such that patient treatment at an effective dose of the nucleic acid is not associated with unacceptable toxicity and/or risk to the patient. The present invention provides these and related advantages.

### Summary of the Invention

The present invention provides mRNA comprising lipid nanoparticles or pharmaceutical compositions comprising said nanoparticles as well as the uses thereof. mRNA comprising lipid nanoparticles according to the invention comprise:
(i) a cationic lipid with the formula (I):
   or a pharmaceutically acceptable salt, tautomer or stereoisomer thereof, wherein R^{1a}, R^{1b}, R^{2a}, R^{2b}, R^{3a}, R^{3b}, R^{4a}, R^{4b}, R⁵, R⁶, R⁷, R⁸, R⁹, L¹, L², a, b, c, d and e are as defined herein;
      and or
   a cationic lipid with the formula (II):
   or a pharmaceutically acceptable salt, tautomer, prodrug or stereoisomer thereof, wherein R^{1a}, R^{1b}, R^{2a}, R^{2b}, R^{3a}, R^{3b}, R^{4a}, R^{4b}, R⁵, R⁶, R⁷, R⁸, R⁹, L¹, L², G¹, G², G³, a, b, c and d are as defined herein;
   and/or preferably
   a cationic lipid with the formula III:
   or a pharmaceutically acceptable salt, tautomer, prodrug or stereoisomer thereof, wherein R¹ , R² , R³, L¹ , L² , G¹ , G² , and G³ are as defined herein.
   and/or a PEG lipid with the formula (IV)
   wherein R⁸ and R⁹ are each independently a straight or branched, saturated or unsaturated alkyl chain containing from 10 to 30 carbon atoms, wherein the alkyl chain is optionally interrupted by one or more ester bonds;
   and w has a mean value ranging from 30 to 60;
   and optionally a neutral lipid and/or a steroid or sterioid analogue, wherein the mRNA compound is encapsulated in or associated with said lipid nanoparticle.

The present invention further provides for pharmaceutical compositions comprising said lipid nanoparticles, as well as methods for producing said nanoparticles. In a further aspect the invention relates to medical uses of the lipid nanoparticles or the pharmaceutical composition comprising the same.

In a further aspect, the invention relates to methods of medical prophylaxis or treatment using said mRNA comprising lipid nanoparticles.

### Definitions

For the sake of clarity and readability, the following scientific background information and definitions are provided. Any technical features disclosed thereby can be part of each and every embodiment of the invention. Additional definitions and explanations can be provided in the context of this disclosure.

Unless defined otherwise, or unless the specific context requires otherwise, all technical terms used herein have the same meaning as is commonly understood by a person skilled in the relevant technical field.

Unless the context indicates or requires otherwise, the words "comprise", "comprises" and "comprising" and similar expressions are to be construed in an open and inclusive sense, as "including, but not limited to" in this description and in the claims.

The expressions, "one embodiment", "an embodiment", "a specific embodiment" and the like mean that a particular feature, property or characteristic, or a particular group or combination of features, properties or characteristics, as referred to in combination with the respective expression, is present in at least one of the embodiments of the invention. The occurrence of these expressions in various places throughout this description do not necessarily refer to the same embodiment. Moreover, the particular features, properties or characteristics may be combined in any suitable manner in one or more embodiments.

The singular forms "a", "an" and "the" should be understood as to include plural references unless the context clearly dictates otherwise.

Percentages in the context of numbers should be understood as relative to the total number of the respective items. In other cases, and unless the context dictates otherwise, percentages should be understood as percentages by weight (wt.-%).

In the context of the invention, a "composition" refers to any type of composition in which the specified ingredients may be incorporated, optionally along with any further constituents, usually with at least one pharmaceutically acceptable carrier or excipient. Thus, the composition may be a dry composition such as a powder or granules, or a solid unit such as a lyophilised form or a tablet. Alternatively, the composition may be in liquid form, and each constituent may be independently incorporated in dissolved or dispersed (e.g. suspended or emulsified) form. In one of the preferred embodiments, the composition is formulated as a sterile solid composition, such as a powder or lyophilised form for reconstitution with an aqueous liquid carrier.

Such formulation is also preferred for those versions of the composition which comprise a nucleic acid cargo as described in further detail below.

As used herein, a "compound" means a chemical substance, which is a material consisting of molecules having essentially the same chemical structure and properties. For a small molecular compound, the molecules are typically identical with respect to their atomic composition and structural configuration. For a macromolecular or polymeric compound, the molecules of a compound are highly similar but not all of them are necessarily identical. For example, a segment of a polymer that is designated to consist of 50 monomeric units may also contain individual molecules with e.g. 48 or 53 monomeric units.

A lipidoid compound, also simply referred to as lipidoid, is a lipid-like compound, i.e. an amphiphilic compound with lipid-like physical properties. In the context of the present invention the term lipid is considered to encompass lipidoids.

Unless a different meaning is clear from the specific context, the term "cationic" means that the respective structure bears a positive charge, either permanently, or not permanently but in response to certain conditions such as pH. Thus, the term "cationic" covers both "permanently cationic" and "cationisable".

As used herein, "permanently cationic" means that the respective compound, or group or atom, is positively charged at any pH value or hydrogen ion activity of its environment. Typically, the positive charge is results from the presence of a quaternary nitrogen atom. Where a compound carries a plurality of such positive charges, it may be referred to as permanently polycationic, which is a subcategory of permanently cationic.

Cationic component/compound:The term "cationic component/compound" typically refers to a charged molecule, which is positively charged (cation) at a pH value of typically about 1 to 9. In some embodiments, the cationic component/compound is preferably charged at a pH value of or below 9 (e.g. 5 to 9), of or below 8 (e.g. 5 to 8), of or below 7 (e.g. 5 to 7), most preferably at physiological pH values, e.g. about 7.3 to 7.4. Accordingly, a cationic peptide, protein, polysaccharide, lipid or polymer according to one embodiment of the present invention is positively charged under physiological conditions, particularly under physiological salt conditions of the cell in vivo. In another preferred embodiment, the lipid nanoparticle, the cationic peptide, protein, polysaccharide, lipid or polymer according to the present invention is uncharged, has a neutral charge or is respectivley electrically neutral under physiological conditions, particularly under physiological salt conditions of the cell in vivo. A cationic peptide or protein preferably contains a larger number of cationic amino acids, e.g. a larger number of Arg, His, Lys or Orn than other amino acid residues (in particular more cationic amino acids than anionic amino acid residues like Asp or Glu) or contains blocks predominantly formed by cationic amino acid residues. The expression "cationic" may also refer to "polycationic" components/compounds.

The cationic component/compound may also refer to a cationic lipid capable of being positively charged. Exemplary cationic lipids include one or more amine group(s) which bear the positive charge. Preferred cationic lipids are ionizable such that they can exist in a positively charged or neutral form depending on pH. The ionization of the cationic lipid affects the surface charge of a lipid nanoparticle (LNP) under different pH conditions. This charge state can influence plasma protein absorption, blood clearance and tissue distribution (Semple, S.C., et al., Adv. Drug Deliv Rev 32:3-17 (1998)) as well as the ability to form non-bilayer structures (Hafez, I.M., et al., Gene Ther 8:1188-1196 (2001)) critical to the intracellular delivery of nucleic acids. As described elsewhere, the pKa of formulated cationic lipids is correlated with the effectiveness of LNPs for delivery of nucleic acids (see Jayaraman et al, Angewandte Chemie, International Edition (2012), 51(34), 8529-8533; Semple et al, Nature Biotechnology 28, 172-176 (2010)). In some embodiments of the present invention, the preferred range of pKa is about 5 to about 7.

In this context, the prefix "poly-" refers to a plurality of atoms or groups having the respective property in a compound. If put in parenthesis, the presence of a plurality is optional. For example, (poly)cationic means cationic and/or polycationic. However, the absence of the prefix should not be interpreted such as to exclude a plurality. For example, a polycationic compound is also a cationic compound and may be referred to as such.

"Cationisable" means that a compound, or group or atom, is positively charged at a lower pH and uncharged at a higher pH of its environment. Also in non-aqueous environments where no pH value can be determined, a cationisable compound, group or atom is positively charged at a high hydrogen ion concentration and uncharged at a low concentration or activity of hydrogen ions. It depends on the individual properties of the cationisable or polycationisable compound, in particular the pKₐ of the respective cationisable group or atom, at which pH or hydrogen ion concentration it is charged or uncharged. In diluted aqueous environments, the fraction of cationisable compounds, groups or atoms bearing a positive charge may be estimated using the so-called Henderson-Hasselbalch equation which is well-known to a person skilled in the art.

For example, in some embodiments, if a compound or moiety is cationisable, it is preferred that it is positively charged at a pH value of about 1 to 9, preferably 4 to 9, 5 to 8 or even 6 to 8, more preferably of a pH value of or below 9, of or below 8, of or below 7, most preferably at physiological pH values, e.g. about 7.3 to 7.4, i.e. under physiological conditions, particularly under physiological salt conditions of the cell in vivo. In other embodiments, it is preffered that the cationisable compound or moiety is predominantly neutral at phyisiological pH values, e.g. about 7.0-7.4, but becomes positively charged at lower pH values. In some embodiments, the preferred range of pKa for the cationisable compound or moiety is about 5 to about 7.

Nucleic acid: The term nucleic acid means any DNA- or RNA-molecule. The term may be used for a polynucleotide and/or oligonucleotide. Wherever herein reference is made to a nucleic acid or nucleic acid sequence encoding a particular protein and/or peptide, said nucleic acid or nucleic acid sequence, respectively, preferably also comprises regulatory sequences allowing in a suitable host, e.g. a human being, its expression, i.e. transcription and/or translation of the nucleic acid sequence encoding the particular protein or peptide.

Nucleoside modification: in the context of the present invention the term nucleoside modification refers to mRNA molecules or compounds comprising nucleosides, which are not usually part of mRNA, preferably non-natural nucleosides. In particular, the term preferably refers to mRNA nucleosides other than adenine, guanine, cytosine, uracil and in some cases thymine.

Peptide: A peptide is an oligomer or polymer of at least two amino acid monomers. Usually the monomers are linked by peptide bonds. The term "peptide" does not limit the length of the polymer chain of amino acids. In some embodiments of the present invention a peptide may for example contain less than 50 monomer units. Longer peptides are also called polypeptides, typically having 50 to 600 monomeric units, more specifically 50 to 300 monomeric units.

Protein: A protein typically consists of one or more peptides and/or polypeptides folded into a 3-dimensional form, facilitating a biological function.

Influenza pandemic or pandemic flu: An influenza pandemic can occur when a non-human (novel) influenza virus gains the ability for efficient and sustained human-to-human transmission and then spreads globally. Influenza viruses that have the potential to cause a pandemic are referred to as "influenza viruses with pandemic potential" or "pandemic influenza virus".

Examples of influenza viruses with pandemic potential include avian influenza A (H5N1) and avian influenza A (H7N9), which are two different "bird flu" viruses. These are non-human viruses (i.e., they are novel among humans and circulate in birds in parts of the world) so there is little to no immunity against these viruses among people. Human infections with these viruses have occurred rarely, but if either of these viruses was to change in such a way that it was able to infect humans easily and spread easily from person to person, an influenza pandemic could result.

Vaccine for pandemic influenza/flu or pandemic influenza/flu vaccine: A vaccine directed against a pandemic influenza virus is called herein as a vaccine for pandemic influenza/flu or pandemic influenza/flu vaccine.

Flu/influenza season: Flu season is an annually recurring time period characterized by the prevalence of outbreaks of influenza (flu). The season occurs during the cold half of the year in each hemisphere. Influenza activity can sometimes be predicted and even tracked geographically. While the beginning of major flu activity in each season varies by location, in any specific location these minor epidemics usually take about 3 weeks to peak and another 3 weeks to significantly diminish. Flu vaccinations have been used to diminish the effects of the flu season; pneumonia vaccinations additionally diminishes the effects and complications of flu season. Since the Northern and Southern Hemisphere have winter at different times of the year, there are actually two flu seasons each year.

Vaccine for seasonal influenza/flu or seasonal influenza/flu vaccine: A vaccine directed against the seasonal occurring influenza viruses in a flu season is termed herein "vaccine for seasonal influenza/flu or seasonal influenza/flu vaccine".

Immune system: The immune system may protect organisms from infection. If a pathogen breaks through a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts contains so called humoral and cellular components.

Immune response: An immune response may typically either be a specific reaction of the adaptive immune system to a particular antigen (so called specific or adaptive immune response) or an unspecific reaction of the innate immune system (so called unspecific or innate immune response). The invention relates to the core to specific reactions (adaptive immune responses) of the adaptive immune system. Particularly, it relates to adaptive immune responses to infections by viruses like e.g. Influenza viruses. However, this specific response can be supported by an additional unspecific reaction (innate immune response). Therefore, the invention also relates to a compound for simultaneous stimulation of the innate and the adaptive immune system to evoke an efficient adaptive immune response.

Adaptive immune system: The adaptive immune system is composed of highly specialized, systemic cells and processes that eliminate or prevent pathogenic growth. The adaptive immune response provides the vertebrate immune system with the ability to recognize and remember specific pathogens (to generate immunity), and to mount stronger attacks each time the pathogen is encountered. The system is highly adaptable because of somatic hypermutation (a process of increased frequency of somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of that cell will then inherit genes encoding the same receptor specificity, including the Memory B cells and Memory T cells that are the keys to long-lived specific immunity. Immune network theory is a theory of how the adaptive immune system works, that is based on interactions between the variable regions of the receptors of T cells, B cells and of molecules made by T cells and B cells that have variable regions.

Adaptive immune response: The adaptive immune response is typically understood to be antigen-specific. Antigen specificity allows for the generation of responses that are tailored to specific antigens, pathogens or pathogen-infected cells. The ability to mount these tailored responses is maintained in the body by "memory cells". Should a pathogen infect the body more than once, these specific memory cells are used to quickly eliminate it. In this context, the first step of an adaptive immune response is the activation of naïve antigen-specific T cells or different immune cells able to induce an antigen-specific immune response by antigen-presenting cells. This occurs in the lymphoid tissues and organs through which naïve T cells are constantly passing. Cell types that can serve as antigen-presenting cells are inter alia dendritic cells, macrophages, and B cells. Each of these cells has a distinct function in eliciting immune responses. Dendritic cells take up antigens by phagocytosis and macropinocytosis and are stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. Presenting the antigen on MHC molecules leads to activation of T cells which induces their proliferation and differentiation into armed effector T cells. The most important function of effector T cells is the killing of infected cells by CD8+ cytotoxic T cells and the activation of macrophages by Th1 cells which together make up cell-mediated immunity, and the activation of B cells by both Th2 and Th1 cells to produce different classes of antibody, thus driving the humoral immune response. T cells recognize an antigen by their T cell receptors which do not recognize and bind antigen directly, but instead recognize short peptide fragments e.g. of pathogen-derived protein antigens, which are bound to MHC molecules on the surfaces of other cells.

Cellular immunity/cellular immune response: Cellular immunity relates typically to the activation of macrophages, natural killer cells (NK), antigen-specific cytotoxic T-lymphocytes, and the release of various cytokines in response to an antigen. In a more general way, cellular immunity is not related to antibodies but to the activation of cells of the immune system. A cellular immune response is characterized e.g. by activating antigen-specific cytotoxic T-lymphocytes that are able to induce apoptosis in body cells displaying epitopes of an antigen on their surface, such as virus-infected cells, cells with intracellular bacteria, and cancer cells displaying tumor antigens; activating macrophages and natural killer cells, enabling them to destroy pathogens; and stimulating cells to secrete a variety of cytokines that influence the function of other cells involved in adaptive immune responses and innate immune responses.

Humoral immunity/humoral immune response: Humoral immunity refers typically to antibody production and the accessory processes that may accompany it. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Innate immune system: The innate immune system, also known as non-specific immune system, comprises the cells and mechanisms that defend the host from infection by other organisms in a non-specific manner. This means that the cells of the innate system recognize and respond to pathogens in a generic way, but unlike the adaptive immune system, it does not confer long-lasting or protective immunity to the host. The innate immune system may be e.g. activated by ligands of pathogen-associated molecular patterns (PAMP) receptors, e.g. Toll-like receptors (TLRs) or other auxiliary substances such as lipopolysaccharides, TNF-alpha, CD40 ligand, or cytokines, monokines, lymphokines, interleukins or chemokines, IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta, TNF-alpha, growth factors, and hGH, a ligand of human Toll-like receptor TLR1, TLR2, TLR3, TLR4, TLR5, TLR6, TLR7, TLR8, TLR9, TLR10, a ligand of murine Toll-like receptor TLR1, TLR2, TLR3, TLR4, TLR5, TLR6, TLR7, TLR8, TLR9, TLR10, TLR11, TLR12 or TLR13, a ligand of a NOD-like receptor, a ligand of a RIG-I like receptor, an immunostimulatory nucleic acid, an immunostimulatory RNA (isRNA), a CpG-DNA, an antibacterial agent, or an anti-viral agent. Typically a response of the innate immune system includes recruiting immune cells to sites of infection, through the production of chemical factors, including specialized chemical mediators, called cytokines; activation of the complement cascade; identification and removal of foreign substances present in organs, tissues, the blood and lymph, by specialized white blood cells; activation of the adaptive immune system through a process known as antigen presentation; and/or acting as a physical and chemical barrier to infectious agents.

Adjuvant/adjuvant component: An adjuvant or an adjuvant component in the broadest sense is typically a (e.g. pharmacological or immunological) agent or composition that may modify, e.g. enhance, the efficacy of other agents, such as a drug or vaccine. Conventionally the term refers in the context of the invention to a compound or composition that serves as a carrier or auxiliary substance for immunogens and/or other pharmaceutically active compounds. It is to be interpreted in a broad sense and refers to a broad spectrum of substances that are able to increase the immunogenicity of antigens incorporated into or co-administered with an adjuvant in question. In the context of the present invention an adjuvant will preferably enhance the specific immunogenic effect of the active agents of the present invention. Typically, "adjuvant" or "adjuvant component" has the same meaning and can be used mutually. Adjuvants may be divided, e.g., into immuno potentiators, antigenic delivery systems or even combinations thereof.

The term "adjuvant" is typically understood not to comprise agents which confer immunity by themselves. An adjuvant assists the immune system unspecifically to enhance the antigen-specific immune response by e.g. promoting presentation of an antigen to the immune system or induction of an unspecific innate immune response. Furthermore, an adjuvant may preferably e.g. modulate the antigen-specific immune response by e.g. shifting the dominating Th2-based antigen specific response to a more Th1-based antigen specific response or vice versa. Accordingly, an adjuvant may favourably modulate cytokine expression/secretion, antigen presentation, type of immune response etc.

Immunostimulatory RNA: An immunostimulatory RNA (isRNA) in the context of the invention may typically be an RNA that is able to induce an innate immune response itself. It usually does not have an open reading frame and thus does not provide a peptide-antigen or immunogen but elicits an innate immune response e.g. by binding to a specific kind of Toll-like-receptor (TLR) or other suitable receptors. However, of course also mRNAs having an open reading frame and coding for a peptide/protein (e.g. an antigenic function) may induce an innate immune response.

Antigen: In the context of the present invention "antigen" refers typically to a substance which may be recognized by the immune system, preferably by the adaptive immune system, and is capable of triggering an antigen-specific immune response, e.g. by formation of antibodies and/or antigen-specific T cells as part of an adaptive immune response. Typically, an antigen may be or may comprise a peptide or protein which may be presented by the MHC to T-cells. In the sense of the present invention an antigen may be the product of translation of a provided nucleic acid molecule, preferably an mRNA as defined herein. In this context, also fragments, variants and derivatives of peptides and proteins comprising at least one epitope are understood as antigen.

Epitope (also called "antigen determinant"): T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule.

B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form.

Such epitopes of proteins or peptides may furthermore be selected from any of the herein mentioned variants of such proteins or peptides. In this context antigenic determinants can be conformational or discontinuous epitopes which are composed of segments of the proteins or peptides as defined herein that are discontinuous in the amino acid sequence of the proteins or peptides as defined herein but are brought together in the three-dimensional structure or continuous or linear epitopes which are composed of a single polypeptide chain.

Vaccine: A vaccine is typically understood to be a prophylactic or therapeutic material providing at least one antigen or antigenic function. The antigen or antigenic function may stimulate the body's adaptive immune system to provide an adaptive immune response.

Antigen-providing mRNA: An antigen-providing mRNA in the context of the invention may typically be an mRNA, having at least one open reading frame that can be translated by a cell or an organism provided with that mRNA. The product of this translation is a peptide or protein that may act as an antigen, preferably as an immunogen. The product may also be a fusion protein composed of more than one immunogen, e.g. a fusion protein that consist of two or more epitopes, peptides or proteins derived from the same or different virus-proteins, wherein the epitopes, peptides or proteins may be linked by linker sequences.

Artificial mRNA (sequence): An artificial mRNA (sequence) may typically be understood to be an mRNA molecule, that does not occur naturally. In other words, an artificial mRNA molecule may be understood as a non-natural mRNA molecule. Such mRNA molecule may be non-natural due to its individual sequence (which does not occur naturally) and/or due to other modifications, e.g. structural modifications of nucleotides which do not occur naturally. Typically, artificial mRNA molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot.

Bi-/multicistronic mRNA: mRNA, that typically may have two (bicistronic) or more (multicistronic) open reading frames (ORF) (coding regions or coding sequences). An open reading frame in this context is a sequence of several nucleotide triplets (codons) that can be translated into a peptide or protein. Translation of such an mRNA yields two (bicistronic) or more (multicistronic) distinct translation products (provided the ORFs are not identical). For expression in eukaryotes such mRNAs may for example comprise an internal ribosomal entry site (IRES) sequence.

Monocistronic mRNA: A monocistronic mRNA may typically be an mRNA, that comprises only one open reading frame (coding sequence or coding region). An open reading frame in this context is a sequence of several nucleotide triplets (codons) that can be translated into a peptide or protein.

5'-CAP structure: A 5'-CAP is typically a modified nucleotide (CAP analogue), particularly a guanine nucleotide, added to the 5'-end of an mRNA molecule. Preferably, the 5'-CAP is added using a 5'-5'-triphosphate linkage (also named m7GpppN). Further examples of 5'-CAP structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-CAP structures may be used in the context of the present invention to modify the mRNA sequence of the inventive composition. Further modified 5'-CAP structures which may be used in the context of the present invention are CAP1 (additional methylation of the ribose of the adjacent nucleotide of m7GpppN), CAP2 (additional methylation of the ribose of the 2nd nucleotide downstream of the m7GpppN), cap3 (additional methylation of the ribose of the 3rd nucleotide downstream of the m7GpppN), cap4 (additional methylation of the ribose of the 4th nucleotide downstream of the m7GpppN), ARCA (anti-reverse CAP analogue), modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In the context of the present invention, a 5'-CAP structure may also be formed in chemical RNA synthesis or RNA in vitro transcription (co-transcriptional capping) using cCAP analogues, or a CAP structure may be formed in vitro using capping enzymes (e.g., commercially available capping kits).

CAP analogue: A CAP analogue refers to a non-polymerizable di-nucleotide that has CAP functionality in that it facilitates translation or localization, and/or prevents degradation of the RNA molecule when incorporated at the 5'-end of the RNA molecule. Non-polymerizable means that the CAP analogue will be incorporated only at the 5'-terminus because it does not have a 5' triphosphate and therefore cannot be extended in the 3'-direction by a template-dependent RNA polymerase.

CAP analogues include, but are not limited to, a chemical structure selected from the group consisting of m7GpppG, m7GpppA, m7GpppC; unmethylated CAP analogues (e.g., GpppG); dimethylated CAP analogue (e.g., m2,7GpppG), trimethylated CAP analogue (e.g., m2,2,7GpppG), dimethylated symmetrical CAP analogues (e.g., m7Gpppm7G), or anti reverse CAP analogues (e.g., ARCA; m7,2'OmeGpppG, m7,2'dGpppG, m7,3'OmeGpppG, m7,3'dGpppG and their tetraphosphate derivatives) (Stepinski et al., 2001. RNA 7(10):1486-95).

Further CAP analogues have been described previously (US7,074,596, WO2008/016473, WO2008/157688, WO2009/149253, WO2011/015347, and WO2013/059475). The synthesis of N7-(4-chlorophenoxyethyl) substituted dinucleotide CAP analogues has been described recently (Kore et al. (2013) Bioorg. Med. Chem. 21(15): 4570-4).

Poly (C) sequence: A poly-(C)-sequence is typically a long sequence of cytosine nucleotides, typically about 10 to about 200 cytosine nucleotides, preferably about 10 to about 100 cytosine nucleotides, more preferably about 10 to about 70 cytosine nucleotides or even more preferably about 20 to about 50 or even about 20 to about 30 cytosine nucleotides. A poly(C) sequence may preferably be located 3' of the coding region comprised by a nucleic acid.

Poly-A-tail/sequence: A poly-A-tail also called "3'-poly(A) tail or poly(A) sequence" is typically a long sequence of adenosine nucleotides of up to about 400 adenosine nucleotides, e.g. from about 25 to about 400, preferably from about 50 to about 400, more preferably from about 50 to about 300, even more preferably from about 50 to about 250, most preferably from about 60 to about 250 adenosine nucleotides, added to the 3'-end of a RNA. Moreover, poly(A) sequences, or poly(A) tails may be generated in vitro by enzymatic polyadenylation of the RNA, e.g. using Poly(A)polymerases derived from E.coli or yeast.

Polyadenylation: Polyadenylation is typically understood to be the addition of a poly(A) sequence to a nucleic acid molecule, such as an RNA molecule, e.g. to a premature mRNA. Polyadenylation may be induced by a so called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

3'-untranslated region (3'-UTR): A 3'-UTR is typically the part of an mRNA which is located between the protein coding region (i.e. the open reading frame) and the poly(A) sequence of the mRNA. A 3'-UTR of the mRNA is not translated into an amino acid sequence. The 3'-UTR sequence is generally encoded by the gene which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'-Capping, splicing the pre-mature mRNA to excise optional introns and modifications of the 3'-end, such as polyadenylation of the 3'-end of the pre-mature mRNA and optional endo- or exonuclease cleavages etc. In the context of the present invention, a 3'-UTR corresponds to the sequence of a mature mRNA which is located 3' to the stop codon of the protein coding region, preferably immediately 3' to the stop codon of the protein coding region, and which extends to the 5'-side of the poly(A) sequence, preferably to the nucleotide immediately 5' to the poly(A) sequence. The term "corresponds to" means that the 3'-UTR sequence may be an RNA sequence, such as in the mRNA sequence used for defining the 3'-UTR sequence, or a DNA sequence which corresponds to such RNA sequence. In the context of the present invention, the term "a 3'-UTR of a gene", such as "a 3'-UTR of an albumin gene", is the sequence which corresponds to the 3'-UTR of the mature mRNA derived from this gene, i.e. the mRNA obtained by transcription of the gene and maturation of the pre- mature mRNA. The term "3'-UTR of a gene" encompasses the DNA sequence and the RNA sequence of the 3'-UTR.

5'-untranslated region (5'-UTR): A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites or a 5'-Terminal Oligopyrimidine Tract. The 5'-UTR may be posttranscriptionally modified, for example by addition of a 5'-CAP. In the context of the present invention, a 5'-UTR corresponds to the sequence of a mature mRNA which is located between the 5'-CAP and the start codon. Preferably, the 5'-UTR corresponds to the sequence which extends from a nucleotide located 3' to the 5'-CAP, preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence which corresponds to such RNA sequence. In the context of the present invention, the term "a 5'-UTR of a gene", such as "a 5'-UTR of a TOP gene", is the sequence which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence of the 5'-UTR.

5'-Terminal Oligopyrimidine Tract (TOP): The 5'-terminal oligopyrimidine tract (TOP) is typically a stretch of pyrimidine nucleotides located at the 5'-terminal region of a nucleic acid molecule, such as the 5'-terminal region of certain mRNA molecules or the 5'-terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 29, 30 or even more nucleotides. The pyrimidine stretch and thus the 5'-TOP ends one nucleotide 5' to the first purine nucleotide located downstream of the TOP. Messenger RNA that contains a 5'-terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'-TOP as defined above. Thus, a TOP motif in the context of the present invention is preferably a stretch of pyrimidine nucleotides having a length of 3-30 nucleotides. Preferably, the TOP motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'-end with a cytosine nucleotide. In TOP genes and TOP mRNAs, the TOP motif preferably starts at its 5'-end with the transcriptional start site and ends one nucleotide 5' to the first purin residue in said gene or mRNA. A TOP motif in the sense of the present invention is preferably located at the 5'-end of a sequence which represents a 5'-UTR or at the 5'-end of a sequence which codes for a 5'-UTR. Thus, preferably, a stretch of 3 or more pyrimidine nucleotides is called "TOP motif" in the sense of the present invention if this stretch is located at the 5'end of a respective sequence, such as the inventive mRNA, the 5'-UTR element of the inventive mRNA, or the nucleic acid sequence which is derived from the 5'-UTR of a TOP gene as described herein. In other words, a stretch of 3 or more pyrimidine nucleotides which is not located at the 5'-end of a 5'-UTR or a 5'-UTR element but anywhere within a 5'-UTR or a 5'-UTR element is preferably not referred to as "TOP motif".

TOP gene: TOP genes are typically characterised by the presence of a 5'-terminal oligopyrimidine tract. Furthermore, most TOP genes are characterized by a growth-associated translational regulation. However, also TOP genes with a tissue specific translational regulation are known. As defined above, the 5'-UTR of a TOP gene corresponds to the sequence of a 5'-UTR of a mature mRNA derived from a TOP gene, which preferably extends from the nucleotide located 3' to the 5'-CAP to the nucleotide located 5' to the start codon. A 5'-UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5'-UTRs of TOP genes are generally rather short. The lengths of 5'-UTRs of TOP genes may vary between 20 nucleotides up to 500 nucleotides, and are typically less than about 200 nucleotides, preferably less than about 150 nucleotides, more preferably less than about 100 nucleotides. Exemplary 5'-UTRs of TOP genes in the sense of the present invention are the nucleic acid sequences extending from the nucleotide at position 5 to the nucleotide located immediately 5' to the start codon (e.g. the ATG) in the sequences according to SEQ ID NOs: 1-1363, SEQ ID NO: 1395, SEQ ID NO: 1421 and SEQ ID NO: 1422 of the international patent application WO2013/143700 or homologs or variants thereof, whose disclosure is incorporated herewith by reference. In this context a particularly preferred fragment of a 5'-UTR of a TOP gene is a 5'-UTR of a TOP gene lacking the 5'-TOP motif. The term "5'-UTR of a TOP gene" preferably refers to the 5'-UTR of a naturally occurring TOP gene.

Fragment of a nucleic acid sequence, particularly an mRNA: A fragment of a nucleic acid sequence consists of a continuous stretch of nucleotides corresponding to a continuous stretch of nucleotides in the full-length nucleic acid sequence which is the basis for the nucleic acid sequence of the fragment, which represents at least 20%, preferably at least 30%, more preferably at least 40%, more preferably at least 50%, even more preferably at least 60%, even more preferably at least 70%, even more preferably at least 80%, and most preferably at least 90% of the full-length nucleic acid sequence. Such a fragment, in the sense of the present invention, is preferably a functional fragment of the full-length nucleic acid sequence.

In this context, a "fragment of a nucleic acid sequence" e.g. a fragment of an mRNA sequence is preferably a nucleic acid sequence encoding a fragment of a protein or of a variant thereof as described herein. More preferably, the expression 'fragment of a nucleic acid sequence' refers to a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with a respective full-length nucleic acid sequence.

Variant of a nucleic acid sequence, particularly an mRNA: A variant of a nucleic acid sequence refers to a variant of nucleic acid sequences which forms the basis of a nucleic acid sequence. For example, a variant nucleic acid sequence may exhibit one or more nucleotide deletions, insertions, additions and/or substitutions compared to the nucleic acid sequence from which the variant is derived. Preferably, a variant of a nucleic acid sequence is at least 40%, preferably at least 50%, more preferably at least 60%, more preferably at least 70%, even more preferably at least 80%, even more preferably at least 90%, most preferably at least 95% identical to the nucleic acid sequence the variant is derived from. Preferably, the variant is a functional variant. A "variant" of a nucleic acid sequence may have at least 70%, 75%, 80%, 85%, 90%, 95%, 98% or 99% nucleotide identity over a stretch of 10, 20, 30, 50, 75 or 100 nucleotide of such nucleic acid sequence.

Stabilized nucleic acid, preferably mRNA: A stabilized nucleic acid, preferably mRNA typically, exhibits a modification increasing resistance to in vivo degradation (e.g. degradation by an exo- or endo-nuclease) and/or ex vivo degradation (e.g. by the manufacturing process prior to vaccine administration, e.g. in the course of the preparation of the vaccine solution to be administered). Stabilization of RNA can, e.g., be achieved by providing a 5'-CAP-Structure, a Poly-A-Tail, or any other UTR-modification. It can also be achieved by chemical modification or modification of the G/C-content of the nucleic acid. Various other methods are known in the art and conceivable in the context of the invention.

RNA In vitro transcription: The terms "RNA in vitro transcription" or "in vitro transcription" relate to a process wherein RNA is synthesized in a cell-free system (in vitro). DNA, particularly plasmid DNA, is used as template for the generation of RNA transcripts. RNA may be obtained by DNA-dependent in vitro transcription of an appropriate DNA template, which according to the present invention is preferably a linearized plasmid DNA template. The promoter for controlling in vitro transcription can be any promoter for any DNA-dependent RNA polymerase. Particular examples of DNA-dependent RNA polymerases are the T7, T3, and SP6 RNA polymerases. A DNA template for in vitro RNA transcription may be obtained by cloning of a nucleic acid, in particular cDNA corresponding to the respective RNA to be in vitro transcribed, and introducing it into an appropriate vector for in vitro transcription, for example into plasmid DNA. In a preferred embodiment of the present invention the DNA template is linearized with a suitable restriction enzyme, before it is transcribed in vitro. The cDNA may be obtained by reverse transcription of mRNA or chemical synthesis. Moreover, the DNA template for in vitro RNA synthesis may also be obtained by gene synthesis.

Methods for in vitro transcription are known in the art (see, e.g., Geall et al. (2013) Semin. Immunol. 25(2): 152-159; Brunelle et al. (2013) Methods Enzymol. 530:101-14). Reagents used in said method typically include:
1) a linearized DNA template with a promoter sequence that has a high binding affinity for its respective RNA polymerase such as bacteriophage-encoded RNA polymerases;
2) ribonucleoside triphosphates (NTPs) for the four bases (adenine, cytosine, guanine and uracil);
3) optionally a CAP analogue as defined above (e.g. m7G(5')ppp(5')G (m7G));
4) a DNA-dependent RNA polymerase capable of binding to the promoter sequence within the linearized DNA template (e.g. T7, T3 or SP6 RNA polymerase);
5) optionally a ribonuclease (RNase) inhibitor to inactivate any contaminating RNase;
6) optionally a pyrophosphatase to degrade pyrophosphate, which may inhibit transcription;
7) MgCl2, which supplies Mg2+ ions as a co-factor for the polymerase;
8) a buffer to maintain a suitable pH value, which can also contain antioxidants (e.g. DTT), and/or polyamines such as spermidine at optimal concentrations.

Full-length protein: The term "full-length protein" as used herein typically refers to a protein that substantially comprises the entire amino acid sequence of the naturally occuring protein. Nevertheless, substitutions of amino acids e.g. due to mutation in the protein are also encompassed in the term full-length protein.

Fragments of proteins: "Fragments" of proteins or peptides in the context of the present invention may, typically, comprise a sequence of a protein or peptide as defined herein, which is, with regard to its amino acid sequence (or its encoded nucleic acid molecule), N-terminally and/or C-terminally truncated compared to the amino acid sequence of the original (native) protein (or its encoded nucleic acid molecule). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid molecule of such a protein or peptide.

In this context a fragment of a protein may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective naturally occuring full-length protein.

Fragments of proteins or peptides in the context of the present invention may furthermore comprise a sequence of a protein or peptide as defined herein, which has a length of for example at least 5 amino acids, preferably a length of at least 6 amino acids, preferably at least 7 amino acids, more preferably at least 8 amino acids, even more preferably at least 9 amino acids; even more preferably at least 10 amino acids; even more preferably at least 11 amino acids; even more preferably at least 12 amino acids; even more preferably at least 13 amino acids; even more preferably at least 14 amino acids; even more preferably at least 15 amino acids; even more preferably at least 16 amino acids; even more preferably at least 17 amino acids; even more preferably at least 18 amino acids; even more preferably at least 19 amino acids; even more preferably at least 20 amino acids; even more preferably at least 25 amino acids; even more preferably at least 30 amino acids; even more preferably at least 35 amino acids; even more preferably at least 50 amino acids; or most preferably at least 100 amino acids. For example such fragment may have a length of about 6 to about 20 or even more amino acids, e.g. fragments as processed and presented by MHC class I molecules, preferably having a length of about 8 to about 10 amino acids, e.g. 8, 9, or 10, (or even 6, 7, 11, or 12 amino acids), or fragments as processed and presented by MHC class II molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T-cells in form of a complex consisting of the peptide fragment and an MHC molecule, i.e. the fragments are typically not recognized in their native form. Fragments of proteins or peptides may comprise at least one epitope of those proteins or peptides. Furthermore also domains of a protein, like the extracellular domain, the intracellular domain or the transmembrane domain and shortened or truncated versions of a protein may be understood to comprise a fragment of a protein.

Variants of proteins: "Variants" of proteins or peptides as defined in the context of the present invention may be generated, having an amino acid sequence which differs from the original sequence in one or more mutation(s), such as one or more substituted, inserted and/or deleted amino acid(s). Preferably, these fragments and/or variants have the same biological function or specific activity compared to the full-length native protein, e.g. its specific antigenic property. "Variants" of proteins or peptides as defined in the context of the present invention may comprise conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

A "variant" of a protein or peptide may have at least 70%, 75%, 80%, 85%, 90%, 95%, 98% or 99% amino acid identity over a stretch of 10, 20, 30, 50, 75 or 100 amino acids of such protein or peptide.

Furthermore, variants of proteins or peptides as defined herein, which may be encoded by a nucleic acid molecule, may also comprise those sequences, wherein nucleotides of the encoding nucleic acid sequence are exchanged according to the degeneration of the genetic code, without leading to an alteration of the respective amino acid sequence of the protein or peptide, i.e. the amino acid sequence or at least part thereof may not differ from the original sequence in one or more mutation(s) within the above meaning.

Identity of a sequence: In order to determine the percentage to which two sequences are identical, e.g. nucleic acid sequences or amino acid sequences as defined herein, preferably the amino acid sequences encoded by a nucleic acid sequence of the polymeric carrier as defined herein or the amino acid sequences themselves, the sequences can be aligned in order to be subsequently compared to one another. Therefore, e.g. a position of a first sequence may be compared with the corresponding position of the second sequence. If a position in the first sequence is occupied by the same component (residue) as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences of the present invention to a certain extent can be identified by this program.

Derivative of a protein or peptide: A derivative of a peptide or protein is typically understood to be a molecule that is derived from another molecule, such as said peptide or protein. A "derivative" of a peptide or protein also encompasses fusions comprising a peptide or protein used in the present invention. For example, the fusion comprises a label, such as, for example, an epitope, e.g., a FLAG epitope or a V5 epitope. For example, the epitope is a FLAG epitope. Such a tag is useful for, for example, purifying the fusion protein.

Pharmaceutically effective amount: A pharmaceutically effective amount in the context of the invention is typically understood to be an amount that is sufficient to induce an immune response.

Carrier/polymeric carrier: A carrier in the context of the invention may typically be a compound that facilitates transport and/or complexation of another compound. Said carrier may form a complex with said other compound. A polymeric carrier is a carrier that is formed of a polymer.

Vehicle: An agent, e.g. a carrier, that may typically be used within a pharmaceutical composition or vaccine for facilitating administering of the components of the pharmaceutical composition or vaccine to an individual.

Jet injection: The term "jet injection", as used herein, refers to a needle-free injection method, wherein a fluid containing at least one inventive mRNA sequence and, optionally, further suitable excipients is forced through an orifice, thus generating an ultra-fine liquid stream of high pressure that is capable of penetrating mammalian skin and, depending on the injection settings, subcutaneous tissue or muscle tissue. In principle, the liquid stream forms a hole in the skin, through which the liquid stream is pushed into the target tissue. Preferably, jet injection is used for intradermal, subcutaneous or intramuscular injection of the mRNA sequence according to the invention. In a preferred embodiment, jet injection is used for intramuscular injection of the mRNA sequence according to the invention. In a further preferred embodiment, jet injection is used for intradermal injection of the mRNA sequence according to the invention.

### Detailed Description of the Invention

The present invention is based on the inventors' surprising finding that mRNA encoding at least one antigenic peptide or protein comprised in lipid nanoparticles (LNPs) induces very efficiently antigen-specific immune responses against the encoded antigenic peptide or protein at a very low dosages and dosing regimen which do not require frequent administration.

Further advantages of the inventive mRNA encoding at least one antigenic peptide or protein comprised in lipid nanoparticles (LNPs) are:
- Induction of a strong humoral immune response
- Induction of B-cell memory
- Faster onset of immune protection
- Longevity of the induced immune responses
- Induction of broad cellular T-cell responses
- Induction of a (local and transient) pro-inflammatory environment
- No induction of systemic cytokine or chemokine response
- Well tolarability, no side-effects, non toxic
- Advantageous stability characteristics
- Formulation compatible with many different antigens: larger antigen cocktails feasible based on the same (production) technology
- No vector immunity, i.e. technology can be used to vaccinate the same subject multiple times against multiple (different) antigens
- Speed, adaptability, simplicity and scalability of production

In particular, the invention relates to mRNA comprising lipid nanoparticles and uses thereof. In order to be suitable for the present invention, the lipid nanoparticles comprise at least:
(i) a cationic lipid and/or a PEG-lipid as defined below; and
(ii) an mRNA compound comprising an mRNA sequence encoding an antigenic peptide or protein.

The mRNA comprising lipid nanoparticle may comprise further compounds, such as one or more neutral lipids, steroids and combinations of said compounds. Suitable compounds will be described in detail below.

The mRNA compound comprising an mRNA sequence encoding an antigenic peptide or protein may be a mRNA molecule. In one embodiment of the invention, the mRNA compound is a natural and non-modified mRNA. Within the context of the present invention, natural and non-modified mRNA encompasses mRNA generated in vitro, without chemical modifications or changes in the sequence.

In an alternative embodiment of the invention, the mRNA compound comprises an artificial mRNA. In the context of the present invention artificial mRNA encompasses mRNA with chemical modifications, sequence modifications or non-natural sequences.

In a preferred embodiment of the invention, the mRNA compound does not comprise nucleoside modifications, in particular no base modifications. In a further embodiment, the mRNA compound does not comprise 1-methylpseudouridine modifications. In one preferred embodiment, the mRNA comprises only the naturally existing nucleosides. In a further preferred embodiment, the mRNA compound does not comprise any chemical modification and optionally comprises sequence modifications. In a further preferred embodiment of the invention the mRNA comnpound only comprises the naturally existing nucleosides adenine, uracil, guanine and cytosine.

According to certain embodiments of the present invention, the mRNA sequence is mono-, bi-, or multicistronic, preferably as defined herein. The coding sequences in a bi- or multicistronic mRNA preferably encode distinct peptides or proteins as defined herein or a fragment or variant thereof. Preferably, the coding sequences encoding two or more peptides or proteins may be separated in the bi- or multicistronic mRNA by at least one IRES (internal ribosomal entry site) sequence, as defined below. Thus, the term "encoding two or more peptides or proteins" may mean, without being limited thereto, that the bi- or even multicistronic mRNA, may encode e.g. at least two, three, four, five, six or more (preferably different) peptides or proteins or their fragments or variants within the definitions provided herein. More preferably, without being limited thereto, the bi- or even multicistronic mRNA, may encode, for example, at least two, three, four, five, six or more (preferably different) peptides or proteins as defined herein or their fragments or variants as defined herein. In this context, a so-called IRES (internal ribosomal entry site) sequence as defined above can function as a sole ribosome binding site, but it can also serve to provide a bi- or even multicistronic mRNA as defined above, which encodes several peptides or proteins which are to be translated by the ribosomes independently of one another. Examples of IRES sequences, which can be used according to the invention, are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

According to a further embodiment the at least one coding region of the mRNA sequence according to the invention may encode at least two, three, four, five, six, seven, eight and more peptides or proteins (or fragments and derivatives thereof) as defined herein linked with or without an amino acid linker sequence, wherein said linker sequence can comprise rigid linkers, flexible linkers, cleavable linkers (e.g., self-cleaving peptides) or a combination thereof. Therein, the peptides or proteins may be identical or different or a combination thereof. Particular peptide or protein combinations can be encoded by said mRNA encoding at least two peptides or proteins as explained herein (also referred to herein as "multi-antigen-constructs/ mRNA").

In a particular aspect of the invention, the lipid nanoparticles comprise an mRNA compound, comprising an mRNA sequence encoding an antigenic peptide or protein, or a fragment, variant or derivative thereof.

These antigenic peptides or proteins may be derived from pathogenic antigens, tumour antigens, allergenic antigens or autoimmune self-antigens, preferably as defined herein. In the context of the present invention, antigenic peptides or proteins preferably exclude luciferases.

### Pathogenic antigens:

Such pathogenic antigens are derived from pathogenic organisms, in particular bacterial, viral or protozoological (multicellular) pathogenic organisms, which evoke an immunological reaction by subject, in particular a mammalian subject, more particularly a human. More specifically, pathogenic antigens are preferably surface antigens, e.g. proteins (or fragments of proteins, e.g. the exterior portion of a surface antigen) located at the surface of the virus or the bacterial or protozoological organism.

Pathogenic antigens are peptide or protein antigens preferably derived from a pathogen associated with infectious disease which are preferably selected from antigens derived from the pathogens Acinetobacter baumannii, Anaplasma genus, Anaplasma phagocytophilum, Ancylostoma braziliense, Ancylostoma duodenale, Arcanobacterium haemolyticum, Ascaris lumbricoides, Aspergillus genus, Astroviridae, Babesia genus, Bacillus anthracis, Bacillus cereus, Bartonella henselae, BK virus, Blastocystis hominis, Blastomyces dermatitidis, Bordetella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomallei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium perfringens, Clostridium spp, Clostridium tetani, Coccidioides spp, coronaviruses, Corynebacterium diphtheriae, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and Enterovirus 71 (EV71), Epidermophyton spp, Epstein-Barr Virus (EBV), Escherichia coli O157:H7, O111 and O104:H4, Fasciola hepatica and Fasciola gigantica, FFI prion, Filarioidea superfamily, Flaviviruses, Francisella tularensis, Fusobacterium genus, Geotrichum candidum, Giardia intestinalis, Gnathostoma spp, GSS prion, Guanarito virus, Haemophilus ducreyi, Haemophilus influenzae, Helicobacter pylori, Henipavirus (Hendra virus Nipah virus), Hepatitis A Virus, Hepatitis B Virus (HBV), Hepatitis C Virus (HCV), Hepatitis D Virus, Hepatitis E Virus, Herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and Human herpesvirus 7 (HHV-7), Human metapneumovirus (hMPV), Human papillomavirus (HPV), Human parainfluenza viruses (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitidis, Nocardia asteroides, Nocardia spp, Onchocerca volvulus, Orientia tsutsugamushi, Orthomyxoviridae family (Influenza), Paracoccidioides brasiliensis, Paragonimus spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, rhinoviruses, Rickettsia akari, Rickettsia genus, Rickettsia prowazekii, Rickettsia rickettsii, Rickettsia typhi, Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), Variola major or Variola minor, vCJD prion, Venezuelan equine encephalitis virus, Vibrio cholerae, West Nile virus, Western equine encephalitis virus, Wuchereria bancrofti, Yellow fever virus, Yersinia enterocolitica, Yersinia pestis, and Yersinia pseudotuberculosis.

In this context particularly preferred are antigens from the pathogens selected from Influenza virus, respiratory syncytial virus (RSV), Herpes simplex virus (HSV), human Papilloma virus (HPV), Human immunodeficiency virus (HIV), Plasmodium, Staphylococcus aureus, Dengue virus, Chlamydia trachomatis, Cytomegalovirus (CMV), Hepatitis B virus (HBV), Mycobacterium tuberculosis, Rabies virus, and Yellow Fever Virus.

Furthermore, the pathogenic antigen (antigen derived from a pathogen associated with infectious disease) may be preferably selected from the following antigens: Outer membrane protein A OmpA, biofilm associated protein Bap, transport protein MucK (Acinetobacter baumannii, Acinetobacter infections)); variable surface glycoprotein VSG, microtubule-associated protein MAPP15, trans-sialidase TSA (Trypanosoma brucei, African sleeping sickness (African trypanosomiasis)); HIV p24 antigen, HIV envelope proteins (Gp120, Gp41, Gp160), polyprotein GAG, negative factor protein Nef, trans-activator of transcription Tat (HIV (Human immunodeficiency virus), AIDS (Acquired immunodeficiency syndrome)); galactose-inhibitable adherence protein GIAP, 29 kDa antigen Eh29, Gal/GalNAc lectin, protein CRT, 125 kDa immunodominant antigen, protein M17, adhesin ADH112, protein STIRP (Entamoeba histolytica, Amoebiasis); Major surface proteins 1-5 (MSP1a, MSP1b, MSP2, MSP3, MSP4, MSP5), type IV secreotion system proteins (VirB2, VirB7, VirB11, VirD4) (Anaplasma genus, Anaplasmosis); protective Antigen PA, edema factor EF, lethal facotor LF, the S-layer homology proteins SLH (Bacillus anthracis, Anthrax); acranolysin, phospholipase D, collagen-binding protein CbpA (Arcanobacterium haemolyticum, Arcanobacterium haemolyticum infection); nucleocapsid protein NP, glycoprotein precursor GPC, glycoprotein GP1, glycoprotein GP2 (Junin virus, Argentine hemorrhagic fever); chitin-protein layer proteins, 14 kDa suarface antigen A14, major sperm protein MSP, MSP polymerization-organizing protein MPOP, MSP fiber protein 2 MFP2, MSP polymerization-activating kinase MPAK, ABA-1-like protein ALB, protein ABA-1, cuticulin CUT-1 (Ascaris lumbricoides, Ascariasis); 41 kDa allergen Asp v13, allergen Asp f3, major conidial surface protein rodlet A, protease Pep1p, GPI-anchored protein Gel1p, GPI-anchored protein Crf1p (Aspergillus genus, Aspergillosis); family VP26 protein, VP29 protein (Astroviridae, Astrovirus infection); Rhoptry-associated protein 1 RAP-1, merozoite surface antigens MSA-1, MSA-2 (a1, a2, b, c), 12D3, 11C5, 21B4, P29, variant erythrocyte surface antigen VESA1, Apical Membrane Antigen 1 AMA-1 (Babesia genus, Babesiosis); hemolysin, enterotoxin C, PXO1-51, glycolate oxidase, ABC-transporter, penicillin-bingdn protein, zinc transporter family protein, pseudouridine synthase Rsu, plasmid replication protein RepX, oligoendopeptidase F, prophage membrane protein, protein HemK, flagellar antigen H, 28.5-kDa cell surface antigen (Bacillus cereus, Bacillus cereus infection); large T antigen LT, small T antigen, capsid protein VP1, capsid protein VP2 (BK virus, BK virus infection); 29 kDa-protein, caspase-3-like antigens, glycoproteins (Blastocystis hominis, Blastocystis hominis infection); yeast surface adhesin WI-1 (Blastomyces dermatitidis, Blastomycosis); nucleoprotein N, polymerase L, matrix protein Z, glycoprotein GP (Machupo virus, Bolivian hemorrhagic fever); outer surface protein A OspA, outer surface protein OspB, outer surface protein OspC, decorin binding protein A DbpA, decorin binding protein B DbpB, flagellar filament 41 kDa core protein Fla, basic membrane protein A precursor BmpA (Immunodominant antigen P39), outer surface 22 kDa lipoprotein precursor (antigen IPLA7), variable surface lipoprotein visE (Borrelia genus, Borrelia infection); Botulinum neurotoxins BoNT/A1, BoNT/A2, BoNT/A3, BoNT/B, BoNT/C, BoNT/D, BoNT/E, BoNT/F, BoNT/G, recombinant botulinum toxin F Hc domain FHc (Clostridium botulinum, Botulism (and Infant botulism)); nucleocapsid, glycoprotein precursor (Sabia virus, Brazilian hemorrhagic fever); copper/Zinc superoxide dismutase SodC, bacterioferritin Bfr, 50S ribosomal protein RplL, OmpA-like transmembrane domain-containing protein Omp31, immunogenic 39-kDa protein M5 P39, zinc ABC transporter periplasmic zinc-bnding protein znuA, periplasmic immunogenic protein Bp26, 30S ribosomal protein S12 RpsL, glyceraldehyde-3-phosphate dehydrogenase Gap, 25 kDa outer-membrane immunogenic protein precursor Omp25, invasion protein B lalB, trigger factor Tig, molecular chaperone DnaK, putative peptidyl-prolyl cis-trans isomerase SurA, lipoprotein Omp19, outer membrane protein MotY Omp16, conserved outer membrane protein D15, malate dehydrogenase Mdh, component of the Type-IV secretion system (T4SS) VirJ, lipoprotein of unknown function BAB1_0187 (Brucella genus, Brucellosis); members of the ABC transporter family (LolC, OppA, and PotF), putative lipoprotein releasing system transmembrane protein LolC/E, flagellin FliC, Burkholderia intracellular motility A BimA, bacterial Elongation factor-Tu EF-Tu, 17 kDa OmpA-like protein, boaA coding protein, boaB coding protein (Burkholderia cepacia and other Burkholderia species, Burkholderia infection); mycolyl-transferase Ag85A, heat-shock protein Hsp65, protein TB10.4, 19 kDa antigen, protein PstS3, heat-shock protein Hsp70 (Mycobacterium ulcerans, Buruli ulcer); norovirus major and minor viral capsid proteins VP1 and VP2, genome polyprotein, Sapoviurus capsid protein VP1, protein Vp3, geome polyprotein (Caliciviridae family, Calicivirus infection (Norovirus and Sapovirus)); major outer membrane protein PorA, flagellin FlaA, surface antigen CjaA, fibronectin binding protein CadF, aspartate/glutamate-binding ABC transporter protein Peb1A, protein FspA1, protein FspA2 (Campylobacter genus, Campylobacteriosis); glycolytic enzyme enolase, secreted aspartyl proteinases SAP1-10, glycophosphatidylinositol (GPI)-linked cell wall protein, protein Hyr1, complement receptor 3-related protein CR3-RP, adhesin Als3p, heat shock protein 90 kDa hsp90, cell surface hydrophobicity protein CSH (usually Candida albicans and other Candida species, Candidiasis); 17-kDa antigen, protein P26, trimeric autotransporter adhesins TAAs, Bartonella adhesin A BadA, variably expressed outer-membrane proteins Vomps, protein Pap3, protein HbpA, envelope-associated protease HtrA, protein OMP89, protein GroEL, protein LalB, protein OMP43, dihydrolipoamide succinyltransferase SucB (Bartonella henselae, Cat-scratch disease); amastigote surface protein-2, amastigote-specific surface protein SSP4, cruzipain, trans-sialidase TS, trypomastigote surface glycoprotein TSA-1, complement regulatory protein CRP-10, protein G4, protein G2, paraxonemal rod protein PAR2, paraflagellar rod component Par1, mucin-Associated Surface Proteins MPSP (Trypanosoma cruzi, Chagas Disease (American trypanosomiasis)); envelope glycoproteins (gB, gC, gE, gH, gI, gK, gL) (Varicella zoster virus (VZV), Chickenpox); major outer membrane protein MOMP, probable outer membrane protein PMPC, outer membrane complex protein B OmcB, heat shock proteins Hsp60 HSP10, protein IncA, proteins from the type III secretion system, ribonucleotide reductase small chain protein NrdB, plasmid protein Pgp3, chlamydial outer protein N CopN, antigen CT521, antigen CT425, antigen CT043, antigen TC0052, antigen TC0189, antigen TC0582, antigen TC0660, antigen TC0726, antigen TC0816, antigen TC0828 (Chlamydia trachomatis, Chlamydia); low calcium response protein E LCrE, chlamydial outer protein N CopN, serine/threonine-protein kinase PknD, acyl-carrier-protein S-malonyltransferase FabD, single-stranded DNA-binding protein Ssb, major outer membrane protein MOMP, outer membrane protein 2 Omp2, polymorphic membrane protein family (Pmp1, Pmp2, Pmp3, Pmp4, Pmp5, Pmp6, Pmp7, Pmp8, Pmp9, Pmp10, Pmp11, Pmp12, Pmp13, Pmp14, Pmp15, Pmp16, Pmp17, Pmp18, Pmp19, Pmp20, Pmp21) (Chlamydophila pneumoniae, Chlamydophila pneumoniae infection); cholera toxin B CTB, toxin coregulated pilin A TcpA, toxin coregulated pilin TcpF, toxin co-regulated pilus biosynthesis ptrotein F TcpF, cholera enterotoxin subunit A, cholera enterotoxin subunit B, Heat-stable enterotoxin ST, mannose-sensitive hemagglutinin MSHA, outer membrane protein U Porin ompU, Poring B protein, polymorphic membrane protein-D (Vibrio cholerae, Cholera); propionyl-CoA carboxylase PCC, 14-3-3 protein, prohibitin, cysteine proteases, glutathione transferases, gelsolin, cathepsin L proteinase CatL, Tegumental Protein 20.8 kDa TP20.8, tegumental protein 31.8 kDa TP31.8, lysophosphatidic acid phosphatase LPAP, (Clonorchis sinensis, Clonorchiasis); surface layer proteins SLPs, glutamate dehydrogenase antigen GDH, toxin A, toxin B, cysteine protease Cwp84, cysteine protease Cwp13, cysteine protease Cwp19, Cell Wall Protein CwpV, flagellar protein FliC, flagellar protein FliD (Clostridium difficile, Clostridium difficile infection); rhinoviruses: capsid proteins VP1, VP2, VP3, VP4; coronaviruses: sprike proteins S, envelope proteins E, membrane proteins M, nucleocapsid proteins N (usually rhinoviruses and coronaviruses, Common cold (Acute viral rhinopharyngitis; Acute coryza)); prion protein Prp (CJD prion, Creutzfeldt-Jakob disease (CJD)); envelope protein Gc, envelope protein Gn, nucleocapsid proteins (Crimean-Congo hemorrhagic fever virus, Crimean-Congo hemorrhagic fever (CCHF)); virulence-associated DEAD-box RNA helicase VAD1, galactoxylomannan-protein GalXM, glucuronoxylomannan GXM, mannoprotein MP (Cryptococcus neoformans, Cryptococcosis); acidic ribosomal protein P2 CpP2, mucin antigens Muc1, Muc2, Muc3 Muc4, Muc5, Muc6, Muc7, surface adherence protein CP20, surface adherence protein CP23, surface protein CP12, surface protein CP21, surface protein CP40, surface protein CP60, surface protein CP15, surface-associated glycopeptides gp40, surface-associated glycopeptides gp15, oocyst wall protein AB, profilin PRF, apyrase (Cryptosporidium genus, Cryptosporidiosis); fatty acid and retinol binding protein-1 FAR-1, tissue inhibitor of metalloproteinase TIMP (TMP), cysteine proteinase ACEY-1, cysteine proteinase ACCP-1, surface antigen Ac-16, secreted protein 2 ASP-2, metalloprotease 1 MTP-1, aspartyl protease inhibitor API-1, surface-associated antigen SAA-1, adult-specific secreted factor Xa serine protease inhibitor anticoagulant AP, cathepsin D-like aspartic protease ARR-1 (usually Ancylostoma braziliense; multiple other parasites, Cutaneous larva migrans (CLM)); cathepsin L-like proteases, 53/25-kDa antigen, 8kDa family members, cysticercus protein with a marginal trypsin-like activity TsAg5, oncosphere protein TSOL18, oncosphere protein TSOL45-1A, lactate dehydrogenase A LDHA, lactate dehydrogenase B LDHB (Taenia solium, Cysticercosis); pp65 antigen, membrane protein pp15, capsid-proximal tegument protein pp150, protein M45, DNA polymerase UL54, helicase UL105, glycoprotein gM, glycoprotein gN, glcoprotein H, glycoprotein B gB, protein UL83, protein UL94, protein UL99 (Cytomegalovirus (CMV), Cytomegalovirus infection); capsid protein C, premembrane protein prM, membrane protein M, envelope protein E (domain I, domain II, domain II), protein NS1, protein NS2A, protein NS2B, protein NS3, protein NS4A, protein 2K, protein NS4B, protein NS5 (Dengue viruses (DEN-1, DEN-2, DEN-3 and DEN-4)-Flaviviruses, Dengue fever); 39 kDa protein (Dientamoeba fragilis, Dientamoebiasis); diphtheria toxin precursor Tox, diphteria toxin DT, pilin-specific sortase SrtA, shaft pilin protein SpaA, tip pilin protein SpaC, minor pilin protein SpaB, surface-associated protein DIP1281 (Corynebacterium diphtheriae, Diphtheria); glycoprotein GP, nucleoprotein NP, minor matrix protein VP24, major matrix protein VP40, transcription activator VP30, polymerase cofactor VP35, RNA polymerase L (Ebolavirus (EBOV), Ebola hemorrhagic fever); prion protein (vCJD prion, Variant Creutzfeldt-Jakob disease (vCJD, nvCJD)); UvrABC system protein B, protein Flp1, protein Flp2, protein Flp3, protein TadA, hemoglobin receptor HgbA, outer membrane protein TdhA, protein CpsRA, regulator CpxR, protein SapA, 18 kDa antigen, outer membrane protein NcaA, protein LspA, protein LspA1, protein LspA2, protein LspB, outer membrane component DsrA, lectin DltA, lipoprotein Hlp, major outer membrane protein OMP, outer membrane protein OmpA2 (Haemophilus ducreyi, Chancroid); aspartyl protease 1 Pep1, phospholipase B PLB, alpha-mannosidase 1 AMN1, glucanosyltransferase GEL1, urease URE, peroxisomal matrix protein Pmp1, proline-rich antigen Pra, humal T-cell reative protein TcrP (Coccidioides immitis and Coccidioides posadasii, Coccidioidomycosis); allergen Tri r 2, heat shock protein 60 Hsp60, fungal actin Act, antigen Tri r2, antigen Tri r4, antigen Tri t1, protein IV, glycerol-3-phosphate dehydrogenase Gpd1, osmosensor HwSho1A, osmosensor HwSho1B, histidine kinase HwHhk7B, allergen Mala s 1, allergen Mala s 11, thioredoxin Trx Mala s 13, allergen Mala f, allergen Mala s (usually Trichophyton spp, Epidermophyton spp., Malassezia spp., Hortaea werneckii, Dermatophytosis); protein EG95, protein EG10, protein EG18, protein EgA31, protein EM18, antigen EPC1, antigen B, antigen 5, protein P29, protein 14-3-3, 8-kDa protein, myophilin, heat shock protein 20 HSP20, glycoprotein GP-89, fatty acid binding protein FAPB (Echinococcus genus, Echinococcosis); major surface protein 2 MSP2, major surface protein 4 MSP4, MSP variant SGV1, MSP variant SGV2, outer membrane protein OMP, outer membrande protein 19 OMP-19, major antigenic protein MAP1, major antigenic protein MAP1-2, major antigenic protein MAP1B, major antigenic protein MAP1-3, Erum2510 coding protein, protein GroEL, protein GroES, 30-kDA major outer membrane proteins, GE 100-kDa protein, GE 130-kDa protein, GE 160-kDa protein (Ehrlichia genus, Ehrlichiosis); secreted antigen SagA, sagA-like proteins SalA and SalB, collagen adhesin Scm, surface proteins Fms1 (EbpA(fm), Fms5 (EbpB(fm), Fms9 (EpbC(fm) and Fms10, protein EbpC(fm), 96 kDa immunoprotective glycoprotein G1 (Enterococcus genus, Enterococcus infection); genome polyprotein, polymerase 3D, viral capsid protein VP1, viral capsid protein VP2, viral capsid protein VP3, viral capsid protein VP4, protease 2A, protease 3C (Enterovirus genus, Enterovirus infection); outer membrane proteins OM, 60 kDa outer membrane protein, cell surface antigen OmpA, cell surface antigen OmpB (sca5), 134 kDa outer membrane protein, 31 kDa outer membrane protein, 29.5 kDa outer membrane protein, cell surface protein SCA4, cell surface protein Adr1 (RP827), cell surface protein Adr2 (RP828), cell surface protein SCA1, Invasion protein invA, cell division protein fts, secretion proteins sec 0family, virulence proteins virB, tlyA, tlyC, parvulin-like protein Plp, preprotein translocase SecA, 120-kDa surface protein antigen SPA, 138 kD complex antigen, major 100-kD protein (protein I), intracytoplasmic protein D, protective surface protein antigen SPA (Rickettsia prowazekii, Epidemic typhus); Epstein-Barr nuclear antigens (EBNA-1, EBNA-2, EBNA-3A, EBNA-3B, EBNA-3C, EBNA-leader protein (EBNA-LP)), latent membrane proteins (LMP-1, LMP-2A, LMP-2B), early antigen EBV-EA, membrane antigen EBV-MA, viral capsid antigen EBV-VCA, alkaline nuclease EBV-AN, glycoprotein H, glycoprotein gp350, glycoprotein gp110, glycoprotein gp42, glycoprotein gHgL, glycoprotein gB (Epstein-Barr Virus (EBV), Epstein-Barr Virus Infectious Mononucleosis); cpasid protein VP2, capsid protein VP1, major protein NS1 (Parvovirus B19, Erythema infectiosum (Fifth disease)); pp65 antigen, glycoprotein 105, major capsid protein, envelope glycoprotein H, protein U51 (Human herpesvirus 6 (HHV-6) and Human herpesvirus 7 (HHV-7), Exanthem subitum); thioredoxin-glutathione reductase TGR, cathepsins L1 and L2, Kunitz-type protein KTM, leucine aminopeptidase LAP, cysteine proteinase Fas2, saposin-like protein-2 SAP-2, thioredoxin peroxidases TPx, Prx-1, Prx-2, cathepsin I cysteine proteinase CL3, protease cathepsin L CL1, phosphoglycerate kinase PGK, 27-kDa secretory protein, 60 kDa protein HSP35alpha, glutathione transferase GST, 28.5 kDa tegumental antigen 28.5 kDa TA, cathepsin B3 protease CatB3, Type I cystatin stefin-1, cathepsin L5, cathepsin L1g and cathepsin B, fatty acid binding protein FABP, leucine aminopeptidases LAP (Fasciola hepatica and Fasciola gigantica, Fasciolosis); prion protein (FFI prion, Fatal familial insomnia (FFI)); venom allergen homolog-like protein VAL-1, abundant larval transcript ALT-1, abundant larval transcript ALT-2, thioredoxin peroxidase TPX, vespid allergen homologue VAH, thiordoxin peroxidase 2 TPX-2, antigenic protein SXP (peptides N, N1, N2, and N3), activation associated protein-1 ASP-1, Thioredoxin TRX, transglutaminase BmTGA, glutathione-S-transferases GST, myosin, vespid allergen homologue VAH, 175 kDa collagenase, glyceraldehyde-3-phosphate dehydrogenase GAPDH, cuticular collagen Col-4, secreted larval acidic proteins SLAPs, chitinase CHI-1, maltose binding protein MBP, glycolytic enzyme fructose-1,6-bisphosphate aldolase Fba, tropomyosin TMY-1, nematode specific gene product OvB20, onchocystatin CPI-2, Cox-2 (Filarioidea superfamily, Filariasis); phospholipase C PLC, heat-labile enterotoxin B, Iota toxin component Ib, protein CPE1281 , pyruvate ferredoxin oxidoreductase, elongation factor G EF-G, perfringolysin O Pfo, glyceraldehyde-3-phosphate dehydrogenase GapC, Fructose-bisphosphate aldolase Alf2, clostridium perfringens enterotoxin CPE, alpha toxin AT, alpha toxoid ATd, epsilon-toxoid ETd, protein HP, large cytotoxin TpeL, endo-beta-N-acetylglucosaminidase Naglu, phosphoglyceromutase Pgm (Clostridium perfringens, Food poisoning by Clostridium perfringens); leukotoxin lkta, adhesion FadA, outer membrane protein RadD, high-molecular weight arginine-binding protein (Fusobacterium genus, Fusobacterium infection); phospholipase C PLC, heat-labile enterotoxin B, Iota toxin component Ib, protein CPE1281, pyruvate ferredoxin oxidoreductase, elongation factor G EF-G, perfringolysin O Pfo, glyceraldehyde-3-phosphate dehydrogenase GapC, fructose-bisphosphate aldolase Alf2, clostridium perfringens enterotoxin CPE, alpha toxin AT, alpha toxoid ATd, epsilon-toxoid ETd, protein HP, large cytotoxin TpeL, endo-beta-N-acetylglucosaminidase Naglu, phosphoglyceromutase Pgm (usually Clostridium perfringens; other Clostridium species, Gas gangrene (Clostridial myonecrosis)); lipase A, lipase B, peroxidase Dec1 (Geotrichum candidum, Geotrichosis); prion protein (GSS prion, Gerstmann-Sträussler-Scheinker syndrome (GSS)); cyst wall proteins CWP1, CWP2, CWP3, variant surface protein VSP, VSP1, VSP2, VSP3, VSP4, VSP5, VSP6, 56 kDa antigen, pyruvate ferredoxin oxidoreductase PFOR, alcohol dehydrogenase E ADHE, alpha-giardin, alpha8-giardin, alpha1-guiardin, beta-giardin, cystein proteases, glutathione-S-transferase GST, arginine deiminase ADI, fructose-1,6-bisphosphat aldolase FBA, Giardia trophozoite antigens GTA (GTA1, GTA2), ornithine carboxyl transferase OCT, striated fiber-asseblin-like protein SALP, uridine phosphoryl-like protein UPL, alpha-tubulin, beta-tubulin (Giardia intestinalis, Giardiasis); members of the ABC transporter family (LolC, OppA, and PotF), putative lipoprotein releasing system transmembrane protein LolC/E, flagellin FliC, Burkholderia intracellular motility A BimA, bacterial Elongation factor-Tu EF-Tu, 17 kDa OmpA-like protein, boaA coding protein (Burkholderia mallei, Glanders); cyclophilin CyP, 24 kDa third-stage larvae protien GS24, excretion-secretion products ESPs (40, 80, 120 and 208 kDa) (Gnathostoma spinigerum and Gnathostoma hispidum, Gnathostomiasis); pilin proteins, minor pilin-associated subunit pilC, major pilin subunit and variants pilE, piIS, phase variation protein porA, Porin B PorB, protein TraD, Neisserial outer membrane antigen H.8, 70kDa antigen, major outer membrane protein PI, outer membrane proteins PIA and PIB, W antigen, surface protein A NspA, transferrin binding protein TbpA, transferrin binding protein TbpB , PBP2, mtrR coding protein, ponA coding protein, membrane permease FbpBC, FbpABC protein system, LbpAB proteins, outer membrane protein Opa, outer membrane transporter FetA, iron-repressed regulator MpeR (Neisseria gonorrhoeae, Gonorrhea); outer membrane protein A OmpA, outer membrane protein C OmpC, outer membrane protein K17 OmpK17 (Klebsiella granulomatis, Granuloma inguinale (Donovanosis)); fibronectin-binding protein Sfb, fibronectin/fibrinogen-binding protein FBP54, fibronectin-binding protein FbaA, M protein type 1 Emm1, M protein type 6 Emm6, immunoglobulin-binding protein 35 Sib35, Surface protein R28 Spr28, superoxide dismutase SOD, C5a peptidase ScpA, antigen I/II AgI/II, adhesin AspA, G-related alpha2-macroglobulin-binding protein GRAB, surface fibrillar protein M5 (Streptococcus pyogenes, Group A streptococcal infection); C protein β antigen, arginine deiminase proteins, adhesin BibA, 105 kDA protein BPS, surface antigens c, surface antigens R, surface antigens X, trypsin-resistant protein R1, trypsin-resistant protein R3, trypsin-resistant protein R4, surface immunogenic protein Sip, surface protein Rib, Leucine-rich repeats protein LrrG, serine-rich repeat protein Srr-2, C protein alpha-antigen Bca, Beta antigen Bag, surface antigen Epsilon, alpha-like protein ALP1, alpha-like protein ALPS surface antigen delta, alpha-like protein ALP2, alpha-like protein ALP3, alpha-like protein ALP4, Cbeta protein Bac (Streptococcus agalactiae, Group B streptococcal infection); transferrin-binding protein 2 Tbp2, phosphatase P4, outer membrane protein P6, peptidoglycan-associated lipoprotein Pal, protein D, protein E, adherence and penetration protein Hap, outer membrane protein 26 Omp26, outer membrane protein P5 (Fimbrin), outer membrane protein D15, outer membrane protein OmpP2, 5'-nucleotidase NucA, outer membrane protein P1, outer membrane protein P2, outer membrane lipoprotein Pcp, Lipoprotein E, outer membrane protein P4, fuculokinase FucK, [Cu,Zn]-superoxide dismutase SodC, protease HtrA, protein 0145, alpha-galactosylceramide (Haemophilus influenzae, Haemophilus influenzae infection); polymerase 3D, viral capsid protein VP1, viral capsid protein VP2, viral capsid protein VP3, viral capsid protein VP4, protease 2A, protease 3C (Enteroviruses, mainly Coxsackie A virus and Enterovirus 71 (EV71), Hand, foot and mouth disease (HFMD)); RNA polymerase L, protein L, glycoprotein Gn, glycoprotein Gc, nucleocapsid protein S, envelope glycoprotein G1, nucleoprotein NP, protein N, polyprotein M (Sin Nombre virus, Hantavirus, Hantavirus Pulmonary Syndrome (HPS)); heat shock protein HspA, heat shock protein HspB, citrate synthase GltA, protein UreB, heat shock protein Hsp60, neutrophil-activating protein NAP, catalase KatA, vacuolating cytotoxin VacA, urease alpha UreA, urease beta Ureb, protein Cpn10, protein groES, heat shock protein Hsp10, protein MopB, cytotoxicity-associated 10 kDa protein CAG, 36 kDa antigen, beta-lactamase HcpA, Beta-lactamase HcpB (Helicobacter pylori, Helicobacter pylori infection); integral membrane proteins, aggregation-prone proteins, O-antigen, toxin-antigens Stx2B, toxin-antigen Stx1B, adhesion-antigen fragment Int28, protein EspA, protein EspB, Intimin, protein Tir, protein IntC300, protein Eae (Escherichia coli O157:H7, O111 and O104:H4, Hemolytic-uremic syndrome (HUS)); RNA polymerase L, protein L, glycoprotein Gn, glycoprotein Gc, nucleocapsid protein S, envelope glycoprotein G1, nucleoprotein NP, protein N, polyprotein M (Bunyaviridae family, Hemorrhagic fever with renal syndrome (HFRS)); glycoprotein G, matrix protein M, nucleoprotein N, fusion protein F, polymerase L, protein W, proteinC, phosphoprotein p, non-structural protein V (Henipavirus (Hendra virus Nipah virus), Henipavirus infections); polyprotein, glycoproten Gp2, hepatitis A surface antigen HBAg, protein 2A, virus protein VP1, virus protein VP2, virus protein VP3, virus protein VP4, protein P1B, protein P2A, protein P3AB, protein P3D (Hepatitis A Virus, Hepatitis A); hepatitis B surface antigen HBsAg, Hepatitis B core antigen HbcAg, polymerase, protein Hbx, preS2 middle surface protein, surface protein L, large S protein, virus protein VP1, virus protein VP2, virus protein VP3, virus protein VP4 (Hepatitis B Virus (HBV), Hepatitis B); envelope glycoprotein E1 gp32 gp35 , envelope glycoprotein E2 NS1 gp68 gp70, capsid protein C , core protein Core, polyprotein, virus protein VP1, virus protein VP2, virus protein VP3, virus protein VP4, antigen G, protein NS3, protein NS5A, (Hepatitis C Virus, Hepatitis C); virus protein VP1, virus protein VP2, virus protein VP3, virus protein VP4, large hepaptitis delta antigen, small hepaptitis delta antigen (Hepatitis D Virus, Hepatitis D); virus protein VP1, virus protein VP2, virus protein VP3, virus protein VP4, capsid protein E2 (Hepatitis E Virus, Hepatitis E); glycoprotein L UL1, uracil-DNA glycosylase UL2, protein UL3, protein UL4, DNA replication protein UL5, portal protein UL6, virion maturation protein UL7, DNA helicase UL8, replication origin-binding protein UL9, glycoprotein M UL10, protein UL11, alkaline exonuclease UL12, serine-threonine protein kinase UL13, tegument protein UL14, terminase UL15, tegument protein UL16, protein UL17, capsid protein VP23 UL18, major capsid protein VP5 UL19, membrane protein UL20, tegument protein UL21, Glycoprotein H (UL22), Thymidine Kinase UL23, protein UL24, protein UL25, capsid protein P40 (UL26, VP24, VP22A), glycoprotein B (UL27), ICP18.5 protein (UL28), major DNA-binding protein ICP8 (UL29), DNA polymerase UL30, nuclear matrix protein UL31, envelope glycoprotein UL32, protein UL33, inner nuclear membrane protein UL34, capsid protein VP26 (UL35), large tegument protein UL36, capsid assembly protein UL37, VP19C protein (UL38), ribonucleotide reductase (Large subunit) UL39, ribonucleotide reductase (Small subunit) UL40, tegument protein/virion host shutoff VHS protein (UL41), DNA polymerase processivity factor UL42, membrane protein UL43, glycoprotein C (UL44), membrane protein UL45, tegument proteins VP11/12 (UL46), tegument protein VP13/14 (UL47), virion maturation protein VP16 (UL48, Alpha-TIF), envelope protein UL49, dUTP diphosphatase UL50, tegument protein UL51, DNA helicase/primase complex protein UL52, glycoprotein K (UL53), transcriptional regulation protein IE63 (ICP27, UL54), protein UL55, protein UL56, viral replication protein ICP22 (IE68, US1), protein US2, serine/threonine-protein kinase US3, glycoprotein G (US4), glycoprotein J (US5), glycoprotein D (US6), glycoprotein I (US7), glycoprotein E (US8), tegument protein US9, capsid/tegument protein US10, Vmw21 protein (US11), ICP47 protein (IE12, US12), major transcriptional activator ICP4 (IE175, RS1), E3 ubiquitin ligase ICP0 (IE110), latency-related protein 1 LRP1, latency-related protein 2 LRP2, neurovirulence factor RL1 (ICP34.5), latency-associated transcript LAT (Herpes simplex virus 1 and 2 (HSV-1 and HSV-2), Herpes simplex); heat shock protein Hsp60, cell surface protein H1C, dipeptidyl peptidase type IV DppIV, M antigen, 70 kDa protein, 17 kDa histone-like protein (Histoplasma capsulatum, Histoplasmosis); fatty acid and retinol binding protein-1 FAR-1, tissue inhibitor of metalloproteinase TIMP (TMP), cysteine proteinase ACEY-1, cysteine proteinase ACCP-1, surface antigen Ac-16, secreted protein 2 ASP-2, metalloprotease 1 MTP-1, aspartyl protease inhibitor API-1, surface-associated antigen SAA-1, surface-associated antigen SAA-2, adult-specific secreted factor Xa, serine protease inhibitor anticoagulant AP, cathepsin D-like aspartic protease ARR-1, glutathione S-transferase GST, aspartic protease APR-1, acetylcholinesterase AChE (Ancylostoma duodenale and Necator americanus, Hookworm infection); protein NS1, protein NP1, protein VP1, protein VP2, protein VP3 (Human bocavirus (HBoV), Human bocavirus infection); major surface protein 2 MSP2, major surface protein 4 MSP4, MSP variant SGV1, MSP variant SGV2, outer membrane protein OMP, outer membrande protein 19 OMP-19, major antigenic protein MAP1, major antigenic protein MAP1-2, major antigenic protein MAP1B, major antigenic protein MAP1-3, Erum2510 coding protein, protein GroEL, protein GroES, 30-kDA major outer membrane proteins, GE 100-kDa protein, GE 130-kDa protein, GE 160-kDa protein (Ehrlichia ewingii, Human ewingii ehrlichiosis); major surface proteins 1-5 (MSP1a, MSP1b, MSP2, MSP3, MSP4, MSP5), type IV secreotion system proteins VirB2, VirB7, VirB11, VirD4 (Anaplasma phagocytophilum, Human granulocytic anaplasmosis (HGA)); protein NS1, small hydrophobic protein NS2, SH protein, fusion protein F, glycoprotein G, matrix protein M, matrix protein M2-1, matrix protein M2-2, phosphoprotein P, nucleoprotein N, polymerase L (Human metapneumovirus (hMPV), Human metapneumovirus infection); major surface protein 2 MSP2, major surface protein 4 MSP4, MSP variant SGV1, MSP variant SGV2, outer membrane protein OMP, outer membrande protein 19 OMP-19, major antigenic protein MAP1, major antigenic protein MAP1-2, major antigenic protein MAP1B, major antigenic protein MAP1-3, Erum2510 coding protein, protein GroEL, protein GroES, 30-kDA major outer membrane proteins, GE 100-kDa protein, GE 130-kDa protein, GE 160-kDa protein (Ehrlichia chaffeensis, Human monocytic ehrlichiosis); replication protein E1, regulatory protein E2, protein E3, protein E4, protein E5, protein E6, protein E7, protein E8, major capsid protein L1, minor capsid protein L2 (Human papillomavirus (HPV), Human papillomavirus (HPV) infection); fusion protein F, hemagglutinin-neuramidase HN, glycoprotein G, matrix protein M, phosphoprotein P, nucleoprotein N, polymerase L (Human parainfluenza viruses (HPIV), Human parainfluenza virus infection); Hemagglutinin (HA), Neuraminidase (NA), Nucleoprotein (NP), M1 protein, M2 protein, NS1 protein, NS2 protein (NEP protein: nuclear export protein), PA protein, PB1 protein (polymerase basic 1 protein), PB1-F2 protein and PB2 protein (Orthomyxoviridae family, Influenza virus (flu)); genome polyprotein, protein E, protein M, capsid protein C (Japanese encephalitis virus, Japanese encephalitis); RTX toxin, type IV pili, major pilus subunit PilA, regulatory transcription factors PilS and PilR, protein sigma54, outer membrane proteins (Kingella kingae, Kingella kingae infection); prion protein (Kuru prion, Kuru); nucleoprotein N, polymerase L, matrix protein Z, glycoprotein GP (Lassa virus, Lassa fever); peptidoglycan-associated lipoprotein PAL, 60 kDa chaperonin Cpn60 (groEL, HspB), type IV pilin PilE, outer membrane protein MIP, major outer membrane protein MompS, zinc metalloproteinase MSP (Legionella pneumophila, Legionellosis (Legionnaires' disease, Pontiac fever)); P4 nuclease, protein WD, ribonucleotide reductase M2, surface membrane glycoprotein Pg46, cysteine proteinase CP, glucose-regulated protein 78 GRP-78, stage-specific S antigen-like protein A2, ATPase F1, beta-tubulin, heat shock protein 70 Hsp70, KMP-11, glycoprotein GP63, protein BT1, nucleoside hydrolase NH, cell surface protein B1, ribosomal protein P1-like protein P1, sterol 24-c-methyltransferase SMT, LACK protein, histone H1, SPB1 protein, thiol specific antioxidant TSA, protein antigen STI1, signal peptidase SP, histone H2B, suface antigen PSA-2, cystein proteinase b Cpb (Leishmania genus, Leishmaniasis); major membrane protein I, serine-rich antigen- 45 kDa, 10 kDa caperonin GroES, HSP kDa antigen, amino-oxononanoate synthase AONS, protein recombinase A RecA, Acetyl-/propionyl-coenzyme A carboxylase alpha, alanine racemase, 60 kDa chaperonin 2, ESAT-6-like protein EcxB (L-ESAT-6), protein Lsr2, protein ML0276, Heparin-binding hemagglutinin HBHA, heat-shock protein 65 Hsp65, mycP1 or ML0041 coding protein , htrA2 or ML0176 coding protein , htrA4 or ML2659 coding protein, gcp or ML0379 coding protein, clpC or ML0235 coding protein (Mycobacterium leprae and Mycobacterium lepromatosis, Leprosy); outer membrane protein LipL32, membrane protein LIC10258, membrane protein LP30, membrane protein LIC12238, Ompa-like protein Lsa66, surface protein LigA, surface protein LigB, major outer membrane protein OmpL1, outer membrane protein LipL41, protein LigAni, surface protein LcpA, adhesion protein LipL53, outer membrane protein UpL32, surface protein Lsa63, flagellin FlaB1, membran lipoprotein LipL21, membrane protein pL40, leptospiral surface adhesin Lsa27, outer membrane protein OmpL36, outer membrane protein OmpL37, outer membrane protein OmpL47, outer membrane protein OmpL54, acyltransferase LpxA (Leptospira genus, Leptospirosis); listeriolysin O precursor Hly (LLO), invasion-associated protein Iap (P60), Listeriolysin regulatory protein PrfA, Zinc metalloproteinase Mpl, Phosphatidylinositol- specific phospholipase C PLC (PlcA, PlcB), O-acetyltransferase Oat, ABC-transporter permease Im.G_1771, adhesion protein LAP, LAP receptor Hsp60, adhesin LapB, haemolysin listeriolysin O LLO, protein ActA, Internalin A InlA, protein InlB (Listeria monocytogenes, Listeriosis); outer surface protein A OspA, outer surface protein OspB, outer surface protein OspC, decorin binding protein A DbpA, decorin binding protein B DbpB, flagellar filament 41 kDa core protein Fla, basic membrane protein A BmpA (Immunodominant antigen P39), outer surface 22 kDa lipoprotein precursor (antigen IPLA7), variable surface lipoprotein vlsE (usually Borrelia burgdorferi and other Borrelia species, Lyme disease (Lyme borreliosis)); venom allergen homolog-like protein VAL-1, abundant larval transcript ALT-1, abundant larval transcript ALT-2, thioredoxin peroxidase TPX, vespid allergen homologue VAH, thiordoxin peroxidase 2 TPX-2, antigenic protein SXP (peptides N, N1, N2, and N3), activation associated protein-1 ASP-1, thioredoxin TRX, transglutaminase BmTGA, glutathione-S-transferases GST, myosin, vespid allergen homologue VAH, 175 kDa collagenase, glyceraldehyde-3-phosphate dehydrogenase GAPDH, cuticular collagen Col-4, Secreted Larval Acidic Proteins SLAPs, chitinase CHI-1, maltose binding protein MBP, glycolytic enzyme fructose-1,6-bisphosphate aldolase Fba, tropomyosin TMY-1, nematode specific gene product OvB20, onchocystatin CPI-2, protein Cox-2 (Wuchereria bancrofti and Brugia malayi, Lymphatic filariasis (Elephantiasis)); glycoprotein GP, matrix protein Z, polymerase L, nucleoprotein N (Lymphocytic choriomeningitis virus (LCMV), Lymphocytic choriomeningitis); thrombospondin-related anonymous protein TRAP, SSP2 Sporozoite surface protein 2, apical membrane antigen 1 AMA1, rhoptry membrane antigen RMA1, acidic basic repeat antigen ABRA, cell-traversal protein PF, protein Pvs25, merozoite surface protein 1 MSP-1, merozoite surface protein 2 MSP-2, ring-infected erythrocyte surface antigen RESALiver stage antigen 3 LSA-3, protein Eba-175, serine repeat antigen 5 SERA-5, circumsporozoite protein CS, merozoite surface protein 3 MSP3, merozoite surface protein 8 MSP8, enolase PF10, hepatocyte erythrocyte protein 17 kDa HEP17, erythrocyte membrane protein 1 EMP1, protein Kbetamerozoite surface protein 4/5 MSP 4/5, heat shock protein Hsp90, glutamate-rich protein GLURP, merozoite surface protein 4 MSP-4, protein STARP, circumsporozoite protein-related antigen precursor CRA (Plasmodium genus, Malaria); nucleoprotein N, membrane-associated protein VP24, minor nucleoprotein VP30, polymerase cofactor VP35, polymerase L, matrix protein VP40, envelope glycoprotein GP (Marburg virus, Marburg hemorrhagic fever (MHF)); protein C, matrix protein M, phosphoprotein P, non-structural protein V, hemagglutinin glycoprotein H, polymerase L, nucleoprotein N, fusion protein F (Measles virus, Measles); members of the ABC transporter family (LolC, OppA, and PotF), putative lipoprotein releasing system transmembrane protein LolC/E, flagellin FliC, Burkholderia intracellular motility A BimA, bacterial Elongation factor-Tu EF-Tu, 17 kDa OmpA-like protein, boaA coding protein, boaB coding protein (Burkholderia pseudomallei, Melioidosis (Whitmore's disease)); pilin proteins, minor pilin-associated subunit pilC, major pilin subunit and variants pilE, pilS, phase variation protein porA, Porin B PorB, protein TraD, Neisserial outer membrane antigen H.8, 70kDa antigen, major outer membrane protein PI, outer membrane proteins PIA and PIB, W antigen, surface protein A NspA, transferrin binding protein TbpA, transferrin binding protein TbpB , PBP2, mtrR coding protein, ponA coding protein, membrane permease FbpBC, FbpABC protein system, LbpAB proteins, outer membrane protein Opa, outer membrane transporter FetA, iron-repressed regulator MpeR, factor H-binding protein fHbp, adhesin NadA, protein NhbA, repressor FarR (Neisseria meningitidis, Meningococcal disease); 66 kDa protein, 22 kDa protein (usually Metagonimus yokagawai, Metagonimiasis); polar tube proteins (34, 75, and 170 kDa in Glugea, 35, 55 and 150kDa in Encephalitozoon), kinesin-related protein, RNA polymerase II largest subunit, similar ot integral membrane protein YIPA, anti-silencing protein 1, heat shock transcription factor HSF, protein kinase, thymidine kinase, NOP-2 like nucleolar protein (Microsporidia phylum, Microsporidiosis); CASP8 and FADD-like apoptosis regulator, Glutathione peroxidase GPX1, RNA helicase NPH-II NPH2, Poly(A) polymerase catalytic subunit PAPL, Major envelope protein P43K, early transcription factor 70 kDa subunit VETFS, early transcription factor 82 kDa subunit VETFL, metalloendopeptidase G1-type, nucleoside triphosphatase I NPH1, replication protein A28-like MC134L, RNA polymease 7 kDa subunit RPO7 (Molluscum contagiosum virus (MCV), Molluscum contagiosum (MC)); matrix protein M, phosphoprotein P/V, small hydrophobic protein SH, nucleoprotein N, protein V, fusion glycoprotein F, hemagglutinin-neuraminidase HN, RNA polymerase L (Mumps virus, Mumps); Outer membrane proteins OM, cell surface antigen OmpA, cell surface antigen OmpB (sca5), cell surface protein SCA4, cell surface protein SCA1, intracytoplasmic protein D, crystalline surface layer protein SLP, protective surface protein antigen SPA (Rickettsia typhi, Murine typhus (Endemic typhus)); adhesin P1, adhesion P30, protein p116, protein P40, cytoskeletal protein HMW1, cytoskeletal protein HMW2, cytoskeletal protein HMW3, MPN152 coding protein, MPN426 coding protein, MPN456 coding protein, MPN-500coding protein (Mycoplasma pneumoniae, Mycoplasma pneumonia); NocA, Iron dependent regulatory protein, VapA, VapD, VapF, VapG, caseinolytic protease, filament tip-associated 43-kDa protein, protein P24, protein P61, 15-kDa protein, 56-kDa protein (usually Nocardia asteroides and other Nocardia species, Nocardiosis); venom allergen homolog-like protein VAL-1, abundant larval transcript ALT-1, abundant larval transcript ALT-2, thioredoxin peroxidase TPX, vespid allergen homologue VAH, thiordoxin peroxidase 2 TPX-2, antigenic protein SXP (peptides N, N1, N2, and N3), activation associated protein-1 ASP-1, Thioredoxin TRX, transglutaminase BmTGA, glutathione-S-transferases GST, myosin, vespid allergen homologue VAH, 175 kDa collagenase, glyceraldehyde-3-phosphate dehydrogenase GAPDH, cuticular collagen Col-4, Secreted Larval Acidic Proteins SLAPs, chitinase CHI-1, maltose binding protein MBP, glycolytic enzyme fructose-1,6-bisphosphate aldolase Fba, tropomyosin TMY-1, nematode specific gene product OvB20, onchocystatin CPI-2, Cox-2 (Onchocerca volvulus, Onchocerciasis (River blindness)); 43 kDa secreted glycoprotein, glycoprotein gp0, glycoprotein gp75, antigen Pb27, antigen Pb40, heat shock protein Hsp65, heat shock protein Hsp70, heat shock protein Hsp90, protein P10, triosephosphate isomerase TPI, N-acetyl-glucosamine-binding lectin Paracoccin, 28 kDa protein Pb28 (Paracoccidioides brasiliensis, Paracoccidioidomycosis (South American blastomycosis)); 28-kDa cruzipain-like cystein protease Pw28CCP (usually Paragonimus westermani and other Paragonimus species, Paragonimiasis); outer membrane protein OmpH, outer membrane protein Omp28, protein PM1539, protein PM0355, protein PM1417, repair protein MutL, protein BcbC, prtein PM0305, formate dehydrogenase-N, protein PM0698, protein PM1422, DNA gyrase, lipoprotein PlpE, adhesive protein Cp39, heme aquisition system receptor HasR, 39 kDa capsular protein, iron-regulated OMP IROMP, outer membrane protein OmpA87, fimbrial protein Ptf, fimbrial subunit protein PtfA, transferrin binding protein Tbpl, esterase enzyme MesA, Pasteurella multocida toxin PMT, adhesive protein Cp39 (Pasteurella genus, Pasteurellosis); "filamentous hemagglutinin FhaB, adenylate cyclase CyaA, pertussis toxin subunit 4 precursor PtxD, pertactin precursor Prn, toxin subunit 1 PtxA, protein Cpn60, protein brkA, pertussis toxin subunit 2 precursor PtxB, pertussis toxin subunit 3 precursor PtxC, pertussis toxin subunit 5 precursor PtxE, pertactin Prn, protein Fim2, protein Fim3; " (Bordetella pertussis, Pertussis (Whooping cough)); "F1 capsule antigen, virulence-associated V antigen, secreted effector protein LcrV, V antigen, outer membrane protease Pla,secreted effector protein YopD, putative secreted protein-tyrosine phosphatase YopH, needle complex major subunit YscF, protein kinase YopO, putative autotransporter protein YapF, inner membrane ABC-transporter YbtQ (Irp7), putative sugar binding protein YPO0612, heat shock protein 90 HtpG, putative sulfatase protein YdeN, outer-membrane lipoprotein carrier protein LolA, secretion chaperone YerA, putative lipoprotein YPO0420, hemolysin activator protein HpmB, pesticin/yersiniabactin outer membrane receptor Psn, secreted effector protein YopE, secreted effector protein YopF, secreted effector protein YopK, outer membrane protein YopN , outer membrane protein YopM, Coagulase/fibrinolysin precursor Pla ; " (Yersinia pestis, Plague); protein PhpA, surface adhesin PsaA, pneumolysin Ply, ATP-dependent protease Clp, lipoate-protein ligase LplA, cell wall surface anchored protein psrP, sortase SrtA, glutamyl-tRNA synthetase GltX, choline binding protein A CbpA, pneumococcal surface protein A PspA, pneumococcal surface protein C PspC, 6-phosphogluconate dehydrogenase Gnd, iron-binding protein PiaA, Murein hydrolase LytB, proteon LytC, protease A1 (Streptococcus pneumoniae, Pneumococcal infection); major surface protein B, kexin-like protease KEX1, protein A12, 55 kDa antigen P55, major surface glycoprotein Msg (Pneumocystis jirovecii, Pneumocystis pneumonia (PCP)); genome polyprotein, polymerase 3D, viral capsid protein VP1, viral capsid protein VP2, viral capsid protein VP3, viral capsid protein VP4, protease 2A, protease 3C (Poliovirus, Poliomyelitis); protein Nfa1, exendin-3, secretory lipase, cathepsin B-like protease, cysteine protease, cathepsin, peroxiredoxin, protein Cry1Ac (usually Naegleria fowleri, Primary amoebic meningoencephalitis (PAM)); agnoprotein, large T antigen, small T antigen, major capsid protein VP1, minor capsid protein Vp2 (JC virus, Progressive multifocal leukoencephalopathy); low calcium response protein E LCrE, chlamydial outer protein N CopN, serine/threonine-protein kinase PknD, acyl-carrier-protein S-malonyltransferase FabD, single-stranded DNA-binding protein Ssb, major outer membrane protein MOMP, outer membrane protein 2 Omp2, polymorphic membrane protein family (Pmp1, Pmp2, Pmp3, Pmp4, Pmp5, Pmp6, Pmp7, Pmp8, Pmp9, Pmp10, Pmp11, Pmp12, Pmp13, Pmp14, Pmp15, Pmp16, Pmp17, Pmp18, Pmp19, Pmp20, Pmp21) (Chlamydophila psittaci, Psittacosis); outer membrane protein P1, heat shock protein B HspB, peptide ABC transporter, GTP-binding protein, protein IcmB, ribonuclease R, phosphatas SixA, protein DsbD, outer membrane protein TolC, DNA-binding protein PhoB, ATPase DotB, heat shock protein B HspB, membrane protein Com1, 28 kDa protein, DNA-3-methyladenine glycosidase I, pouter membrane protein OmpH, outer membrane protein AdaA, glycine cleavage system T-protein (Coxiella burnetii, Q fever); nucleoprotein N, large structural protein L, phophoprotein P, matrix protein M, glycoprotein G (Rabies virus, Rabies); fusionprotein F, nucleoprotein N, matrix protein M, matrix protein M2-1, matrix protein M2-2, phophoprotein P, small hydrophobic protein SH, major surface glycoprotein G, polymerase L, non-structural protein 1 NS1, non-structural protein 2 NS2 (Respiratory syncytial virus (RSV), Respiratory syncytial virus infection); genome polyprotein, polymerase 3D, viral capsid protein VP1, viral capsid protein VP2, viral capsid protein VP3, viral capsid protein VP4, protease 2A, protease 3C (Rhinovirus, Rhinovirus infection); outer membrane proteins OM, cell surface antigen OmpA, cell surface antigen OmpB (sca5), cell surface protein SCA4, cell surface protein SCA1, protein PS120, intracytoplasmic protein D, protective surface protein antigen SPA (Rickettsia genus, Rickettsial infection); outer membrane proteins OM, cell surface antigen OmpA, cell surface antigen OmpB (sca5), cell surface protein SCA4, cell surface protein SCA1, intracytoplasmic protein D (Rickettsia akari, Rickettsialpox); envelope glycoprotein GP, polymerase L, nucleoprotein N, non-structural protein NSS (Rift Valley fever virus, Rift Valley fever (RVF)); outer membrane proteins OM, cell surface antigen OmpA, cell surface antigen OmpB (sca5), cell surface protein SCA4, cell surface protein SCA1, intracytoplasmic protein D (Rickettsia rickettsii, Rocky mountain spotted fever (RMSF)); non-structural protein 6 NS6, non-structural protein 2 NS2, intermediate capsid protein VP6, inner capsid protein VP2, non-structural protein 3 NS3, RNA-directed RNA polymerase L, protein VP3, non-structural protein 1 NS1, non-structural protein 5 NS5, outer capsid glycoprotein VP7, non-structural glycoprotein 4 NS4, outer capsid protein VP4;(Rotavirus, Rotavirus infection); polyprotein P200, glycoprotein E1, glycoprotein E2, protein NS2, capsid protein C (Rubella virus, Rubella); chaperonin GroEL (MopA), inositol phosphate phosphatase SopB, heat shock protein HslU, chaperone protein DnaJ, protein TviB, protein IroN, flagellin FliC, invasion protein SipC, glycoprotein gp43, outer membrane protein LamB, outer membrane protein PagC, outer membrane protein TolC, outer membrane protein NmpC, outer membrane protein FadL, transport protein SadA, transferase WgaP, effector proteins SifA, SteC, SseL, SseJ and SseF (Salmonella genus, Salmonellosis); "protein 14, non-structural protein NS7b, non-structural protein NS8a, protein 9b, protein 3a, nucleoprotein N, non-structural protein NS3b, non-structural protein NS6, protein 7a, non-structural protein NS8b, membrane protein M, envelope small membrane protein EsM, replicase polyprotein 1a, spike glycoprotein S, replicase polyprotein 1ab; SARS coronavirus, SARS (Severe Acute Respiratory Syndrome)); serin protease, Atypical Sarcoptes Antigen 1 ASA1, glutathione S-transferases GST, cystein protease, serine protease, apolipoprotein (Sarcoptes scabiei, Scabies); glutathione S-transferases GST, paramyosin, hemoglbinase SM32, major egg antigen, 14 kDa fatty acid-binding protein Sm14, major larval surface antigen P37, 22,6 kDa tegumental antigen, calpain CANP, triphospate isomerase Tim, surface protein 9B, outer capsid protein VP2, 23 kDa integral membrane protein Sm23, Cu/Zn-superoxide dismutase, glycoprotein Gp, myosin (Schistosoma genus, Schistosomiasis (Bilharziosis)); 60 kDa chaperonin, 56 kDa type-specific antigen, pyruvate phosphate dikinase, 4-hydroxybenzoate octaprenyltransferase (Orientia tsutsugamushi, Scrub typhus); dehydrogenase GuaB, invasion protein Spa32, invasin IpaA, invasin IpaB, invasin IpaC, invasin IpaD, invasin IpaH, invasin IpaJ (Shigella genus, Shigellosis (Bacillary dysentery)); protein P53, virion protein US10 homolog, transcriptional regulator IE63, transcriptional transactivator IE62, protease P33, alpha trans-inducing factor 74 kDa protein, deoxyuridine 5'-triphosphate nucleotidohydrolase, transcriptional transactivator IE4, membrane protein UL43 homolog, nuclear phosphoprotein UL3 homolog, nuclear protein UL4 homolog, replication origin-binding protein, membrane protein 2, phosphoprotein 32, protein 57,DNA polymerase processivity factor, portal protein 54, DNA primase, tegument protein UL14 homolog, tegument protein UL21 homolog, tegument protein UL55 homolog,tripartite terminase subunit UL33 homolog,tripartite terminase subunit UL15 homolog, capsid-binding protein 44, virion-packaging protein 43 (Varicella zoster virus (VZV), Shingles (Herpes zoster)); truncated 3-beta hydroxy-5-ene steroid dehydrogenase homolog, virion membrane protein A13, protein A19, protein A31, truncated protein A35 homolog, protein A37.5 homolog, protein A47, protein A49, protein A51, semaphorin-like protein A43, serine proteinase inhibitor 1, serine proteinase inhibitor 2, serine proteinase inhibitor 3, protein A6, protein B15, protein C1, protein C5, protein C6, protein F7, protein F8, protein F9, protein F11, protein F14, protein F15, protein F16 (Variola major or Variola minor, Smallpox (Variola)); adhesin/glycoprotein gp70, proteases (Sporothrix schenckii, Sporotrichosis); heme-iron binding protein IsdB, collagen adhesin Cna, clumping factor A ClfA, protein MecA, fibronectin-binding protein A FnbA, enterotoxin type A EntA, enterotoxin type B EntB, enterotoxin type C EntC1, enterotoxin type C EntC2, enterotoxin type D EntD, enterotoxin type E EntE, Toxic shock syndrome toxin-1 TSST-1, Staphylokinase, Penicillin binding protein 2a PBP2a (MecA), secretory antigen SssA (Staphylococcus genus, Staphylococcal food poisoning); heme-iron binding protein IsdB, collagen adhesin Cna, clumping factor A ClfA, protein MecA, fibronectin-binding protein A FnbA, enterotoxin type A EntA, enterotoxin type B EntB, enterotoxin type C EntC1, enterotoxin type C EntC2, enterotoxin type D EntD, enterotoxin type E EntE, Toxic shock syndrome toxin-1 TSST-1, Staphylokinase, Penicillin binding protein 2a PBP2a (MecA), secretory antigen SssA (Staphylococcus genus e.g. aureus, Staphylococcal infection); antigen Ss-IR, antigen NIE, strongylastacin, Na+-K+ ATPase Sseat-6, tropomysin SsTmy-1, protein LEC-5, 41 kDa aantigen P5, 41-kDa larval protein, 31-kDa larval protein, 28-kDa larval protein (Strongyloides stercoralis, Strongyloidiasis); glycerophosphodiester phosphodiesterase GlpQ (Gpd), outer membrane protein TmpB, protein Tp92, antigen TpF1, repeat protein Tpr, repeat protein F TprF, repeat protein G TprG, repeat protein I TprI, repeat protein J TprJ, repeat protein K TprK, treponemal membrane protein A TmpA, lipoprotein, 15 kDa Tpp15, 47 kDa membrane antigen, miniferritin TpF1, adhesin Tp0751, lipoprotein TP0136, protein TpN17, protein TpN47, outer membrane protein TP0136, outer membrane protein TP0155, outer membrane protein TP0326, outer membrane protein TP0483, outer membrane protein TP0956 (Treponema pallidum, Syphilis); Cathepsin L-like proteases, 53/25-kDa antigen, 8kDa family members, cysticercus protein with a marginal trypsin-like activity TsAg5, oncosphere protein TSOL18, oncosphere protein TSOL45-1A, lactate dehydrogenase A LDHA, lactate dehydrogenase B LDHB (Taenia genus, Taeniasis); tetanus toxin TetX, tetanus toxin C TTC, 140 kDa S layer protein, flavoprotein beta-subunit CT3, phospholipase (lecithinase), phosphocarrier protein HPr (Clostridium tetani, Tetanus (Lockjaw)); genome polyprotein, protein E, protein M, capsid protein C (Tick-borne encephalitis virus (TBEV), Tick-borne encephalitis); 58-kDa antigen, 68-kDa antigens, Toxocara larvae excretory-secretory antigen TES, 32-kDa glycoprotein, glycoprotein TES-70, glycoprotein GP31, excretory-secretory antigen TcES-57, perienteric fluid antigen Pe, soluble extract antigens Ex, excretory/secretory larval antigens ES, antigen TES-120, polyprotein allergen TBA-1, cathepsin L-like cysteine protease c-cpl-1, 26-kDa protein (Toxocara canis or Toxocara cati, Toxocariasis (Ocular Larva Migrans (OLM) and Visceral Larva Migrans (VLM))); microneme proteins ( MIC1, MIC2, MIC3, MIC4, MIC5, MIC6, MIC7, MIC8), rhoptry protein Rop2, rhoptry proteins (Rop1, Rop2, Rop3, Rop4, Rop5, Rop6, Rop7, Rop16, Rjop17), protein SR1,surface antigen P22, major antigen p24, major surface antigen p30, dense granule proteins (GRA1, GRA2, GRA3, GRA4, GRA5, GRA6, GRA7, GRA8, GRA9, GRA10), 28 kDa antigen, surface antigen SAG1, SAG2 related antigen, nucleoside-triphosphatase 1, nucleoside-triphosphatase 2, protein Stt3, HesB-like domain-containing protein, rhomboid-like protease 5, toxomepsin 1 (Toxoplasma gondii, Toxoplasmosis); 43 kDa secreted glycoprotein, 53 kDa secreted glycoprotein, paramyosin, antigen Ts21, antigen Ts87, antigen p46000, TSL-1 antigens, caveolin-1 CAV-1, 49 kDa newborn larva antigen, prosaposin homologue, serine protease, serine proteinase inhibitor, 45 -kDa glycoprotein Gp45 (Trichinella spiralis, Trichinellosis); Myb-like transcriptional factors (Myb1, Myb2, Myb3), adhesion protein AP23, adhesion protein AP33, adhesin protein AP33-3, adhesins AP51, adhesin AP65, adhesion protein AP65-1, alpha-actinin, kinesin-associated protein, teneurin, 62 kDa proteinase, subtilisin-like serine protease SUB1, cysteine proteinase gene 3 CP3, alpha-enolase Enol, cysteine proteinase CP30, heat shock proteins (Hsp70, Hsp60) , immunogenic protein P270, (Trichomonas vaginalis, Trichomoniasis); beta-tubulin, 47-kDa protein, secretory leucocyte-like proteinase-1 SLP-1, 50-kDa protein TT50, 17 kDa antigen, 43/47 kDa protein (Trichuris trichiura, Trichuriasis (Whipworm infection)); protein ESAT-6 (EsxA), 10 kDa filtrate antigen EsxB, secreted antigen 85-B FBPB, fibronectin-binding protein A FbpA (Ag85A), serine protease PepA, PPE family protein PPE18, fibronectin-binding protein D FbpD, immunogenic protein MPT64, secreted protein MPT51, catalase-peroxidase-peroxynitritase T KATG, periplasmic phosphate-binding lipoprotein PSTS3 (PBP-3, Phos-1), iron-regulated heparin binding hemagglutinin Hbha, PPE family protein PPE14, PPE family protein PPE68, protein Mtb72F, protein Apa, immunogenic protein MPT63, periplasmic phosphate-binding lipoprotein PSTS1 (PBP-1), molecular chaperone DnaK, cell surface lipoprotein Mpt83, lipoprotein P23, phosphate transport system permease protein pstA, 14 kDa antigen, fibronectin-binding protein C FbpC1, Alanine dehydrogenase TB43, Glutamine synthetase 1, ESX-1 protein, protein CFP10, TB10.4 protein, protein MPT83, protein MTB12, protein MTB8, Rpf-like proteins, protein MTB32, protein MTB39, crystallin, heat-shock protein HSP65, protein PST-S (usually Mycobacterium tuberculosis, Tuberculosis); outer membrane protein FobA, outer membrane protein FobB, intracellular growth locus IgIC1, intracellular growth locus IglC2, aminotransferase Wbtl, chaperonin GroEL, 17 kDa major membrane protein TUL4, lipoprotein LpnA, chitinase family 18 protein, isocitrate dehydrogenase, Nif3 family protein, type IV pili glycosylation protein, outer membrane protein tolC, FAD binding family protein, type IV pilin multimeric outer membrane protein, two component sensor protein KdpD, chaperone protein DnaK, protein TolQ (Francisella tularensis, Tularemia); "MB antigen, urease, protein GyrA, protein GyrB, protein ParC, protein ParE, lipid associated membrane proteins LAMP, thymidine kinase TK, phospholipase PL-A1, phospholipase PL-A2, phospholipase PL-C, surface-expressed 96-kDa antigen; " (Ureaplasma urealyticum, Ureaplasma urealyticum infection); non-structural polyprotein, structural polyprotein, capsid protein CP, protein E1, protein E2, protein E3, protease P1, protease P2, protease P3 (Venezuelan equine encephalitis virus, Venezuelan equine encephalitis); glycoprotein GP, matrix protein Z, polymerase L, nucleoprotein N (Guanarito virus, Venezuelan hemorrhagic fever); polyprotein, protein E, protein M, capsid protein C, protease NS3, protein NS1, protein NS2A, protein AS2B, brotein NS4A, protein NS4B, protein NS5 (West Nile virus, West Nile Fever); cpasid protein CP, protein E1, protein E2, protein E3, protease P2 (Western equine encephalitis virus, Western equine encephalitis); genome polyprotein, protein E, protein M, capsid protein C, protease NS3, protein NS1, protein NS2A, protein AS2B, protein NS4A, protein NS4B, protein NS5 (Yellow fever virus, Yellow fever); putative Yop targeting protein YobB, effector protein YopD, effector protein YopE, protein YopH, effector protein YopJ, protein translocation protein YopK, effector protein YopT, protein YpkA, flagellar biosyntheses protein FlhA, peptidase M48, potassium efflux system KefA, transcriptional regulatoer RovA, adhesin Ifp, translocator portein LcrV, protein PcrV, invasin Inv, outer membrane protein OmpF-like porin, adhesin YadA, protein kinase C, phospholipase C1, protein PsaA, mannosyltransferase-like protein WbyK, protein YscU, antigen YPMa (Yersinia pseudotuberculosis, Yersinia pseudotuberculosis infection); effector protein YopB, 60 kDa chaperonin, protein WbcP, tyrosin-protein phosphatase YopH, protein YopQ, enterotoxin, Galactoside permease, reductaase NrdE, protein YasN, Invasin Inv, adhesin YadA, outer membrane porin F OmpF, protein UspA1, protein EibA, protein Hia, cell surface protein Ail, chaperone SycD, protein LcrD, protein LcrG, protein LcrV, protein SycE, protein YopE, regulator protein TyeA, protein YopM, protein YopN, protein YopO, protein YopT, protein YopD, protease ClpP, protein MyfA, protein FilA, and protein PsaA (Yersinia enterocolitica, Yersiniosis).

The brackets in the preceding section indicate the particular pathogen or the family of pathogens of which the antigen(s) is/are derived and the infectious disease with which the pathogen is associated.

### Influenza:

In a particularly preferred embodiment of the first aspect of the invention the mRNA compound comprises a mRNA sequence comprises a coding region, encoding at least one antigenic peptide or protein derived from hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, or polymerase basic protein 2 (PB2) of an influenza virus or a fragment or variant thereof.

In this context, the amino acid sequence of the at least one antigenic peptide or protein may be selected from any peptide or protein derived from hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, or polymerase basic protein 2 (PB2) of an influenza virus or a fragment or variant or from any synthetically engineered influenza virus peptide or protein.

In a preferred embodiment of the present invention the coding region encodes at least one antigenic peptide or protein derived from hemagglutinin (HA) and/or neuraminidase (NA) of an influenza virus or a fragment or variant thereof. In this context the hemagglutinin (HA) and the neuraminidase (NA) may be chosen from the same influenza virus or from different influenza viruses.

In this context it is particularly preferred that the at least one coding region encodes at least one full-length protein of hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, or polymerase basic protein 2 (PB2) of an influenza virus or a variant thereof.

In particularly preferred embodiments the at least one coding region encodes at least one full-length protein of hemagglutinin (HA), and/or at least one full-length protein of neuraminidase (NA) of an influenza virus or a variant thereof.

The term "full-length protein" as used herein typically refers to a protein that substantially comprises the entire amino acid sequence of the naturally occurring protein. As used herein, the term "full-length protein" preferably relates to the full-length sequence of a protein as indicated in the sequence listing of the present inventioni.e. to an amino acid sequence as defined by any one of the SEQ ID NOs listed in the sequence listing (SEQ ID NOs: 1-30504 or SEQ ID NO: 224269 or SEQ ID NO: 224309) or to an amino acid provided in the database under the respective database accession number.

### Preferred sequences of the present invention:

In this context it is further preferred that the at least one coding sequence of the mRNA sequence of the present invention encodes at least one antigenic peptide or protein which is derived from
a hemagglutinin (HA) protein of an influenza A virus; or
a hemagglutinin (HA) protein of an influenza B virus; or
a neuraminidase (NA) protein of an influenza A virus; or
a neuraminidase (NA) protein of an influenza B virus; or
or a fragment or variant thereof, wherein the hemagglutinin (HA) protein of an influenza A virus or the hemagglutinin (HA) protein of an influenza B virus or the neuraminidase (NA) protein of an influenza A virus or the neuraminidase (NA) protein of an influenza B virus is selected from the hemagglutinin (HA) proteins or the neuraminidase (NA) proteins as listed in the sequence listing of the present invention.

The sequence listing discloses all influenza A or influenza B virus hemagglutinin (HA) proteins and all influenza A or influenza B virus neuraminidase (NA) proteins which are preferred in the present invention. Each preferred antigenic peptide or protein and its coding sequence can be identified with the data element shown under the numeric identifier <223>. In other words, each preferred hemagglutinin (HA) or neuraminidase (NA) sequence from an influenza A or B virus can be identified through the specific database accession number (i.e. a GenBank Protein or Nucleic Acid Accession No.) by reading through the sequence listing entries under numeric identifier <223>.

In sum, each preferred sequence is depicted by its GenBank Protein or Nucleic Acid Accession No. which again is depicted with seven distinct preferred SEQ ID NO in the sequence listing (protein, nucleic acid wild type, nucleic acid optimizations 1 to 5). This is apparent from the numeric identifier <223>.
I.e. the first consecutive entry of a specific GenBank Protein or Nucleic Acid Accession No. in the sequence listing under numeric identifier <223> indicates the SEQ ID NO: corresponding to the respective AMINO ACID SEQUENCE (i.e. Protein Sequence wild type SEQ ID NO).

Further, the second consecutive entry of a GenBank Protein or Nucleic Acid Accession No. in the sequence listing under numeric identifier <223> corresponds to the NUCLEIC ACID SEQUENCE of the wild type mRNA encoding the protein (i.e. Nucleotide Sequence wild type SEQ ID NO).

Further, the next five consecutive entries of a GenBank Protein or Nucleic Acid Accession No. in the sequence listing under numeric identifier <223> provide the SEQ ID NOs corresponding to five different MODIFIED/OPTIMIZED NUCLEIC ACID SEQUENCES of the sequences as described herein that encode the protein preferably having the amino acid sequence as defined by the first consecutive entry for a specific GenBank Protein or Nucleic Acid Accession No. in the sequence listing (i.e. Optimized Nucleotide Sequence SEQ ID NO).

Accordingly, a reference to a specific GenBank Protein or Nucleic Acid Accession No equals to a reference to the block of seven sequences as described above (protein = 1 , nucleic acid = 2, optimized sequences = 3-7).

One example would be the first GenBank Protein or Nucleic Acid Accession No. which is mentioned in the sequence listing, i.e. under SEQ ID NO:1 numeric identifier <223>: AAA16879. If Accession No. AAA16879 is searched throughout the sequence listing it is apparent that, as described above, seven SEQ ID NO are connected to this Accession No.: SEQ ID NO:1, SEQ ID NO:32013, SEQ ID NO:64025, SEQ ID NO:96037, SEQ ID NO:128049, SEQ ID NO:160061, and SEQ ID NO:192073.

In accordance with the above explanation,
for SEQ ID NO:1, the numeric identifier <223> reads "derived and/or modified protein sequence (wt) from hemagglutinin_InfluenzaA_AAA16879";
for SEQ ID NO: 32013, the numeric identifier <223> reads "derived and/or modified CDS sequence (wt) from hemagglutinin_InfluenzaA_AAA16879" ;
for SEQ ID NO: 64025, the numeric identifier <223> reads "derived and/or modified CDS sequence (opt1) from hemagglutinin_InfluenzaA_AAA16879" ;
for SEQ ID NO: 96037, the numeric identifier <223> reads "derived and/or modified CDS sequence (opt2) from hemagglutinin__InfluenzaA__AAA16879" ;
for SEQ ID NO: 128049, the numeric identifier <223> reads "derived and/or modified CDS sequence (opt3) from hemagglutinin_InfluenzaA_AAA16879" ;
for SEQ ID NO: 160061, the numeric identifier <223> reads "derived and/or modified CDS sequence (opt4) from hemagglutinin_InfluenzaA_AAA16879" ; and
for SEQ ID NO: 192073, the numeric identifier <223> reads "derived and/or modified CDS sequence (opt5) from hemagglutinin_InfluenzaA_AAA16879".

Therefore, a reference to AAA16879 equals to a reference to the seven sequences as described above (protein = 1^{st} sequence , nucleic acid = 2^{nd} sequence, five different optimized sequences = 3^{rd} -7^{th} sequence).

A second example would be the second GenBank Protein or Nucleic Acid Accession No. which is mentioned in the sequence listing, i.e. under SEQ ID NO:2 numeric identifier <223>: "AAA16880". Accession No. AAA16880 is connected to these seven sequences in the sequence listing: SEQ ID NOs:2 (protein), 32014 (nucleic acid wild type), 64026 (optimization 1), 96038 (optimization 2), 128050 (optimization 3), 160062 (optimization 4), and 192074 (optimization 5). Accordingly, a reference to AAA16880 equals to a reference to the seven sequences as described above.

A further illustration of this circumstance can be seen in exemplary figures 20-24, which show the structure of the sequence listing by exemplifying hemagglutinin (HA) proteins and neuraminidase (NA) proteins of influenza A and B viruses and glycoproteins of Rabies virus:
- exemplary hemagglutinin (HA) proteins of influenza A virus (Figure 20);
- exemplary hemagglutinin (HA) proteins of influenza B virus (Figure 21);
- exemplary neuraminidase (NA) proteins of influenza A virus (Figure 22);
- exemplary neuraminidase (NA) proteins of influenza B virus (Figure 23);
- exemplary glycoproteins of Rabies virus (Figure 24).

Reference is made herein to the content of figures 20-24 of PCT/EP2016/075843 filed on October 26, 2016, i.e. the priority application of the present international patent application, which is incorporated herein by reference.

In specific embodiments the influenza virus peptide or protein is derived from an influenza A, B or C virus (strain).

The influenza A virus may be selected from influenza A viruses characterized by a hemagglutinin (HA) selected from the group consisting of H1, H2, H3, H4, H5, H6, H7, H8, H9, H10, H11, H12, H13, H14, H15, H16, H17 and H18. Preferably the influenza A virus is selected from an influenza virus characterized by a hemagglutinin (HA) selected from the group consisting of H1, H3, H5 or H9.

Furthermore, particularly preferred are influenza A viruses characterized by a neuraminidase (NA) selected from the group consisting of N1, N2, N3, N4, N5, N6, N7, N8, N9, N10, and N11. Most preferably the influenza A virus is characterized by a neuraminidase (NA) selected from the group consisting of N1, N2, and N8.

In particularly preferred embodiments the influenza A virus is selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, H10N8, and H10N7, preferably from H1N1, H3N2, H5N1, and H5N8.

In this context it is particularly preferred that the at least one coding region of the inventive mRNA sequence encodes at least one antigenic peptide or protein derived from hemagglutinin (HA) and/or at least one antigenic peptide or protein derived from neuraminidase (NA) of an influenza A virus selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, H10N8 and H10N7, preferably from H1N1, H3N2, H5N1, H5N8 or a fragment or variant thereof.

In the context of the present invention a fragment of a protein or a variant thereof encoded by the at least one coding region of the mRNA sequence according to the invention may typically comprise an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with an amino acid sequence of the respective naturally occurring full-length protein or a variant thereof, preferably according to SEQ ID NOs: 1-30504.

In specific embodiments the antigenic peptide or protein is derived from a hemagglutinin (HA) protein of an influenza A virus according to SEQ ID NOs: 1-14031.

In this context it is further preferred that the at least one coding sequence of the mRNA sequence of the present invention encodes at least one antigenic peptide or protein which is derived from a hemagglutinin (HA) protein of an influenza A virus, or a fragment or variant thereof, wherein the hemagglutinin (HA) protein of an influenza A virus is selected from the hemagglutinin (HA) proteins listed in the sequence listing (see SEQ ID NOs: 1-32012 or SEQ ID NO: 224269 or SEQ ID NO: 224309 and explanation under the section "Preferred sequences of the present invention"). Therein, each hemagglutinin (HA) is identified by the database accession number of the corresponding protein (see sequence listing numeric identifier <223> which indicates the Protein or Nucleic Acid Accession No. (GenBank)). If the respective Protein or Nucleic Acid Accession No. (GenBank) is searched further on in the sequence listing, the next SEQ ID NO: which show said Protein or Nucleic Acid Accession No. (GenBank) under numeric identifier <223> corresponding to the nucleic acid sequence of the wild type mRNA encoding said protein. If again the respective Protein or Nucleic Acid Accession No. (GenBank) is searched further on in the sequence listing, the next five SEQ ID NOs which show the respective Protein or Nucleic Acid Accession No. under numeric idenfifier <223> correspond to five modified/optimized nucleic acid sequences of the mRNAs as described herein that encode the protein preferably having the respective amino acid sequence as mentioned before (first entry having the respective Protein or Nucleic Acid Accession No. (GenBank)).

Particularly preferred in this context are the following HA protein sequences:
HA protein of influenza A/Vietnam/1203/2004 (H5N1) (SEQ ID NOs: 13861-13871)
- HA protein of influenza A/Vietnam/1194/2004 (H5N1); (SEQ ID NOs: 13859-13860)
- HA protein of influenza A/Hong Kong/4801/2014 (H3N2) (SEQ ID NOs: 13853-13856)
- HA protein of influenza A/Netherlands/602/2009 (H1N1) (SEQ ID NOs: 13848-13850)
- HA protein of influenza A/California/07/2009 (H1N1) (SEQ ID NOs: 13836-13844)
- HA protein of influenza A/Michigan/45/2015 (H1N1) (SEQ ID NOs: 13845-13847)

In specific embodiments the antigenic peptide or protein is derived from a hemagglutinin (HA) protein of an influenza B virus according to SEQ ID NOs: 26398-28576.

In this context it is further preferred that the at least one coding sequence of the mRNA sequence of the present invention encodes at least one antigenic peptide or protein which is derived from a hemagglutinin (HA) protein of an influenza B virus, or a fragment or variant thereof, wherein the hemagglutinin (HA) protein of an influenza B virus is selected from the hemagglutinin (HA) proteins listed in the sequence listing (see SEQ ID NOs: 1-32012 or SEQ ID NO: 224269 or SEQ ID NO: 224309 and explanation under the section "Preferred sequences of the present invention"). Therein, each hemagglutinin (HA) is identified by the database accession number of the corresponding protein (see sequence listing numeric identifier <223> which indicates the Protein or Nucleic Acid Accession No. (GenBank)). If the respective Protein or Nucleic Acid Accession No. (GenBank) is searched further on in the sequence listing, the next SEQ ID NO: which show said Protein or Nucleic Acid Accession No. (GenBank) under numeric identifier <223> corresponding to the nucleic acid sequence of the wild type mRNA encoding said protein. If again the respective Protein or Nucleic Acid Accession No. (GenBank) is searched further on in the sequence listing, the next five SEQ ID NOs which show the respective Protein or Nucleic Acid Accession No. under numeric idenfifier <223> correspond to five modified/optimized nucleic acid sequences of the mRNAs as described herein that encode the protein preferably having the respective amino acid sequence as mentioned before (first entry having the respective Protein or Nucleic Acid Accession No. (GenBank)).Particularly preferred in this context are the following HA protein sequences:
- HA protein of influenza B/Phuket/3037/2013 (EPI540671; SEQ ID NOs: 28530-28532)
- HA protein of influenza B/Brisbane/60/2008 (SEQ ID NOs: 28524-28529)

In further specific embodiments the antigenic peptide or protein is derived from a neuraminidase (NA) protein of an influenza A virus according to SEQ ID NOs: 14032-26397, 224309, or 224310.

In this context it is further preferred that the at least one coding sequence of the mRNA sequence of the present invention encodes at least one antigenic peptide or protein which is derived from a neuraminidase (NA) protein of an influenza A virus, or a fragment or variant thereof, wherein the neuraminidase (NA) protein of an influenza A virus is selected from the neuraminidase (NA) proteins listed in the sequence listing (see SEQ ID NOs: 1-32012 or SEQ ID NO: 224269 or SEQ ID NO: 224309 and explanation under the section "Preferred sequences of the present invention"). Therein, each neuraminidase (NA) is identified by the database accession number of the corresponding protein (see sequence listing numeric identifier <223> which indicates the Protein or Nucleic Acid Accession No. (GenBank)). If the respective Protein or Nucleic Acid Accession No. (GenBank) is searched further on in the sequence listing, the next SEQ ID NO: which show said Protein or Nucleic Acid Accession No. (GenBank) under numeric identifier <223> corresponding to the nucleic acid sequence of the wild type mRNA encoding said protein. If again the respective Protein or Nucleic Acid Accession No. (GenBank) is searched further on in the sequence listing, the next five SEQ ID NOs which show the respective Protein or Nucleic Acid Accession No. under numeric idenfifier <223> correspond to five modified/optimized nucleic acid sequences of the mRNAs as described herein that encode the protein preferably having the respective amino acid sequence as mentioned before (first entry having the respective Protein or Nucleic Acid Accession No. (GenBank)).

Particularly preferred in this context are the following NA protein sequences:
- NA protein of influenza A/Hong Kong/4801/2014 (H3N2): SEQ ID NOs: 26251-26254
- NA protein of influenza A/California/7/2009 (H1N1)pdm09: SEQ ID NOs: 26238-26243
- NA protein of influenza A/Vietnam/1194/2004 (H5N1): SEQ ID NOs: 224310
- NA protein of influenza A/Vietnam/1203/2004) (H5N1): SEQ ID NOs: 26255-26257
- NA protein of influenza A/Netherlands/602/2009 (H1N1): SEQ ID NOs: 26246-26250
- NA protein of influenza A/Michigan/45/2015 (H1N1) (SEQ ID NOs: 26244-26245)

In further specific embodiments the antigenic peptide or protein is derived from a neuraminidase (NA) protein of an influenza B virus according to SEQ ID NOs: 28577-30504.

In this context it is further preferred that the at least one coding sequence of the mRNA sequence of the present invention encodes at least one antigenic peptide or protein which is derived from a neuraminidase (NA) protein of an influenza B virus, or a fragment or variant thereof, wherein the neuraminidase (NA) protein of an influenza B virus is selected from the neuraminidase (NA) proteins listed in the sequence listing (see SEQ ID NOs: 1-32012 or SEQ ID NO: 224269 or SEQ ID NO: 224309 and explanation under the section "Preferred sequences of the present invention"). Therein, each neuraminidase (NA) is identified by the database accession number of the corresponding protein (see sequence listing numeric identifier <223> which indicates the Protein or Nucleic Acid Accession No. (GenBank)). If the respective Protein or Nucleic Acid Accession No. (GenBank) is searched further on in the sequence listing, the next SEQ ID NO: which show said Protein or Nucleic Acid Accession No. (GenBank) under numeric identifier <223> corresponding to the nucleic acid sequence of the wild type mRNA encoding said protein. If again the respective Protein or Nucleic Acid Accession No. (GenBank) is searched further on in the sequence listing, the next five SEQ ID NOs which show the respective Protein or Nucleic Acid Accession No. under numeric idenfifier <223> correspond to five modified/optimized nucleic acid sequences of the mRNAs as described herein that encode the protein preferably having the respective amino acid sequence as mentioned before (first entry having the respective Protein or Nucleic Acid Accession No. (GenBank)).

Particularly preferred in this context are the following NA protein sequences:
- NA protein of influenza B/Brisbane/60/2008: SEQ ID NOs: 30455-30460
- NA protein of influenza B/Phuket/3037/2013: SEQ ID NOs: 30461-30462

Furthermore, in this context the coding region encoding at least one antigenic peptide or protein derived from hemagglutinin (HA) and/or neuraminidase (NA) of an influenza virus or a fragment, variant or derivative thereof, may be selected from any nucleic acid sequence comprising a coding region encoding hemagglutinin (HA) or neuraminidase (NA) derived from any influenza virus isolate or a fragment or variant thereof.

In a preferred embodiment, the present invention thus provides an mRNA sequence comprising at least one coding region, wherein the coding region encoding hemagglutinin (HA) of an influenza A virus comprises or consists any one of the nucleic acid sequences as disclosed in the sequence listing, (i.e. SEQ ID NOs: 32013-46043; 64025-78055, 224085-224106, 96037-110067, 128049-142079, 160061-174091, 192073-206103; see above explanation and explanation under the section "Preferred sequences of the present invention") or a fragment or variant of any one of these sequences.

In these context it is particularly preferred that the mRNA sequence according to the invention comprises at least one coding region encoding hemagglutinin (HA) of an influenza A virus comprising an RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences as disclosed in the sequence listing, see above explanation and explanation under the section "Preferred sequences of the present invention", (SEQ ID NOs: 32013-46043; 64025-78055, 224085-224106, 96037-110067, 128049-142079, 160061-174091, 192073-206103) or a fragment or variant thereof.

In particularly preferred embodiments the mRNA sequence comprises at least one coding region encoding hemagglutinin (HA) of an influenza A virus comprising an RNA sequence selected from the following RNA sequences:
- mRNA encoding HA protein of influenza A/Vietnam/1203/2004 (H5N1), preferably mRNA sequences according to SEQ ID NOs: 45873-45883, 77885-77895, 109897-109907, 141909-141919, 173921-173931, 205933-205943.
- mRNA encoding HA protein of influenza A/Vietnam/1194/2004 (H5N1), preferably mRNA sequences according to SEQ ID NOs: 45871, 45872, 77883, 77884, 109895, 109896, 141907, 141908, 173919, 173920, 205931, 205932.
- mRNA encoding HA protein of influenza A/Hong Kong/4801/2014 (H3N2) preferably mRNA sequences according to SEQ ID NOs: 45865-45868, 77877-77877, 109889-109889, 141901-141901, 173913-173913, 205925-205925.
- mRNA encoding HA protein of influenza A/Netherlands/602/2009 (H1N1) preferably mRNA sequences according to SEQ ID NOs: 45860-45862, 77872-77874, 109884-109886, 173908-173910, 205920-205922.
- mRNA encoding HA protein of influenza A/California/07/2009 (H1N1) preferably mRNA sequences according to SEQ ID NOs: 45848-45856, 77860-77868, 109872-109880, 141884-141892, 173896-173904, 205908-205916
- mRNA encoding HA protein of influenza A/Michigan/45/2015 (H1N1) preferably mRNA sequences according to SEQ ID NOs: 45857-45859, 77869-77871, 109881-109883, 141893-141895, 173905-173907, 205917-205919.

In a preferred embodiment, the present invention thus provides an mRNA sequence comprising at least one coding region, wherein the coding region encoding hemagglutinin (HA) of an influenza B virus comprises or consists any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Table 2 or Figures 21 of PCT/EP2016/075843, SEQ ID NOs: 58410-60588, 90422-92600, 224107-224112, 122434-124612, 154446-156624, 186458-188636, 218470-220648 or of a fragment or variant of any one of these sequences.

In these context it is particularly preferred that the mRNA sequence according to the invention comprises at least one coding region encoding hemagglutinin (HA) of an influenza B virus comprising an RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Table 2 or Figures 21 of PCT/EP2016/075843, SEQ ID NOs: 58410-60588, 90422-92600, 224107-224112, 122434-124612, 154446-156624, 186458-188636, 218470-220648 or of a fragment or variant of any one of these sequences

In particularly preferred embodiments the mRNA sequence comprises at least one coding region encoding hemagglutinin (HA) of an influenza B virus comprising an RNA sequence selected from the following RNA sequences:
- mRNA encoding HA protein of influenza B/Phuket/3037/2013 preferably mRNA sequences according to SEQ ID NOs: 60542-60544, 92554-92556, 124566-124568, 156578-156580, 188590-188592, 220602-220604.
- mRNA encoding HA protein of influenza B/Brisbane/60/2008 (GI: 223950973; FJ766840.1) preferably mRNA sequences according to SEQ ID NOs: 60536-60541, 92548-92553, 124560-124565, 156572-156577, 188584-188589, 220596-220601.

In a preferred embodiment, the present invention thus provides an mRNA sequence comprising at least one coding region, wherein the coding region encoding neuraminidase (NA) of an influenza A virus comprises or consists any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Table 3 or Figures 22 of PCT/EP2016/075843, SEQ ID NOs: 46044-58409, 224311, 224312, 78056-90421, 224113, 224313-224317, 110068-122433, 142080-154445, 174092-186457, 206104-218469 or of a fragment or variant of any one of these sequences.

In these context it is particularly preferred that the mRNA sequence according to the invention comprises at least one coding region encoding neuraminidase (NA) of an influenza A virus comprising an RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Table 3 or Figures 22 of PCT/EP2016/075843, SEQ ID NOs: 46044-58409, 224311, 224312, 78056-90421, 224113, 224313-224317, 110068-122433, 142080-154445, 174092-186457, 206104-218469 or of a fragment or variant of any one of these sequences.

In particularly preferred embodiments the mRNA sequence comprises at least one coding region encoding neuraminidase (NA) of an influenza A virus comprising an RNA sequence selected from the following RNA sequences:
- mRNA encoding NA protein of influenza A/Hong Kong/4801/2014 (H3N2): SEQ ID NOs: 58263-58266, 90275-90278, 122287-122290, 154299-154302, 186311-186314, 218323-218326.
- mRNA encoding NA protein of influenza A/California/7/2009 (H1N1)pdm09: SEQ ID NOs: 58250-58255, 90262-90267, 122274-122279, 154286-154291, 186298-186303, 218310-218315.
- mRNA encoding NA protein of influenza A/Vietnam/1194/2004 (H5N1): SEQ ID NO: 224312.
- mRNA encoding NA protein of influenza A/Vietnam/1203/2004) (H5N1): SEQ ID NOs: 58267-58269, 90279-90281, 122291-122293, 154303-154305, 186315-186317, 218327-218329.
- mRNA encoding NA protein of influenza A/Michigan/45/2015 (H1N1) preferably mRNA sequences according to SEQ ID NOs: 58256-58257, 90268-90269, 122280-122281, 154292-154293, 186304-186305, 218316-218317.
- mRNA encoding NA protein of influenza A/Netherlands/602/2009 (H1N1) preferably mRNA sequences according to SEQ ID NOs: 58258-58262, 90270-90274, 122282-122286, 154294-154298, 186306-186310, 218318-218322.

In a preferred embodiment, the present invention thus provides an mRNA sequence comprising at least one coding region, wherein the coding region encoding neuraminidase (NA) of an influenza B virus comprises or consists any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Table 4 or Figures 23 of PCT/EP2016/075843, SEQ ID NOs: 60589-62516, 92601-94528, 124613-126540, 156625-158552, 188637-190564, 220649-222576 or of a fragment or variant of any one of these sequences.

In these context it is particularly preferred that the mRNA sequence according to the invention comprises at least one coding region encoding neuraminidase (NA) of an influenza B virus comprising an RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Table 4 or Figures 23 of PCT/EP2016/075843, SEQ ID NOs: 60589-62516, 92601-94528, 124613-126540, 156625-158552, 188637-190564, 220649-222576 or of a fragment or variant of any one of these sequences.

In particularly preferred embodiments the mRNA sequence comprises at least one coding region encoding neuraminidase (NA) of an influenza B virus comprising an RNA sequence selected from the following RNA sequences:
- mRNA encoding NA protein of influenza B/Brisbane/60/2008 (GI: 223950973; FJ766840.1): SEQ ID NOs: 62467-62472, 94479-94484, 126491-126496, 158503-158508, 190515-190520, 222527-222532.
- mRNA encoding NA protein of influenza B/Phuket/3073/2013): SEQ ID NOs: 62473-62474, 94485-94486, 126497-126498, 158509-158510, 190521-190522, 222533-222534.

### Rabies:

In a particularly preferred embodiment of the first aspect of the invention the mRNA compound comprising an mRNA sequence comprises a coding region, encoding at least one antigenic peptide or protein derived from glycoprotein G of a Rabies virus or a fragment or variant thereof.

In this context, the amino acid sequence of the at least one antigenic peptide or protein may be selected from any peptide or protein derived from a glycoprotein of a Rabies virus or a fragment or variant or from any synthetically engineered Rabies virus peptide or protein.

In a preferred embodiment of the present invention the coding region encodes at least one antigenic peptide or protein derived from a glycoprotein of a Rabies virus or a fragment or variant thereof.

In this context it is particularly preferred that the at least one coding region encodes at least one full-length protein of a glycoprotein of a Rabies virus or a variant thereof.

As used herein, the term "full-length protein" preferably relates to the full-length sequence of protein indicated in the sequence listing of the present invention. More preferably, the term "full-length protein" preferably refers to an amino acid sequence as defined by any one of the SEQ ID NOs listed in the sequence listing (SEQ ID NOs: 30505-32012) or to an amino acid provided in the database under the respective database accession number.

In this context it is further preferred that the at least one coding sequence of the mRNA sequence of the present invention encodes at least one antigenic peptide or protein which is derived from a glycoprotein of a Rabies virus, or a fragment or variant thereof, wherein the glycoprotein of a Rabies virus is selected from the glycoprotein of a Rabies virus proteins listed in the sequence listing (see SEQ ID NOs: 1-32012 or SEQ ID NO: 224269 or SEQ ID NO: 224309 and explanation under the section "Preferred sequences of the present invention"). Therein, each glycoprotein of a Rabies virus is identified by the database accession number of the corresponding protein (see sequence listing numeric identifier <223> which indicates the Protein or Nucleic Acid Accession No. (GenBank)). If the respective Protein or Nucleic Acid Accession No. (GenBank) is searched further on in the sequence listing, the next SEQ ID NO: which show said Protein or Nucleic Acid Accession No. (GenBank) under numeric identifier <223> corresponding to the nucleic acid sequence of the wild type mRNA encoding said protein. If again the respective Protein or Nucleic Acid Accession No. (GenBank) is searched further on in the sequence listing, the next five SEQ ID NOs which show the respective Protein or Nucleic Acid Accession No. under numeric idenfifier <223> correspond to five modified/optimized nucleic acid sequences of the mRNAs as described herein that encode the protein preferably having the respective amino acid sequence as mentioned before (first entry having the respective Protein or Nucleic Acid Accession No. (GenBank)).

Particularly preferred in this context are the following glycoprotein sequences: SEQ ID NOs: 31986, 31073, 31102.

Furthermore, in this context the coding region encoding at least one antigenic peptide or protein derived from glycoprotein of a Rabies virus or a fragment, variant or derivative thereof, may be selected from any nucleic acid sequence comprising a coding region encoding glycoprotein derived from any Rabies virus isolate or a fragment or variant thereof.

In a preferred embodiment, the present invention thus provides an mRNA sequence comprising at least one coding region, wherein the coding region encoding glycoprotein of a Rabies virus comprises or consists any one of the nucleic acid sequences disclosed in the sequence listing (see explanation above; preferably SEQ ID NOs: 62517-64024; 224270, 224274, 94529-96036, 224271-224273, 126541-128048, 158553-160060, 190565-192072, 222577-224084) or a fragment or variant of any one of these sequences.

In these context it is particularly preferred that the mRNA sequence according to the invention comprises at least one coding region encoding a glycoprotein derived from any Rabies virus comprising an RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences as disclosed in the sequence listing (see explanation above; preferably SEQ ID NOs: 62517-64024; 224270, 224274, 94529-96036, 224271-224273, 126541-128048, 158553-160060, 190565-192072, 222577-224084) or a fragment or variant thereof.

In particularly preferred embodiments the mRNA sequence comprises at least one coding region encoding glycoprotein of a Rabies virus (RABV-G) comprising an RNA sequence selected from the following RNA sequences:
mRNA encoding glycoprotein of Rabies virus (Pasteur strain), preferably mRNA sequences according to SEQ ID NOs: 63998, 96010, 128022, 160034, 192046, 224058.

### Ebola:

Ebola virus: Ebolaviruses and the genetically-related Marburgviruses are human pathogens that cause severe diseases. Ebolaviruses and Marburgviruses are filoviruses, which are enveloped viruses featuring a negative-stranded RNA genome. The family of Filoviridae comprises three genera: Ebolavirus, Marburgvirus and Cuevavirus. The genus of Cuevaviruses as well as Marburgviruses include only one species, i.e. Lloviu cuevavirus (Lloviu virus - LLOV) and Marburg marburgvirus, respectively, which is subdivided in Marburg virus (MARV) and Ravn virus (RAVV). The genus of Ebolaviruses comprises five known species, i.e. Bundibugyo ebolavirus (Bundibugyo virus - BDBV), Reston ebolavirus (Reston virus - RESTV), Sudan ebolavirus (Sudan virus - SUDV), Taï Forest ebolavirus (Taï Forest virus - TAFV) (= Côte d'Ivoire ebolavirus), and Zaire ebolavirus (Ebola virus - EBOV). While Cuevaviruses have been isolated from bats and their potential as a pathogen in humans remains unknown, both Ebolaviruses and Marburgviruses are human pathogens that cause Ebolavirus disease (EVD) and Marburgvirus disease, respectively, characterised by haemorrhagic fever and an extremely high mortality rate. In the context of the present invention, any virus, virus member, virus strain, virus type, virus sub-type, virus isolate, virus variant, or virus serotype or genetic reassortant of a virus belonging to or being related to or being derived from viruses of the families and genera listed above are considered to be a "Ebola virus".

In a particularly preferred embodiment of the first aspect of the invention the mRNA compound comprising an mRNA sequence comprises a coding region, encoding at least one antigenic peptide or protein derived from the glycoprotein (GP) and/or the matrix protein 40 (VP40) and/or the nucleoprotein (NP) of a virus of the genus Ebolavirus or Marburgvirus or a fragment, variant or derivative thereof.

In this context, the amino acid sequence of the at least one antigenic peptide or protein may be selected from any peptide or protein derived from glycoprotein (GP) and/or the matrix protein 40 (VP40) and/or the nucleoprotein (NP) a glycoprotein of an Ebola virus or a fragment or variant or from any synthetically engineered Ebola virus peptide or protein.

In a preferred embodiment of the present invention the coding region encodes at least one antigenic peptide or protein derived from a glycoprotein of an Ebola virus or a fragment or variant thereof. In this context it is particularly preferred that the at least one coding region encodes at least one full-length protein of a glycoprotein of an Ebola virus or a variant thereof.

Particularly preferred in this context are the following Ebola glycoprotein amino acid sequences: SEQ ID NOs: 1 to 6 of the patent application WO2016097065, or fragments or variants of these sequences. In this context, SEQ ID NOs: 1 to 6 of WO2016097065 and the disclosure relating to SEQ ID NOs: 1 to 6 of WO2016097065 are incorporated herein by reference.

Particularly preferred in this context are the following Ebola VP40 amino acid sequences: SEQ ID NOs: 7 to 12 of the patent application WO2016097065, or fragments or variants of these sequences. In this context, SEQ ID NOs: 7 to 12 of WO2016097065 and the disclosure relating to SEQ ID NOs: 7 to 12 of WO2016097065 are incorporated herein by reference.

Particularly preferred in this context are the following Ebola NP amino acid sequences: SEQ ID NOs: 13 to 18 of the patent application WO2016097065, or fragments or variants of these sequences. In this context, SEQ ID NOs: 13 to 18 of WO2016097065 and the disclosure relating to SEQ ID NOs: 13 to 18 of WO2016097065 are incorporated herein by reference.

In a preferred embodiment, the present invention provides an mRNA sequence comprising at least one coding region, wherein the coding region encoding an antigenic peptide or protein as specified herein of a Ebola virus comprises or consists any one of the nucleic acid sequences according to SEQ ID NOs: 20 to 27 of the patent application WO2016097065, or fragments or variants of these sequences. In this context, SEQ ID NOs: 20 to 27 of WO2016097065 and the disclosure relating to SEQ ID NOs: 20 to 27 of WO2016097065 are incorporated herein by reference.

In particularly preferred embodiments the mRNA sequence comprises at least one coding region encoding glycoprotein of a Ebola virus. In particularly preferred embodiments the mRNA sequence comprises at least one coding region encoding VP40 of a Ebola virus. In particularly preferred embodiments the mRNA sequence comprises at least one coding region encoding NP of a Ebola virus.

In particularly preferred embodiments the mRNA sequence comprises at least one coding region encoding an antigenic peptide or protein of an Ebola virus comprising an RNA sequence selected from the following RNA sequences:
- mRNA encoding GP protein of Ebola virus: SEQ ID NOs: 37-39 of the patent application WO2016097065, or fragments or variants of these sequences. In this context, SEQ ID NOs: 37-39 of WO2016097065 and the disclosure relating to SEQ ID NOs: 37-39 of WO2016097065 are incorporated herein by reference.
- mRNA encoding VP40 of Ebola virus: SEQ ID NOs: 40-42 of the patent application WO2016097065, or fragments or variants of these sequences. In this context, SEQ ID NOs: 40-42 of WO2016097065 and the disclosure relating to SEQ ID NOs: 40-42 of WO2016097065 are incorporated herein by reference.
- mRNA encoding NP of Ebola virus: SEQ ID NOs: 43-44 of the patent application WO2016097065, or fragments or variants of these sequences. In this context, SEQ ID NOs: 43-44 of WO2016097065 and the disclosure relating to SEQ ID NOs: 43-44 of WO2016097065 are incorporated herein by reference.

Particularily preferrerd is the mRNA sequence comprising a coding sequence encoding GP according to SEQ ID NO: 224362.

### Tumour antigens:

Preferably, the at least one coding sequence of the mRNA compound comprising an mRNA sequence according to the invention encodes a tumor antigen, preferably as defined herein, or a fragment or variant thereof, wherein the tumor antigen is preferably selected from the group consisting of 1A01_HLA-A/m; 1A02; 5T4; ACRBP; AFP; AKAP4; alpha-actinin-_4/m; alpha-methylacyl-coenzyme_A_racemase; ANDR; ART-4; ARTC1/m; AURKB; B2MG; B3GN5; B4GN1; B7H4; BAGE-1; BASI; BCL-2; bcr/abl; beta-catenin/m; BING-4; BIRC7; BRCA1/m; BY55; calreticulin; CAMEL; CASP-8/m; CASPA; cathepsin_B; cathepsin_L; CD1A; CD1B; CD1C; CD1D; CD1E; CD20; CD22; CD276; CD33; CD3E; CD3Z; CD44_Isoform_1; CD44_Isoform_6; CD4; CD52; CD55; CD56; CD80; CD86; CD8A; CDC27/m; CDE30; CDK4/m; CDKN2A/m; CEA; CEAM6; CH3L2; CLCA2; CML28; CML66; COA-1/m; coactosin-like_protein; collagen_XXIII; COX-2; CP1B1; CSAG2; CT45A1; CT55; CT-_9/BRD6; CTAG2_Isoform_LAGE-1A; CTAG2_Isoform_LAGE-1B; CTCFL; Cten; cyclin_B1; cyclin_D1; cyp-B; DAM-10; DEP1A; E7; EF1A2; EFTUD2/m; EGFR; EGLN3; ELF2/m; EMMPRIN; EpCam; EphA2; EphA3; ErbB3; ERBB4; ERG; ETV6; EWS; EZH2; FABP7; FCGR3A_Version_1; FCGR3A_Version_2; FGF5; FGFR2; fibronectin; FOS; FOXP3; FUT1; G250; GAGE-1; GAGE-2; GAGE-3; GAGE-4; GAGE-5; GAGE-6; GAGE7b; GAGE-8_(GAGE-2D); GASR; GnT-V; GPC3; GPNMB/m; GRM3; HAGE; hepsin; Her2/neu; HLA-A2/m; homeobox_NKX3.1; HOM-TES-85; HPG1; HS71A; HS71B; HST-2; hTERT; iCE; IF2B3; IL10; IL-13Ra2; IL2-RA; IL2-RB; IL2-RG; IL-5; IMP3; ITA5; ITB1; ITB6; kallikrein-2; kallikrein-4; KI20A; KIAA0205; KIF2C; KK-LC-1; LDLR; LGMN; LIRB2; LY6K; MAGA5; MAGA8; MAGAB; MAGE-A10; MAGE-A12; MAGE-A1; MAGE-A2; MAGE-A3; MAGE-A4; MAGE-A6; MAGE-A9; MAGE-B10; MAGE-B16; MAGE-B17; MAGE-_B1; MAGE-B2; MAGE-B3; MAGE-B4; MAGE-B5; MAGE-B6; MAGE-C1; MAGE-C2; MAGE-C3; MAGE-D1; MAGE-D2; MAGE-D4; MAGE-_E1; MAGE-E1_(MAGE1); MAGE-E2; MAGE-F1; MAGE-H1; MAGEL2; mammaglobin_A; MART-1/melan-A; MART-2; MC1_R; M-CSF; mesothelin; MITF; MMP1_1; MMP7; MUC-1; MUM-1/m; MUM-2/m; MYCN; MYO1A; MYO1B; MYO1C; MYO1D; MYO1E; MYO1F; MYO1G; MYO1H; NA17; NA88-A; Neo-PAP; NFYC/m; NGEP; NPM; NRCAM; NSE; NUF2; NY-ESO-1; OA1; OGT; OS-9; osteocalcin; osteopontin; p53; PAGE-4; PAI-1; PAI-2; PAP; PATE; PAX3; PAX5; PD1L1; PDCD1; PDEF; PECA1; PGCB; PGFRB; Pim-1_-Kinase; Pin-1; PLAC1; PMEL; PML; POTEF; POTE; PRAME; PRDX5/m; PRM2; prostein; proteinase-3; PSA; PSB9; PSCA; PSGR; PSM; PTPRC; RAB8A; RAGE-1; RARA; RASH; RASK; RASN; RGS5; RHAMM/CD168; RHOC; RSSA; RU1; RU2; RUNX1; S-100; SAGE; SART-_1; SART-2; SART-3; SEPR; SERPINB5; SIA7F; SIA8A; SIAT9; SIRT2/m; SOX10; SP17; SPNXA; SPXN3; SSX-1; SSX-2; SSX3; SSX-4; ST1A1; STAG2; STAMP-1; STEAP-1; Survivin-2B; survivin; SYCP1; SYT-SSX-1; SYT-SSX-2; TARP; TCRg; TF2AA; TGFB1; TGFR2; TGM-4; TIE2; TKTL1; TPI/m; TRGV11; TRGV9; TRPC1; TRP-p8; TSG10; TSPY1; TVC_(TRGV3); TX101; tyrosinase; TYRP1; TYRP2; UPA; VEGFR1; WT1; and XAGE1.

Further antigens useful for the present invention are shown herein below (gene names followed by bracket with protein accession NOs):
1A01_HLA-A/m (UniProtKB: P30443); 1A02 (UniProtKB: P01892); 5T4 (UniProtKB: Q13641); ACRBP (UniProtKB: Q8NEB7); AFP (UniProtKB: P02771); AKAP4 (UniProtKB: Q5JQC9); alpha-actinin-_4/m (UniProtKB: B4DSX0); alpha-actinin-_4/m (UniProtKB: B4E337); alpha-actinin-_4/m (UniProtKB: O43707); alpha-methylacyl-coenzyme_A_racemase (UniProtKB: A0A024RE16); alpha-methylacyl-coenzyme_A_racemase (UniProtKB: A8KAC3); ANDR (UniProtKB: P10275); ART-4 (UniProtKB: Q9ULX3); ARTC1/m (UniProtKB: P52961); AURKB (UniProtKB: Q96GD4); B2MG (UniProtKB: P61769); B3GN5 (UniProtKB: Q9BYG0); B4GN1 (UniProtKB: Q00973); B7H4 (UniProtKB: Q7Z7D3); BAGE-1 (UniProtKB: Q13072); BASI (UniProtKB: P35613); BCL-2 (UniProtKB: A9QXG9); bcr/abl (UniProtKB: A9UEZ4); bcr/abl (UniProtKB: A9UEZ7); bcr/abl (UniProtKB: A9UEZ8); bcr/abl (UniProtKB: A9UEZ9); bcr/abl (UniProtKB: A9UF00); bcr/abl (UniProtKB: A9UF01); bcr/abl (UniProtKB: A9UF03); bcr/abl (UniProtKB: A9UF04); bcr/abl (UniProtKB: A9UF05); bcr/abl (UniProtKB: A9UF06); bcr/abl (UniProtKB: A9UF08); beta-catenin/m (UniProtKB: P35222); beta-catenin/m (UniProtKB: Q8WYA6); BING-4 (UniProtKB: 015213); BIRC7 (UniProtKB: Q96CA5); BRCA1/m (UniProtKB: A0A024R1V0); BRCA1/m (UniProtKB: A0A024R1V7); BRCA1/m (UniProtKB: A0A024R1Z8); BRCA1/m (UniProtKB: A0A068BFX7); BRCA1/m (UniProtKB: C6YB45); BRCA1/m (UniProtKB: C6YB47); BRCA1/m (UniProtKB: G3XAC3); BY55 (UniProtKB: 095971); calreticulin (UniProtKB: B4DHR1); calreticulin (UniProtKB: B4E2Y9); calreticulin (UniProtKB: P27797); calreticulin (UniProtKB: Q96L12); CAMEL (UniProtKB: O95987); CASP-8/m (UniProtKB: Q14790); CASPA (UniProtKB: Q92851-4); cathepsin_B (UniProtKB: A0A024R374); cathepsin_B (UniProtKB: P07858); cathepsin_L (UniProtKB: A0A024R276); cathepsin_L (UniProtKB: P07711); cathepsin_L (UniProtKB: Q9HBQ7); CD1A (UniProtKB: P06126); CD1B (UniProtKB: P29016); CD1C (UniProtKB: P29017); CD1D (UniProtKB: P15813); CD1E (UniProtKB: P15812); CD20 (UniProtKB: P11836); CD22 (UniProtKB: O60926); CD22 (UniProtKB: P20273); CD22 (UniProtKB: Q0EAF5); CD276 (UniProtKB: Q5ZPR3); CD33 (UniProtKB: B4DF51); CD33 (UniProtKB: P20138); CD33 (UniProtKB: Q546G0); CD3E (UniProtKB: P07766); CD3Z (UniProtKB: P20963); CD44_Isoform_1 (UniProtKB: P16070); CD44_Isoform_6 (UniProtKB: P16070-6); CD4 (UniProtKB: P01730); CD52 (UniProtKB: P31358); CD52 (UniProtKB: Q6IBD0); CD52 (UniProtKB: V9HWN9); CD55 (UniProtKB: B1AP15); CD55 (UniProtKB: D3DT85); CD55 (UniProtKB: D3DT86); CD55 (UniProtKB: P08174); CD56 (UniProtKB: P13591); CD80 (UniProtKB: A0N0P2); CD80 (UniProtKB: P33681); CD86 (UniProtKB: P42081); CD8A (UniProtKB: P01732); CDC27/m (UniProtKB: G5EA36); CDC27/m (UniProtKB: P30260); CDE30 (UniProtKB: P28908); CDK4/m (UniProtKB: A0A024RBB6); CDK4/m (UniProtKB: P11802); CDK4/m (UniProtKB: Q6LC83); CDK4/m (UniProtKB: Q96BE9); CDKN2A/m (UniProtKB: D1LYX3); CDKN2A/m (UniProtKB: G3XAG3); CDKN2A/m (UniProtKB: K7PML8); CDKN2A/m (UniProtKB: L8E941); CDKN2A/m (UniProtKB: Q8N726); CEA (RefSeq: NP_004354); CEAM6 (UniProtKB: P40199); CH3L2 (UniProtKB: Q15782); CLCA2 (UniProtKB: Q9UQC9); CML28 (UniProtKB: Q9NQT4); CML66 (UniProtKB: Q96RS6); COA-1/m (UniProtKB: Q5T124); coactosin-like_protein (UniProtKB: Q14019); collagen_XXIII (UniProtKB: L8EAS4); collagen_XXIII (UniProtKB: Q86Y22); COX-2 (UniProtKB: Q6ZYK7); CP1B1 (UniProtKB: Q16678); CSAG2 (UniProtKB: Q9Y5P2-2); CSAG2 (UniProtKB: Q9Y5P2); CT45A1 (UniProtKB: Q5HYN5); CT55 (UniProtKB: Q8WUE5); CT-_9/BRD6 (UniProtKB: Q58F21); CTAG2_Isoform_LAGE-1A (UniProtKB: O75638-2); CTAG2_Isoform_LAGE-1B (UniProtKB: O75638); CTCFL (UniProtKB: Q8NI51); Cten (UniProtKB: Q8IZW8); cyclin_B1 (UniProtKB: P14635); cyclin_D1 (UniProtKB: P24385); cyp-B (UniProtKB: P23284); DAM-10 (UniProtKB: P43366); DEP1A (UniProtKB: Q5TB30); E7 (UniProtKB: P03129); E7 (UniProtKB: P06788); E7 (UniProtKB: P17387); E7 (UniProtKB: P06429); E7 (UniProtKB: P27230); E7 (UniProtKB: P24837); E7 (UniProtKB: P21736); E7 (UniProtKB: P26558); E7 (UniProtKB: P36831); E7 (UniProtKB: P36833); E7 (UniProtKB: Q9QCZ1); E7 (UniProtKB: Q81965); E7 (UniProtKB: Q80956); EF1A2 (UniProtKB: Q05639); EFTUD2/m (UniProtKB: Q15029); EGFR (UniProtKB: A0A0B4J1Y5); EGFR (UniProtKB: E7BSV0); EGFR (UniProtKB: L0R6G1); EGFR (UniProtKB: P00533-2); EGFR (UniProtKB: P00533); EGFR (UniProtKB: Q147T7); EGFR (UniProtKB: Q504U8); EGFR (UniProtKB: Q8NDU8); EGLN3 (UniProtKB: Q9H6Z9); ELF2/m (UniProtKB: B7Z720); EMMPRIN (UniProtKB: Q54A51); EpCam (UniProtKB: P16422); EphA2 (UniProtKB: P29317); EphA3 (UniProtKB: P29320); EphA3 (UniProtKB: Q6P4R6); ErbB3 (UniProtKB: B3KWG5); ErbB3 (UniProtKB: B4DGQ7); ERBB4 (UniProtKB: Q15303); ERG (UniProtKB: P11308); ETV6 (UniProtKB: P41212); EWS (UniProtKB: Q01844); EZH2 (UniProtKB: F2YMM1); EZH2 (UniProtKB: G3XAL2); EZH2 (UniProtKB: L0R855); EZH2 (UniProtKB: Q15910); EZH2 (UniProtKB: S4S3R8); FABP7 (UniProtKB: 015540); FCGR3A_Version_1 (UniProtKB: P08637); FCGR3A_Version_2 (CCDS: CCDS1232.1); FGF5 (UniProtKB: P12034); FGF5 (UniProtKB: Q60518); FGFR2 (UniProtKB: P21802); fibronectin (UniProtKB: A0A024R5I6); fibronectin (UniProtKB: A0A024RB01); fibronectin (UniProtKB: A0A024RDT9); fibronectin (UniProtKB: A0A024RDV5); fibronectin (UniProtKB: A6NH44); fibronectin (UniProtKB: A8K6A5); fibronectin (UniProtKB: B2R627); fibronectin (UniProtKB: B3KXM5); fibronectin (UniProtKB: B4DIC5); fibronectin (UniProtKB: B4DN21); fibronectin (UniProtKB: B4DS98); fibronectin (UniProtKB: B4DTH2); fibronectin (UniProtKB: B4DTK1); fibronectin (UniProtKB: B4DU16); fibronectin (UniProtKB: B7Z3W5); fibronectin (UniProtKB: B7Z939); fibronectin (UniProtKB: G5E9X3); fibronectin (UniProtKB: Q9H382); FOS (UniProtKB: P01100); FOXP3 (UniProtKB: Q9BZS1); FUT1 (UniProtKB: P19526); G250 (UniProtKB: Q16790); GAGE-1 (Genbank: AAA82744); GAGE-2 (UniProtKB: Q6NT46); GAGE-3 (UniProtKB: Q13067); GAGE-4 (UniProtKB: Q13068); GAGE-5 (UniProtKB: Q13069); GAGE-6 (UniProtKB: Q13070); GAGE7b (UniProtKB: O76087); GAGE-8_(GAGE-2D) (UniProtKB: Q9UEU5); GASR (UniProtKB: P32239); GnT-V (UniProtKB: Q09328); GPC3 (UniProtKB: I6QTG3); GPC3 (UniProtKB: P51654); GPC3 (UniProtKB: Q8IYG2); GPNMB/m (UniProtKB: A0A024RA55); GPNMB/m (UniProtKB: Q14956); GPNMB/m (UniProtKB: Q8IXJ5); GPNMB/m (UniProtKB: Q96F58); GRM3 (UniProtKB: Q14832); HAGE (UniProtKB: Q9NXZ2); hepsin (UniProtKB: B2ZDQ2); hepsin (UniProtKB: P05981); Her2/neu (UniProtKB: B4DTR1); Her2/neu (UniProtKB: L8E8G2); Her2/neu (UniProtKB: P04626); Her2/neu (UniProtKB: Q9UK79); HLA-A2/m (UniProtKB: Q95387); HLA-A2/m (UniProtKB: Q9MYF8); homeobox_NKX3.1 (UniProtKB: Q99801); HOM-TES-85 (UniProtKB: B2RBQ6); HOM-TES-85 (UniProtKB: Q9P127); HPG1 (Pubmed: 12543784); HS71A (UniProtKB: P0DMV8); HS71B (UniProtKB: P0DMV9); HST-2 (UniProtKB: P10767); hTERT (UniProtKB: O94807); iCE (UniProtKB: O00748); IF2B3 (UniProtKB: O00425); IL10 (UniProtKB: P22301); IL-13Ra2 (UniProtKB: Q14627); IL2-RA (UniProtKB: P01589); IL2-RB (UniProtKB: P14784); IL2-RG (UniProtKB: P31785); IL-5 (UniProtKB: P05113); IMP3 (UniProtKB: Q9NV31); ITA5 (UniProtKB: P08648); ITB1 (UniProtKB: P05556); ITB6 (UniProtKB: P18564); kallikrein-2 (UniProtKB: A0A024R4J4); kallikrein-2 (UniProtKB: A0A024R4N3); kallikrein-2 (UniProtKB: B0AZU9); kallikrein-2 (UniProtKB: B4DU77); kallikrein-2 (UniProtKB: P20151); kallikrein-2 (UniProtKB: Q6T774); kallikrein-2 (UniProtKB: Q6T775); kallikrein-4 (UniProtKB: A0A0C4DFQ5); kallikrein-4 (UniProtKB: Q5BQA0); kallikrein-4 (UniProtKB: Q96PT0); kallikrein-4 (UniProtKB: Q96PT1); kallikrein-4 (UniProtKB: Q9Y5K2); KI20A (UniProtKB: O95235); KIAA0205 (UniProtKB: Q92604); KIF2C (UniProtKB: Q99661); KK-LC-1 (UniProtKB: Q5H943); LDLR (UniProtKB: P01130); LGMN (UniProtKB: Q99538); LIRB2 (UniProtKB: Q8N423); LY6K (UniProtKB: Q17RY6); MAGA5 (UniProtKB: P43359); MAGA8 (UniProtKB: P43361); MAGAB (UniProtKB: P43364); MAGE-A10 (UniProtKB: AOA024RC14); MAGE-A12 (UniProtKB: P43365); MAGE-A1 (UniProtKB: P43355); MAGE-A2 (UniProtKB: P43356); MAGE-A3 (UniProtKB: P43357); MAGE-A4 (UniProtKB: A0A024RC12); MAGE-A4 (UniProtKB: P43358); MAGE-A4 (UniProtKB: Q1RN33); MAGE-A6 (UniProtKB: A8K072); MAGE-A6 (UniProtKB: P43360); MAGE-A6 (UniProtKB: Q6FHI5); MAGE-A9 (UniProtKB: P43362); MAGE-B10 (UniProtKB: Q96LZ2); MAGE-B16 (UniProtKB: A2A368); MAGE-B17 (UniProtKB: A8MXT2); MAGE-_B1 (UniProtKB: Q96TG1); MAGE-B2 (UniProtKB: 015479); MAGE-B3 (UniProtKB: 015480); MAGE-B4 (UniProtKB: 015481); MAGE-B5 (UniProtKB: Q9BZ81); MAGE-B6 (UniProtKB: Q8N7X4); MAGE-C1 (UniProtKB: O60732); MAGE-C2 (UniProtKB: Q9UBF1); MAGE-C3 (UniProtKB: Q8TD91); MAGE-D1 (UniProtKB: Q9Y5V3); MAGE-D2 (UniProtKB: Q9UNF1); MAGE-D4 (UniProtKB: Q96JG8); MAGE-_E1 (UniProtKB: Q6IAI7); MAGE-E1_(MAGE1) (UniProtKB: Q9HCI5); MAGE-E2 (UniProtKB: Q8TD90); MAGE-F1 (UniProtKB: Q9HAY2); MAGE-H1 (UniProtKB: Q9H213); MAGEL2 (UniProtKB: Q9UJ55); mammaglobin_A (UniProtKB: Q13296); mammaglobin_A (UniProtKB: Q6NX70); MART-1/melan-A (UniProtKB: Q16655); MART-2 (UniProtKB: Q5VTY9); MC1_R (UniProtKB: Q01726); MC1_R (UniProtKB: Q1JUL4); MC1_R (UniProtKB: Q1JUL6); MC1_R (UniProtKB: Q1JUL8); MC1_R (UniProtKB: Q1JUL9); MC1_R (UniProtKB: Q1JUM0); MC1_R (UniProtKB: Q1JUM2); MC1_R (UniProtKB: Q1JUM3); MC1_R (UniProtKB: Q1JUM4); MC1_R (UniProtKB: Q1JUM5); MC1_R (UniProtKB: Q6UR92); MC1_R (UniProtKB: Q6UR94); MC1_R (UniProtKB: Q6UR95); MC1_R (UniProtKB: Q6UR96); MC1_R (UniProtKB: Q6UR97); MC1_R (UniProtKB: Q6UR98); MC1_R (UniProtKB: Q6UR99); MC1_R (UniProtKB: Q6URA0); MC1_R (UniProtKB: Q86YW1); MC1_R (UniProtKB: V9Q5S2); MC1_R (UniProtKB: V9Q671); MC1_R (UniProtKB: V9Q783); MC1_R (UniProtKB: V9Q7F1); MC1_R (UniProtKB: V9Q8N1); MC1_R (UniProtKB: V9Q977); MC1_R (UniProtKB: V9Q9P5); MC1_R (UniProtKB: V9Q9R8); MC1_R (UniProtKB: V9QAE0); MC1_R (UniProtKB: V9QAR2); MC1_R (UniProtKB: V9QAW3); MC1_R (UniProtKB: V9QB02); MC1_R (UniProtKB: V9QB58); MC1_R (UniProtKB: V9QBY6); MC1_R (UniProtKB: V9QC17); MC1_R (UniProtKB: V9QC66); MC1_R (UniProtKB: V9QCQ4); MC1_R (UniProtKB: V9QDF4); MC1_R (UniProtKB: V9QDN7); MC1_R (UniProtKB: V9QDQ6); M-CSF (UniProtKB: P09603); mesothelin (UniProtKB: Q13421); MITF (UniProtKB: O75030-8); MITF (UniProtKB: O75030-9); MITF (UniProtKB: O75030); MMP1_1 (UniProtKB: B3KQS8); MMP7 (UniProtKB: P09237); MUC-1 (Genbank: AAA60019); MUM-1/m (RefSeq: NP_116242); MUM-2/m (UniProtKB: Q9Y5R8); MYCN (UniProtKB: P04198); MYO1A (UniProtKB: Q9UBC5); MYO1B (UniProtKB: O43795); MYO1C (UniProtKB: 000159); MYO1D (UniProtKB: O94832); MYO1E (UniProtKB: Q12965); MYO1F (UniProtKB: 000160); MYO1G (UniProtKB: B0I1T2); MYO1H (RefSeq: NP_001094891); NA17 (UniProtKB: Q3V5L5); NA88-A (Pubmed: 10790436); Neo-PAP (UniProtKB: Q9BWT3); NFYC/m (UniProtKB: Q13952); NGEP (UniProtKB: Q6IWH7); NPM (UniProtKB: P06748); NRCAM (UniProtKB: Q92823); NSE (UniProtKB: P09104); NUF2 (UniProtKB: Q9BZD4); NY-ESO-1 (UniProtKB: P78358); OA1 (UniProtKB: P51810); OGT (UniProtKB: 015294); OS-9 (UniProtKB: B4DH11); OS-9 (UniProtKB: B4E321); OS-9 (UniProtKB: B7Z8E7); OS-9 (UniProtKB: Q13438); osteocalcin (UniProtKB: P02818); osteopontin (UniProtKB: A0A024RDE2); osteopontin (UniProtKB: A0A024RDE6); osteopontin (UniProtKB: A0A024RDJ0); osteopontin (UniProtKB: B7Z351); osteopontin (UniProtKB: F2YQ21); osteopontin (UniProtKB: P10451); p53 (UniProtKB: P04637); PAGE-4 (UniProtKB: O60829); PAI-1 (UniProtKB: P05121); PAI-2 (UniProtKB: P05120); PAP (UniProtKB: Q06141); PAP (UniProtKB: Q53S56); PATE (UniProtKB: Q8WXA2); PAX3 (UniProtKB: P23760); PAX5 (UniProtKB: Q02548); PD1L1 (UniProtKB: Q9NZQ7); PDCD1 (UniProtKB: Q15116); PDEF (UniProtKB: O95238); PECA1 (UniProtKB: P16284); PGCB (UniProtKB: Q96GW7); PGFRB (UniProtKB: P09619); Pim-1_-Kinase (UniProtKB: A0A024RD25); Pin-1 (UniProtKB: 015428); Pin-1 (UniProtKB: Q13526); Pin-1 (UniProtKB: Q49AR7); PLAC1 (UniProtKB: Q9HBJ0); PMEL (UniProtKB: P40967); PML (UniProtKB: P29590); POTEF (UniProtKB: A5A3E0); POTE (UniProtKB: Q86YR6); PRAME (UniProtKB: A0A024R1E6); PRAME (UniProtKB: P78395); PRDX5/m (UniProtKB: P30044); PRM2 (UniProtKB: P04554); prostein (UniProtKB: Q96JT2); proteinase-3 (UniProtKB: D6CHE9); proteinase-3 (UniProtKB: P24158); PSA (UniProtKB: P55786); PSB9 (UniProtKB: P28065); PSCA (UniProtKB: D3DWI6); PSCA (UniProtKB: O43653); PSGR (UniProtKB: Q9H255); PSM (UniProtKB: Q04609); PTPRC (RefSeq: NP_002829); RAB8A (UniProtKB: P61006); RAGE-1 (UniProtKB: Q9UQ07); RARA (UniProtKB: P10276); RASH (UniProtKB: P01112); RASK (UniProtKB: P01116); RASN (UniProtKB: P01111); RGS5 (UniProtKB: 015539); RHAMM/CD168 (UniProtKB: O75330); RHOC (UniProtKB: P08134); RSSA (UniProtKB: P08865); RU1 (UniProtKB: Q9UHJ3); RU2 (UniProtKB: Q9UHG0); RUNX1 (UniProtKB: Q01196); S-100 (UniProtKB: V9HW39); SAGE (UniProtKB: Q9NXZ1); SART-_1 (UniProtKB: O43290); SART-2 (UniProtKB: Q9UL01); SART-3 (UniProtKB: Q15020); SEPR (UniProtKB: Q12884); SERPINB5 (UniProtKB: P36952); SIA7F (UniProtKB: Q969X2); SIA8A (UniProtKB: Q92185); SIAT9 (UniProtKB: Q9UNP4); SIRT2/m (UniProtKB: A0A024R0G8); SIRT2/m (UniProtKB: Q8IXJ6); SOX10 (UniProtKB: P56693); SP17 (UniProtKB: Q15506); SPNXA (UniProtKB: Q9NS26); SPXN3 (UniProtKB: Q5MJ09); SSX-1 (UniProtKB: Q16384); SSX-2 (UniProtKB: Q16385); SSX3 (UniProtKB: Q99909); SSX-4 (UniProtKB: O60224); ST1A1 (UniProtKB: P50225); STAG2 (UniProtKB: Q8N3U4-2); STAMP-1 (UniProtKB: Q8NFT2); STEAP-1 (UniProtKB: A0A024RA63); STEAP-1 (UniProtKB: Q9UHE8); Survivin-2B (UniProtKB: 015392-2); survivin (UniProtKB: 015392); SYCP1 (UniProtKB: A0A024R0I2); SYCP1 (UniProtKB: B7ZLS9); SYCP1 (UniProtKB: Q15431); SYCP1 (UniProtKB: Q3MHC4); SYT-SSX-1 (UniProtKB: A4PIV7); SYT-SSX-1 (UniProtKB: A4PIV8); SYT-SSX-2 (UniProtKB: A4PIV9); SYT-SSX-2 (UniProtKB: A4PIW0); TARP (UniProtKB: Q0VGM3); TCRg (UniProtKB: A2JGV3); TF2AA (UniProtKB: P52655); TGFB1 (UniProtKB: P01137); TGFR2 (UniProtKB: P37173); TGM-4 (UniProtKB: B2R7D1); TIE2 (UniProtKB: Q02763); TKTL1 (UniProtKB: P51854); TPI/m (UniProtKB: P60174); TRGV11 (UniProtKB: Q99601); TRGV9 (UniProtKB: A4D1X2); TRGV9 (UniProtKB: Q99603); TRGV9 (UniProtKB: Q99604); TRPC1 (UniProtKB: P48995); TRP-p8 (UniProtKB: Q7Z2W7); TSG10 (UniProtKB: Q9BZW7); TSPY1 (UniProtKB: Q01534); TVC_(TRGV3) (Genbank: M13231.1); TX101 (UniProtKB: Q9BY14-2); tyrosinase (UniProtKB: ADA024DBG7); tyrosinase (UniProtKB: L8B082); tyrosinase (UniProtKB: L8B086); tyrosinase (UniProtKB: L8B0B9); tyrosinase (UniProtKB: O75767); tyrosinase (UniProtKB: P14679); tyrosinase (UniProtKB: U3M8N0); tyrosinase (UniProtKB: U3M9D5); tyrosinase (UniProtKB: U3M9J2); TYRP1 (UniProtKB: P17643); TYRP2 (UniProtKB: P40126); UPA (UniProtKB: Q96NZ9); VEGFR1 (UniProtKB: B5A924); WT1 (UniProtKB: A0A0H5AUY0); WT1 (UniProtKB: P19544); WT1 (UniProtKB: Q06250); XAGE1 (UniProtKB: Q9HD64).

### Checkpoint inhibitors

Negative regulatory T cell surface molecules were discovered, which are upregulated in activated T cells in order to dampen their activity, thus reducing the effectiveness of said activated T cells in the killing of tumor cells. These inhibitory molecules were termed negative co-stimulatory molecules due to their homology to the T cell co-stimulatory molecule CD28. These proteins, also referred to as immune checkpoint proteins, function in multiple pathways including the attenuation of early activation signals, competition for positive co-stimulation and direct inhibition of antigen presenting cells (Bour-Jordan et al., 2011. Immunol Rev. 241(1):180-205).

In the context of the present invention, a checkpoint modulator is typically a molecule, such as a protein (e.g. an antibody), a dominant negative receptor, a decoy receptor, or a ligand or a fragment or variant thereof, which modulates the function of an immune checkpoint protein, e.g. it inhibits or reduces the activity of checkpoint inhibitors (or inhibitory checkpoint molecules) or it stimulates or enhances the activity of checkpoint stimulators (or stimulatory checkpoint molecules). Therefore, checkpoint modulators as defined herein, influence the activity of checkpoint molecules.

In this context, inhibitory checkpoint molecules are defined as checkpoint inhibitors and can be used synonymously. In addition, stimulatory checkpoint molecules are defined as checkpoint stimulators and can be used synonymously.

Preferably, the checkpoint modulator is selected from agonistic antibodies, antagonistic antibodies, ligands, dominant negative receptors, and decoy receptors or combinations thereof.

Methods for generating and using mRNA-encoded antibodies are known in the art (e.g. WO2008/083949 or PCT/EP2017/060226).

Preferred inhibitory checkpoint molecules that may be inhibited by a checkpoint modulator in the context of the invention are PD-1, PD-L1, CTLA-4, PD-L2, LAG3, TIM3/HAVCR2, 2B4, A2aR, B7H3, B7H4, BTLA, CD30, CD160, CD155, GAL9, HVEM, IDO1, IDO2, KIR, LAIR1 and VISTA.

Preferred stimulatory checkpoint molecules that may be stimulated by a checkpoint modulator in the context of the invention are CD2, CD27, CD28, CD40, CD137, CD226, CD276, GITR, ICOS, OX40 and CD70.

According to a preferred embodiment, the pharmaceutical composition or vaccine comprising RNAs of the invention is for use as described herein, wherein the use comprises - as an additional pharmaceutically active ingredient - a checkpoint modulator selected from the group consisting of the checkpoint modulator is selected from the group consisting of a PD-1 inhibitor, a PD-L1 inhibitor, a PD-L2 inhibitor, a CTLA-4 inhibitor, a LAG3 inhibitor, a TIM3 inhibitor, a TIGIT-inhibitor, an OX40 stimulator, a 4-1BB stimulator, a CD40L stimulator, a CD28 stimulator and a GITR stimulator.

According to a preferred embodiment, the checkpoint modulator as used herein targets a member of the PD-1 pathway. Members of the PD-1 pathway are typically proteins, which are associated with PD-1 signaling. On the one hand, this group comprises proteins, which induce PD-1 signaling upstream of PD-1 as e.g. the ligands of PD-1, PD-L1 and PD-L2, and the signal transduction receptor PD-1. On the other hand, this group comprises signal transduction proteins downstream of PD-1 receptor. Particularly preferred as members of the PD-1 pathway in the context of the present invention are PD-1, PD-L1 and PD-L2.

In the context of the present invention, a PD-1 pathway antagonist (or PD-1 inhibitor) is preferably defined herein as a compound capable to impair the PD-1 pathway signaling, preferably signaling mediated by the PD-1 receptor. Therefore, the PD-1 pathway antagonist may be any antagonist directed against any member of the PD-1 pathway capable of antagonizing PD-1 pathway signaling.

In a preferred embodiment, the checkpoint modulator used herein is a PD-1 inhibitor or a PD-L1 inhibitor, wherein the PD-1 inhibitor is preferably an antagonistic antibody directed against PD-1 and the PD-L1 inhibitor is preferably an antagonistic antibody directed against PD-L1.

In this context, the antagonist may be an antagonistic antibody as defined herein, targeting any member of the PD-1 pathway, preferably an antagonistic antibody directed against PD-1 receptor, PD-L1 or PD-L2. Such an antagonistic antibody may also be encoded by a nucleic acid. Also, the PD-1 pathway antagonist may be a fragment of the PD-1 receptor blocking the activity of PD1 ligands. B7-1 or fragments thereof may act as PD1-antagonizing ligands as well. Additionally, a PD-1 pathway antagonist may be a protein comprising (or a nucleic acid coding for) an amino acid sequence capable of binding to PD-1 but preventing PD-1 signaling, e.g. by inhibiting PD-1 and B7-H1 or B7-DL interaction (WO 2014/127917; WO2012062218).

Particularly preferred are the anti-PD1 antibodies Nivolumab (MDX-1106/BMS-936558/ONO-4538), (Brahmer et al., 2010. J Clin Oncol. 28(19):3167-75; PMID: 20516446); Pidilizumab (CT-011), (Berger et al., 2008. Clin Cancer Res. 14(10):3044-51; PMID: 18483370); Pembrolizumab (MK-3475, SCH 900475); AMP-224, and MEDI0680 (AMP-514).

Particularly preferred are also the anti-PD-L1 antibodies MDX-1105/BMS-936559 (Brahmer et al. 2012. N Engl J Med. 366(26):2455-65; PMID: 22658128); atezolizumab (MPDL3280A/RG7446); durvalumab (MEDI4736); and avelumab (MSB0010718).

According to another embodiment, the checkpoint modulator used herein is an OX40 stimulator. OX40 is a member of the TNFR-superfamily of receptors, and is expressed on the surface of antigen-activated mammalian CD4+ and CD8+ T lymphocytes. OX40 ligand (OX40L, also known as gp34, ACT-4-L, and CD252) is a protein that specifically interacts with the OX40 receptor. The term OX40L includes the entire OX40 ligand, soluble OX40 ligand, and fusion proteins comprising a functionally active portion of OX40 ligand covalently linked to a second moiety, e.g., a protein domain. Also included within the definition of OX40L are variants which vary in amino acid sequence from naturally occurring OX40L, but which retain the ability to specifically bind to the OX40 receptor. Further included within the definition of OX40L are variants thereof, which enhance the biological activity of OX40. An OX40 agonist is a molecule which induces or enhances the biological activity of OX40, e.g. signal transduction mediated by OX40. An OX40 agonist is preferably defined herein as a binding molecule capable of specific binding to OX40. Therefore, the OX40 agonist may be any agonist binding to OX40 and capable of stimulating OX40 signaling. In this context, the OX40 agonist may be an agonistic antibody binding to OX40.

OX40 agonists and anti-OX40 monoclonal antibodies are described in WO1995/021251, WO1995/012673 and WO1995/21915. Particularly preferred is the anti-OX40 antibody 9B12, a murine anti-OX40 monoclonal antibody directed against the extracellular domain of human OX40 (Weinberg et al., 2006. J. Immunother. 29(6):575-585).

In another embodiment, the checkpoint modulator as used herein is an antagonistic antibody is selected from the group consisting of anti-CTLA4, anti-PD1, anti-PD-L1, anti-Vista, anti-Tim-3, anti-TIGIT, anti-LAG-3, and anti-BTLA.

Preferably, an anti-CTLA4 antibody that may be used as a checkpoint modulator is directed against Cytotoxic T lymphocyte antigen-4 (CTLA-4). CTLA-4 is mainly expressed within the intracellular compartment of T cells. After a potent or long-lasting stimulus to a naive T cell via the T cell receptor (TCR), CTLA-4 is transported to the cell surface and concentrated at the immunological synapse. CTLA-4 then competes with CD28 for CD80/CD86 and down-modulates TCR signaling via effects on Akt signaling. Thus CTLA-4 functions physiologically as a signal dampener (Weber, J. 2010. Semin. Oncol. 37(5):430-9).

In preferred embodiments, the pharmaceutical composition or vaccine comprising RNAs of the invention is for use as described herein, wherein the use comprises - as an additional pharmaceutically active ingredient - a CTLA4 antagonist, which is preferably an antagonistic antibody directed against CTLA4 (anti-CTLA4 antibody). The term 'CTLA4 antagonist' as used herein comprises any compound, such as an antibody, that antagonizes the physiological function of CTLA4. In the context of the present invention, the term 'anti-CTLA4 antibody' may refer to an antagonistic antibody directed against CTLA4 (or a functional fragment or variant of said antibody) or to a nucleic acid, preferably an RNA, encoding said antagonistic antibody (or a functional fragment thereof). A functional fragment or variant of an anti-CTLA4 antibody preferably acts as a CTLA4 antagonist. More preferably, the term 'anti-CTLA4 antibody' refers to a monoclonal antibody directed against CTLA4 (or a functional fragment or variant of such an antibody) or to a nucleic acid encoding a monoclonal antibody directed against CTLA4 (or a functional fragment or variant of such an antibody). The term 'anti-CTLA4 antibody' as used herein may refer to the heavy or light antibody chain, respectively, or also refer to both antibody chains (heavy and light chain), or to a fragment or variant of any one of these chains. Preferably, the fragment or variant of an anti-CTLA4 antibody as used herein is a functional fragment or variant, preferably as described herein.

Particularly preferred are the anti-CTLA-4 antibodies ipilimumab (Yervoy^{®}), tremelimumab, and AGEN-1884. Further preferred anti-CTLA4 antibodies as used herein comprise BMS 734016; BMS-734016; BMS734016; MDX 010; MDX 101; MDX-010; MDX-101; MDX-CTLA-4; MDX-CTLA4; MDX010; Winglore; and Yervoy, or a functional fragment or variant of any one of these antibodies.

According to a further embodiment, the checkpoint modulator as used herein is at least one antibody described in Table 1 or a fragment or variant thereof

**Table 1: Antibodies directed against checkpoint molecules**

| **Name** | **Target** |
|---|---|
| Urelumab | 4-1BB/CD137 |
| PF-05082566 | 4-1BB/CD137 |
| 8H9 | B7-H3 |
| Enoblituzumab | B7-H3 |
| Ipilimumab | CD152/CTLA-4 |
| Ticilimumab (= tremelimumab) | CD152/CTLA-4 |
| Tremelimumab | CD152/CTLA-4 |
| Varlilumab | CD27 |
| Teneliximab | CD40 |
| Vorsetuzumab mafodotin | CD70 |
| Lirilumab | KIR2D |
| GSK-3174998 | OX40 |
| MEDI-6469 | OX40 |
| MEDI-6383 | OX40 |
| MEDI-0562 | OX40 |
| PF-04518600 | OX40 |
| RG-7888 | OX40 |
| PF-06801591 | PD-1 |
| BGBA-317 | PD-1 |
| MEDI-0680 | PD-1 |
| MK-3475 | PD-1 |
| Nivolumab | PD-1 |
| PDR-001 | PD-1 |
| Pembrolizumab | PD-1 |
| Pidilizumab | PD-1 |
| REGN-2810 | PD-1 |
| SHR-1210 | PD-1 |
| TSR-042 | PD-1 |
| MDX-1106 | PD-1 |
| Merck 3745 | PD-1 |
| CT- 011 | PD-1 |
| MEDI-0680 | PD-1 |
| PDR001 | PD-1 |
| REGN2810 | PD-1 |
| BGB-108 | PD-1 |
| BGB-A317 | PD-1 |
| AMP-224 | PD-1 |
| Atezolizumab | PD-L1 (CD274) |
| Avelumab | PD-L1 (CD274) |
| BMS-936559 | PD-L1 (CD274) |
| Durvalumab | PD-L1 (CD274) |
| MEDI-4736 | PD-L1 (CD274) |
| MPDL33280A | PD-L1 (CD274) |
| YW243.55.S70 | PD-L1 (CD274) |
| MDX-1105 | PD-L1 (CD274) |
| MSB0010718C | PD-L1 (CD274) |

### Standard therapy

More preferably, the subject receiving the pharmaceutical composition or vaccine comprising RNAs of the invention, the combination thereof or the pharmaceutical composition or vaccine comprising said RNA(s) is a patient suffering from a tumor or cancer disease as described herein and who received or receives chemotherapy (e.g. first-line or second-line chemotherapy), radiotherapy, chemoradiation (combination of chemotherapy and radiotherapy), kinase inhibitors, antibody therapy and/or checkpoint modulators (e.g. CTLA4 inhibitors, PD1 pathway inhibitors), or a patient, who has achieved partial response or stable disease after having received one or more of the treatments specified above. More preferably, the subject is a patient suffering from a tumor or cancer disease as described herein and who received or receives a compound conventionally used in any of these diseases as described herein, more preferably a patient who receives or received a checkpoint modulator.

The following compounds are preferred compounds which preferably are used in standard therapies and can be applied in combination with the pharmaceutical compositions or vaccines comprising RNAs of the invention: Cetuximab (Erbitux), paclitaxel albumin bound (Abraxane), (gimeracil + oteracil + tegafur) (TS-1), Docetaxel (Docetaxel, Doxel, Taxotere, Docetaxel An, Docel, Nanoxel M, Tautax, Docetaxel -AS, Docetaxel-M, Qvidadotax, Relidoce, Taxelo, Oncodocel, Doxotel, Pacancer, Docetrust, Dodetax, Dodabur, Soulaxcin, Taxedol, Docefim, Docetaxel, Ribodocel, Critidoc, Asodoc, Chemodoc, Docelibbs, Docenat, Dincilezan, Dostradixinol, Docefrez, Camitotic, Oncotaxel, Somatixel, Belotaxel, Qvidadotax, Taxceus, Cetadocure, Docetaxel CT, Tevaxter, Docirena, Eurotere, Axtere, Celotax, Taxanit, Drobanos, Cetado, Doxocad, Taxceus, Egidox, Tedocad, Docecad, Docelex, Docetax, Docetaxel, Docetere, Dotax, ,Taxuba, Monotaxel, Taceedo, Detaxl, Docet, Docetaxel, Ferdotax, Wintaxel), (tegafur + uracil) (Uft, Uft E, Tefudex, Unitoral, Luporal, Tagracil), Fluorouracil (5-FU), (gimeracil + oteracil + tegafur) ODT (TS-1 Combination OD), bleomycin sulfate (Tecnomicina, Cinaleo, Bleomycin, Bloicin-S, Bonar, Bleocin, Bleomycin Sulfate, Bleo, Bleocel, Bleotex, Oncobleo, Bleonco, Bleosol, Lyoble, Bleomycin Sulfate, Blenamax, Bleomycin, Blenoxane, Bleomicina, Bleomycine Bellon, Bleoprim), carboplatin (Carboplatin, Platamine CS, Carbaccord, Carboplatina, Carboplatino, Paraplatin, Carbosin, Tecnocarb, Carbomerck, Paract, Carboplatine CTRS, Carboplatine Intsel Chimos, Carboplatin, Carbokem, Carbotinol, Fauldcarbo, Evocarb, Citoplatina, Platin), ciprofloxacin (Hypoflox, Ufexil), ciprofloxacin hydrochloride (Ciprofloxacin Pharma, Prodin, Ciproxin), cisplatin (Cisplatin, Stritin, Ifapla, Accocit, Unistin, Cancertin, Cisplan, Citoplax, Nuoxin, Placis, Cisplatino, Displanor, Randa, Cispla, Fauldcispla, Briplatin, Platinex, Platinol, Platinex, Riboplatin, Cisplatine, Platistine CS, Platosin, Accocit, Cisplatino) cyclophosphamide (Endoxan, Cyclophosphamide), doxifluridine (Doxifluridine, May Vladimir), doxorubicin (Doxorubicin Hydrochloride, Adriamycin RDF, Doxorubicin, Doxorubicin PFS), epirubicin, hydrochloride (Brecila, Cloridrato De Epirrubicina, Epirubicin, Farmorubicina, Nuovodox, Adnexa, 4-Eppedo, Favicin), fluorouracil (Agicil, Fluorouracil, Fauldfluor, Oncourcil, Flocil, 5 Flucel), folic acid + methotrexate (Truxofol), human adenovirus type 5 (recombinant) (Oncorine), hydroxyurea (Oxyrea, Durea, Myelostat, Riborea, Unidrea, Ondrea, Hydran, Leukocel, Hydroxyurea, Hydrea), ifosfamide (Holoxan, Ifosfamide EG), levamisole (Zirsol), methotrexate Methotrexate (Tratoben, Methotrexate, Fresexate, Neometho, Fauldmetro, Methotrexate Sodium, Methocel, Hytas, Methaccord, Methofill, Metotrexato, Traxacord, Plastomet, Tevatrex, Metrex, Caditrex, Carditrex, Vibzi, Imutrex, Biotrexate, Methorex, Mexate, Neotrexate, Oncotrex, Remtrex, Trixilem, Hi-Trex, Metorex, Trex, Unitrexate, Ebetrexac, Fauldexato, Lantarel, Maxtrex, Miantrex CS, Rheumatrex, Folex, Folex PFS, Abitrexate, Tevametho, Trexall, Emthexate, Abitrexate, Meadow), mitomycin (Mitomycin C, Mitomycin, Mitonco, Lyomit), nedaplatin (Jiebaishu, Aoxianda, Aqupla), nimesulide (Nimulid), nimotuzumab (Biomab EGFR, Laedemab), nitrofurantoin (Furatsilin), ofloxacin (Entof), paclitaxel (Paclitaxel, Taxol), peplomycin sulfate (Pepleo), picibanil (Picibanil), pirarubicin (Pirarubicin Hydrochloride, Therarubicin, Pinorubin), sodium glycididazole (CMNa), tegafur (Utefos, Icarus, Futraful, Tegafur Gimeracil Oteracil Potassium), temoporfin (Foscan), topotecan hydrochloride (Topotecan), ubenimex (Ubenimex), vinblastine sulfate (Vinblastine, Vblastin), vincristine sulfate (Vincristine, Vincristine Sulfate, Vincristin, Sutivin, vindesine sulfate (Eldisine), carboplatin (Carboplatine Qualimed, Carboplatine, Carboplatino, Carboplatin), cisplatin (Cisplatin), docetaxel (Kamdocon, Naltoxater, Docetaxel), fluorouracil (Fluorouracil, Fluorouracile, Fluorouracil), methotrexate (Methotrexate Sodium, Mexate, Mexate Aq, Biometrox, Medsatrexate, Otaxem), vincristine sulfate (Oncovin), fluorouracil, sunitinib malate, acitretin, fibrin sealant, cetuximab, cetuximab, erlotinib, cisplatin;docetaxel;fluorouracil, undisclosed anti-cancer drug, gefitinib, pravastatin sodium, sirolimus, undisclosed chemotherapy, cisplatin;docetaxel;fluorouracil, sirolimus, fluorouracil;undisclosed taxane, methyl aminolevulinate hydrochloride, cisplatin;docetaxel;fluorouracil, erlotinib hydrochloride, cetuximab, imiquimod, undisclosed Chinese herbal medicine, aspirin;enalapril maleate, undisclosed chemotherapy, cetuximab, (gimeracil + oteracil + tegafur);carboplatin;cisplatin, cisplatin;fluorouracil;nimotuzumab, carboplatin;paclitaxel albumin bound, cisplatin;nedaplatin, bleomycin, nedaplatin, cisplatin;paclitaxel, paclitaxel albumin bound, (gimeracil + oteracil + tegafur), bleomycin;undisclosed chemotherapy, apatinib;docetaxel, undisclosed immunomodulatory supplement, BCM-95, aminolevulinic acid hydrochloride, nedaplatin, cisplatin;palifermin, cetuximab, gefitinib, bevacizumab, belagenpumatucel-L, cisplatin;tirapazamine, cisplatin;tirapazamine, cisplatin; gemcitabine; paclitaxel; topotecan;vinorelbine, cisplatin;fluorouracil, panitumumab, carboplatin;docetaxel;gemcitabine hydrochloride;vinorelbine tartrate, amifostine; fluorouracil, cisplatin;fluorouracil, carboplatin;paclitaxel, tirapazamine, cisplatin;epoetin alfa, figitumumab, melphalan;tumor necrosis factor alf, cisplatin, cisplatin;fluorouracil, cisplatin;undisclosed chemotherapy, docetaxel, contusugene ladenovec, cisplatin; fluorouracil;paclitaxel, docetaxel, human papillomavirus [serotypes 16, 18] (bivalent) vaccine, isotretinoin, cisplatin;fluorouracil, misonidazole, paclitaxel, palifermin, endostatin, pilocarpine, cisplatin; docetaxel; filgrastim; fluorouracil; paclitaxel, cisplatin; docetaxel; filgrastim;fluorouracil;paclitaxel, cisplatin;irinotecan hydrochloride, cisplatin;gemcitabine, cisplatin;epirubicin;fluorouracil;undisclosed chemotherapy, methyl aminolevulinate hydrochloride, carboplatin;paclitaxel, carbogen;carbon dioxide; niacinamide, cisplatin; fluorouracil, talimogene laherparepvec, epoetin alfa, cisplatin;fluorouracil;panitumumab, cisplatin;fluorouracil, cisplatin;fluorouracil, aldesleukin, cisplatin;fluorouracil, cisplatin; paclitaxel, cisplatin;fluorouracil, fluorouracil;leucovorin;lobaplatin, cisplatin, cisplatin; ethyl mercaptan;ifosfamide;mesna;mitolactol, doxorubicin;levamisole, (tegafur + uracil), cisplatin;fluorouracil, cisplatin;vinorelbine, carboplatin;cisplatin;gemcitabine hydrochloride, Corynebacterium parvum;doxorubicin, capecitabine; cisplatin; fluorouracil; paclitaxel, fluorouracil;leucovorin;methotrexate, rAd-p53, cetuximab;cisplatin;docetaxel, PV-10, methyl aminolevulinate hydrochloride, cisplatin;fluorouracil, paclitaxel;topotecan hydrochloride, carboplatin;cisplatin;paclitaxel, cisplatin;topatecan hydrochloride, cisplatin;etoposide, docetaxel;fluorouracil, aspirin, cisplatin;gemcitabine, Lactobacillus brevis CD2,cisplatin;docetaxel, fosbretabulin tromethamine, panitumumab, fluorouracil, paclitaxel, carboplatin;cisplatin;docetaxel;fluorouracil, fluorouracil, erlotinib hydrochloride, cisplatin; undisclosed chemotherapy;vinorelbine, (gimeracil + oteracil + tegafur);carboplatin, cetuximab, contusugene ladenovec, cetuximab, methyl aminolevulinate hydrochloride, cyclophosphamide, (gimeracil + oteracil + tegafur);cisplatin, paclitaxel albumin bound, carboplatin;paclitaxel, cisplatin;gemcitabine, capecitabine;cisplatin, docetaxel, Z-100, cisplatin;ifosfamide;paclitaxel, nimotuzumab, irinotecan hydrochloride, celecoxib;methotrexate, Nutrison, carboplatin; cisplatin; fluorouracil; paclitaxel, cisplatin; paclitaxel, cisplatin; docetaxel; vinorelbine, paclitaxel,(gimeracil + oteracil + tegafur); cisplatin, carboplatin;paclitaxel, methyl aminolevulinate hydrochloride, Aibin, cisplatin;fluorouracil, porfimer sodium, carboplatin; cisplatin;tocotrienol;vinorelbine, (gimeracil + oteracil + tegafur);cisplatin;paclitaxel, docetaxel, ipilimumab, cisplatin, VB-4847, celecoxib;thalidomide, cisplatin; epirubicin;fluorouracil, cisplatin;fluorouracil, fluorouracil, carboplatin;paclitaxel, cetuximab;cisplatin;docetaxel, autologous cytokine induced killer cells, cisplatin;docetaxel;fluorouracil, cisplatin;epirubicin;fluorouracil, tergenpumatucel-L, cetuximab;cisplatin;docetaxel, Elental, cisplatin;nimotuzumab;paclitaxel, eicosapentaenoic acid; undisclosed nutritional supplement, palbociclib, pembrolizumab (Keytruda), nimotuzumab, apatorsen and dacomitinib.

### Tumor indications

As used herein, the terms "tumor", "cancer" or "cancer disease" refer to a malignant disease, which is preferably selected from the group consisting of Adenocystic carcinoma (Adenoid cystic carcinoma), Adrenocortical carcinoma, AIDS-related cancers, AIDS-related lymphoma, Anal cancer, Appendix cancer, Astrocytoma, Basal cell carcinoma, Bile duct cancer, Bladder cancer, Bone cancer, Osteosarcoma/Malignant fibrous histiocytoma, Brainstem glioma, Brain tumor, cerebellar astrocytoma, cerebral astrocytoma/malignant glioma, ependymoma, medulloblastoma, supratentorial primitive neuroectodermal tumors, visual pathway and hypothalamic glioma, Breast cancer, Bronchial adenomas/carcinoids, Burkitt lymphoma, childhood Carcinoid tumor, gastrointestinal Carcinoid tumor, Carcinoma of unknown primary, primary Central nervous system lymphoma, childhood Cerebellar astrocytoma, childhood Cerebral astrocytoma/Malignant glioma, Cervical cancer, Childhood cancers, Chronic lymphocytic leukemia, Colon Cancer, Cutaneous T-cell lymphoma including Mycosis Fungoides and Sezary Syndrome, Desmoplastic small round cell tumor, Endometrial cancer, Ependymoma, Esophageal cancer, Ewing's sarcoma in the Ewing family of tumors, Childhood Extracranial germ cell tumor, Extragonadal Germ cell tumor, Extrahepatic bile duct cancer, Intraocular melanoma, Retinoblastoma, Gallbladder cancer, Gastric (Stomach) cancer, Gastrointestinal Carcinoid Tumor, Gastrointestinal stromal tumor (GIST), extracranial, extragonadal, or ovarian Germ cell tumor, Gestational trophoblastic tumor, Glioma of the brain stem, Childhood Cerebral Astrocytoma, Childhood Visual Pathway and Hypothalamic Glioma, Gastric carcinoid, Hairy cell leukemia, Head and neck cancer, Heart cancer, Hepatocellular (liver) cancer, Hodgkin lymphoma, Human Papilloma Virus (HPV)-related cancer, Hypopharyngeal cancer, childhood Hypothalamic and visual pathway glioma, Intraocular Melanoma, Islet Cell Carcinoma (Endocrine Pancreas), Kaposi sarcoma, Kidney cancer (renal cell cancer), Laryngeal Cancer, Lip and Oral Cavity Cancer, Liposarcoma, Liver Cancer, Non-Small Cell Lung Cancer, Small Cell Lung Cancer, Lymphomas, AIDS-related Lymphoma, Burkitt Lymphoma, Hodgkin Lymphoma, Non-Hodgkin Lymphomas, Primary Central Nervous System Lymphoma, Malignant Fibrous Histiocytoma of Bone/Osteosarcoma, Childhood Medulloblastoma, Melanoma, Intraocular (Eye) Melanoma, Merkel Cell Carcinoma, Adult Malignant Mesothelioma, Childhood Mesothelioma, Head or Neck Cancer, Mouth Cancer, Childhood Multiple Endocrine Neoplasia Syndrome, Multiple Myeloma/Plasma Cell Neoplasm, Multiple Myeloma (Cancer of the Bone-Marrow), Nasal cavity and paranasal sinus cancer, Nasopharyngeal carcinoma, Neuroblastoma, Oral Cancer, Oropharyngeal cancer, Osteosarcoma/malignant fibrous histiocytoma of bone, Ovarian cancer, Ovarian epithelial cancer (Surface epithelial-stromal tumor), Ovarian germ cell tumor, Ovarian low malignant potential tumor, Pancreatic cancer, islet cell Pancreatic cancer, Paranasal sinus and nasal cavity cancer, Parathyroid cancer, Penile cancer, Pharyngeal cancer, Pheochromocytoma, Pineal astrocytoma, Pineal germinoma, childhood Pineoblastoma and supratentorial primitive neuroectodermal tumors, Pituitary adenoma, Plasma cell neoplasia/ plasmocytoma/Multiple myeloma, Pleuropulmonary blastoma, Primary central nervous system lymphoma, Prostate cancer, Rectal cancer, Renal cell carcinoma (kidney cancer), Cancer of the Renal pelvis and ureter, Retinoblastoma, childhood Rhabdomyosarcoma, Salivary gland cancer, Sarcoma of the Ewing family of tumors, Kaposi Sarcoma, soft tissue Sarcoma, uterine Sarcoma, Skin cancer (nonmelanoma), Skin cancer (melanoma), Merkel cell Skin carcinoma, Small intestine cancer, Squamous cell carcinoma, metastatic Squamous neck cancer with occult primary, soft tissue sarcoma (STS), childhood Supratentorial primitive neuroectodermal tumor, Testicular cancer (seminoma and non-seminoma), Throat cancer, childhood Thymoma, Thymoma and Thymic carcinoma, Thyroid cancer, childhood Thyroid cancer, Transitional cell cancer of the renal pelvis and ureter, gestational Trophoblastic tumor, Urethral cancer, endometrial Uterine cancer, Uterine sarcoma, Vaginal cancer, childhood Visual pathway and hypothalamic glioma, Vulvar cancer, and childhood Wilms tumor (kidney cancer).

### Allergenic antigens:

Antigens associated with allergy or allergic disease (allergens or allergenic antigens) are preferably derived from a source selected from the list consisting of:
Acarus spp (Aca s 1, Aca s 10, Aca s 10.0101, Aca s 13, Aca s 13.0101, Aca s 2, Aca s 3, Aca s 7, Aca s 8), Acanthocybium spp (Aca so 1), Acanthocheilonema spp (Aca v 3, Aca v 3.0101), Acetes spp (Ace ja 1), Actinidia spp (Act a 1, Act c 1, Act c 10, Act c 10.0101, Act c 2, Act c 4, Act c 5, Act c 5.0101, Act c 8, Act c 8.0101, Act c Chitinase, Act d 1, Act d 1.0101, Act d 10, Act d 10.0101, Act d 10.0201, Act d 11, Act d 11.0101, Act d 2, Act d 2.0101, Act d 3, Act d 3.0101, Act d 3.02, Act d 4, Act d 4.0101, Act d 5, Act d 5.0101, Act d 6, Act d 6.0101, Act d 7, Act d 7.0101, Act d 8, Act d 8.0101, Act d 9, Act d 9.0101, Act d Chitinase, Act e 1, Act e 5), Acyrthosiphon spp (Acy pi 7, Acy pi 7.0101, Acy pi 7.0102), Adenia spp (Ade v RIP), Aedes spp (Aed a 1, Aed a 1.0101, Aed a 2, Aed a 2.0101, Aed a 3, Aed a 3.0101, Aed a 4, Aed a 7, Aed a 7.0101, Aed a 7.0102, Aed a 7.0103, Aed a 7.0104, Aed a 7.0105, Aed a 7.0106, Aed a 7.0107, Aed a 7.0108, Aed a 7.0109, Aed a 7.0110, Aed a 7.0111, Aed al 1, Aed al 3, Aed al 37kD, Aed v 37kD, Aed v 63kD), Aegilops spp (Aeg ta 28, Aeg ta alpha_Gliadin, Aeg um 28, Aeg un 28), Aethaloperca spp (Aet ro 1), Agropyron spp (Agr c 7), Agrostis spp (Agr ca 1, Agr ca 5, Agr g 1, Agr g 4, Agr s 5), Agrobacterium spp (Agr sp CP4 EPSPS), Ailuropoda spp (Ail me Phosvitin, Ail me TCTP), Aix spp (Aix ga 1, Aix sp 1), Aleuroglyphus spp (Ale o 1, Ale o 10, Ale o 10.0101, Ale o 10.0102, Ale o 13, Ale o 14, Ale o 2, Ale o 20, Ale o 3, Ale o 5, Ale o 7, Ale o 8, Ale o 9), Allium spp (All a 3, All a Alliin lyase, All c 3, All c 30kD, All c 4, All c Alliin lyase, All p Alliin lyase, All s Alliin lyase), Alnus spp (Aln g 1, Aln g 1.0101, Aln g 1/Bet v 1/Cor a 1 TPC7, Aln g 1/Bet v 1/Cor a 1 TPC9, Aln g 2, Aln g 4, Aln g 4.0101), Alopochen spp (Alo ae 1), Alopecurus spp (Alo p 1, Alo p 5), Alternaria spp (Alt a 1, Alt a 1.0101, Alt a 1.0102, Alt a 10, Alt a 10.0101, Alt a 12, Alt a 12.0101, Alt a 13, Alt a 13.0101, Alt a 2, Alt a 3, Alt a 3.0101, Alt a 4, Alt a 4.0101, Alt a 5, Alt a 5.0101, Alt a 6, Alt a 6.0101, Alt a 7, Alt a 7.0101, Alt a 70kD, Alt a 8, Alt a 8.0101, Alt a 9, Alt a MnSOD, Alt a NTF2, Alt a TCTP, Alt ar 1, Alt arg 1, Alt b 1, Alt bl 1, Alt br 1, Alt c 1, Alt ca 1, Alt ce 1, Alt ch 1, Alt ci 1, Alt co 1, Alt cr 1, Alt ct 1, Alt cu 1, Alt cy 1, Alt d 1, Alt du 1, Alt e 1, Alt et 1, Alt eu 1, Alt ga 1, Alt gr 1, Alt j 1, Alt l 1, Alt lo 1, Alt m 1, Alt me 1, Alt mi 1, Alt mo 1, Alt o 1, Alt p 1, Alt ph 1, Alt po 1, Alt ps 1, Alt r 1, Alt s 1, Alt se 1, Alt sm 1, Alt so 1, Alt su 1, Alt t 1, Alt te 1, Alt to 1), Amaranthus spp (Ama r 2, Ama r 2.0101, Ama v 2, Ama v 2.0101, Ama v 2.0201), Ambrosia spp (Amb a 1, Amb a 1.0101, Amb a 1.0201, Amb a 1.0202, Amb a 1.0301, Amb a 1.0302, Amb a 1.0303, Amb a 1.0304, Amb a 1.0305, Amb a 1.0401, Amb a 1.0402, Amb a 1.0501, Amb a 1.0502, Amb a 10, Amb a 10.0101, Amb a 3, Amb a 3.0101, Amb a 4, Amb a 4.0101, Amb a 5, Amb a 5.0101, Amb a 6, Amb a 6.0101, Amb a 7, Amb a 7.0101, Amb a 8, Amb a 8.0101, Amb a 8.0102, Amb a 9, Amb a 9.0101, Amb a 9.0102, Amb a CPI, Amb p 1, Amb p 5, Amb p 5.0101, Amb p 5.0201, Amb t 5, Amb t 5.0101, Amb t 8), Ammothea spp (Amm h 7, Amm h 7.0101), Anadara spp (Ana br 1), Ananas spp (Ana c 1, Ana c 1.0101, Ana c 2, Ana c 2.0101, Ana c 2.0101 (MUXF3)), Anas spp (Ana ca 1), Anarhichas spp (Ana l 1), Anacardium spp (Ana o 1, Ana o 1.0101, Ana o 1.0102, Ana o 2, Ana o 2.0101, Ana o 3, Ana o 3.0101), Anas spp (Ana p 1, Ana p 2, Ana p 3), Anguilla spp (Ang a 1, Ang j 1), Anisakis spp (Ani s 1, Ani s 1.0101, Ani s 10, Ani s 10.0101, Ani s 11, Ani s 11.0101, Ani s 12, Ani s 12.0101, Ani s 2, Ani s 2.0101, Ani s 24kD, Ani s 3, Ani s 3.0101, Ani s 4, Ani s 4.0101, Ani s 5, Ani s 5.0101, Ani s 6, Ani s 6.0101, Ani s 7, Ani s 7.0101, Ani s 8, Ani s 8.0101, Ani s 9, Ani s 9.0101, Ani s CCOS3, Ani s Cytochrome B, Ani s FBPP, Ani s NADHDS4L, Ani s NARaS, Ani s PEPB, Ani s Troponin), Annona spp (Ann c Chitinase), Anopheles spp (Ano da 17, Ano da 17.0101, Ano da 27, Ano da 27.0101, Ano da 7, Ano da 7.0101, Ano g 7, Ano g 7.0101), Anser spp (Ans a 1, Ans a 2, Ans a 3, Ans in 1), Anthoxanthum spp (Ant o 1, Ant o 1.0101, Ant o 12, Ant o 13, Ant o 2, Ant o 4, Ant o 5, Ant o 6, Ant o 7), Apis spp (Api c 1, Api c 1.0101, Api c 10, Api c 2, Api c 4, Api d 1, Api d 1.0101, Api d 4, Api fl 4), Apium spp (Api g 1, Api g 1.0101, Api g 1.0201, Api g 2, Api g 2.0101, Api g 3, Api g 3.0101, Api g 4, Api g 4.0101, Api g 5, Api g 5.0101, Api g 6, Api g 6.0101), Apis spp (Api m 1, Api m 1.0101, Api m 10, Api m 10.0101, Api m 11, Api m 11.0101, Api m 11.0201, Api m 13kD, Api m 2, Api m 2.0101, Api m 3, Api m 3.0101, Api m 4, Api m 4.0101, Api m 5, Api m 5.0101, Api m 6, Api m 6.0101, Api m 7, Api m 7.0101, Api m 8, Api m 8.0101, Api m 9, Api m 9.0101, Api m A1-A2, Api m A1-A2-A3, Api m Apalbumin 1, Api m Apalbumin 2, Api me 1, Api me 4), Arachis spp (Ara d 2, Ara d 6, Ara f 3, Ara f 4, Ara h 1, Ara h 1.0101, Ara h 10, Ara h 10.0101, Ara h 10.0102, Ara h 11, Ara h 11.0101, Ara h 2, Ara h 2.0101, Ara h 2.0102, Ara h 2.0201, Ara h 2.0202, Ara h 3, Ara h 3.0101, Ara h 4, Ara h 4.0101, Ara h 5, Ara h 5.0101, Ara h 6, Ara h 6.0101, Ara h 7, Ara h 7.0101, Ara h 7.0201, Ara h 7.0202, Ara h 8, Ara h 8.0101, Ara h 8.0201, Ara h 9, Ara h 9.0101, Ara h 9.0201, Ara h Agglutinin, Ara h Oleosin 18kD, Ara i 2, Ara i 6), Arabidopsis spp (Ara t 3, Ara t 8, Ara t GLP), Archosargus spp (Arc pr 1), Archaeopotamobius spp (Arc s 8, Arc s 8.0101), Aequipecten spp (Arg i 1), Argas spp (Arg r 1, Arg r 1.0101), Ariopsis spp (Ari fe 1), Armoracia spp (Arm r HRP), Arrhenatherum spp (Arr e 1, Arr e 5), Artemisia spp (Art a 1, Art ap 1), Artemia spp (Art fr 1, Art fr 1.0101, Art fr 5, Art fr 5.0101), Arthrobacter spp (Art gl CO), Achorion spp (Art gy 7), Artocarpus spp (Art h 17kD, Art h 4), Arthrospira spp (Art pl beta_Phycocyanin), Artemisia spp (Art v 1, Art v 1.0101, Art v 1.0102, Art v 1.0103, Art v 1.0104, Art v 1.0105, Art v 1.0106, Art v 1.0107, Art v 2, Art v 2.0101, Art v 3, Art v 3.0101, Art v 3.0201, Art v 3.0202, Art v 3.0301, Art v 4, Art v 4.0101, Art v 4.0201, Art v 47kD, Art v 5, Art v 5.0101, Art v 6, Art v 6.0101, Art v 60kD), Arthroderma spp (Art va 4), Ascaris spp (Asc l 3, Asc l 3.0101, Asc l 3.0102, Asc l 34kD, Asc s 1, Asc s 1.0101, Asc s 3, Asc s 3.0101, Asc s GST), Aspergillus spp (Asp aw Glucoamylase, Asp c 22, Asp f 1, Asp f 1.0101, Asp f 10, Asp f 10.0101, Asp f 11, Asp f 11.0101, Asp f 12, Asp f 12.0101, Asp f 13, Asp f 13.0101, Asp f 15, Asp f 15.0101, Asp f 16, Asp f 16.0101, Asp f 17, Asp f 17.0101, Asp f 18, Asp f 18.0101, Asp f 2, Asp f 2.0101, Asp f 22, Asp f 22.0101, Asp f 23, Asp f 23.0101, Asp f 27, Asp f 27.0101, Asp f 28, Asp f 28.0101, Asp f 29, Asp f 29.0101, Asp f 3, Asp f 3.0101, Asp f 34, Asp f 34.0101, Asp f 4, Asp f 4.0101, Asp f 5, Asp f 5.0101, Asp f 56kD, Asp f 6, Asp f 6.0101, Asp f 7, Asp f 7.0101, Asp f 8, Asp f 8.0101, Asp f 9, Asp f 9.0101, Asp f AfCalAp, Asp f AT_V, Asp f Catalase, Asp f Chitosanase, Asp f CP, Asp f DPPV, Asp f FDH, Asp f gamma_Actin, Asp f Glucosidase, Asp f GPI, Asp f GST, Asp f GT, Asp f IAO, Asp f IPMI, Asp f LPL1, Asp f LPL3, Asp f Mannosidase, Asp f MDH, Asp f PL, Asp f PUP, Asp f RPS3, Asp f SXR, Asp fl 13, Asp fl 13.0101, Asp fl 18, Asp fl 2, Asp fl 21, Asp fl 3, Asp fl 4, Asp fl 7, Asp fl 8, Asp fl 9, Asp me Seaprose, Asp n 14, Asp n 14.0101, Asp n 18, Asp n 18.0101, Asp n 25, Asp n 25.0101, Asp n 30, Asp n Glucoamylase, Asp n Hemicellulase, Asp n Pectinase, Asp o 13, Asp o 13.0101, Asp o 21, Asp o 21.0101, Asp o 3, Asp o 4, Asp o 7, Asp o 8, Asp o Lactase, Asp o Lipase, Asp oc 13, Asp r 1, Asp sa AP, Asp sp Glucoamylase, Asp sp Glucoseoxidase, Asp sp PL, Asp sp PME, Asp sy 13, Asp v 13, Asp v 13.0101, Asp v Catalase A, Asp v Enolase, Asp v GAPDH, Asp v MDH, Asp v SXR), Asparagus spp (Aspa o 1, Aspa o 1.01, Aspa o 1.02, Aspa o 17kD, Aspa o 4), Aspergillus spp (Aspe ni 2, Aspe ni 3, Aspe ni 4, Aspe ni 7, Aspe ni 8, Aspe ni 9), Avena spp (Ave s 1, Ave s 12, Ave s 13, Ave s 2, Ave s 4, Ave s 5, Ave s 7), Babylonia spp (Bab ja 1), Bacillus spp (Bac al Subtilisin, Bac cl Subtilisin, Bac l Subtilisin, Bac li aA, Bac li Subtilisin), Bactrocera spp (Bac ol 27, Bac ol 27.0101), Bacillus spp (Bac sp aA1, Bac sp aA3, Bac sp Decarboxylase, Bac st amyM, Bac su Subtilisin, Bac t Cry1Ab, Bac t Cry1Fa, Bac t Cry3Bb1, Bac t Cry9c), Bagre spp (Bag ma 1), Balistes spp (Bal ca 1), Balanus spp (Bal r 1, Bal r 1.0101), Beauveria spp (Bea b Ald, Bea b Enol, Bea b f2, Bea b Hex), Bertholletia spp (Ber e 1, Ber e 1.0101, Ber e 2, Ber e 2.0101), Beryx spp (Ber sp 1), Betula spp (Bet ab 1, Bet al 1, Bet ch 1, Bet co 1, Bet da 1, Bet gr 1, Bet hu 1, Bet le 1, Bet me 1, Bet n 1, Bet p 1, Bet pa 1, Bet po 1, Bet pu 1, Bet pu 2, Bet pu 4, Bet pu 6, Bet pu 7, Bet sc 1, Bet ut 1, Bet v 1, Bet v 1 B1-B1-B1, Bet v 1 fv Mal 4x, Bet v 1.0101, Bet v 1.0102, Bet v 1.0103, Bet v 1.0201, Bet v 1.0301, Bet v 1.0401, Bet v 1.0402, Bet v 1.0501, Bet v 1.0601, Bet v 1.0602, Bet v 1.0701, Bet v 1.0801, Bet v 1.0901, Bet v 1.1001, Bet v 1.1101, Bet v 1.1201, Bet v 1.1301, Bet v 1.1401, Bet v 1.1402, Bet v 1.1501, Bet v 1.1502, Bet v 1.1601, Bet v 1.1701, Bet v 1.1801, Bet v 1.1901, Bet v 1.2001, Bet v 1.2101, Bet v 1.2201, Bet v 1.2301, Bet v 1.2401, Bet v 1.2501, Bet v 1.2601, Bet v 1.2701, Bet v 1.2801, Bet v 1.2901, Bet v 1.3001, Bet v 1.3101, Bet v 2, Bet v 2.0101, Bet v 3, Bet v 3.0101, Bet v 4, Bet v 4.0101, Bet v 6, Bet v 6.0101, Bet v 6.0102, Bet v 7, Bet v 7.0101, Bet v 8, Bet v Glucanase), Beta spp (Beta v 1, Beta v 1.0101, Beta v 2, Beta v 2.0101), Blattella spp (Bla g 1, Bla g 1.0101, Bla g 1.0102, Bla g 1.0103, Bla g 1.0201, Bla g 1.0202, Bla g 2, Bla g 2.0101, Bla g 2.0201, Bla g 36kD, Bla g 4, Bla g 4.0101, Bla g 4.0201, Bla g 5, Bla g 5.0101, Bla g 5.0201, Bla g 6, Bla g 6.0101, Bla g 6.0201, Bla g 6.0301, Bla g 7, Bla g 7.0101, Bla g 8, Bla g 8.0101, Bla g 9, Bla g Enolase, Bla g GSTD1, Bla g RACK1, Bla g TPI, Bla g Trypsin, Bla g Vitellogenin), Blatta spp (Bla o 1, Bla o 7), Blomia spp (Blo t 1, Blo t 1.0101, Blo t 1.0201, Blo t 10, Blo t 10.0101, Blo t 10.0102, Blo t 11, Blo t 11.0101, Blo t 12, Blo t 12.0101, Blo t 12.0102, Blo t 13, Blo t 13.0101, Blo t 14, Blo t 15, Blo t 18, Blo t 19, Blo t 19.0101, Blo t 2, Blo t 2.0101, Blo t 2.0102, Blo t 2.0103, Blo t 20, Blo t 21, Blo t 21.0101, Blo t 3, Blo t 3.0101, Blo t 4, Blo t 4.0101, Blo t 5, Blo t 5.0101, Blo t 6, Blo t 6.0101, Blo t 7, Blo t 8, Blo t 9, Blo t HSP70), Bombus spp (Bom ar 4, Bom hy 4, Bom p 1, Bom p 1.0101, Bom p 2, Bom p 3, Bom p 4, Bom p 4.0101, Bom t 1, Bom t 1.0101, Bom t 4, Bom t 4.0101), Bombyx spp (Bomb m 1, Bomb m 1.0101, Bomb m 7, Bomb m 7.0101, Bomb m 7.0102, Bomb m 7.0103, Bomb m 7.0104, Bomb m 7.0105, Bomb m 7.0106), Boophilus spp (Boo m 1, Boo m 7, Boo m 7.0101), Bos spp (Bos d 2, Bos d 2.0101, Bos d 2.0102, Bos d 2.0103, Bos d 3, Bos d 3.0101, Bos d 4, Bos d 4.0101, Bos d 5, Bos d 5.0101, Bos d 5.0102, Bos d 6, Bos d 6 (MDA), Bos d 6.0101, Bos d 7, Bos d 7.0101, Bos d 8, Bos d 8 alphaS1, Bos d 8 alphaS2, Bos d 8 beta, Bos d 8 kappa, Bos d alpha2I, Bos d alpha2I.0101, Bos d Chymosin, Bos d Fibrin, Bos d Gelatin, Bos d HG, Bos d Insulin, Bos d Lactoferrin, Bos d Lactoperoxidase, Bos d Myoglobin, Bos d OBP, Bos d OSCP, Bos d Phosvitin, Bos d PLA2, Bos d PRVB, Bos d Thrombin, Bos d TI, Bos gr ALA, Bos gr Myoglobin), Bothrops spp (Bot as 1, Bot at 1), Bouteloua spp (Bou g 1), Biting spp (Bov ov 1), Brama spp (Bra du 1), Brassica spp (Bra j 1, Bra j 1.0101, Bra n 1, Bra n 1.0101, Bra n 4, Bra n 7, Bra n 8, Bra n PG, Bra ni 8, Bra o 3, Bra o 3.0101, Bra r 1, Bra r 1.0101, Bra r 2, Bra r 2.0101, Bra r 3, Bra r 4, Bra r 7), Bromus spp (Bro a 1, Bro a 4), Brosme spp (Bro br 1), Bromus spp (Bro i 1, Bro i 5, Bro i 7), Brugia spp (Bru m 3, Bru m 3.0101, Bru m Bm33), Bubalus spp (Bub b ALA, Bub b BLG, Bub b Casein, Bub b Casein alphaS1, Bub b Casein alphaS2, Bub b Casein beta, Bub b Casein kappa), Caenorhabditis spp (Cae b 3, Cae b 3.0101, Cae br 3, Cae br 3.0101, Cae e 3, Cae e 3.0101, Cae e 3.0102, Cae re 13, Cae re 13.0101), Cajanus spp (Caj c 1), Caligus spp (Cal cl 1, Cal cl 1.0101, Cal cl 1.0102), Calamus spp (Cal le 1), Callinectes spp (Cal s 2), Camelus spp (Cam d ALA, Cam d Casein, Cam d Casein alphaS1, Cam d Casein alphaS2, Cam d Casein beta, Cam d Casein kappa), Camponotus spp (Cam fl 7, Cam fl 7.0101), Canis spp (Can f 1, Can f 1.0101, Can f 2, Can f 2.0101, Can f 3, Can f 3.0101, Can f 4, Can f 4.0101, Can f 5, Can f 5.0101, Can f 6, Can f 6.0101, Can f Feld1-like, Can f Homs2-like, Can f Phosvitin, Can f TCTP), Canthidermis spp (Can ma 1), Cancer spp (Can mg 2, Can p 1), Cannabis spp (Can s 3), Candida spp (Cand a 1, Cand a 1.0101, Cand a 3, Cand a 3.0101, Cand a CAAP, Cand a CyP, Cand a Enolase, Cand a FPA, Cand a MnSOD, Cand a PGK, Cand b 2, Cand b 2.0101, Cand b FDH, Cand r Lipase), Capsicum spp (Cap a 1, Cap a 1.0101, Cap a 17kD, Cap a 2, Cap a 2.0101, Cap a 30kD, Cap a Glucanase, Cap ch 17kD), Caprella spp (Cap e 1), Capra spp (Cap h ALA, Cap h BLG, Cap h Casein, Cap h Casein alphaS1, Cap h Casein alphaS2, Cap h Casein beta, Cap h Casein kappa, Cap h GSA), Capitulum spp (Cap m 1), Carassius spp (Car au 1), Carpinus spp (Car b 1, Car b 1.0101, Car b 1.0102, Car b 1.0103, Car b 1.0104, Car b 1.0105, Car b 1.0106, Car b 1.0107, Car b 1.0108, Car b 1.0109, Car b 1.0110, Car b 1.0111, Car b 1.0112, Car b 1.0113, Car b 1.0201, Car b 1.0301, Car b 1.0302, Car b 2, Car b 4), Caranx spp (Car cr 1), Carya spp (Car i 1, Car i 1.0101, Car i 2, Car i 4, Car i 4.0101), Carcinus spp (Car ma 2), Caryota spp (Car mi 2), Carica spp (Car p 1, Car p Chitinase, Car p Chymopapain, Car p Endoproteinase), Castanea spp (Cas c 24kD, Cas s 1, Cas s 1.0101, Cas s 1.0102, Cas s 1.0103, Cas s 2, Cas s 5, Cas s 5.0101, Cas s 8, Cas s 8.0101, Cas s 9, Cas s 9.0101), Catharanthus spp (Cat r 1, Cat r 1.0101, Cat r 17kD, Cat r 2), Caulolatilus spp (Cau ch 1), Cavia spp (Cav p 1, Cav p 1.0101, Cav p 2, Cav p 2.0101, Cav p 3, Cav p 3.0101, Cav p Gelatin, Cav p GSA), Centropristis spp (Cen s 1), Cephalopholis spp (Cep so 1), Charybdis spp (Cha f 1, Cha f 1.0101), Chaetodipterus spp (Cha fa 1), Chamaecyparis spp (Cha o 1, Cha o 1.0101, Cha o 2, Cha o 2.0101), Chenopodium spp (Che a 1, Che a 1.0101, Che a 2, Che a 2.0101, Che a 3, Che a 3.0101), Chironomus spp (Chi k 1, Chi k 10, Chi k 10.0101), Chinchilla spp (Chi l 21kD_a, Chi l 21kD_b), Chionoecetes spp (Chi o 1, Chi o 1.0101, Chi o 2, Chi o 4, Chi o 6, Chi o alpha_Actin, Chi o SERCA), Chironomus spp (Chi t 1, Chi t 1.0101, Chi t 1.0201, Chi t 2, Chi t 2.0101, Chi t 2.0102, Chi t 3, Chi t 3.0101, Chi t 4, Chi t 4.0101, Chi t 5, Chi t 5.0101, Chi t 6, Chi t 6.0101, Chi t 6.0201, Chi t 7, Chi t 7.0101, Chi t 8, Chi t 8.0101, Chi t 9, Chi t 9.0101), Chlamys spp (Chl n 1), Chloephaga spp (Chl pi 1), Chortoglyphus spp (Cho a 10), Chrysomela spp (Chr tr 7, Chr tr 7.0101), Cicer spp (Cic a 2S Albumin, Cic a Albumin), Cichorium spp (Cic i 1), Cimex spp (Cim l Nitrophorin), Citrus spp (Cit l 1, Cit l 3, Cit l 3.0101), Citrullus spp (Cit la 2, Cit la MDH, Cit la TPI), Citrus spp (Cit r 3, Cit r 3.0101, Cit s 1, Cit s 1.0101, Cit s 2, Cit s 2.0101, Cit s 3, Cit s 3.0101, Cit s 3.0102, Cit s IFR), Cladosporium spp (Cla c 14, Cla c 14.0101, Cla c 9, Cla c 9.0101, Cla h 1, Cla h 10, Cla h 10.0101, Cla h 12, Cla h 12.0101, Cla h 2, Cla h 2.0101, Cla h 42kD, Cla h 5, Cla h 5.0101, Cla h 6, Cla h 6.0101, Cla h 7, Cla h 7.0101, Cla h 8, Cla h 8 CSP, Cla h 8.0101, Cla h 9, Cla h 9.0101, Cla h abH, Cla h GST, Cla h HCh1, Cla h HSP70, Cla h NTF2, Cla h TCTP), Clostridium spp (Clo hi Collagenase, Clo t Toxoid), Clupea spp (Clu h 1, Clu h 1.0101, Clu h 1.0201, Clu h 1.0301), Cocos spp (Coc n 2, Coc n 4, Coc n 5), Coccidioides spp (Coc po 8), Coffea spp (Cof a 1, Cof a 1.0101), Columba spp (Col l PSA), Coprinus spp (Cop c 1, Cop c 1.0101, Cop c 2, Cop c 2.0101, Cop c 3, Cop c 3.0101, Cop c 4, Cop c 5, Cop c 5.0101, Cop c 6, Cop c 7, Cop c 7.0101), Corylus spp (Cor a 1, Cor a 1.0101, Cor a 1.0102, Cor a 1.0103, Cor a 1.0104, Cor a 1.0201, Cor a 1.0301, Cor a 1.0401, Cor a 1.0402, Cor a 1.0403, Cor a 1.0404, Cor a 10, Cor a 10.0101, Cor a 11, Cor a 11.0101, Cor a 12, Cor a 12.0101, Cor a 13, Cor a 13.0101, Cor a 14, Cor a 14.0101, Cor a 2, Cor a 2.0101, Cor a 2.0102, Cor a 8, Cor a 8.0101, Cor a 9, Cor a 9.0101), Corynebacterium spp (Cor d Toxoid), Corylus spp (Cor he 1), Coryphaena spp (Cor hi 1), Coriandrum spp (Cor s 1, Cor s 11kD, Cor s 2), Cotoneaster spp (Cot l 3), Crangon spp (Cra c 1, Cra c 1.0101, Cra c 2, Cra c 2.0101, Cra c 4, Cra c 4.0101, Cra c 5, Cra c 5.0101, Cra c 6, Cra c 6.0101, Cra c 8, Cra c 8.0101), Crassostrea spp (Cra g 1), Cricetus spp (Cri c HSA), Crivellia spp (Cri pa 1), Crocus spp (Cro s 1, Cro s 1.0101, Cro s 2, Cro s 2.0101, Cro s 3, Cro s 3.01, Cro s 3.02), Cryptomeria spp (Cry j 1, Cry j 1.0101, Cry j 1.0102, Cry j 1.0103, Cry j 2, Cry j 2.0101, Cry j 2.0102, Cry j 3, Cry j 3.1, Cry j 3.2, Cry j 3.3, Cry j 3.4, Cry j 3.5, Cry j 3.6, Cry j 3.7, Cry j 3.8, Cry j 4, Cry j AP, Cry j Chitinase, Cry j CPA9, Cry j IFR, Cry j LTP, Cry j P1-P2), Cryphonectria spp (Cry p AP), Ctenocephalides spp (Cte f 1, Cte f 1.0101, Cte f 2, Cte f 2.0101, Cte f 3, Cte f 3.0101), Ctenopharyngodon spp (Cte id 1), Cucumis spp (Cuc m 1, Cuc m 1.0101, Cuc m 2, Cuc m 2.0101, Cuc m 3, Cuc m 3.0101, Cuc m Lec17, Cuc m MDH), Cucurbita spp (Cuc ma 18kD, Cuc ma 2, Cuc p 2, Cuc p AscO), Cucumis spp (Cuc s 2), Culicoides spp (Cul n 1, Cul n 10, Cul n 11, Cul n 2, Cul n 3, Cul n 4, Cul n 5, Cul n 6, Cul n 7, Cul n 8, Cul n 9, Cul n HSP70), Culex spp (Cul q 28kD, Cul q 35kD, Cul q 7, Cul q 7.0101, Cul q 7.0102), Culicoides spp (Cul so 1), Cuminum spp (Cum c 1, Cum c 2), Cupressus spp (Cup a 1, Cup a 1.0101, Cup a 1.02, Cup a 2, Cup a 3, Cup a 4, Cup a 4.0101, Cup s 1, Cup s 1.0101, Cup s 1.0102, Cup s 1.0103, Cup s 1.0104, Cup s 1.0105, Cup s 3, Cup s 3.0101, Cup s 3.0102, Cup s 3.0103, Cup s 8), Cochliobolus spp (Cur l 1, Cur l 1.0101, Cur l 2, Cur l 2.0101, Cur l 3, Cur l 3.0101, Cur l 4, Cur l 4.0101, Cur l ADH, Cur l GST, Cur l MnSOD, Cur l Oryzin, Cur l Trx, Cur l ZPS1), Cyanochen spp (Cya cy 1), Cynoscion spp (Cyn ar 1), Cynosurus spp (Cyn cr 1, Cyn cr 5), Cynodon spp (Cyn d 1, Cyn d 1.0101, Cyn d 1.0102, Cyn d 1.0103, Cyn d 1.0104, Cyn d 1.0105, Cyn d 1.0106, Cyn d 1.0107, Cyn d 1.0201, Cyn d 1.0202, Cyn d 1.0203, Cyn d 1.0204, Cyn d 10, Cyn d 11, Cyn d 12, Cyn d 12.0101, Cyn d 13, Cyn d 15, Cyn d 15.0101, Cyn d 2, Cyn d 22, Cyn d 22.0101, Cyn d 23, Cyn d 23.0101, Cyn d 24, Cyn d 24.0101, Cyn d 4, Cyn d 5, Cyn d 6, Cyn d 7, Cyn d 7.0101), Cynoscion spp (Cyn ne 1), Cynomys spp (Cyn sp Lipocalin), Cyprinus spp (Cyp c 1, Cyp c 1.01, Cyp c 1.02), Daboia spp (Dab ru 1), Dactylis spp (Dac g 1, Dac g 1.01, Dac g 1.0101, Dac g 1.02, Dac g 12, Dac g 13, Dac g 2, Dac g 2.0101, Dac g 3, Dac g 3.0101, Dac g 4, Dac g 4.0101, Dac g 5, Dac g 5.0101, Dac g 7), Dama spp (Dam d CSA), Danio spp (Dan re 1, Dan re 2, Dan re alpha2I, Dan re CK), Dasyatis spp (Das ak 1, Das am 1, Das sa 1), Daucus spp (Dau c 1, Dau c 1.0101, Dau c 1.0102, Dau c 1.0103, Dau c 1.0104, Dau c 1.0105, Dau c 1.0201, Dau c 1.0301, Dau c 3, Dau c 4, Dau c 4.0101, Dau c CyP), Decapterus spp (Dec ru 1), Dendronephthya spp (Den n 1, Den n 1.0101), Dermatophagoides spp (Der f 1, Der f 1.0101, Der f 1.0102, Der f 1.0103, Der f 1.0104, Der f 1.0105, Der f 1.0106, Der f 1.0107, Der f 1.0108, Der f 1.0109, Der f 1.0110, Der f 10, Der f 10.0101, Der f 10.0102, Der f 11, Der f 11.0101, Der f 13, Der f 13.0101, Der f 14, Der f 14.0101, Der f 15, Der f 15.0101, Der f 16, Der f 16.0101, Der f 17, Der f 17.0101, Der f 18, Der f 18.0101, Der f 2, Der f 2.0101, Der f 2.0102, Der f 2.0103, Der f 2.0104, Der f 2.0105, Der f 2.0106, Der f 2.0107, Der f 2.0108, Der f 2.0109, Der f 2.0110, Der f 2.0111, Der f 2.0112, Der f 2.0113, Der f 2.0114, Der f 2.0115, Der f 2.0116, Der f 2.0117, Der f 20, Der f 21, Der f 22, Der f 22.0101, Der f 3, Der f 3.0101, Der f 4, Der f 5, Der f 6, Der f 6.0101, Der f 7, Der f 7.0101, Der f 8, Der f 9, Der f HSP70), Dermanyssus spp (Der g 10, Der g 10.0101), Dermatophagoides spp (Der m 1, Der m 1.0101, Der p 1, Der p 1.0101, Der p 1.0102, Der p 1.0103, Der p 1.0104, Der p 1.0105, Der p 1.0106, Der p 1.0107, Der p 1.0108, Der p 1.0109, Der p 1.0110, Der p 1.0111, Der p 1.0112, Der p 1.0113, Der p 1.0114, Der p 1.0115, Der p 1.0116, Der p 1.0117, Der p 1.0118, Der p 1.0119, Der p 1.0120, Der p 1.0121, Der p 1.0122, Der p 1.0123, Der p 1.0124, Der p 10, Der p 10.0101, Der p 10.0102, Der p 10.0103, Der p 11, Der p 11.0101, Der p 13, Der p 14, Der p 14.0101, Der p 15, Der p 18, Der p 2, Der p 2.0101, Der p 2.0102, Der p 2.0103, Der p 2.0104, Der p 2.0105, Der p 2.0106, Der p 2.0107, Der p 2.0108, Der p 2.0109, Der p 2.0110, Der p 2.0111, Der p 2.0112, Der p 2.0113, Der p 2.0114, Der p 2.0115, Der p 20, Der p 20.0101, Der p 21, Der p 21.0101, Der p 23, Der p 23.0101, Der p 3, Der p 3.0101, Der p 4, Der p 4.0101, Der p 5, Der p 5.0101, Der p 5.0102, Der p 6, Der p 6.0101, Der p 7, Der p 7.0101, Der p 8, Der p 8.0101, Der p 9, Der p 9.0101, Der p 9.0102, Der p P1-P2, Der p P2-P1, Der s 1, Der s 2, Der s 3), Dianthus spp (Dia c RIP), Dicranopteris spp (Dic l 2S Albumin), Diospyros spp (Dio k 17kD, Dio k 4, Dio k IFR), Dioscorea spp (Dio p TSP), Diplodus spp (Dip ho 1), Distichlis spp (Dis s 1, Dis s 7), Ditrema spp (Dit te 1), Dolichovespula spp (Dol a 1, Dol a 2, Dol a 5, Dol a 5.0101), Dolichos spp (Dol b Agglutinin), Dolichovespula spp (Dol m 1, Dol m 1.0101, Dol m 1.02, Dol m 2, Dol m 2.0101, Dol m 5, Dol m 5.0101, Dol m 5.02), Drosophila spp (Dro an 7, Dro an 7.0101, Dro er 7, Dro er 7.0101, Dro er 7.0102, Dro gr 7, Dro gr 7.0101, Dro gr 7.0102, Dro m 7, Dro m 7.0101, Dro m 7.0102, Dro m 7.0103, Dro m 7.0104, Dro m 7.0105, Dro m 7.0106, Dro m 7.0107, Dro m 7.0108, Dro m 7.0109, Dro m 7.0110, Dro m 7.0111, Dro m 7.0112, Dro m 7.0113, Dro m 9, Dro m MnSOD, Dro mo 7, Dro mo 7.0101, Dro pp 7, Dro pp 7.0101, Dro se 7, Dro se 7.0101, Dro si 7, Dro si 7.0101, Dro si 7.0102, Dro vi 7, Dro vi 7.0101, Dro wi 7, Dro wi 7.0101, Dro y 7, Dro y 7.0101, Dro y 7.0102, Dro y 7.0103), Echium spp (Ech p Cytochrome C), Elaeis spp (Ela g 2, Ela g Bd31kD), Elops spp (Elo sa 1), Embellisia spp (Emb a 1, Emb i 1, Emb nz 1, Emb t 1), Engraulis spp (Eng e 1), Enteroctopus spp (Ent d 1), Epinephelus spp (Epi bl 1, Epi co 1, Epi fl 1, Epi mc 1, Epi mo 1), Epicoccum spp (Epi p 1, Epi p 1.0101, Epi p 12kD, Epi p GST), Epinephelus spp (Epi po 1, Epi un 1), Equisetum spp (Equ a 17kD), Equus spp (Equ as 4, Equ as DSA, Equ bu 4, Equ c 1, Equ c 1.0101, Equ c 2, Equ c 2.0101, Equ c 2.0102, Equ c 3, Equ c 3.0101, Equ c 4, Equ c 4.0101, Equ c 5, Equ c 5.0101, Equ c ALA, Equ c BLG, Equ c Casein, Equ c Casein beta, Equ c Casein kappa, Equ c PRVB, Equ he 4, Equ z ZSA), Erimacrus spp (Eri i 1, Eri i 1.0101, Eri i 1.0102), Eriocheir spp (Eri s 1, Eri s 1.0101, Eri s 2), Erwinia spp (Erw ch Asparaginase), Escherichia spp (Esc c Asparaginase, Esc c beta GAL), Esox spp (Eso l 1), Euphausia spp (Eup p 1, Eup p 1.0101), Euphasia spp (Eup s 1, Eup s 1.0101), Euroglyphus spp (Eur m 1, Eur m 1.0101, Eur m 1.0102, Eur m 1.0103, Eur m 10, Eur m 14, Eur m 14.0101, Eur m 2, Eur m 2.0101, Eur m 2.0102, Eur m 3, Eur m 3.0101, Eur m 4, Eur m 4.0101), Evynnis spp (Evy j 1), Fagopyrum spp (Fag e 1, Fag e 1.0101, Fag e 10kD, Fag e 19kD, Fag e 2, Fag e 2.0101, Fag e TI), Fagus spp (Fag s 1, Fag s 1.0101, Fag s 2, Fag s 4), Fagopyrum spp (Fag t 1, Fag t 10kD, Fag t 2, Fag t 2.0101), Felis spp (Fel d 1, Fel d 1.0101, Fel d 2, Fel d 2.0101, Fel d 3, Fel d 3.0101, Fel d 4, Fel d 4.0101, Fel d 5, Fel d 5.0101, Fel d 6, Fel d 6.0101, Fel d 7, Fel d 7.0101, Fel d 8, Fel d 8.0101, Fel d IgG), Fenneropenaeus spp (Fen c 1, Fen c 2, Fen me 1, Fen me 1.0101), Festuca spp (Fes e 1, Fes e 13, Fes e 4, Fes e 5, Fes e 7, Fes p 1, Fes p 13, Fes p 4, Fes p 4.0101, Fes p 5, Fes r 1, Fes r 5), Ficus spp (Fic c 17kD, Fic c 4, Fic c Ficin), Foeniculum spp (Foe v 1, Foe v 2), Forsythia spp (For s 1), Forcipomyia spp (For t 1, For t 1.0101, For t 2, For t 2.0101, For t 7, For t FPA, For t Myosin, For t TPI), Fragaria spp (Fra a 1, Fra a 1.0101, Fra a 3, Fra a 3.0101, Fra a 3.0102, Fra a 3.0201, Fra a 3.0202, Fra a 3.0203, Fra a 3.0204, Fra a 3.0301, Fra a 4, Fra a 4.0101, Fra c 1), Fraxinus spp (Fra e 1, Fra e 1.0101, Fra e 1.0102, Fra e 1.0201, Fra e 12, Fra e 2, Fra e 3, Fra e 9), Fragaria spp (Fra v 1), Fusarium spp (Fus c 1, Fus c 1.0101, Fus c 2, Fus c 2.0101, Fus c 3, Fus s 1, Fus s 45kD, Fus sp Lipase), Gadus spp (Gad c 1, Gad c 1.0101, Gad c APDH, Gad m 1, Gad m 1.0101, Gad m 1.0102, Gad m 1.0201, Gad m 1.0202, Gad m 45kD, Gad m Gelatin, Gad ma 1), Gallus spp (Gal d 1, Gal d 1.0101, Gal d 2, Gal d 2.0101, Gal d 3, Gal d 3.0101, Gal d 4, Gal d 4.0101, Gal d 5, Gal d 5.0101, Gal d 6, Gal d 6.0101, Gal d Apo I, Gal d Apo VI, Gal d GPI, Gal d HG, Gal d IgY, Gal d L-PGDS, Gal d Ovomucin, Gal d Phosvitin, Gal d PRVB, Gal la 4), Galleria spp (Gal m 18kD, Gal m 24kD), Gallus spp (Gal so 4), Gammarus spp (Gam s TM), Gelonium spp (Gel m RIP), Geothelphusa spp (Geo de 1), Glossina spp (Glo m 5, Glo m 5.0101, Glo m 7, Glo m 7.0101, Glo m 7.0102, Glo m 7.0103), Glycine spp (Gly a Bd30K, Gly ar Bd30K, Gly ca Bd30K, Gly cl Bd30K, Gly cu Bd30K, Gly cy Bd30K), Glycyphagus spp (Gly d 10, Gly d 10.0101, Gly d 13, Gly d 2, Gly d 2.0101, Gly d 2.0201, Gly d 2.03, Gly d 2/Lep d 2 L1, Gly d 2/Lep d 2 L2, Gly d 2/Lep d 2 L3, Gly d 2/Lep d 2 L4, Gly d 2/Lep d 2 R1, Gly d 2/Lep d 2 R2, Gly d 2/Lep d 2 R3, Gly d 2/Lep d 2 R4, Gly d 2/Lep d 2 R5, Gly d 20, Gly d 3, Gly d 5, Gly d 5.01, Gly d 5.02, Gly d 7, Gly d 8), Glycine spp (Gly f Bd30K, Gly l Bd30K, Gly m 1, Gly m 1.0101, Gly m 1.0102, Gly m 2, Gly m 2.0101, Gly m 2S Albumin, Gly m 3, Gly m 3.0101, Gly m 3.0102, Gly m 39kD, Gly m 4, Gly m 4.0101, Gly m 5, Gly m 5.0101, Gly m 5.0201, Gly m 5.0301, Gly m 5.0302, Gly m 50kD, Gly m 6, Gly m 6.0101, Gly m 6.0201, Gly m 6.0301, Gly m 6.0401, Gly m 6.0501, Gly m 68kD, Gly m Agglutinin, Gly m Bd28K, Gly m Bd30K, Gly m Bd60K, Gly m CPI, Gly m EAP, Gly m TI, Gly mi Bd30K, Gly s Bd30K, Gly t Bd30K, Gly to Bd30K), Gossypium spp (Gos h Vicilin), Haemophilus spp (Hae in P6), Haemaphysalis spp (Hae l 7, Hae l 7.0101, Hae q 7, Hae q 7.0101), Haliotis spp (Hal a 1, Hal d 1, Hal di 1, Hal di PM, Hal m 1, Hal m 1.0101, Hal r 1, Hal r 49kD, Hal ru 1), Harmonia spp (Har a 1, Har a 1.0101, Har a 2, Har a 2.0101), Harpegnathos spp (Har sa 7, Har sa 7.0101, Har sa 7.0102), Helianthus spp (Hel a 1, Hel a 1.0101, Hel a 2, Hel a 2.0101, Hel a 2S Albumin, Hel a 3, Hel a 3.0101, Hel a 4), Helix spp (Hel ap 1, Hel as 1, Hel as 1.0101), Heligmosomoides spp (Hel p 3, Hel p 3.0101), Helianthus spp (Hel tu 1), Hemanthias spp (Hem le 1), Hemifusus spp (Hem t 1), Heterodera spp (Het g 3, Het g 3.0101), Hevea spp (Hev b 1, Hev b 1.0101, Hev b 10, Hev b 10.0101, Hev b 10.0102, Hev b 10.0103, Hev b 11, Hev b 11.0101, Hev b 11.0102, Hev b 12, Hev b 12.0101, Hev b 13, Hev b 13.0101, Hev b 14, Hev b 14.0101, Hev b 2, Hev b 2.0101, Hev b 3, Hev b 3.0101, Hev b 4, Hev b 4.0101, Hev b 5, Hev b 5.0101, Hev b 6, Hev b 6.01, Hev b 6.02, Hev b 6.0202, Hev b 6.03, Hev b 7, Hev b 7.01, Hev b 7.02, Hev b 7.D2, Hev b 7.S2, Hev b 8, Hev b 8.0101, Hev b 8.0102, Hev b 8.0201, Hev b 8.0202, Hev b 8.0203, Hev b 8.0204, Hev b 9, Hev b 9.0101, Hev b Citrate binding Protein, Hev b GAPDH, Hev b HSP80, Hev b IFR, Hev b Proteasome subunit, Hev b Rotamase, Hev b SPI, Hev b Trx, Hev b UDPGP), Hexagrammos spp (Hex ot 1), Hippoglossus spp (Hip h 1), Hippoglossoides spp (Hip pl 1), Hippoglossus spp (Hip st 1), Hirudo spp (Hir me Hirudin), Holcus spp (Hol l 1, Hol l 1.0101, Hol l 1.0102, Hol l 2, Hol l 4, Hol l 5, Hol l 5.0101, Hol l 5.0201), Holocnemus spp (Hol pl 9, Hol pl Hemocyanin), Homarus spp (Hom a 1, Hom a 1.0101, Hom a 1.0102, Hom a 1.0103, Hom a 3, Hom a 3.0101, Hom a 4, Hom a 6, Hom a 6.0101, Hom g 1, Hom g 2), Homo spp (Hom s 1, Hom s 1.0101, Hom s 2, Hom s 2.0101, Hom s 3, Hom s 3.0101, Hom s 4, Hom s 4.0101, Hom s 5, Hom s 5.0101, Hom s AAT, Hom s ACTH, Hom s Adalimumab, Hom s ALA, Hom s alpha_Actin, Hom s alpha-Galactosidase, Hom s APDH, Hom s Arylsulfatase B, Hom s Casein, Hom s CyP A, Hom s CyP B, Hom s CyP C, Hom s DSF70, Hom s DSG3, Hom s eIF6, Hom s Etanercept, Hom s Factor IX, Hom s Factor VII, Hom s Factor VIII, Hom s G-CSF, Hom s Glucocerebrosidase, Hom s Glucosidase, Hom s HLA-DR-alpha, Hom s HSA, Hom s Iduronidase, Hom s Idursulfase, Hom s IgA, Hom s Insulin, Hom s Lactoferrin, Hom s Laminin gamma_2, Hom s MnSOD, Hom s Oxytocin, Hom s P2, Hom s Phosvitin, Hom s Profilin, Hom s PSA, Hom s RP1, Hom s TCTP, Hom s TL, Hom s TPA, Hom s TPO, Hom s Transaldolase, Hom s Trx, Hom s Tubulin-alpha, Hom s/Mus m Basiliximab, Hom s/Mus m Cetuximab, Hom s/Mus m Cetuximab (Gal-Gal), Hom s/Mus m Infliximab, Hom s/Mus m Natalizumab, Hom s/Mus m Omalizumab, Hom s/Mus m Palivizumab, Hom s/Mus m Rituximab, Hom s/Mus m Tocilizumab, Hom s/Mus m Trastuzumab), Hoplostethus spp (Hop a 1), Hordeum spp (Hor v 1, Hor v 12, Hor v 12.0101, Hor v 13, Hor v 14, Hor v 15, Hor v 15.0101, Hor v 16, Hor v 16.0101, Hor v 17, Hor v 17.0101, Hor v 18kD, Hor v 2, Hor v 21, Hor v 21.0101, Hor v 28, Hor v 33, Hor v 4, Hor v 5, Hor v 5.0101, Hor v BDAI, Hor v BTI), Humicola spp (Hum in Cellulase), Humulus spp (Hum j 1, Hum j 1.0101, Hum j 10kD, Hum j 2), Huso spp (Hus h 1), Hylocereus spp (Hyl un LTP), Hymenocephalus spp (Hym st 1), Hyperoglyphe spp (Hyp by 1), Hypophthalmichthys spp (Hyp mo 1), Hypophthalmichthy spp (Hyp no 1), Ictalurus spp (Ict fu 1, Ict p 1), Imperata spp (Imp c 4, Imp c 5, Imp c VIIIe1), Ixodes spp (Ixo r 2, Ixo sc 7, Ixo sc 7.0101), Jasus spp (Jas la 1, Jas la 1.0101, Jas la 1.0102), Juglans spp (Jug ca 1, Jug ca 2, Jug ci 1, Jug ci 2, Jug n 1, Jug n 1.0101, Jug n 2, Jug n 2.0101, Jug r 1, Jug r 1.0101, Jug r 2, Jug r 2.0101, Jug r 3, Jug r 3.0101, Jug r 4, Jug r 4.0101, Jug r 5), Juniperus spp (Jun a 1, Jun a 1.0101, Jun a 1.0102, Jun a 2, Jun a 2.0101, Jun a 3, Jun a 3.0101, Jun c 1, Jun o 1, Jun o 4, Jun o 4.0101, Jun r 3, Jun r 3.1, Jun r 3.2, Jun v 1, Jun v 1.0101, Jun v 1.0102, Jun v 3, Jun v 3.0101, Jun v 3.0102, Jun v 4), Katsuwonus spp (Kat p 1), Kyphosus spp (Kyp se 1), Lachnolaimus spp (Lac ma 1), Lachesis spp (Lac mu 1), Lactuca spp (Lac s 1, Lac s 1.0101), Lagocephalus spp (Lag la 1), Larus spp (Lar a 1, Lar a 2, Lar a 3), Larimichthys spp (Lar po 1), Lates spp (Lat c 1), Lateolabrax spp (Lat ja 1), Lathyrus spp (Lat oc Agglutinin), Leiostomus spp (Lei xa 1), Lens spp (Len c 1, Len c 1.0101, Len c 1.0102, Len c 1.0103, Len c 2, Len c 2.0101, Len c 3, Len c 3.0101, Len c Agglutinin), Leopardus spp (Leo p 1), Lepidoglyphus spp (Lep d 10, Lep d 10.0101, Lep d 12, Lep d 13, Lep d 13.0101, Lep d 2, Lep d 2.0101, Lep d 2.0102, Lep d 2.0201, Lep d 2.0202, Lep d 3, Lep d 39kD, Lep d 5, Lep d 5.0101, Lep d 5.0102, Lep d 5.0103, Lep d 7, Lep d 7.0101, Lep d 8, Lep d alpha Tubulin), Lepomis spp (Lep gi 1), Leptomelanosoma spp (Lep i 1), Lepomis spp (Lep ma 1), Lepisma spp (Lep s 1, Lep s 1.0101, Lep s 1.0102), Lepeophtheirus spp (Lep sa 1, Lep sa 1.0101, Lep sa 1.0102, Lep sa 1.0103), Leptailurus spp (Lep se 1), Lepidorhombus spp (Lep w 1, Lep w 1.0101), Lethocerus spp (Let in 7, Let in 7.0101, Let in 7.0102), Leuciscus spp (Leu ce 1), Lewia spp (Lew in 1), Ligustrum spp (Lig v 1, Lig v 1.0101, Lig v 1.0102, Lig v 2), Lilium spp (Lil l 2, Lil l PG), Limanda spp (Lim fe 1), Limnonectes spp (Lim m 1), Limulus spp (Lim p 1, Lim p 1.0101, Lim p 2, Lim p LPA), Liposcelis spp (Lip b 1, Lip b 1.0101), Litchi spp (Lit c 1, Lit c 1.0101, Lit c IFR, Lit c TPI), Lithobates spp (Lit ca 1), Litopenaeus spp (Lit se 1, Lit v 1, Lit v 1.0101, Lit v 2, Lit v 2.0101, Lit v 3, Lit v 3.0101, Lit v 4, Lit v 4.0101), Filiaria spp (Loa lo 3, Loa lo 3.0101), Lobotes spp (Lob su 1), Locusta spp (Loc m 7, Loc m 7.0101), Loligo spp (Lol b 1, Lol e 1), Lolium spp (Lol m 2, Lol m 5, Lol p 1, Lol p 1.0101, Lol p 1.0102, Lol p 1.0103, Lol p 10, Lol p 11, Lol p 11.0101, Lol p 12, Lol p 13, Lol p 2, Lol p 2.0101, Lol p 3, Lol p 3.0101, Lol p 4, Lol p 4.0101, Lol p 5, Lol p 5.0101, Lol p 5.0102, Lol p 7, Lol p CyP, Lol p FT, Lol p Legumin), Lonomia spp (Lon o 7, Lon o 7.0101), Lophodytes spp (Lop cu 1), Lophonetta spp (Lop sp 1), Lupinus spp (Lup a 1, Lup a alpha_Conglutin, Lup a delta_Conglutin, Lup a gamma_Conglutin, Lup an 1, Lup an 1.0101, Lup an alpha_Conglutin, Lup an delta_Conglutin, Lup an gamma_Conglutin, Lup l 17kD), Lutjanus spp (Lut a 1, Lut c 1, Lut cy 1, Lut gr 1, Lut gu 1, Lut jo 1), Lutraria spp (Lut p 1), Lutjanus spp (Lut pu 1, Lut sy 1), Lycopersicon spp (Lyc e 1, Lyc e 1.0101, Lyc e 11S Globulin, Lyc e 2, Lyc e 2.0101, Lyc e 2.0102, Lyc e 3, Lyc e 3.0101, Lyc e 4, Lyc e 4.0101, Lyc e ARP60S, Lyc e Chitinase, Lyc e Glucanase, Lyc e Peroxidase, Lyc e PG, Lyc e PME, Lyc e PR23, Lyc e Vicilin), Maconellicoccus spp (Mac h 7, Mac h 7.0101), Macruronus spp (Mac ma 1, Mac n 1), Maclura spp (Mac po 17kD), Macrobrachium spp (Mac ro 1, Mac ro 1.0101, Mac ro Hemocyanin), Macropus spp (Macr s Gelatin), Malus spp (Mal d 1, Mal d 1.0101, Mal d 1.0102, Mal d 1.0103, Mal d 1.0104, Mal d 1.0105, Mal d 1.0106, Mal d 1.0107, Mal d 1.0108, Mal d 1.0109, Mal d 1.0201, Mal d 1.0202, Mal d 1.0203, Mal d 1.0204, Mal d 1.0205, Mal d 1.0206, Mal d 1.0207, Mal d 1.0208, Mal d 1.0301, Mal d 1.0302, Mal d 1.0303, Mal d 1.0304, Mal d 1.0401, Mal d 1.0402, Mal d 1.0403, Mal d 2, Mal d 2.0101, Mal d 3, Mal d 3.0101, Mal d 3.0102, Mal d 3.0201, Mal d 3.0202, Mal d 3.0203, Mal d 4, Mal d 4.0101, Mal d 4.0102, Mal d 4.0201, Mal d 4.0202, Mal d 4.0301, Mal d 4.0302), Malpighia spp (Mal g 4, Mal g Hevein), Malus spp (Mal p 1), Malassezia spp (Mala f 2, Mala f 2.0101, Mala f 3, Mala f 3.0101, Mala f 4, Mala f 4.0101, Mala g 10, Mala s 1, Mala s 1.0101, Mala s 10, Mala s 10.0101, Mala s 11, Mala s 11.0101, Mala s 12, Mala s 12.0101, Mala s 13, Mala s 13.0101, Mala s 5, Mala s 5.0101, Mala s 6, Mala s 6.0101, Mala s 7, Mala s 7.0101, Mala s 8, Mala s 8.0101, Mala s 9, Mala s 9.0101), Manihot spp (Man e 5, Man e 5.0101, Man e FPA, Man e GAPDH), Mangifera spp (Man i 1, Man i 14kD, Man i 2, Man i 3, Man i 3.01, Man i 3.02, Man i Chitinase), Marsupenaeus spp (Mar j 1, Mar j 1.0101, Mar j 2, Mar j 4), Matricaria spp (Mat c 17kD), Mecopoda spp (Mec e 7), Megalobrama spp (Meg am 2, Meg am CK), Megathura spp (Meg c Hemocyanin), Megalops spp (Meg sp 1), Melanogrammus spp (Mel a 1), Meleagris spp (Mel g 1, Mel g 2, Mel g 3, Mel g PRVB, Mel g TSA), Melicertus spp (Mel l 1), Menticirrhus spp (Men am 1), Mercurialis spp (Mer a 1, Mer a 1.0101), Merluccius spp (Mer ap 1, Mer au 1, Mer bi 1, Mer ca 1, Mer ga 1, Mer hu 1), Merlangius spp (Mer me 1), Merluccius spp (Mer mr 1, Mer pa 1, Mer po 1, Mer pr 1, Mer se 1), Meriones spp (Mer un 23kD), Metarhizium spp (Met a 30), Metapenaeopsis spp (Met ba 1), Metapenaeus spp (Met e 1, Met e 1.0101, Met e 2), Metasequoia spp (Met gl 2), Metapenaeus spp (Met j 1, Met j 2), Metanephrops spp (Met ja 1), Metapenaeopsis spp (Met la 1), Metanephrops spp (Met t 2), Micromesistius spp (Mic po 1), Micropogonias spp (Mic un 1), Mimachlamys spp (Mim n 1), Momordica spp (Mom c RIP), Morus spp (Mor a 17kD, Mor a 4), Morone spp (Mor am 1), Morus spp (Mor n 3, Mor n 3.0101), Morone spp (Mor sa 1, Mor sc 1), Mugil spp (Mug c 1), Muraenolepis spp (Mur mi 1), Musa spp (Mus a 1, Mus a 1.0101, Mus a 2, Mus a 2.0101, Mus a 3, Mus a 3.0101, Mus a 4, Mus a 4.0101, Mus a 5, Mus a 5.0101, Mus a 5.0102), Mus spp (Mus m 1, Mus m 1.0101, Mus m 1.0102, Mus m 2, Mus m Gelatin, Mus m IgG, Mus m MSA, Mus m Muromonab, Mus m Phosvitin), Mustela spp (Mus p 17kD), Musa spp (Mus xp 1, Mus xp 2, Mus xp 5), Mycteroperca spp (Myc bo 1, Myc mi 1, Myc ph 1), Myceliophthora spp (Myc sp Laccase), Myrmecia spp (Myr p 1, Myr p 1.0101, Myr p 2, Myr p 2.0101, Myr p 2.0102, Myr p 3, Myr p 3.0101), Mytilus spp (Myt e 1, Myt g 1, Myt g PM), Myzus spp (Myz p 7, Myz p 7.0101), Nemorhedus spp (Nae go Hya), Necator spp (Nec a Calreticulin), Nemipterus spp (Nem vi 1), Neosartorya spp (Neo fi 1, Neo fi 22), Neochen spp (Neo ju 1), Neoscona spp (Neo n 7, Neo n 7.0101), Nephelium spp (Nep l GAPDH), Nephrops spp (Nep n 1, Nep n DF9), Neptunea spp (Nep po 1, Nep po 1.0101), Nicotiana spp (Nic t 8, Nic t Osmotin, Nic t Villin), Nimbya spp (Nim c 1, Nim s 1), Nippostrongylus spp (Nip b Ag1), Nycticebus spp (Nyc c 1), Octopus spp (Oct f 1, Oct l 1, Oct v 1, Oct v 1.0101, Oct v PM), Ocyurus spp (Ocy ch 1), Olea spp (Ole e 1, Ole e 1.0101, Ole e 1.0102, Ole e 1.0103, Ole e 1.0104, Ole e 1.0105, Ole e 1.0106, Ole e 1.0107, Ole e 10, Ole e 10.0101, Ole e 11, Ole e 11.0101, Ole e 11.0102, Ole e 12, Ole e 13, Ole e 2, Ole e 2.0101, Ole e 3, Ole e 3.0101, Ole e 36kD, Ole e 4, Ole e 4.0101, Ole e 5, Ole e 5.0101, Ole e 6, Ole e 6.0101, Ole e 7, Ole e 7.0101, Ole e 8, Ole e 8.0101, Ole e 9, Ole e 9.0101), Ommastrephes spp (Omm b 1, Omm b 1.0101), Oncorhynchus spp (Onc ke 1, Onc ke 18 kD, Onc ke alpha2I, Onc ke Vitellogenin, Onc m 1, Onc m 1.0101, Onc m 1.0201, Onc m alpha2I, Onc m Protamine, Onc m Vitellogenin, Onc ma 1, Onc ma FPA, Onc ma FSA, Onc ma TPI, Onc n 1), Onchocerca spp (Onc o 3, Onc o 3.0101), Oncorhynchus spp (Onc ts 1), Onchocerca spp (Onc v 3, Onc v 3.0101), Oratosquilla spp (Ora o 1, Ora o 1.0101), Oreochromis spp (Ore a 1, Ore mo 1, Ore mo 2, Ore mo FPA, Ore mo SCAF7145, Ore ni 1, Ore ni 18kD, Ore ni 45kD), Ornithonyssus spp (Orn sy 10, Orn sy 10.0101, Orn sy 10.0102), Oryctolagus spp (Ory c 1, Ory c 1.0101, Ory c 2, Ory c Casein, Ory c Phosvitin, Ory c RSA), Oryza spp (Ory s 1, Ory s 1.0101, Ory s 11, Ory s 12, Ory s 12.0101, Ory s 13, Ory s 14, Ory s 17kD, Ory s 19kD, Ory s 2, Ory s 23, Ory s 3, Ory s 7, Ory s aA_TI, Ory s GLP52, Ory s GLP63, Ory s Glyoxalase I, Ory s NRA), Ostrya spp (Ost c 1, Ost c 1.0101), Ovis spp (Ovi a ALA, Ovi a BLG, Ovi a Casein, Ovi a Casein alphaS1, Ovi a Casein alphaS2, Ovi a Casein beta, Ovi a Casein kappa, Ovi a Phosvitin, Ovi a SSA), Pachycondyla spp (Pac c 3), Pagrus spp (Pag m 1, Pag pa 1), Pampus spp (Pam ar 1, Pam c 1), Pandalus spp (Pan b 1, Pan b 1.0101), Pangasius spp (Pan bo 1), Pandalus spp (Pan e 1, Pan e 1.0101, Pan e 4), Panulirus spp (Pan h 1, Pan hy 1), Pangasius spp (Pan hy 18kD, Pan hy 45kD), Panulirus spp (Pan j 1), Panthera spp (Pan l 1, Pan o 1, Pan p 1), Panulirus spp (Pan s 1, Pan s 1.0101), Panthera spp (Pan t 1), Pan spp (Pan tr TCTP), Papaver spp (Pap s 17kD, Pap s 2, Pap s 34kD), Papilio spp (Pap xu 7, Pap xu 7.0101, Pap xu 7.0102), Paralichthys spp (Par a 1), Parasilurus spp (Par as 1, Par c 1), Paralithodes spp (Par c 1.0101, Par c 1.0102, Par f 1), Parthenium spp (Par h 1), Parietaria spp (Par j 1, Par j 1.0101, Par j 1.0102, Par j 1.0103, Par j 1.0201, Par j 2, Par j 2.0101, Par j 2.0102, Par j 3, Par j 3.0101, Par j 3.0102, Par j 4, Par j 4.0101, Par j J1-J2), Paralichthys spp (Par le 1), Parietaria spp (Par m 1, Par o 1, Par o 1.0101), Paralichthys spp (Par ol 1, Par ol alpha2I), Parahucho spp (Par pe Vitellogenin), Passiflora spp (Pas e Chitinase, Pas e Hevein), Paspalum spp (Pas n 1, Pas n 1.0101, Pas n 13), Patinopecten spp (Pat y 1), Pediculus spp (Ped h 7, Ped h 7.0101), Penaeus spp (Pen a 1, Pen a 1.0101, Pen a 1.0102, Pen a 1.0102 (103-117), Pen a 1.0102 (109-123), Pen a 1.0102 (1-15), Pen a 1.0102 (115-129), Pen a 1.0102 (121-135), Pen a 1.0102 (127-141), Pen a 1.0102 (13-27), Pen a 1.0102 (133-147), Pen a 1.0102 (139-153), Pen a 1.0102 (145-159)), Farfantepenaeus spp (Pen a 1.0102 (151-165)), Penaeus spp (Pen a 1.0102 (157-171), Pen a 1.0102 (163-177), Pen a 1.0102 (169-183), Pen a 1.0102 (175-189), Pen a 1.0102 (181-195), Pen a 1.0102 (187-201), Pen a 1.0102 (193-207), Pen a 1.0102 (19-33), Pen a 1.0102 (199-213), Pen a 1.0102 (205-219), Pen a 1.0102 (211-225), Pen a 1.0102 (217-231), Pen a 1.0102 (223-237), Pen a 1.0102 (229-243)), Farfantepenaeus spp (Pen a 1.0102 (235-249)), Penaeus spp (Pen a 1.0102 (241-255), Pen a 1.0102 (247-261), Pen a 1.0102 (253-267), Pen a 1.0102 (25-39), Pen a 1.0102 (259-273), Pen a 1.0102 (265-279), Pen a 1.0102 (270-284), Pen a 1.0102 (31-45), Pen a 1.0102 (37-51), Pen a 1.0102 (43-57), Pen a 1.0102 (49-63)), Farfantepenaeus spp (Pen a 1.0102 (55-69)), Penaeus spp (Pen a 1.0102 (61-75), Pen a 1.0102 (67-81), Pen a 1.0102 (7-21), Pen a 1.0102 (73-87), Pen a 1.0102 (79-93), Pen a 1.0102 (85-99), Pen a 1.0102 (91-105), Pen a 1.0102 (97-111), Pen a 1.0103), Penicillium spp (Pen b 13, Pen b 13.0101, Pen b 26, Pen b 26.0101, Pen c 1, Pen c 13, Pen c 13.0101, Pen c 18, Pen c 19, Pen c 19.0101, Pen c 2, Pen c 22, Pen c 22.0101, Pen c 24, Pen c 24.0101, Pen c 3, Pen c 3.0101, Pen c 30, Pen c 30.0101, Pen c 32, Pen c 32.0101, Pen c MnSOD, Pen ch 13, Pen ch 13.0101, Pen ch 18, Pen ch 18.0101, Pen ch 20, Pen ch 20.0101, Pen ch 31, Pen ch 31.0101, Pen ch 33, Pen ch 33.0101, Pen ch 35, Pen ch 35.0101, Pen ch MnSOD), Penaeus spp (Pen i 1, Pen i 1.0101, Pen m 1, Pen m 1.0101, Pen m 1.0102, Pen m 2, Pen m 2.0101, Pen m 3, Pen m 3.0101, Pen m 4, Pen m 4.0101, Pen m 6, Pen m 6.0101), Penicillium spp (Pen o 18, Pen o 18.0101), Penaeus spp (Pena o 1, Pena o 1.0101), Periplaneta spp (Per a 1, Per a 1.0101, Per a 1.0102, Per a 1.0103, Per a 1.0104, Per a 1.0105, Per a 1.0201, Per a 10, Per a 10.0101, Per a 2, Per a 3, Per a 3.0101, Per a 3.0201, Per a 3.0202, Per a 3.0203, Per a 4, Per a 5, Per a 6, Per a 6.0101, Per a 7, Per a 7.0101, Per a 7.0102, Per a 7.0103, Per a 9, Per a 9.0101, Per a Cathepsin, Per a FABP, Per a Trypsin, Per f 1, Per f 7, Per f 7.0101), Perna spp (Per v 1), Persea spp (Pers a 1, Pers a 1.0101, Pers a 4), Petroselinum spp (Pet c 1, Pet c 2, Pet c 3), Phalaris spp (Pha a 1, Pha a 1.0101, Pha a 5, Pha a 5.0101, Pha a 5.02, Pha a 5.03, Pha a 5.04), Phaseolus spp (Pha v 3, Pha v 3.0101, Pha v 3.0201, Pha v aAI, Pha v aAI.0101, Pha v Chitinase, Pha v PHA, Pha v Phaseolin), Phleum spp (Phl p 1, Phl p 1.0101, Phl p 1.0102, Phl p 11, Phl p 11.0101, Phl p 12, Phl p 12.0101, Phl p 12.0102, Phl p 12.0103, Phl p 13, Phl p 13.0101, Phl p 2, Phl p 2.0101, Phl p 3, Phl p 3.0101, Phi p 3.0102, Phl p 4, Phl p 4.0101, Phl p 4.0102, Phl p 4.0201, Phl p 4.0202, Phl p 4.0203, Phl p 4.0204, Phl p 5, Phl p 5.0101, Phl p 5.0102, Phl p 5.0103, Phl p 5.0104, Phl p 5.0105, Phl p 5.0106, Phl p 5.0107, Phl p 5.0108, Phl p 5.0109, Phl p 5.0201, Phl p 5.0202, Phl p 5.0203, Phl p 5.0204, Phl p 5.0205, Phl p 5.0206, Phl p 5.0207, Phl p 6, Phl p 6.0101, Phl p 6.0102, Phl p 7, Phl p 7.0101, Phl p P1-P2-P5-P6, Phl p P2-P6, Phl p P5-P1, Phl p P6-P2), Phoenix spp (Pho d 2, Pho d 2.0101, Pho d 40kD, Pho d 90kD), Phodopus spp (Pho s 21kD), Phoma spp (Pho t 1), Phragmites spp (Phr a 1, Phr a 12, Phr a 13, Phr a 4, Phr a 5), Phytolacca spp (Phy a RIP), Pimpinella spp (Pim a 1, Pim a 2), Pinna spp (Pin a 1), Piper spp (Pip n 14kD, Pip n 28kD), Pisum spp (Pis s 1, Pis s 1.0101, Pis s 1.0102, Pis s 2, Pis s 2.0101, Pis s 5, Pis s Agglutinin, Pis s Albumin), Pistacia spp (Pis v 1, Pis v 1.0101, Pis v 2, Pis v 2.0101, Pis v 2.0201, Pis v 3, Pis v 3.0101, Pis v 4, Pis v 4.0101, Pis v 5, Pis v 5.0101), Platanus spp (Pla a 1, Pla a 1.0101, Pla a 2, Pla a 2.0101, Pla a 3, Pla a 3.0101, Pla a 8), Platichthys spp (Pla f 1), Plantago spp (Pla l 1, Pla l 1.0101, Pla l 1.0102, Pla l 1.0103, Pla l Cytochrome C), Platanus spp (Pla oc 1, Pla or 1, Pla or 1.0101, Pla or 2, Pla or 2.0101, Pla or 3, Pla or 3.0101, Pla or 4, Pla or CyP, Pla r 1), Plectropomus spp (Ple ar 1), Pleospora spp (Ple h 1), Plectropomus spp (Ple le 1), Plodia spp (Plo i 1, Plo i 1.0101, Plo i 2, Plo i 2.0101), Poa spp (Poa p 1, Poa p 1.0101, Poa p 10, Poa p 12, Poa p 13, Poa p 2, Poa p 4, Poa p 5, Poa p 5.0101, Poa p 6, Poa p 7), Polistes spp (Pol a 1, Pol a 1.0101, Pol a 2, Pol a 2.0101, Pol a 5, Pol a 5.0101, Pol d 1, Pol d 1.0101, Pol d 1.0102, Pol d 1.0103, Pol d 1.0104, Pol d 4, Pol d 4.0101, Pol d 5, Pol d 5.0101, Pol e 1, Pol e 1.0101, Pol e 2, Pol e 4, Pol e 4.0101, Pol e 5, Pol e 5.0101, Pol f 5, Pol f 5.0101, Pol g 1, Pol g 1.0101, Pol g 2, Pol g 4, Pol g 5, Pol g 5.0101, Pol he MLT, Pol m 5, Pol m 5.0101), Polypedilum spp (Pol n 1), Pollicipes spp (Pol po 1), Pollachius spp (Pol vi 1), Polybia spp (Poly p 1, Poly p 1.0101, Poly p 2, Poly p 5, Poly s 5, Poly s 5.0101), Pomatomus spp (Pom sa 1), Pongo spp (Pon ab HSA), Pontastacus spp (Pon l 4, Pon l 4.0101, Pon l 7, Pon l 7.0101), Portunus spp (Por s 1, Por s 1.0101, Por s 1.0102, Por tr 1, Por tr 1.0101), Protortonia spp (Pro ca 38kD), Procumbarus spp (Pro cl 1, Pro cl 1.0101, Pro cl 21kD), Prosopis spp (Pro j 20kD), Prunus spp (Pru ar 1, Pru ar 1.0101, Pru ar 3, Pru ar 3.0101, Pru av 1, Pru av 1.0101, Pru av 1.0201, Pru av 1.0202, Pru av 1.0203, Pru av 2, Pru av 2.0101, Pru av 3, Pru av 3.0101, Pru av 4, Pru av 4.0101, Pru c 1, Pru d 1, Pru d 2, Pru d 3, Pru d 3.0101, Pru d 4, Pru du 1, Pru du 2, Pru du 2S Albumin, Pru du 3, Pru du 3.0101, Pru du 4, Pru du 4.0101, Pru du 4.0102, Pru du 5, Pru du 5.0101, Pru du 6, Pru du 6.0101, Pru du 6.0201, Pru du Conglutin, Pru p 1, Pru p 1.0101, Pru p 2, Pru p 2.0101, Pru p 2.0201, Pru p 2.0301, Pru p 3, Pru p 3.0101, Pru p 3.0102, Pru p 4, Pru p 4.0101, Pru p 4.0201, Pru sa 3), Psilocybe spp (Psi c 1, Psi c 1.0101, Psi c 2, Psi c 2.0101), Psoroptes spp (Pso o 1, Pso o 10, Pso o 10.0101, Pso o 11, Pso o 13, Pso o 14, Pso o 2, Pso o 21, Pso o 3, Pso o 5, Pso o 7), Puma spp (Pum c 1), Punica spp (Pun g 3), Pyrus spp (Pyr c 1, Pyr c 1.0101, Pyr c 3, Pyr c 3.0101, Pyr c 4, Pyr c 4.0101, Pyr c 5, Pyr c 5.0101, Pyr py 2), Quercus spp (Que a 1, Que a 1.0101, Que a 1.0201, Que a 1.0301, Que a 1.0401, Que a 2, Que a 4), Rachycentron spp (Rac ca 1), Rana spp (Ran e 1, Ran e 1.0101, Ran e 2, Ran e 2.0101), Ranina spp (Ran ra 1), Rangifer spp (Ran t BLG), Rattus spp (Rat n 1, Rat n 1.0101, Rat n Casein, Rat n Gelatin, Rat n IgG, Rat n Phosvitin, Rat n RSA, Rat n Transferrin), Rhizomucor spp (Rhi m AP), Rhizopus spp (Rhi nv Lipase, Rhi o Lipase), Rhomboplites spp (Rho au 1), Rhodotorula spp (Rho m 1, Rho m 1.0101, Rho m 2, Rho m 2.0101), Ricinus spp (Ric c 1, Ric c 1.0101, Ric c 2, Ric c 3, Ric c 8, Ric c RIP), Rivulus spp (Riv ma 1), Robinia spp (Rob p 2, Rob p 4, Rob p Glucanase), Rosa spp (Ros r 3), Roystonea spp (Roy e 2), Rubus spp (Rub i 1, Rub i 1.0101, Rub i 3, Rub i 3.0101, Rub i Chitinase, Rub i CyP), Saccharomyces spp (Sac c Carboxypeptidase Y, Sac c CyP, Sac c Enolase, Sac c Glucosidase, Sac c Invertase, Sac c MnSOD, Sac c P2, Sac c Profilin), Salvelinus spp (Sal f 1), Salsola spp (Sal k 1, Sal k 1.0101, Sal k 1.0201, Sal k 1.0301, Sal k 1.0302, Sal k 2, Sal k 2.0101, Sal k 3, Sal k 3.0101, Sal k 4, Sal k 4.0101, Sal k 4.0201, Sal k 5, Sal k 5.0101), Salvelinus spp (Sal le Vitellogenin), Salmo spp (Sal s 1, Sal s 1.0101, Sal s 1.0201, Sal s 2, Sal s 2.0101, Sal s Gelatin), Sambucus spp (Sam n 1), Sander spp (San lu 1), Saponaria spp (Sap o RIP), Sardinops spp (Sar m 1), Sarkidiornis spp (Sar ml 1), Sardina spp (Sar p 1), Sarcoptes spp (Sar s 1, Sar s 14, Sar s 3, Sar s GST, Sar s PM), Sardinops spp (Sar sa 1, Sar sa 1.0101), Schistosoma spp (Sch j GST, Sch j PM, Sch j Sj22, Sch j Sj67, Sch ma Sm20, Sch ma Sm21, Sch ma Sm22, Sch ma Sm31), Sciaenops spp (Sci oc 1), Scomber spp (Sco a 1), Scombermorus spp (Sco ca 1), Scomberomorus spp (Sco g 1), Scomber spp (Sco j 1, Sco ma 1, Sco s 1), Scolopendra spp (Sco y 7, Sco y 7.0101), Scylla spp (Scy o 1, Scy o 1.0101, Scy o 2, Scy pa 1, Scy pa 2, Scy s 1, Scy s 1.0101, Scy s 2), Sebastes spp (Seb fa 1, Seb in 1, Seb m 1, Seb m 1.0101, Seb m 1.0201), Secale spp (Sec c 1, Sec c 12, Sec c 13, Sec c 2, Sec c 20, Sec c 20.0101, Sec c 20.0201, Sec c 28, Sec c 3, Sec c 4, Sec c 4.0101, Sec c 4.0201, Sec c 5, Sec c 5.0101, Sec c aA_TI, Sec c aA_TI.0101), Senecio spp (Sen j MDH, Sen j PL), Sepia spp (Sep e 1, Sep e 1.0101), Sepioteuthis spp (Sep l 1, Sep l 1.0101), Sepia spp (Sep m 1), Seriola spp (Ser d 1, Ser la 1), Sergestes spp (Ser lu 1), Seriola spp (Ser q 1, Ser ri 1), Sesamum spp (Ses i 1, Ses i 1.0101, Ses i 2, Ses i 2.0101, Ses i 3, Ses i 3.0101, Ses i 4, Ses i 4.0101, Ses i 5, Ses i 5.0101, Ses i 6, Ses i 6.0101, Ses i 7, Ses i 7.0101, Ses i 8), Shigella spp (Shi bo GST, Shi dy GST), Simulia spp (Sim vi 1, Sim vi 2, Sim vi 3, Sim vi 4, Sim vi 70kD), Sinapis spp (Sin a 1, Sin a 1.0101, Sin a 1.0104, Sin a 1.0105, Sin a 1.0106, Sin a 1.0107, Sin a 1.0108, Sin a 2, Sin a 2.0101, Sin a 3, Sin a 3.0101, Sin a 4, Sin a 4.0101), Sinonovacula spp (Sin c 1, Sin c 1.0101), Solenopsis spp (Sol g 2, Sol g 2.0101, Sol g 3, Sol g 3.0101, Sol g 4, Sol g 4.0101, Sol g 4.0201, Sol i 1, Sol i 1.0101, Sol i 2, Sol i 2.0101, Sol i 3, Sol i 3.0101, Sol i 4, Sol i 4.0101), Solenocera spp (Sol me 1), Solenopsis spp (Sol r 1, Sol r 2, Sol r 2.0101, Sol r 3, Sol r 3.0101, Sol s 2, Sol s 2.0101, Sol s 3, Sol s 3.0101, Sol s 4), Solea spp (Sol so 1, Sol so TPI), Solanum spp (Sola t 1, Sola t 1.0101, Sola t 2, Sola t 2.0101, Sola t 3, Sola t 3.0101, Sola t 3.0102, Sola t 4, Sola t 4.0101, Sola t 8, Sola t Glucanase), Sorghum spp (Sor b 1, Sor h 1, Sor h 1.0101, Sor h 12, Sor h 7), Sparus spp (Spa a 1), Sphyrna spp (Sph ti 1), Spirulina spp (Spi mx beta_Phycocyanin), Spinacia spp (Spi o 2, Spi o RuBisCO), Squilla spp (Squ ac 1, Squ ac 1.0101, Squ o 1, Squ o 1.0101), Staphylococcus spp (Sta a FBP, Sta a SEA, Sta a SEB, Sta a SEC, Sta a SED, Sta a SEE, Sta a TSST), Stachybotrys spp (Sta c 3, Sta c 3.0101, Sta c Cellulase, Sta c Hemolysin, Sta c SchS34, Sta c Stachyrase A), Stemphylium spp (Ste b 1, Ste c 1, Ste v 1), Stolephorus spp (Sto i 1), Struthio spp (Str c 1, Str c 2, Str c 3), Streptococcus spp (Str dy Streptokinase), Streptomyces spp (Str g Pronase), Streptococcus spp (Str pn PspC), Strongylocentrotus spp (Str pu 18kD, Str pu Vitellogenin), Streptococcus spp (Str py SPEA, Str py SPEC, Str py Streptokinase), Strongyloides spp (Str st 45kD), Streptomyces spp (Str v PAT), Styela spp (Sty p 1), Suidasia spp (Sui m 1, Sui m 13, Sui m 2, Sui m 3, Sui m 5, Sui m 5.01, Sui m 5.02, Sui m 5.03, Sui m 6, Sui m 7, Sui m 8, Sui m 9), Sus spp (Sus s ACTH, Sus s ALA, Sus s Amylase, Sus s BLG, Sus s Casein, Sus s Casein alphaS1, Sus s Casein alphaS2, Sus s Casein beta, Sus s Casein kappa, Sus s Gelatin, Sus s HG, Sus s Insulin, Sus s Lipase, Sus s Pepsin, Sus s Phosvitin, Sus s PRVB, Sus s PSA, Sus s TCTP), Syntelopodeuma spp (Syn y 7, Syn y 7.0101), Syringa spp (Syr v 1, Syr v 1.0101, Syr v 1.0102, Syr v 1.0103, Syr v 2, Syr v 3, Syr v 3.0101), Tabanus spp (Tab y 1, Tab y 1.0101, Tab y 2, Tab y 2.0101, Tab y 5, Tab y 5.0101), Tadorna spp (Tad ra 1), Talaromyces spp (Tal st 22, Tal st 3, Tal st 8), Taraxacum spp (Tar o 18kD), Taxodium spp (Tax d 2), Tegenaria spp (Teg d Hemocyanin), Teladorsagia spp (Tel ci 3), Thaumetopoea spp (Tha p 1, Tha p 1.0101, Tha p 2, Tha p 2.0101), Theragra spp (The c 1), Thermomyces spp (The l Lipase, The sp Lipase, The sp Xylanase), Thunnus spp (Thu a 1, Thu a 1.0101, Thu a Collagen, Thu al 1, Thu at 1, Thu o 1, Thu o Collagen), Thuja spp (Thu oc 3, Thu p 1), Thunnus spp (Thu t 1, Thu to 1), Thyrsites spp (Thy at 1), Thyrophygus spp (Thy y 7, Thy y 7.0101), Todarodes spp (Tod p 1, Tod p 1.0101, Tod p 1.0102), Toxoptera spp (Tox c 7, Tox c 7.0101), Toxocara spp (Tox ca TES120, Tox ca TES26, Tox ca TES30), Toxoplasma spp (Tox g HSP70), Trachypenaeus spp (Tra c 1), Trachinotus spp (Tra ca 1), Trachurus spp (Tra j 1, Tra j Gelatin, Tra tr Gelatin), Triticum spp (Tri a 1, Tri a 10kD, Tri a 12, Tri a 12.0101, Tri a 12.0102, Tri a 12.0103, Tri a 12.0104, Tri a 13, Tri a 14, Tri a 14.0101, Tri a 14.0201, Tri a 15, Tri a 15.0101, Tri a 18, Tri a 18.0101, Tri a 19, Tri a 19.0101, Tri a 2, Tri a 21, Tri a 21.0101, Tri a 23kd, Tri a 25, Tri a 25.0101, Tri a 26, Tri a 26.0101, Tri a 27, Tri a 27.0101, Tri a 28, Tri a 28.0101, Tri a 29, Tri a 29.0101, Tri a 29.0201, Tri a 3, Tri a 30, Tri a 30.0101, Tri a 31, Tri a 31.0101, Tri a 32, Tri a 32.0101, Tri a 33, Tri a 33.0101, Tri a 34, Tri a 34.0101, Tri a 35, Tri a 35.0101, Tri a 36, Tri a 36.0101, Tri a 37, Tri a 37.0101, Tri a 4, Tri a 4.0101, Tri a 4.0201, Tri a 5, Tri a 7, Tri a aA_SI, Tri a alpha_Gliadin, Tri a bA, Tri a Bd36K, Tri a beta_Gliadin, Tri a Chitinase, Tri a CM16, Tri a DH, Tri a Endochitinase, Tri a gamma_Gliadin, Tri a Germin, Tri a Gliadin, Tri a GST, Tri a LMW Glu, Tri a LMW-GS B16, Tri a LMW-GS P42, Tri a LMW-GS P73, Tri a LTP2, Tri a omega2_Gliadin, Tri a Peroxidase, Tri a Peroxidase 1, Tri a SPI, Tri a TLP, Tri a Tritin, Tri a XI), Tritirachium spp (Tri al Proteinase K), Tribolium spp (Tri ca 17, Tri ca 17.0101, Tri ca 7, Tri ca 7.0101), Trichostrongylus spp (Tri co 3, Tri co 3.0101), Trichophyton spp (Tri eq 4), Trigonella spp (Tri fg 1, Tri fg 2, Tri fg 3, Tri fg 4), Trichosanthes spp (Tri k RIP), Trichiurus spp (Tri le 1), Triticum spp (Tri m Peroxidase), Trichophyton spp (Tri me 2, Tri me 4), Trisetum spp (Tri p 1, Tri p 5), Trichinella spp (Tri ps 3, Tri ps 3.0101), Trichophyton spp (Tri r 2, Tri r 2.0101, Tri r 4, Tri r 4.0101), Trichoderma spp (Tri rs Cellulase), Triticum spp (Tri s 14), Trichophyton spp (Tri sc 2, Tri sc 4, Tri so 2), Trichinella spp (Tri sp 3, Tri sp 3.0101, Tri sp 3.0102, Tri sp 3.0103, Tri sp 3.0104, Tri sp 3.0105, Tri sp 3.0106), Trichophyton spp (Tri t 1, Tri t 1.0101, Tri t 4, Tri t 4.0101), Triticum spp (Tri td 14, Tri td aA_TI), Trichoderma spp (Tri v Cellulase), Trichophyton spp (Tri ve 4), Triatoma spp (Tria p 1, Tria p 1.0101), Triplochiton spp (Trip s 1), Turbo spp (Tur c 1, Tur c PM), Tyrophagus spp (Tyr p 1, Tyr p 10, Tyr p 10.0101, Tyr p 10.0102, Tyr p 13, Tyr p 13.0101, Tyr p 2, Tyr p 2.0101, Tyr p 24, Tyr p 24.0101, Tyr p 3, Tyr p 3.0101, Tyr p 4, Tyr p 5, Tyr p 5.01, Tyr p 5.02, Tyr p 5.03, Tyr p 7, Tyr p alpha Tubulin), Ulocladium spp (Ulo a 1, Ulo at 1, Ulo b 1, Ulo c 1, Ulo co 1, Ulo cu 1, Ulo mu 1, Ulo ob 1, Ulo se 1, Ulo su 1, Ulo tu 1), Uncia spp (Unc u 1), Urophycis spp (Uro te 1), Vaccinium spp (Vac m 3), Varroa spp (Var j 13kD), Venerupis spp (Ven ph 1, Ven ph 1.0101), Vespula spp (Ves f 1, Ves f 2, Ves f 5, Ves f 5.0101, Ves g 1, Ves g 2, Ves g 5, Ves g 5.0101, Ves m 1, Ves m 1.0101, Ves m 2, Ves m 2.0101, Ves m 5, Ves m 5.0101, Ves m MLT, Ves p 1, Ves p 2, Ves p 5, Ves p 5.0101, Ves s 1, Ves s 1.0101, Ves s 2, Ves s 5, Ves s 5.0101, Ves v 1, Ves v 1.0101, Ves v 2, Ves v 2.0101, Ves v 2.0201, Ves v 3, Ves v 3.0101, Ves v 5, Ves v 5.0101, Ves v 5-Pol a 5, Ves vi 5, Ves vi 5.0101), Vespa spp (Vesp c 1, Vesp c 1.0101, Vesp c 2, Vesp c 5, Vesp c 5.0101, Vesp c 5.0102, Vesp m 1, Vesp m 1.0101, Vesp m 5, Vesp m 5.0101, Vesp ma 1, Vesp ma 2, Vesp ma 5, Vesp ma MLT, Vesp v MLT), Vigna spp (Vig r 1, Vig r 1.0101, Vig r 17kD, Vig r 5, Vig r 8S Globulin, Vig r Albumin, Vig r beta-Conglycinin), Vitis spp (Vit v 1, Vit v 1.0101, Vit v 4, Vit v 5, Vit v Glucanase, Vit v TLP), Xiphias spp (Xip g 1, Xip g 1.0101, Xip g 25kD), Zea spp (Zea m 1, Zea m 1.0101, Zea m 11, Zea m 12, Zea m 12.0101, Zea m 12.0102, Zea m 12.0103, Zea m 12.0104, Zea m 12.0105, Zea m 13, Zea m 14, Zea m 14.0101, Zea m 14.0102, Zea m 2, Zea m 20S, Zea m 22, Zea m 25, Zea m 25.0101, Zea m 27kD Zein, Zea m 3, Zea m 4, Zea m 5, Zea m 50kD Zein, Zea m 7, Zea m Chitinase, Zea m G1, Zea m G2, Zea m PAO, Zea m Zm13), Zeus spp (Zeu fa 1), Ziziphus spp (Ziz m 1, Ziz m 1.0101), Zoarces spp (Zoa a ISP III), Zygophyllum spp (Zyg f 2).

In this context, the terms in brackets indicate the particular preferred allergenic antigens (allergens) from the particular source.

Most preferably the allergenic antigen is preferably derived from a source (e.g. a plant (e.g. grass or a tree), a natural product (e.g. milk, nuts etc.), a fungal source (e.g. Aspergillus) or a bacterial source or from an animal source or animal poison (e.g. cat, dog, venom of bees etc.), preferably selected from the list consisting of grass pollen (e.g. pollen of rye), tree pollen (e.g. pollen of hazel, birch, alder, ash), flower pollen, herb pollen (e.g. pollen of mugwort), dust mite (e.g. Der f 1, Der p 1, Eur m 1, Der m 1 Der f 2, Der p 2, Eur m 2, Tyr p 2, Lep d 2), mold (e.g. allergens of Acremonium, Aspergillus, Cladosporium, Fusarium, Mucor, Penicillium, Rhizopus, Stachybotrys, Trichoderma, or Alternaria), animals (e.g Fel d1, Fel d 2, Fel d3, or Fel d4 of cats), food (e.g. allergens of fish (e.g. bass, cod, flounder), seafood (e.g. crab, lobster, shrimps), egg, wheat, nuts (e.g. peanuts, almonds, cashews, walnuts), soya, milk, etc.) or insect venom (e.g. allergens from the venom of wasps, bees, hornets, ants, mosquitos, or ticks).

### Autoimmune self-antigens:

Autoimmune self-antigens, i.e. antigens associated with autoimmune disease or autoantigens, may be associated with an autoimmune disease affecting at least one or more of the following organ systems: the circulatory system, the digestive system, the endocrine system, the excretory system, the immune system, the integumentary system, the muscular system, the nervous system, the reproductive system, the respiratory system, the skeletal system, preferably with the the cardiovascular system, the neuroendocrine system, the musculoskeletal system or gastrointestinal system. Therein the circulatory system is the organ system which enables pumping and channeling blood to and from the body and lungs with heart, blood and blood vessels. The digestive system enables digestion and processing food with salivary glands, esophagus, stomach, liver, gallbladder, pancreas, intestines, colon, rectum and anus. The endocrine system enables communication within the body using hormones made by endocrine glands such as the hypothalamus, pituitary or pituitary gland, pineal body or pineal gland, thyroid gland, parathyroid gland and adrenal glands. The excretory system comprises kidneys, ureters, bladder and urethra and is involved in fluid balance, electrolyte balance and excretion of urine. The immune system comprises structures involved in the transfer of lymph between tissues and the blood stream, the lymph and the nodes and vessels wich may be responsible for transport of cellular and humoral components of the immune system. It is responsible for defending against disease-causing agents and comprises amonstg others leukocytes, tonsils, adenoids, thymus and spleen. The integumentary system comprises skin, hair and nails. The muscular system enables movement with muscles together with the skeletal system which comprises bones, cartilage, ligaments and tendons and provides structural support. The nervous system is responsible for collecting, transferring and processing information and comprises the brain, spinal cord and nerves. The reproductive system comprises the sex organs, such as ovaries, fallopian tubes, uterus, vagina, mammary glands, testes, vas deferens, seminal vesicles, prostate and penis. The respiratory system comprises the organs used for breathing, the pharynx, larynx, trachea, bronchi, lungs and diaphragm and acts together with the circulation system.

Autoimmune self-antigens (antigens associated with autoimmune disease or autoantigens) are selected from autoantigens asscociated with autoimmune diseases selected from Addison disease (autoimmune adrenalitis, Morbus Addison), alopecia areata, Addison's anemia (Morbus Biermer), autoimmune hemolytic anemia (AIHA), autoimmune hemolytic anemia (AIHA) of the cold type (cold hemagglutinine disease, cold autoimmune hemolytic anemia (AIHA) (cold agglutinin disease), (CHAD)), autoimmune hemolytic anemia (AIHA) of the warm type (warm AIHA, warm autoimmune haemolytic anemia (AIHA)), autoimmune hemolytic Donath-Landsteiner anemia (paroxysmal cold hemoglobinuria), antiphospholipid syndrome (APS), atherosclerosis, autoimmune arthritis, arteriitis temporalis, Takayasu arteriitis (Takayasu's disease, aortic arch disease), temporal arteriitis/giant cell arteriitis, autoimmune chronic gastritis, autoimmune infertility, autoimmune inner ear disease (AIED), Basedow's disease (Morbus Basedow), Bechterew's disease (Morbus Bechterew, ankylosing spondylitis, spondylitis ankylosans ), Behcet's syndrome (Morbus Behcet), bowel disease including autoimmune inflammatory bowel disease (including colitis ulcerosa (Morbus Crohn, Crohn's disease), cardiomyopathy, particularly autoimmune cardiomyopathy, idiopathic dilated cardiomyopathy (DCM), celiac sprue dermatitis (gluten mediated enteropathia), chronic fatigue immune dysfunction syndrome (CFIDS), chronic inflammatory demyelinating polyneuropathy (CIDP), chronic polyarthritis, Churg-Strauss syndrome, cicatricial pemphigoid, Cogan syndrome, CREST syndrome (syndrom with Calcinosis cutis, Raynaud phenomenon, motility disorders of the esophagus, sklerodaktylia and teleangiectasia), Crohn's disease (Morbus Crohn, colitis ulcerosa), dermatitis herpetiformis during, dermatologic autoimmune diseases, dermatomyositis, Diabetes, Diabetes mellitus Type 1 (type I diabetes, insuline dependent Diabetes mellitus), Diabetes mellitus Type 2 (type II diabetes), essential mixed cryoglobulinemia, essential mixed cryoglobulinemia, fibromyalgia, fibromyositis, Goodpasture syndrome (anti-GBM mediated glomerulonephritis), graft versus host disease, Guillain-Barré syndrome (GBM, Polyradikuloneuritis), haematologic autoimmune diseases, Hashimoto thyroiditis, hemophilia, acquired hemophilia, hepatitis, autoimmune hepatitis, particularly autoimmune forms of chronic hepatitis, idiopathic pulmonary fibrosis (IPF), idiopathic thrombocytopenic purpura, Immuno-thrombocytopenic purpura (Morbus Werlhof; ITP), IgA nephropathy, infertility, autoimmune infertility, juvenile rheumatoid arthritis (Morbus Still, Still syndrome), Lambert-Eaton syndrome, lichen planus, lichen sclerosus, lupus erythematosus, systemic lupus erythematosus (SLE), lupus erythematosus (discoid form), Lyme arthritis (Lyme disease, borrelia arthritis), Ménierè's disease (Morbus Ménierè); mixed connective tissue disease (MCTD) , multiple sclerosis (MS, encephalomyelitis disseminate, Charcot's disease), Myasthenia gravis (myasthenia, MG), myosits, polymyositis, neural autoimmune diseases, neurodermitis, pemphigus vulgaris, bullous pemphigoid, scar forming pemphigoid; polyarteriitis nodosa (periarteiitis nodosa), polychondritis (panchondritis), polyglandular (autoimmune) syndrome (PGA syndrome, Schmidt's syndrome), Polymyalgia rheumatica, primary agammaglobulinemia, primary biliary cirrhosis PBC, primary autoimmune cholangitis), progressive systemic sclerosis (PSS), Psoriasis, Psoriasis vulgaris, Raynaud's phenomena, Reiter's syndrome (Morbus Reiter, urethral conjunctive synovial syndrome)), rheumatoid arthritis (RA, chronic polyarthritis, rheumatic disease of the joints, rheumatic fever), sarcoidosis (Morbus Boeck, Besnier-Boeck-Schaumann disease), stiff-man syndrome, Sclerodermia, Scleroderma, Sjögren's syndrome, sympathetic ophtalmia; Transient gluten intolerance, transplanted organ rejection, uveitis, autoimmune uveiitis, Vasculitis, Vitiligo, (leucoderma, piebold skin), and Wegner's disease (Morbus Wegner, Wegner's granulomatosis).

These and other proteins acting as autoimmune self-antigens are understood to be therapeutic, as they are meant to treat the subject, in particular a mammal, more particularly a human being, by vaccinating with a self-antigen which is expressed by the mammal, e.g. the human, itself and which triggers an undesired immune response, which is not raised in a healthy subject. Accordingly, such proteins acting as self-antigens are typically of mammalian, in particular human origin.

Particularly preferred in this context are autoimmune self-antigens (autoantigens) selected from:
- myelin basic protein (MBP), proteolipid protein (PLP), and myelin oligodendrocyte glycoprotein (MOG), in each case associated with multiple sclerosis (MS);
- CD44, preproinsulin, proinsulin, insulin, glutamic acid decaroxylase (GAD65), tyrosine phosphatase-like insulinoma antigen 2 (IA2), zinc transporter ( (ZnT8), and heat shock protein 60 (HSP60), in each case associated with diabetes Typ I;
- interphotoreceptor retinoid-binding protein (IRBP) associated with autoimmune uveitis;
- acetylcholine receptor AchR, and insulin-like growth factor-1 receptor (IGF-1R), in each case associated with Myasthenia gravis;
- M-protein from beta-hemolytic streptocci (pseudo-autoantigen) associated with Rheumatic Fever;
- Macrophage migration inhibitory factor associated with Arthritis;
- Ro/La RNP complex, alpha- and beta-fodrin, islet cell autoantigen, poly(ADP)ribose polymerase (PARP), NuMA, NOR-90, Ro60 autoantigen, and p27 antigen, in each case associated with Sjögren's syndrome;
- Ro60 autoantigen, low-density lipoproteins, Sm antigens of the U-1 small nuclear ribonucleoprotein complex (B/B', D1, D2, D3, E, F, G), and RNP ribonucleoproteins, in each case associated with lupus erythematosus;
- oxLDL, beta(2)GPI, HSP60/65, and oxLDL/beta(2)GPI, in each case associated with Atherosclerosis;
- cardiac beta(1)-adrenergic receptor associated with idiopathic dilated cardiomyopathy (DCM);
- histidyl-tRNA synthetase (HisRS) associated with myositis;
- topoisomerase I associated with scleroderma disease.

Furthermore, in other embodiments said autoimmune self-antigen is associated with the respective autoimmune disease, like e.g. IL-17, heat shock proteins, and/or any idiotype pathogenic T cell or chemokine receptor which is expressed by immune cells involved in the autoimmune response in said autoimmune disease (such as any autoimmune diseases described herein).

Preferably, the at least one coding region of the mRNA compound comprising an mRNA sequence according to the invention comprises at least two, three, four, five, six, seven, eight or more nucleic acid sequences identical to or having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the nucleic acid sequences disclosed in the sequence listing (or respectively in Tables 1-5 or Figures 20-24 of PCT/EP2016/075843), or a fragment or variant of any one of said nucleic acid sequences.

Preferably, the mRNA sequence comprising at least one coding region as defined herein typically comprises a length of about 50 to about 20000, or 100 to about 20000 nucleotides, preferably of about 250 to about 20000 nucleotides, more preferably of about 500 to about 10000, even more preferably of about 500 to about 5000.

According to a further embodiment, the mRNA sequence according to the invention is an artificial mRNA sequence as defined herein.

According to a further embodiment, the mRNA compound comprising an mRNA sequence according to the invention is a modified mRNA sequence, preferably a modified mRNA sequence as described herein. In this context, a modification as defined herein preferably leads to a stabilization of the mRNA sequence according to the invention. More preferably, the invention thus provides a stabilized mRNA sequence comprising at least one coding region as defined herein.

According to one embodiment, the mRNA compound comprising an mRNA sequence of the present invention may thus be provided as a "stabilized mRNA sequence", that is to say as an mRNA that is essentially resistant to in vivo degradation (e.g. by an exo- or endo-nuclease). Such stabilization can be effected, for example, by a modified phosphate backbone of the mRNA of the present invention. A backbone modification in connection with the present invention is a modification in which phosphates of the backbone of the nucleotides contained in the mRNA are chemically modified. Nucleotides that may be preferably used in this connection contain e.g. a phosphorothioate-modified phosphate backbone, preferably at least one of the phosphate oxygens contained in the phosphate backbone being replaced by a sulfur atom. Stabilized mRNAs may further include, for example: non-ionic phosphate analogues, such as, for example, alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include, without implying any limitation, modifications from the group consisting of methylphosphonates, phosphoramidates and phosphorothioates (e.g. cytidine-5'-O-(1-thiophosphate)).

In the following, specific modifications are described, which are preferably capable of "stabilizing" the mRNA as defined herein.

### Chemical modifications:

The term "mRNA modification" as used herein may refer to chemical modifications comprising backbone modifications as well as sugar modifications or base modifications.

In this context, a modified mRNA (sequence) as defined herein may contain nucleotide analogues/modifications, e.g. backbone modifications, sugar modifications or base modifications. A backbone modification in connection with the present invention is a modification, in which phosphates of the backbone of the nucleotides contained in an mRNA compound comprising an mRNA sequence as defined herein are chemically modified. A sugar modification in connection with the present invention is a chemical modification of the sugar of the nucleotides of the mRNA compound comprising an mRNA sequence as defined herein. Furthermore, a base modification in connection with the present invention is a chemical modification of the base moiety of the nucleotides of the mRNA compound comprising an mRNA sequence. In this context, nucleotide analogues or modifications are preferably selected from nucleotide analogues, which are applicable for transcription and/or translation.

### Sugar Modifications:

The modified nucleosides and nucleotides, which may be incorporated into a modified mRNA compound comprising an mRNA sequence as described herein, can be modified in the sugar moiety. For example, the 2' hydroxyl group (OH) can be modified or replaced with a number of different "oxy" or "deoxy" substituents. Examples of "oxy" -2' hydroxyl group modifications include, but are not limited to, alkoxy or aryloxy (-OR, e.g., R=H, alkyl, cycloalkyl, aryl, aralkyl, heteroaryl or sugar); polyethyleneglycols (PEG), -O(CH2CH2O)nCH2CH2OR; "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH2; alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N, and O.

The sugar group can also contain one or more carbons that possess the opposite stereochemical configuration than that of the corresponding carbon in ribose. Thus, a modified mRNA can include nucleotides containing, for instance, arabinose as the sugar.

### Backbone Modifications:

The phosphate backbone may further be modified in the modified nucleosides and nucleotides, which may be incorporated into a modified mRNA compound comprising an mRNA sequence as described herein. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates), sulfur (bridged phosphorothioates) and carbon (bridged methylene-phosphonates).

### Base Modifications:

The modified nucleosides and nucleotides, which may be incorporated into a modified mRNA compound comprising an mRNA sequence as described herein can further be modified in the nucleobase moiety.

Examples of nucleobases found in mRNA include, but are not limited to, adenine, guanine, cytosine and uracil.

For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl-inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-Iodo-2'-deoxycytidine-5'-tri phosphate, 5-iodouridine-5'-triphosphate, 5-Iodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl- 1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine .

In other embodiments, modified nucleosides include 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group. In specific embodiments, a modified nucleoside is 5'-O-(1-thiophosphate)-adenosine, 5'-O-(1-thiophosphate)-cytidine, 5'-O-(1-thiophosphate)-guanosine, 5'-O-(1-thiophosphate)-uridine or 5'-O-(1-thiophosphate)-pseudouridine.

In further specific embodiments, a modified mRNA may comprise nucleoside modifications selected from 6-aza-cytidine, 2-thio-cytidine, α-thio-cytidine, Pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxythymidine, 5-methyl-uridine, Pyrrolo-cytidine, inosine, α-thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-aminopurine, Pseudo-iso-cytidine, 6-Chloro-purine, N6-methyl-adenosine, α-thio-adenosine, 8-azido-adenosine, 7-deaza-adenosine.

In a very specific embodiment of the invention, the mRNA compound does not comprise a base modification as described above.

### Lipid modification:

According to a further embodiment, a modified mRNA compound comprising an mRNA sequence as defined herein can contain a lipid modification. Such a lipid-modified mRNA typically comprises an mRNA as defined herein. Such a lipid-modified mRNA as defined herein typically further comprises at least one linker covalently linked with that mRNA, and at least one lipid covalently linked with the respective linker. Alternatively, the lipid-modified mRNA comprises at least one mRNA as defined herein and at least one (bifunctional) lipid covalently linked (without a linker) with that mRNA. According to a third alternative, the lipid-modified mRNA comprises an mRNA molecule as defined herein, at least one linker covalently linked with that mRNA, and at least one lipid covalently linked with the respective linker, and also at least one (bifunctional) lipid covalently linked (without a linker) with that mRNA. In this context, it is particularly preferred that the lipid modification is present at the terminal ends of a linear mRNA sequence.

### Sequence Modifications:

### G/C content modification:

According to another embodiment, the mRNA comprising lipid nanoparticles comprises an mRNA compound comprising an mRNA sequence, which may be modified, and thus stabilized, by modifying the guanosine/cytosine (G/C) content of the mRNA sequence, preferably of the at least one coding region of the mRNA compound comprising an mRNA sequence of the present invention.

In a particularly preferred embodiment of the present invention, the G/C content of the coding region of the mRNA compound comprising an mRNA sequence of the present invention is modified, particularly increased, compared to the G/C content of the coding region of the respective wild type mRNA, i.e. the unmodified mRNA. The amino acid sequence encoded by the mRNA is preferably not modified as compared to the amino acid sequence encoded by the respective wild type mRNA. This modification of the mRNA sequence of the present invention is based on the fact that the sequence of any mRNA region to be translated is important for efficient translation of that mRNA. Thus, the composition of the mRNA and the sequence of various nucleotides are important. In particular, sequences having an increased G (guanosine)/C (cytosine) content are more stable than sequences having an increased A (adenosine)/U (uracil) content. According to the invention, the codons of the mRNA are therefore varied compared to the respective wild type mRNA, while retaining the translated amino acid sequence, such that they include an increased amount of G/C nucleotides. In respect to the fact that several codons code for one and the same amino acid (so-called degeneration of the genetic code), the most favourable codons for the stability can be determined (so-called alternative codon usage). Depending on the amino acid to be encoded by the mRNA, there are various possibilities for modification of the mRNA sequence, compared to its wild type sequence. In the case of amino acids, which are encoded by codons, which contain exclusively G or C nucleotides, no modification of the codon is necessary. Thus, the codons for Pro (CCC or CCG), Arg (CGC or CGG), Ala (GCC or GCG) and Gly (GGC or GGG) require no modification, since no A or U is present. In contrast, codons which contain A and/or U nucleotides can be modified by substitution of other codons, which code for the same amino acids but contain no A and/or U. Examples of these are: the codons for Pro can be modified from CCU or CCA to CCC or CCG; the codons for Arg can be modified from CGU or CGA or AGA or AGG to CGC or CGG; the codons for Ala can be modified from GCU or GCA to GCC or GCG; the codons for Gly can be modified from GGU or GGA to GGC or GGG. In other cases, although A or U nucleotides cannot be eliminated from the codons, it is however possible to decrease the A and U content by using codons which contain a lower content of A and/or U nucleotides. Examples of these are: the codons for Phe can be modified from UUU to UUC; the codons for Leu can be modified from UUA, UUG, CUU or CUA to CUC or CUG; the codons for Ser can be modified from UCU or UCA or AGU to UCC, UCG or AGC; the codon for Tyr can be modified from UAU to UAC; the codon for Cys can be modified from UGU to UGC; the codon for His can be modified from CAU to CAC; the codon for Gln can be modified from CAA to CAG; the codons for Ile can be modified from AUU or AUA to AUC; the codons for Thr can be modified from ACU or ACA to ACC or ACG; the codon for Asn can be modified from AAU to AAC; the codon for Lys can be modified from AAA to AAG; the codons for Val can be modified from GUU or GUA to GUC or GUG; the codon for Asp can be modified from GAU to GAC; the codon for Glu can be modified from GAA to GAG; the stop codon UAA can be modified to UAG or UGA. In the case of the codons for Met (AUG) and Trp (UGG), on the other hand, there is no possibility of sequence modification. The substitutions listed above can be used either individually or in all possible combinations to increase the G/C content of the mRNA sequence of the present invention compared to its particular wild type mRNA (i.e. the original sequence). Thus, for example, all codons for Thr occurring in the wild type sequence can be modified to ACC (or ACG). Preferably, however, for example, combinations of the above substitution possibilities are used:
substitution of all codons coding for Thr in the original sequence (wild type mRNA) to ACC (or ACG) and
substitution of all codons originally coding for Ser to UCC (or UCG or AGC);
substitution of all codons coding for Ile in the original sequence to AUC and
substitution of all codons originally coding for Lys to AAG and
substitution of all codons originally coding for Tyr to UAC;
substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Arg to CGC (or CGG);
substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Gly to GGC (or GGG) and
substitution of all codons originally coding for Asn to AAC;
substitution of all codons coding for Val in the original sequence to GUC (or GUG) and
substitution of all codons originally coding for Phe to UUC and
substitution of all codons originally coding for Cys to UGC and
substitution of all codons originally coding for Leu to CUG (or CUC) and
substitution of all codons originally coding for Gln to CAG and
substitution of all codons originally coding for Pro to CCC (or CCG); etc.

Preferably, the G/C content of the coding region of the mRNA compound comprising an mRNA sequence of the present invention is increased by at least 7%, more preferably by at least 15%, particularly preferably by at least 20%, compared to the G/C content of the coding region of the wild type RNA, which codes for an antigen as defined herein or a fragment or variant thereof. According to a specific embodiment at least 5%, 10%, 20%, 30%, 40%, 50%, 60%, more preferably at least 70 %, even more preferably at least 80% and most preferably at least 90%, 95% or even 100% of the substitutable codons in the region coding for a peptide or protein as defined herein or a fragment or variant thereof or the whole sequence of the wild type mRNA sequence are substituted, thereby increasing the GC/content of said sequence. In this context, it is particularly preferable to increase the G/C content of the mRNA sequence of the present invention, preferably of the at least one coding region of the mRNA sequence according to the invention, to the maximum (i.e. 100% of the substitutable codons) as compared to the wild type sequence. According to the invention, a further preferred modification of the mRNA sequence of the present invention is based on the finding that the translation efficiency is also determined by a different frequency in the occurrence of tRNAs in cells. Thus, if so-called "rare codons" are present in the mRNA sequence of the present invention to an increased extent, the corresponding modified mRNA sequence is translated to a significantly poorer degree than in the case where codons coding for relatively "frequent" tRNAs are present. According to the invention, in the modified mRNA sequence of the present invention, the region which codes for a peptide or protein as defined herein or a fragment or variant thereof is modified compared to the corresponding region of the wild type mRNA sequence such that at least one codon of the wild type sequence, which codes for a tRNA which is relatively rare in the cell, is exchanged for a codon, which codes for a tRNA which is relatively frequent in the cell and carries the same amino acid as the relatively rare tRNA. By this modification, the sequence of the mRNA of the present invention is modified such that codons for which frequently occurring tRNAs are available are inserted. In other words, according to the invention, by this modification all codons of the wild type sequence, which code for a tRNA which is relatively rare in the cell, can in each case be exchanged for a codon, which codes for a tRNA which is relatively frequent in the cell and which, in each case, carries the same amino acid as the relatively rare tRNA. Which tRNAs occur relatively frequently in the cell and which, in contrast, occur relatively rarely is known to a person skilled in the art; cf. e.g. Akashi, Curr. Opin. Genet. Dev. 2001, 11(6): 660-666. The codons, which use for the particular amino acid the tRNA which occurs the most frequently, e.g. the Gly codon, which uses the tRNA, which occurs the most frequently in the (human) cell, are particularly preferred. According to the invention, it is particularly preferable to link the sequential G/C content which is increased, in particular maximized, in the modified mRNA sequence of the present invention, with the "frequent" codons without modifying the amino acid sequence of the protein encoded by the coding region of the mRNA sequence. This preferred embodiment allows provision of a particularly efficiently translated and stabilized (modified) mRNA sequence of the present invention. The determination of a modified mRNA sequence of the present invention as described above (increased G/C content; exchange of tRNAs) can be carried out using the computer program explained in WO02/098443 - the disclosure content of which is included in its full scope in the present invention. Using this computer program, the nucleotide sequence of any desired mRNA sequence can be modified with the aid of the genetic code or the degenerative nature thereof such that a maximum G/C content results, in combination with the use of codons which code for tRNAs occurring as frequently as possible in the cell, the amino acid sequence coded by the modified mRNA sequence preferably not being modified compared to the non-modified sequence. Alternatively, it is also possible to modify only the G/C content or only the codon usage compared to the original sequence. The source code in Visual Basic 6.0 (development environment used: Microsoft Visual Studio Enterprise 6.0 with Servicepack 3) is also described in WO02/098443. In a further preferred embodiment of the present invention, the A/U content in the environment of the ribosome binding site of the mRNA sequence of the present invention is increased compared to the A/U content in the environment of the ribosome binding site of its respective wild type mRNA. This modification (an increased A/U content around the ribosome binding site) increases the efficiency of ribosome binding to the mRNA. An effective binding of the ribosomes to the ribosome binding site (Kozak sequence: SEQ ID NO: 224307 or SEQ ID NO: 224308, the AUG forms the start codon) in turn has the effect of an efficient translation of the mRNA. According to a further embodiment of the present invention, the mRNA sequence of the present invention may be modified with respect to potentially destabilizing sequence elements. Particularly, the coding region and/or the 5' and/or 3' untranslated region of this mRNA sequence may be modified compared to the respective wild type mRNA such that it contains no destabilizing sequence elements, the encoded amino acid sequence of the modified mRNA sequence preferably not being modified compared to its respective wild type mRNA. It is known that, for example in sequences of eukaryotic mRNAs, destabilizing sequence elements (DSE) occur, to which signal proteins bind and regulate enzymatic degradation of mRNA in vivo. For further stabilization of the modified mRNA sequence, optionally in the region which encodes at least one peptide or protein as defined herein or a fragment or variant thereof, one or more such modifications compared to the corresponding region of the wild type mRNA can therefore be carried out, so that no or substantially no destabilizing sequence elements are contained there. According to the invention, DSE present in the untranslated regions (3'- and/or 5'-UTR) can also be eliminated from the mRNA sequence of the present invention by such modifications. Such destabilizing sequences are e.g. AU-rich sequences (AURES), which occur in 3'-UTR sections of numerous unstable mRNAs (Caput et al., Proc. Natl. Acad. Sci. USA 1986, 83: 1670 to 1674). The mRNA sequence of the present invention is therefore preferably modified compared to the respective wild type mRNA such that the mRNA sequence of the present invention contains no such destabilizing sequences. This also applies to those sequence motifs which are recognized by possible endonucleases, e.g. the sequence GAACAAG, which is contained in the 3'-UTR segment of the gene encoding the transferrin receptor (Binder et al., EMBO J. 1994, 13: 1969 to 1980). These sequence motifs are also preferably removed in the mRNA sequence of the present invention.

According to a preferred embodiment, the present invention provides mRNA comprising lipid nanoparticles wherein the mRNA comprises an mRNA sequence as defined herein comprising at least one coding region, wherein the coding region comprises or consists of any one of the (modified) RNA sequences as disclosed in the sequence listing having numeric identifier <223> which starts with "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column C" of Tables 1-5 or Figures 20-24 of PCT/EP2016/075843, or of a fragment or variant of any one of these sequences.

According to a particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza A virus, wherein the coding region comprises or consists of any one of the (modified) RNA sequences according to SEQ ID NOs: 64025-78055, 224085-224106, 192073-206103 or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza B virus, wherein the coding region comprises or consists of any one of the (modified) RNA sequences according to SEQ ID NOs: 90422-92600, 224107-224112, 218470-220648, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza A virus, wherein the coding region comprises or consists of any one of the (modified) RNA sequences according to SEQ ID NOs: 78056-90421, 224113, 224313-224317, 206104-218469, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza B virus, wherein the coding region comprises or consists of any one of the (modified) RNA sequences according to SEQ ID NOs: 92601-94528, 220649-222576 or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from glycoprotein of a Rabies virus, wherein the coding region comprises or consists of any one of the (modified) RNA sequences according to SEQ ID NOs: 94529-96036, 224271-224273, 222577-224084 or of a fragment or variant of any one of these sequences.

In a further preferred embodiment, the at least one coding region of the mRNA sequence according to the invention comprises or consists of an RNA sequence identical to or having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the (modified) RNA sequences according to SEQ ID NOs: 64025-96036, 192073-224084, or of a fragment or variant of any one of these sequences.

According to a particularly preferred embodiment, the at least one coding region of the mRNA sequence according to the invention comprises or consists of an RNA sequence having a sequence identity of at least 80% with any one of the (modified) RNA sequences according SEQ ID NOs: 64025-96036, 192073-224084, or of a fragment or variant of any one of these sequences.

### Sequences adapted to human codon usaae:

According to the invention, a further preferred modification of the mRNA compound comprising an mRNA sequence comprised in the mRNA of the mRNA comprising lipid nanoparticles of the present invention is based on the finding that codons encoding the same amino acid typically occur at different frequencies. According to the invention, in the modified mRNA compound comprising an mRNA sequence of the present invention, the coding coding region as defined herein is preferably modified compared to the corresponding coding region of the respective wild type mRNA such that the frequency of the codons encoding the same amino acid corresponds to the naturally occurring frequency of that codon according to the human codon usage as e.g. shown in Table 1a (Human codon usage table).

For example, in the case of the amino acid alanine (Ala) present in an amino acid sequence encoded by the at least one coding region of the mRNA compound comprising an mRNA sequence according to the invention, the wild type coding region is preferably adapted in a way that the codon "GCC" is used with a frequency of 0.40, the codon "GCT" is used with a frequency of 0.28, the codon "GCA" is used with a frequency of 0.22 and the codon "GCG" is used with a frequency of 0.10 etc. (see Table 1a).

**Table 1a: Human codon usage table**

| **Amino acid** | **codon** | **fraction** | **/1000** | **Amino acid** | **codon** | **fraction** | **/1000** |
|---|---|---|---|---|---|---|---|
| Ala | GCG | 0.10 | 7.4 | Pro | CCG | 0.11 | 6.9 |
| Ala | GCA | 0.22 | 15.8 | Pro | CCA | 0.27 | 16.9 |
| Ala | GCT | 0.28 | 18.5 | Pro | CCT | 0.29 | 17.5 |
| Ala | GCC* | 0.40 | 27.7 | Pro | CCC* | 0.33 | 19.8 |
| Cys | TGT | 0.42 | 10.6 | Gln | CAG* | 0.73 | 34.2 |
| Cys | TGC* | 0.58 | 12.6 | Gln | CAA | 0.27 | 12.3 |
| Asp | GAT | 0.44 | 21.8 | Arg | AGG | 0.22 | 12.0 |
| Asp | GAC* | 0.56 | 25.1 | Arg | AGA* | 0.21 | 12.1 |
| Glu | GAG* | 0.59 | 39.6 | Arg | CGG | 0.19 | 11.4 |
| Glu | GAA | 0.41 | 29.0 | Arg | CGA | 0.10 | 6.2 |
| Phe | TTT | 0.43 | 17.6 | Arg | CGT | 0.09 | 4.5 |
| Phe | TTC* | 0.57 | 20.3 | Arg | CGC | 0.19 | 10.4 |
| Gly | GGG | 0.23 | 16.5 | Ser | AGT | 0.14 | 12.1 |
| Gly | GGA | 0.26 | 16.5 | Ser | AGC* | 0.25 | 19.5 |
| Gly | GGT | 0.18 | 10.8 | Ser | TCG | 0.06 | 4.4 |
| Gly | GGC* | 0.33 | 22.2 | Ser | TCA | 0.15 | 12.2 |
| His | CAT | 0.41 | 10.9 | Ser | TCT | 0.18 | 15.2 |
| His | CAC* | 0.59 | 15.1 | Ser | TCC | 0.23 | 17.7 |
| Ile | ATA | 0.14 | 7.5 | Thr | ACG | 0.12 | 6.1 |
| Ile | ATT | 0.35 | 16.0 | Thr | ACA | 0.27 | 15.1 |
| Ile | ATC* | 0.52 | 20.8 | Thr | ACT | 0.23 | 13.1 |
| Lys | AAG* | 0.60 | 31.9 | Thr | ACC* | 0.38 | 18.9 |
| Lys | AAA | 0.40 | 24.4 | Val | GTG* | 0.48 | 28.1 |
| Leu | TTG | 0.12 | 12.9 | Val | GTA | 0.10 | 7.1 |
| Leu | TTA | 0.06 | 7.7 | Val | GTT | 0.17 | 11.0 |
| Leu | CTG* | 0.43 | 39.6 | Val | GTC | 0.25 | 14.5 |
| Leu | CTA | 0.07 | 7.2 | Trp | TGG* | 1 | 13.2 |
| Leu | CTT | 0.12 | 13.2 | Tyr | TAT | 0.42 | 12.2 |
| Leu | CTC | 0.20 | 19.6 | Tyr | TAC* | 0.58 | 15.3 |
| Met | ATG* | 1 | 22.0 | Stop | TGA* | 0.61 | 1.6 |
| Asn | AAT | 0.44 | 17.0 | Stop | TAG | 0.17 | 0.8 |
| Asn | AAC* | 0.56 | 19.1 | Stop | TAA | 0.22 | 1.0 |

| | | | | | | | |
|---|---|---|---|---|---|---|---|
| *most frequent codon | | | | | | | |

According to a preferred embodiment, the present invention provides mRNA comprising lipid nanoparticles wherein the mRNA comprises an mRNA sequence as defined herein comprising at least one coding region, wherein the coding region comprises or consists of any one of the (modified) RNA sequences according to SEQ ID NOs: 128049-160060, or of a fragment or variant of any one of these sequences.

According to a particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza A virus, wherein the coding region comprises or consists of any one of the (modified) RNA sequences according to SEQ ID NOs: 128049-142079, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza B virus, wherein the coding region comprises or consists of any one of the (modified) RNA sequences according to SEQ ID NOs: 154446-156624 or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza A virus, wherein the coding region comprises or consists of any one of the (modified) RNA sequences according to SEQ ID NOs: 142080-154445, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza B virus, wherein the coding region comprises or consists of any one of the (modified) RNA sequences according to SEQ ID NOs: 156625-158552 or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from glycoprotein of a Rabies virus, wherein the coding region comprises or consists of any one of the (modified) RNA sequences according to SEQ ID NOs: 158553-160060 or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from an Ebola virus, wherein the coding region comprises or consists of any one of the (modified) RNA sequences according to SEQ ID NOs: 20-44 of the patent application WO2016097065, or fragments or variants of these sequences. In this context, SEQ ID NOs: 20-44 of WO2016097065 and the disclosure relating to SEQ ID NOs: 20-44 of WO2016097065 are incorporated herein by reference.

In a further preferred embodiment, the at least one coding region of the mRNA sequence according to the invention comprises or consists of an RNA sequence identical to or having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the (modified) RNA sequences according to SEQ ID NOs: 128049-160060, or of a fragment or variant of any one of these sequences.

According to a particularly preferred embodiment, the at least one coding region of the mRNA sequence according to the invention comprises or consists of an RNA sequence having a sequence identity of at least 80% with any one of the (modified) RNA sequences according to SEQ ID NOs: 128049-160060 or of a fragment or variant of any one of these sequences.

### Codon-optimized sequences:

As described above it is preferred according to the invention, that all codons of the wild type sequence which code for a tRNA, which is relatively rare in the cell, are exchanged for a codon which codes for a tRNA, which is relatively frequent in the cell and which, in each case, carries the same amino acid as the relatively rare tRNA. Therefore it is particularly preferred that the most frequent codons are used for each encoded amino acid (see Table 1a, "Human codon usage table", most frequent codons are marked with asterisks). Such an optimization procedure increases the codon adaptation index (CAI) and ultimately maximises the CAI. In the context of the invention, sequences with increased or maximized CAI are typically referred to as "codon-optimized" sequences and/or CAI increased and/or maximized sequences. According to a preferred embodiment, the mRNA compound comprising an mRNA sequence of the present invention comprises at least one coding region, wherein the coding region/sequence is codon-optimized as described herein. More preferably, the codon adaptation index (CAI) of the at least one coding sequence is at least 0.5, at least 0.8, at least 0.9 or at least 0.95. Most preferably, the codon adaptation index (CAI) of the at least one coding sequence is 1.

For example, in the case of the amino acid alanine (Ala) present in the amino acid sequence encoded by the at least one coding sequence of the RNA according to the invention, the wild type coding sequence is adapted in a way that the most frequent human codon "GCC" is always used for said amino acid, or for the amino acid Cysteine (Cys), the wild type sequence is adapted in a way that the most frequent human codon "TGC" is always used for said amino acid etc.

According to a preferred embodiment, the present invention provides mRNA comprising lipid nanoparticles wherein the mRNA comprises an mRNA sequence as defined herein comprising at least one coding region, wherein the coding region comprises or consists of any one of the (modified) RNA sequences according to SEQ ID NOs: 160061-192072 or of a fragment or variant of any one of these sequences.

According to a particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza A virus, wherein the coding region comprises or consists of any one of the (modified) RNA sequences according to SEQ ID NOs: 160061-174091, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza B virus, wherein the coding region comprises or consists of any one of the (modified) RNA sequences according to SEQ ID NOs: 186458-188636, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza A virus, wherein the coding region comprises or consists of any one of the (modified) RNA sequences according to SEQ ID NOs: 174092-186457 or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza B virus, wherein the coding region comprises or consists of any one of the (modified) RNA sequences according to SEQ ID NOs: 188637-190564, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from glycoprotein of a Rabies virus, wherein the coding region comprises or consists of any one of the (modified) RNA sequences according to SEQ ID NOs: 190565-192072 or of a fragment or variant of any one of these sequences.

In a further preferred embodiment, the at least one coding region of the mRNA sequence according to the invention comprises or consists of an RNA sequence identical to or having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the (modified) RNA sequences according to SEQ ID NOs: 160061-192072, or of a fragment or variant of any one of these sequences.

According to a particularly preferred embodiment, the at least one coding region of the mRNA sequence according to the invention comprises or consists of an RNA sequence having a sequence identity of at least 80% with any one of the (modified) RNA sequences according to SEQ ID NOs: 160061-192072or of a fragment or variant of any one of these sequences.

### C-optimized sequences:

According to another embodiment, the mRNA compound comprising an mRNA sequence of the present invention may be modified by modifying, preferably increasing, the cytosine (C) content of the mRNA sequence, preferably of the coding region of the mRNA sequence.

In a particularly preferred embodiment of the present invention, the C content of the coding region of the mRNA sequence of the present invention is modified, preferably increased, compared to the C content of the coding region of the respective wild type mRNA, i.e. the unmodified mRNA. The amino acid sequence encoded by the at least one coding region of the mRNA sequence of the present invention is preferably not modified as compared to the amino acid sequence encoded by the respective wild type mRNA.

In a preferred embodiment of the present invention, the modified mRNA sequence is modified such that at least 10%, 20%, 30%, 40%, 50%, 60%, 70% or 80%, or at least 90% of the theoretically possible maximum cytosine-content or even a maximum cytosine-content is achieved.

In further preferred embodiments, at least 10%, 20%, 30%, 40%, 50%, 60%, 70%, 80%, 90% or even 100% of the codons of the target mRNA wild type sequence, which are "cytosine content optimizable" are replaced by codons having a higher cytosine-content than the ones present in the wild type sequence.

In a further preferred embodiment, some of the codons of the wild type coding sequence may additionally be modified such that a codon for a relatively rare tRNA in the cell is exchanged by a codon for a relatively frequent tRNA in the cell, provided that the substituted codon for a relatively frequent tRNA carries the same amino acid as the relatively rare tRNA of the original wild type codon. Preferably, all of the codons for a relatively rare tRNA are replaced by a codon for a relatively frequent tRNA in the cell, except codons encoding amino acids, which are exclusively encoded by codons not containing any cytosine, or except for glutamine (Gln), which is encoded by two codons each containing the same number of cytosines.

In a further preferred embodiment of the present invention, the modified target mRNA is modified such that at least 80%, or at least 90% of the theoretically possible maximum cytosine-content or even a maximum cytosine-content is achieved by means of codons, which code for relatively frequent tRNAs in the cell, wherein the amino acid sequence remains unchanged.

Due to the naturally occurring degeneracy of the genetic code, more than one codon may encode a particular amino acid. Accordingly, 18 out of 20 naturally occurring amino acids are encoded by more than one codon (with Tryp and Met being an exception), e.g. by 2 codons (e.g. Cys, Asp, Glu), by three codons (e.g. Ile), by 4 codons (e.g. Al, Gly, Pro) or by 6 codons (e.g. Leu, Arg, Ser). However, not all codons encoding the same amino acid are utilized with the same frequency under in vivo conditions. Depending on each single organism, a typical codon usage profile is established.

The term "cytosine content-optimizable codon" as used within the context of the present invention refers to codons, which exhibit a lower content of cytosines than other codons encoding the same amino acid. Accordingly, any wild type codon, which may be replaced by another codon encoding the same amino acid and exhibiting a higher number of cytosines within that codon, is considered to be cytosine-optimizable (C-optimizable). Any such substitution of a C-optimizable wild type codon by the specific C-optimized codon within a wild type coding region increases its overall C-content and reflects a C-enriched modified mRNA sequence. According to a preferred embodiment, the mRNA sequence of the present invention, preferably the at least one coding region of the mRNA sequence of the present invention comprises or consists of a C-maximized mRNA sequence containing C-optimized codons for all potentially C-optimizable codons. Accordingly, 100% or all of the theoretically replaceable C-optimizable codons are preferably replaced by C-optimized codons over the entire length of the coding region.

In this context, cytosine-content optimizable codons are codons, which contain a lower number of cytosines than other codons coding for the same amino acid.

Any of the codons GCG, GCA, GCU codes for the amino acid Ala, which may be exchanged by the codon GCC encoding the same amino acid, and/or
the codon UGU that codes for Cys may be exchanged by the codon UGC encoding the same amino acid, and/or
the codon GAU which codes for Asp may be exchanged by the codon GAC encoding the same amino acid, and/or
the codon that UUU that codes for Phe may be exchanged for the codon UUC encoding the same amino acid, and/or
any of the codons GGG, GGA, GGU that code Gly may be exchanged by the codon GGC encoding the same amino acid, and/or
the codon CAU that codes for His may be exchanged by the codon CAC encoding the same amino acid, and/or any of the codons AUA, AUU that code for Ile may be exchanged by the codon AUC, and/or
any of the codons UUG, UUA, CUG, CUA, CUU coding for Leu may be exchanged by the codon CUC encoding the same amino acid, and/or
the codon AAU that codes for Asn may be exchanged by the codon AAC encoding the same amino acid, and/or
any of the codons CCG, CCA, CCU coding for Pro may be exchanged by the codon CCC encoding the same amino acid, and/or
any of the codons AGG, AGA, CGG, CGA, CGU coding for Arg may be exchanged by the codon CGC encoding the same amino acid, and/or
any of the codons AGU, AGC, UCG, UCA, UCU coding for Ser may be exchanged by the codon UCC encoding the same amino acid, and/or
any of the codons ACG, ACA, ACU coding for Thr may be exchanged by the codon ACC encoding the same amino acid, and/or
any of the codons GUG, GUA, GUU coding for Val may be exchanged by the codon GUC encoding the same amino acid, and/or
the codon UAU coding for Tyr may be exchanged by the codon UAC encoding the same amino acid.

In any of the above instances, the number of cytosines is increased by 1 per exchanged codon. Exchange of all non C-optimized codons (corresponding to C-optimizable codons) of the coding region results in a C-maximized coding sequence. In the context of the invention, at least 70%, preferably at least 80%, more preferably at least 90%, of the non C-optimized codons within the at least one coding region of the mRNA sequence according to the invention are replaced by C-optimized codons.

It may be preferred that for some amino acids the percentage of C-optimizable codons replaced by C-optimized codons is less than 70%, while for other amino acids the percentage of replaced codons is higher than 70% to meet the overall percentage of C-optimization of at least 70% of all C-optimizable wild type codons of the coding region.

Preferably, in a C-optimized mRNA sequence of the invention, at least 50% of the C-optimizable wild type codons for any given amino acid are replaced by C-optimized codons, e.g. any modified C-enriched mRNA sequence preferably contains at least 50% C-optimized codons at C-optimizable wild type codon positions encoding any one of the above mentioned amino acids Ala, Cys, Asp, Phe, Gly, His, Ile, Leu, Asn, Pro, Arg, Ser, Thr, Val and Tyr, preferably at least 60%.

In this context codons encoding amino acids, which are not cytosine content-optimizable and which are, however, encoded by at least two codons, may be used without any further selection process. However, the codon of the wild type sequence that codes for a relatively rare tRNA in the cell, e.g. a human cell, may be exchanged for a codon that codes for a relatively frequent tRNA in the cell, wherein both code for the same amino acid. Accordingly, the relatively rare codon GAA coding for Glu may be exchanged by the relative frequent codon GAG coding for the same amino acid, and/or
the relatively rare codon AAA coding for Lys may be exchanged by the relative frequent codon AAG coding for the same amino acid, and/or
the relatively rare codon CAA coding for Gln may be exchanged for the relative frequent codon CAG encoding the same amino acid.

In this context, the amino acids Met (AUG) and Trp (UGG), which are encoded by only one codon each, remain unchanged. Stop codons are not cytosine-content optimized, however, the relatively rare stop codons amber, ochre (UAA, UAG) may be exchanged by the relatively frequent stop codon opal (UGA).

The single substitutions listed above may be used individually as well as in all possible combinations in order to optimize the cytosine-content of the modified mRNA sequence compared to the wild type mRNA sequence.

Accordingly, the at least one coding sequence as defined herein may be changed compared to the coding region of the respective wild type mRNA in such a way that an amino acid encoded by at least two or more codons, of which one comprises one additional cytosine, such a codon may be exchanged by the C-optimized codon comprising one additional cytosine, wherein the amino acid is preferably unaltered compared to the wild type sequence.

According to a preferred embodiment, the the present invention provides mRNA comprising lipid nanoparticles wherein the mRNA comprises an mRNA sequence as defined herein comprising at least one coding region, wherein the coding region comprises or consists of any one of the (modified) RNA sequences according to SEQ ID NOs: 96037-128048, or of a fragment or variant of any one of these sequences.

According to a particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza A virus, wherein the coding region comprises or consists of any one of the (modified) RNA sequences according to SEQ ID NOs: 96037-110067, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from hemagglutinin (HA) of an influenza B virus, wherein the coding region comprises or consists of any one of the (modified) RNA sequences according to SEQ ID NOs: 122434-124612 or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza A virus, wherein the coding region comprises or consists of any one of the (modified) RNA sequences according to SEQ ID NOs: 110068-122433, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from neuraminidase (NA) of an influenza B virus, wherein the coding region comprises or consists of any one of the (modified) RNA sequences according to SEQ ID NOs: 124613-126540, or of a fragment or variant of any one of these sequences.

According to a further particularly preferred embodiment, the present invention provides mRNA comprising lipid nanoparticles wherein the mRNA comprises an mRNA sequence as defined herein comprising at least one coding region encoding at least one antigenic peptide or protein derived from glycoprotein of a Rabies virus, wherein the coding region comprises or consists of any one of the (modified) RNA sequences according to SEQ ID NOs: 126541-128048 or of a fragment or variant of any one of these sequences.

In a further preferred embodiment, the at least one coding region of the mRNA sequence according to the invention comprises or consists of an RNA sequence identical to or having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the (modified) RNA sequences according to SEQ ID NOs: 96037-128048, or of a fragment or variant of any one of these sequences.

According to a particularly preferred embodiment, the at least one coding region of the mRNA compound comprising an mRNA sequence according to the invention comprises or consists of an RNA sequence having a sequence identity of at least 80% with any one of the (modified) RNA sequences according to SEQ ID NOs: 96037-128048 or of a fragment or variant of any one of these sequences.

According to a particularly preferred embodiment, the present invention provides mRNA comprising lipid nanoparticles wherein the mRNA comprises an mRNA sequence, comprising at least one coding region as defined herein, wherein the G/C content of the at least one coding region of the mRNA sequence is increased compared to the G/C content of the corresponding coding region of the corresponding wild type mRNA, and/or wherein the C content of the at least one coding region of the mRNA sequence is increased compared to the C content of the corresponding coding region of the corresponding wild type mRNA, and/or wherein the codons in the at least one coding region of the mRNA sequence are adapted to human codon usage, wherein the codon adaptation index (CAI) is preferably increased or maximised in the at least one coding region of the mRNA sequence,
and wherein the amino acid sequence encoded by the mRNA sequence is preferably not being modified compared to the amino acid sequence encoded by the corresponding wild type mRNA.

### 5'-CAP structure:

According to another preferred embodiment of the invention, a modified mRNA sequence as defined herein, can be modified by the addition of a so-called "5'-CAP structure", which preferably stabilizes the mRNA as described herein. A 5'-CAP is an entity, typically a modified nucleotide entity, which generally "caps" the 5'-end of a mature mRNA. A 5'-CAP may typically be formed by a modified nucleotide, particularly by a derivative of a guanine nucleotide. Preferably, the 5'-CAP is linked to the 5'-terminus via a 5'-5'-triphosphate linkage. A 5'-CAP may be methylated, e.g. m7GpppN, wherein N is the terminal 5' nucleotide of the nucleic acid carrying the 5'-CAP, typically the 5'-end of an mRNA. m7GpppN is the 5'-CAP structure, which naturally occurs in mRNA transcribed by polymerase II and is therefore preferably not considered as modification comprised in a modified mRNA in this context. Accordingly, a modified mRNA sequence of the present invention may comprise a m7GpppN as 5'-cap, but additionally the modified mRNA sequence typically comprises at least one further modification as defined herein.

Further examples of 5'-CAP structures include glyceryl, inverted deoxy abasic residue (moiety), 4',5' methylene nucleotide, 1-(beta-D-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety, 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-CAP structures are regarded as at least one modification in this context.

Particularly preferred modified 5'-CAP structures are cap1 (methylation of the ribose of the adjacent nucleotide of m7G), cap2 (additional methylation of the ribose of the 2nd nucleotide downstream of the m7G), cap3 (additional methylation of the ribose of the 3rd nucleotide downstream of the m7G), cap4 (methylation of the ribose of the 4th nucleotide downstream of the m7G), ARCA (anti-reverse CAP analogue, modified ARCA (e.g. phosphothioate modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine. Accordingly, the RNA according to the invention preferably comprises a 5'-CAP structure.

### Poly(A) sequence/tail:

According to a further preferred embodiment, the mRNA compound comprising an mRNA sequence of the present invention may contain a poly-A tail on the 3'-terminus of typically about 10 to 200 adenosine nucleotides, preferably about 10 to 100 adenosine nucleotides, more preferably about 40 to 80 adenosine nucleotides or even more preferably about 50 to 70 adenosine nucleotides.

Preferably, the poly(A) sequence in the mRNA compound comprising an mRNA sequence of the present invention is derived from a DNA template by RNA in vitro transcription. Alternatively, the poly(A) sequence may also be obtained in vitro by common methods of chemical-synthesis without being necessarily transcribed from a DNA-progenitor. Moreover, poly(A) sequences, or poly(A) tails may be generated by enzymatic polyadenylation of the RNA according to the present invention using commercially available polyadenylation kits and corresponding protocols known in the art.

Alternatively, the mRNA as described herein optionally comprises a polyadenylation signal, which is defined herein as a signal, which conveys polyadenylation to a (transcribed) RNA by specific protein factors (e.g. cleavage and polyadenylation specificity factor (CPSF), cleavage stimulation factor (CstF), cleavage factors I and II (CF I and CF II), poly(A) polymerase (PAP)). In this context, a consensus polyadenylation signal is preferred comprising the NN(U/T)ANA consensus sequence. In a particularly preferred aspect, the polyadenylation signal comprises one of the following sequences: AA(U/T)AAA or A(U/T)(U/T)AAA (wherein uridine is usually present in RNA and thymidine is usually present in DNA).

### Poly(C) sequence:

According to a further preferred embodiment, the mRNA compound comprising an mRNA sequence of the present invention may contain a poly(C) tail on the 3'-terminus of typically about 10 to 200 cytosine nucleotides, preferably about 10 to 100 cytosine nucleotides, more preferably about 20 to 70 cytosine nucleotides or even more preferably about 20 to 60 or even 10 to 40 cytosine nucleotides.

In one preferred embodiment the mRNA compound comprising an mRNA sequence comprises, preferably in 5'-to 3'-direction:
a) a 5'-CAP structure, preferably m7GpppN;
b) at least one coding region encoding at least one antigenic peptide or protein,
c) optionally, a poly(A) sequence, preferably comprising 64 adenosines;
d) optionally, a poly(C) sequence, preferably comprising 30 cytosines.

In a more preferred embodiment the mRNA sequence comprises, preferably in 5'- to 3'-direction:
a) a 5'-CAP structure, preferably m7GpppN;
b) at least one coding region encoding at least one antigenic peptide or protein derived from a protein of an influenza or rabies virus or a fragment or variant thereof,
c) optionally, a poly(A) sequence, preferably comprising 64 adenosines;
d) optionally, a poly(C) sequence, preferably comprising 30 cytosines.

In a particularly preferred embodiment the mRNA sequence comprises, preferably in 5'- to 3'-direction:
a) a 5'-CAP structure, preferably m7GpppN;
b) at least one coding region encoding at least one antigenic peptide or protein derived from a protein of an influenza virus or a rabies virus or a fragment or variant thereof, preferably comprising or consisting of any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Tables 1-5 or Figures 20-24 of PCT/EP2016/075843, SEQ ID NOs: 32013-64024, or SEQ ID NOs: 64025-224084 or of a fragment or variant of any one of these sequences),
c) optionally, a poly(A) sequence, preferably comprising 64 adenosines;
d) optionally, a poly(C) sequence, preferably comprising 30 cytosines.

### UTRs:

In a preferred embodiment, the mRNA compound comprising an mRNA sequence according to the invention comprises at least one 5'- or 3'-UTR element. In this context, an UTR element comprises or consists of a nucleic acid sequence, which is derived from the 5'- or 3'-UTR of any naturally occurring gene or which is derived from a fragment, a homolog or a variant of the 5'- or 3'-UTR of a gene. Preferably, the 5'- or 3'-UTR element used according to the present invention is heterologous to the at least one coding region of the mRNA sequence of the invention. Even if 5'- or 3'-UTR elements derived from naturally occurring genes are preferred, also synthetically engineered UTR elements may be used in the context of the present invention.

### 3'-UTR elements:

The term "3'-UTR element" typically refers to a nucleic acid sequence, which comprises or consists of a nucleic acid sequence that is derived from a 3'-UTR or from a variant of a 3'-UTR. A 3'-UTR element in the sense of the present invention may represent the 3'-UTR of an RNA, preferably an mRNA. Thus, in the sense of the present invention, preferably, a 3'-UTR element may be the 3'-UTR of an RNA, preferably of an mRNA, or it may be the transcription template for a 3'-UTR of an RNA. Thus, a 3'-UTR element preferably is a nucleic acid sequence which corresponds to the 3'-UTR of an RNA, preferably to the 3'-UTR of an mRNA, such as an mRNA obtained by transcription of a genetically engineered vector construct. Preferably, the 3'-UTR element fulfils the function of a 3'-UTR or encodes a sequence which fulfils the function of a 3'-UTR.

Preferably, the at least one 3'-UTR element comprises or consists of a nucleic acid sequence derived from the 3'-UTR of a chordate gene, preferably a vertebrate gene, more preferably a mammalian gene, most preferably a human gene, or from a variant of the 3'-UTR of a chordate gene, preferably a vertebrate gene, more preferably a mammalian gene, most preferably a human gene.

Preferably, the mRNA compound comprising an mRNA sequence of the present invention comprises a 3'-UTR element, which may be derivable from a gene that relates to an mRNA with an enhanced half-life (that provides a stable mRNA), for example a 3'-UTR element as defined and described below. Preferably, the 3'-UTR element is a nucleic acid sequence derived from a 3'-UTR of a gene, which preferably encodes a stable mRNA, or from a homolog, a fragment or a variant of said gene.

In a particularly preferred embodiment, the 3'-UTR element comprises or consists of a nucleic acid sequence, which is derived from a 3'-UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene, or from a variant of a 3'-UTR of a gene selected from the group consisting of an albumin gene, an α-globin gene, a β-globin gene, a tyrosine hydroxylase gene, a lipoxygenase gene, and a collagen alpha gene, such as a collagen alpha 1(I) gene according to SEQ ID NOs: 1369-1390 of the patent application WO2013/143700, whose disclosure is incorporated herein by reference, or from a homolog, a fragment or a variant thereof. In a particularly preferred embodiment, the 3'-UTR element comprises or consists of a nucleic acid sequence which is derived from a 3'-UTR of an albumin gene, preferably a vertebrate albumin gene, more preferably a mammalian albumin gene, most preferably a human albumin gene according to SEQ ID NO: 224301 or SEQ ID NO: 224303 or the corresponding RNA sequences SEQ ID NO: 224300 or SEQ ID NO: 224304.
Human albumin 3'-UTR SEQ ID NO 224301 CATCACATTTAAAAGCATCTCAGCCTACCATGAGAATAAGAGAAAGAAAATGAAGATCAAAAGCTTATTCATCTGTTTTTC TTTTTCGTTGGTGTAAAGCCAACACCCTGTCTAAAAAACATAAATTTCTTTAATCATTTTGCCTCTTTTCTCTGTGCTTCAA TTAATAAAAAATGGAAAGAATCT (corresponding to SEQ ID NO: 1369 of the patent application WO2013/143700).

In this context it is particularly preferred that the mRNA compound comprising an mRNA sequence according to the invention comprises a 3'-UTR element comprising a corresponding RNA sequence derived from the nucleic acids according to SEQ ID NOs: 1369-1390 of the patent application WO2013/143700 or a fragment, homolog or variant thereof.

Most preferably the 3'-UTR element comprises the nucleic acid sequence derived from a fragment of the human albumin gene according to SEQ ID NO: 224303.
albumin7 3'-UTR
CATCACATTTAAAAGCATCTCAGCCTACCATGAGAATAAGAGAAAGAAAATGAAGATCAATAGCTTATTCATCTCTTTTTC TTTTTCGTTGGTGTAAAGCCAACACCCTGTCTAAAAAACATAAATTTCTTTAATCATTTTGCCTCTTTTCTCTGTGCTTCAA TTAATAAAAAATGGAAAGAACCT (SEQ ID NO: 224303 corresponding to SEQ ID NO: 1376 of the patent application WO2013143700).

In this context, it is particularly preferred that the 3'-UTR element of the mRNA sequence according to the present invention comprises or consists of a corresponding RNA sequence of the nucleic acid sequence according to SEQ ID NO: 224301 or SEQ ID NO: 224303 as shown in SEQ ID NOs: 224302 or SEQ ID NO: 224304.

In another particularly preferred embodiment, the 3'-UTR element comprises or consists of a nucleic acid sequence which is derived from a 3'-UTR of an α-or β-globin gene, preferably a vertebrate α-or β -globin gene, more preferably a mammalian α-or β -globin gene, most preferably a human α-or β globin gene according to SEQ ID NOs: 224291, 224293, 224295, 224297 or the corresponding RNA sequences SEQ ID NOs: 224292, 224294, 224296, 224298.
3'-UTR of Homo sapiens hemoglobin, alpha 1 (HBA1)
   GCTGGAGCCTCGGTGGCCATGCTTCTTGCCCCTTGGGCCTCCCCCCAGCCCCTCCTCCCCTTCCTGCACCCGTACCCCCG TGGTCTTTGAATAAAGTCTGAGTGGGCGGC (SEQ ID NO: 224291 corresponding to SEQ ID NO: 1370 of the patent application WO2013/143700).
3'-UTR of Homo sapiens hemoglobin, alpha 2 (HBA2)
   GCTGGAGCCTCGGTAGCCGTTCCTCCTGCCCGCTGGGCCTCCCAACGGGCCCTCCTCCCCTCCTTGCACCGGCCCTTCCT GGTCTTTGAATAAAGTCTGAGTGGGCAG (SEQ ID NO: 224293 corresponding to SEQ ID NO: 1371 of the patent application WO2013/143700).
3'-UTR of Homo sapiens hemoglobin, beta (HBB)
   GCTCGCTTTCTTGCTGTCCAATTTCTATTAAAGGTTCCTTTGTTCCCTAAGTCCAACTACTAAACTGGGGGATATTATGAA GGGCCTTGAGCATCTGGATTCTGCCTAATAAAAAACATTTATTTTCATTGC (SEQ ID NO: 224295 corresponding to SEQ ID NO: 1372 of the patent application WO2013/143700).

For example, the 3'-UTR element may comprise or consist of the center, α-complex-binding portion of the 3'-UTR of an α-globin gene, such as of a human α-globin gene, or a homolog, a fragment, or a variant of an α-globin gene, preferably according to SEQ ID NO: 224297.

Center, α-complex-binding portion of the 3'-UTR of an α-globin gene (also named herein as "muag") GCCCGATGGGCCTCCCAACGGGCCCTCCTCCCCTCCTTGCACCG (SEQ ID NO: 224297 corresponding to SEQ ID NO: 1393 of the patent application WO2013/143700).

In this context it is particularly preferred that the 3'-UTR element of the mRNA sequence according to the invention comprises or consists of a corresponding RNA sequence of the nucleic acid sequence according to SEQ ID NO: 224297 as shown in SEQ ID NO: 224298, or a homolog, a fragment or variant thereof.

The term "a nucleic acid sequence which is derived from the 3'-UTR of a [...] gene" preferably refers to a nucleic acid sequence which is based on the 3'-UTR sequence of a [...] gene or on a part thereof, such as on the 3'-UTR of an albumin gene, an α-globin gene, a β-globin gene, a tyrosine hydroxylase gene, a lipoxygenase gene, or a collagen alpha gene, such as a collagen alpha 1(I) gene, preferably of an albumin gene or on a part thereof. This term includes sequences corresponding to the entire 3'-UTR sequence, i.e. the full length 3'-UTR sequence of a gene, and sequences corresponding to a fragment of the 3'-UTR sequence of a gene, such as an albumin gene, α-globin gene, β-globin gene, tyrosine hydroxylase gene, lipoxygenase gene, or collagen alpha gene, such as a collagen alpha 1(I) gene, preferably of an albumin gene.

The term "a nucleic acid sequence which is derived from a variant of the 3'-UTR of a [...] gene" preferably refers to a nucleic acid sequence, which is based on a variant of the 3'-UTR sequence of a gene, such as on a variant of the 3'-UTR of an albumin gene, an α-globin gene, a β-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'-UTR of a gene, i.e. the full length variant 3'-UTR sequence of a gene, and sequences corresponding to a fragment of the variant 3'-UTR sequence of a gene. A fragment in this context preferably consists of a continuous stretch of nucleotides corresponding to a continuous stretch of nucleotides in the full-length variant 3'-UTR, which represents at least 20%, preferably at least 30%, more preferably at least 40%, more preferably at least 50%, even more preferably at least 60%, even more preferably at least 70%, even more preferably at least 80%, and most preferably at least 90% of the full-length variant 3'-UTR. Such a fragment of a variant, in the sense of the present invention, is preferably a functional fragment of a variant as described herein.

According to a preferred embodiment, the mRNA compound comprising an mRNA sequence according to the invention comprises a 5'-CAP structure and/or at least one 3'-untranslated region element (3'-UTR element), preferably as defined herein. More preferably, the RNA further comprises a 5'-UTR element as defined herein.

In one preferred embodiment the mRNA compound comprising an mRNA sequence comprises, preferably in 5'-to 3'-direction:
a) a 5'-CAP structure, preferably m7GpppN;
b) at least one coding region encoding at least one antigenic peptide or protein,
c) optionally a 3'-UTR element, preferably comprising or consisting of a nucleic acid sequence which is derived from an alpha globin gene, preferably comprising the corresponding RNA sequence of the nucleic acid sequence according to SEQ ID NO: 224297 as shown in SEQ ID NO: 224298, a homolog, a fragment or a variant thereof;
d) optionally, a poly(A) sequence, preferably comprising 64 adenosines;
e) optionally, a poly(C) sequence, preferably comprising 30 cytosines.

In a preferred embodiment the mRNA sequence comprises, preferably in 5'- to 3'-direction:
a) a 5'-CAP structure, preferably m7GpppN;
b) at least one coding region encoding at least one antigenic peptide or protein, preferably derived from a protein of an influenza virus or a Rabies virus or a fragment or variant thereof,
c) optionally a 3'-UTR element, preferably comprising or consisting of a nucleic acid sequence which is derived from an alpha globin gene, preferably comprising the corresponding RNA sequence of the nucleic acid sequence according to SEQ ID NO: 224297 as shown in SEQ ID NO: 224298, a homolog, a fragment or a variant thereof;
d) optionally, a poly(A) sequence, preferably comprising 64 adenosines;
e) optionally, a poly(C) sequence, preferably comprising 30 cytosines.

In a particularly preferred embodiment the mRNA sequence comprises, preferably in 5'- to 3'-direction:
a) a 5'-CAP structure, preferably m7GpppN;
b) at least one coding region encoding at least one antigenic peptide or protein preferably derived from a protein of an influenza virus or Rabies virus or a fragment or variant thereof, preferably comprising or consisting of any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Tables 1-5 or Figures 20-24 of PCT/EP2016/075843, SEQ ID NOs: 32013-64024, or SEQ ID NOs: 64025-224084 or of a fragment or variant of any one of these sequences,
c) optionally a 3'-UTR element, preferably comprising or consisting of a nucleic acid sequence which is derived from an alpha globin gene, preferably comprising the corresponding RNA sequence of the nucleic acid sequence according to SEQ ID NO: 224297 as shown in SEQ ID NO: 224298, a homolog, a fragment or a variant thereof;
d) optionally, a poly(A) sequence, preferably comprising 64 adenosines;
e) optionally, a poly(C) sequence, preferably comprising 30 cytosines; and

### 5'-UTR elements:

In a particularly preferred embodiment, the at least one mRNA compound comprising an mRNA sequence comprises at least one 5'-untranslated region element (5'-UTR element). Preferably, the at least one 5'-UTR element comprises or consists of a nucleic acid sequence, which is derived from the 5'-UTR of a TOP gene or which is derived from a fragment, homolog or variant of the 5'-UTR of a TOP gene.

It is particularly preferred that the 5'-UTR element does not comprise a TOP motif or a 5'-TOP, as defined above.

In some embodiments, the nucleic acid sequence of the 5'-UTR element, which is derived from a 5'-UTR of a TOP gene, terminates at its 3'-end with a nucleotide located at position 1, 2, 3, 4, 5, 6, 7, 8, 9 or 10 upstream of the start codon (e.g. A(U/T)G) of the gene or mRNA it is derived from. Thus, the 5'-UTR element does not comprise any part of the protein coding region. Thus, preferably, the only protein coding part of the at least one mRNA sequence is provided by the coding region.

The nucleic acid sequence derived from the 5'-UTR of a TOP gene is preferably derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'-UTR element is preferably selected from 5'-UTR elements comprising or consisting of a nucleic acid sequence, which is derived from a nucleic acid sequence selected from the group consisting of SEQ ID NOs: 1-1363, SEQ ID NO: 1395, SEQ ID NO: 1421 and SEQ ID NO: 1422 of the patent application WO2013/143700, whose disclosure is incorporated herein by reference, from the homologs of SEQ ID NOs: 1-1363, SEQ ID NO: 1395, SEQ ID NO: 1421 and SEQ ID NO: 1422 of the patent application WO2013/143700, from a variant thereof, or preferably from a corresponding RNA sequence. The term "homologs of SEQ ID NOs: 1-1363, SEQ ID NO: 1395, SEQ ID NO: 1421 and SEQ ID NO: 1422 of the patent application WO2013/143700" refers to sequences of other species than homo sapiens, which are homologous to the sequences according to SEQ ID NOs: 1-1363, SEQ ID NO: 1395, SEQ ID NO: 1421 and SEQ ID NO: 1422 of the patent application WO2013/143700.

In a preferred embodiment, the 5'-UTR element of the mRNA compound comprising an mRNA sequence according to the invention comprises or consists of a nucleic acid sequence, which is derived from a nucleic acid sequence extending from nucleotide position 5 (i.e. the nucleotide that is located at position 5 in the sequence) to the nucleotide position immediately 5' to the start codon (located at the 3'-end of the sequences), e.g. the nucleotide position immediately 5' to the ATG sequence, of a nucleic acid sequence selected from SEQ ID NOs: 1-1363, SEQ ID NO: 1395, SEQ ID NO: 1421 and SEQ ID NO: 1422 of the patent application WO2013/143700, from the homologs of SEQ ID NOs: 1-1363, SEQ ID NO: 1395, SEQ ID NO: 1421 and SEQ ID NO: 1422 of the patent application WO2013/143700 from a variant thereof, or a corresponding RNA sequence. It is particularly preferred that the 5'-UTR element is derived from a nucleic acid sequence extending from the nucleotide position immediately 3' to the 5'-TOP to the nucleotide position immediately 5' to the start codon (located at the 3'-end of the sequences), e.g. the nucleotide position immediately 5' to the ATG sequence, of a nucleic acid sequence selected from SEQ ID NOs: 1-1363, SEQ ID NO: 1395, SEQ ID NO: 1421 and SEQ ID NO: 1422 of the patent application WO2013/143700, from the homologs of SEQ ID NOs: 1-1363, SEQ ID NO: 1395, SEQ ID NO: 1421 and SEQ ID NO: 1422 of the patent application WO2013/143700, from a variant thereof, or a corresponding RNA sequence.

In a particularly preferred embodiment, the 5'-UTR element comprises or consists of a nucleic acid sequence, which is derived from a 5'-UTR of a TOP gene encoding a ribosomal protein or from a variant of a 5'-UTR of a TOP gene encoding a ribosomal protein. For example, the 5'-UTR element comprises or consists of a nucleic acid sequence, which is derived from a 5'-UTR of a nucleic acid sequence according to any of SEQ ID NOs: 67, 170, 193, 244, 259, 554, 650, 675, 700, 721, 913, 1016, 1063, 1120, 1138, and 1284-1360 of the patent application WO2013/143700, a corresponding RNA sequence, a homolog thereof, or a variant thereof as described herein, preferably lacking the 5'-TOP motif. As described above, the sequence extending from position 5 to the nucleotide immediately 5' to the ATG (which is located at the 3'-end of the sequences) corresponds to the 5'-UTR of said sequences.

Preferably, the 5'-UTR element comprises or consists of a nucleic acid sequence, which is derived from a 5'-UTR of a TOP gene encoding a ribosomal Large protein (RPL) or from a homolog or variant of a 5'-UTR of a TOP gene encoding a ribosomal Large protein (RPL). For example, the 5'-UTR element comprises or consists of a nucleic acid sequence, which is derived from a 5'-UTR of a nucleic acid sequence according to any of SEQ ID NOs: 67, 259, 1284-1318, 1344, 1346, 1348-1354, 1357, 1358, 1421 and 1422of the patent application WO2013/143700, a corresponding RNA sequence, a homolog thereof, or a variant thereof as described herein, preferably lacking the 5'-TOP motif.

In a particularly preferred embodiment, the 5'-UTR element comprises or consists of a nucleic acid sequence which is derived from the 5'-UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, or from a variant of the 5'UTR of a ribosomal protein Large 32 gene, preferably from a vertebrate ribosomal protein Large 32 (L32) gene, more preferably from a mammalian ribosomal protein Large 32 (L32) gene, most preferably from a human ribosomal protein Large 32 (L32) gene, wherein preferably the 5'-UTR element does not comprise the 5'-TOP of said gene.

Accordingly, in a particularly preferred embodiment, the 5'-UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 224287or SEQ ID NO: 224288 (5'-UTR of human ribosomal protein Large 32 lacking the 5'-terminal oligopyrimidine tract:
GGCGCTGCCTACGGAGGTGGCAGCCATCTCCTTCTCGGCATC; corresponding to SEQ ID NO: 1368 of the patent application WO2013/143700) or preferably to a corresponding RNA sequence, or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 224287 or more preferably to a corresponding RNA sequence (SEQ ID NO: 224288), wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the mRNA compound comprising an mRNA sequence according to the invention comprises a 5'-UTR element, which comprises or consists of a nucleic acid sequence, which is derived from the 5'-UTR of a vertebrate TOP gene, such as a mammalian, e.g. a human TOP gene, selected from RPSA, RPS2, RPS3, RPS3A, RPS4, RPS5, RPS6, RPS7, RPS8, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3, RPL4, RPL5, RPL6, RPL7, RPL7A, RPL8, RPL9, RPL10, RPL10A, RPL11, RPL12, RPL13, RPL13A, RPL14, RPL15, RPL17, RPL18, RPL18A, RPL19, RPL21, RPL22, RPL23, RPL23A, RPL24, RPL26, RPL27, RPL27A, RPL28, RPL29, RPL30, RPL31, RPL32, RPL34, RPL35, RPL35A, RPL36, RPL36A, RPL37, RPL37A, RPL38, RPL39, RPL40, RPL41, RPLP0, RPLP1, RPLP2, RPLP3, RPLP0, RPLP1, RPLP2, EEF1A1, EEF1B2, EEF1D, EEF1G, EEF2, EIF3E, EIF3F, EIF3H, EIF2S3, EIF3C, EIF3K, EIF3EIP, EIF4A2, PABPC1, HNRNPA1, TPT1, TUBB1, UBA52, NPM1, ATP5G2, GNB2L1, NME2, UQCRB, or from a homolog or variant thereof, wherein preferably the 5'-UTR element does not comprise a TOP motif or the 5'-TOP of said genes, and wherein optionally the 5'-UTR element starts at its 5'-end with a nucleotide located at position 1, 2, 3, 4, 5, 6, 7, 8, 9 or 10 downstream of the 5'-terminal oligopyrimidine tract (TOP) and wherein further optionally the 5'-UTR element which is derived from a 5'-UTR of a TOP gene terminates at its 3'-end with a nucleotide located at position 1, 2, 3, 4, 5, 6, 7, 8, 9 or 10 upstream of the start codon (A(U/T)G) of the gene it is derived from.

In further particularly preferred embodiments, the 5'-UTR element comprises or consists of a nucleic acid sequence, which is derived from the 5'-UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit 1, cardiac muscle (ATP5A1) gene, an hydroxysteroid (17-beta) dehydrogenase 4 gene (HSD17B4), an androgen-induced 1 gene (AIG1), cytochrome c oxidase subunit VIc gene (COX6C), or a N-acylsphingosine amidohydrolase (acid ceramidase) 1 gene (ASAH1) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit 1, cardiac muscle (ATP5A1) gene, a vertebrate hydroxysteroid (17-beta) dehydrogenase 4 gene (HSD17B4), a vertebrate androgen-induced 1 gene (AIG1), a vertebrate cytochrome c oxidase subunit VIc gene (COX6C), or a vertebrate N-acylsphingosine amidohydrolase (acid ceramidase) 1 gene (ASAH1) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase, H+ transporting, mitochondrial F1 complex, alpha subunit 1, cardiac muscle (ATP5A1) gene, a mammalian hydroxysteroid (17-beta) dehydrogenase 4 gene (HSD17B4), a mammalian androgen-induced 1 gene (AIG1), a mammalian cyto-chrome c oxidase subunit VIc gene (COX6C), or a mammalian N-acylsphingosine ami-dohydrolase (acid ceramidase) 1 gene (ASAH1) or from a variant thereof, most preferably from a human ribosomal protein Large 32 gene (RPL32), a human ribosomal protein Large 35 gene (RPL35), a human ribosomal protein Large 21 gene (RPL21), a human ATP synthase, H+ transporting, mitochondrial F1 complex, alpha subunit 1, cardiac muscle (ATP5A1) gene, a human hydroxysteroid (17-beta) dehydrogenase 4 gene (HSD17B4), a human androgen-induced 1 gene (AIG1), a human cytochrome c oxidase subunit VIc gene (COX6C), or a human N-acylsphingosine amidohydrolase (acid ceramidase) 1 gene (ASAH1) or from a variant thereof, wherein preferably the 5'-UTR element does not comprise the 5'-TOP of said gene.

Accordingly, in a particularly preferred embodiment, the 5'-UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 1368, or SEQ ID NOs: 1412-1420 of the patent application WO2013/143700, or a corresponding RNA sequence, or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 1368, or SEQ ID NOs: 1412-1420 of the patent application WO2013/143700, wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Accordingly, in a particularly preferred embodiment, the 5'-UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 224289 (5'-UTR of ATP5A1 lacking the 5'-terminal oligopyrimidine tract:
GCGGCTCGGCCATTTTGTCCCAGTCAGTCCGGAGGCTGCGGCTGCAGAAGTACCGCCTGCGGAGTAACTGCAAAG; corresponding to SEQ ID NO: 224289of the patent application WO2013/143700) or preferably to a corresponding RNA sequence (SEQ ID NO: 224290), or wherein the at least one 5'-UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 224289 or more preferably to a corresponding RNA sequence (SEQ ID NO: 224290), wherein, preferably, the fragment is as described above, i.e. being a continuous stretch of nucleotides representing at least 20% etc. of the full-length 5'-UTR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the at least one 5'-UTR element and the at least one 3'-UTR element act synergistically to increase protein production from the at least one mRNA sequence as described above.

According to a preferred embodiment the mRNA compound comprising an mRNA sequence according to the invention comprises, preferably in 5'- to 3'-direction:
a) a 5'-CAP structure, preferably m7GpppN;
b) optionally a 5'-UTR element which preferably comprises or consists of a nucleic acid sequence which is derived from the 5'-UTR of a TOP gene, more preferably comprising or consisting of the corresponding RNA sequence of a nucleic acid sequence according to SEQ ID NO: 224287, as shown in SEQ ID NO: 224288, a homolog, a fragment or a variant thereof;
c) at least one coding region encoding at least one antigenic peptide or protein preferably derived from a protein of an influenza virus or a Rabies virus, or a fragment or variant thereof, preferably comprising or consisting of any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Tables 1-5 or Figures 20-24 of PCT/EP2016/075843, SEQ ID NOs: 32013-64024, or SEQ ID NOs: 64025-224084 or of a fragment or variant of any one of these sequences,
d) optionally a 3'-UTR element which preferably comprises or consists of a nucleic acid sequence which is derived from a gene providing a stable mRNA, preferably comprising or consisting of the corresponding RNA sequence of a nucleic acid sequence according to SEQ ID NO: 224291, 224293, 224295, 224297, 224299, 224301 or 224303, preferably according to SEQ ID NO: 224297 or SEQ ID NO: 224303 or a homolog, a fragment or a variant thereof;
e) optionally a poly(A) sequence preferably comprising 64 adenosines; and
f) optionally a poly(C) sequence, preferably comprising 30 cytosines.

### Histone stem-loop:

In a particularly preferred embodiment, the mRNA sequence of the mRNA compound according to the invention comprises a histone stem-loop sequence/structure. Such histone stem-loop sequences are preferably selected from histone stem-loop sequences as disclosed in WO2012/019780, the disclosure of which is incorporated herewith by reference.

A histone stem-loop sequence, suitable to be used within the present invention, is preferably selected from at least one of the following formulae (V) or (VI): wherein:
stem1 or stem2 bordering elements N₁₋₆ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
stem1 [N₀₋₂GN₃₋₅] is reverse complementary or partially reverse complementary with element stem2, and is a consecutive sequence between of 5 to 7 nucleotides;
wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
wherein G is guanosine or an analogue thereof, and may be optionally replaced by a cytidine or an analogue thereof, provided that its complementary nucleotide cytidine in stem2 is replaced by guanosine;
loop sequence [N₀₋₄(U/T)N₀₋₄] is located between elements stem1 and stem2, and is a consecutive sequence of 3 to 5 nucleotides, more preferably of 4 nucleotides;
wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
wherein U/T represents uridine, or optionally thymidine;
stem2 [N₃₋₅CN₀₋₂] is reverse complementary or partially reverse complementary with element stem1, and is a consecutive sequence between of 5 to 7 nucleotides;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
wherein N₀₋₂ is a consecutive sequence of 0 to 2, preferably of 0 to 1, more preferably of 1 N, wherein each N is independently from another selected from a nucleotide selected from A, U, T, G or C or a nucleotide analogue thereof; and
wherein C is cytidine or an analogue thereof, and may be optionally replaced by a guanosine or an analogue thereof provided that its complementary nucleoside guanosine in stem1 is replaced by cytidine;
wherein
stem1 and stem2 are capable of base pairing with each other forming a reverse complementary sequence,
wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one or more bases in one stem do not have a complementary base in the reverse complementary sequence of the other stem.

According to a further preferred embodiment the inventive mRNA sequence of the mRNA compound may comprise at least one histone stem-loop sequence according to at least one of the following specific formulae (Va) or (VIa): wherein:
N, C, G, T and Uare as defined above.

According to a further more particularly preferred embodiment, the at least one mRNA of the inventive composition may comprise at least one histone stem-loop sequence according to at least one of the following specific formulae (Vb) or (VIb): wherein:
N, C, G, T and U are as defined above.

A particular preferred histone stem-loop sequence is the sequence CAAAGGCTCTTTTCAGAGCCACCA (according to SEQ ID NO: 224305) or more preferably the corresponding RNA sequence CAAAGGCUCUUUUCAGAGCCACCA (according to SEQ ID NO: 224306).

Any of the above modifications may be applied to the mRNA compound comprising an mRNA sequence of the present invention, and further to any mRNA as used in the context of the present invention and may be, if suitable or necessary, be combined with each other in any combination, provided, these combinations of modifications do not interfere with each other in the respective mRNA sequence. A person skilled in the art will be able to take his choice accordingly.

The mRNA compound comprising an mRNA sequence according to the invention, which comprises at least one coding region as defined herein, may preferably comprise a 5'-UTR and/or a 3'-UTR preferably containing at least one histone stem-loop. The 3'-UTR of the mRNA sequence according to the invention preferably comprises also a poly(A) and/or a poly(C) sequence as defined herein. The single elements of the 3'-UTR may occur therein in any order from 5' to 3' along the sequence of the mRNA sequence of the present invention. In addition, further elements as described herein, may also be contained, such as a stabilizing sequence as defined herein (e.g. derived from the UTR of a globin gene), IRES sequences, etc. Each of the elements may also be repeated in the mRNA sequence according to the invention at least once (particularly in di- or multicistronic constructs), preferably twice or more. As an example, the single elements may be present in the mRNA sequence according to the invention in the following order:
5' - coding region - histone stem-loop - poly(A)/(C) sequence - 3'; or
5' - coding region - poly(A)/(C) sequence - histone stem-loop - 3'; or
5' - coding region - histone stem-loop - polyadenylation signal - 3'; or
5' - coding region - polyadenylation signal - histone stem-loop - 3'; or
5' - coding region - histone stem-loop - histone stem-loop - poly(A)/(C) sequence - 3'; or
5' - coding region - histone stem-loop - histone stem-loop - polyadenylation signal - 3'; or
5' - coding region - stabilizing sequence - poly(A)/(C) sequence - histone stem-loop - 3'; or
5' - coding region - stabilizing sequence - poly(A)/(C) sequence - poly(A)/(C) sequence - histone stem-loop - 3'; etc.

According to a further embodiment, the mRNA compound comprising an mRNA sequence of the present invention preferably comprises at least one of the following structural elements: a 5'- and/or 3'- untranslated region element (UTR element), particularly a 5'-UTR element, which preferably comprises or consists of a nucleic acid sequence which is derived from the 5'-UTR of a TOP gene or from a fragment, homolog or a variant thereof, or a 5'- and/or 3'-UTR element which may preferably be derivable from a gene that provides a stable mRNA or from a homolog, fragment or variant thereof; a histone-stem-loop structure, preferably a histone-stem-loop in its 3' untranslated region; a 5'-CAP structure; a poly-A tail; or a poly(C) sequence.

In one embodiment the mRNA compound comprising an mRNA sequence comprises, preferably in 5'- to 3'-direction:
a) a 5'-CAP structure, preferably m7GpppN;
b) at least one coding region encoding at least one antigenic peptide or protein,
c) optionally a 3'-UTR element comprising or consisting of a nucleic acid sequence which is derived from an alpha globin gene, preferably comprising the corresponding RNA sequence of the nucleic acid sequence according to SEQ ID NOs: 224291, 224293, or 224297, preferably according to SEQ ID NO: 224297, or a homolog, a fragment or a variant thereof;
d) optionally, a poly(A) sequence, preferably comprising 64 adenosines;
e) optionally, a poly(C) sequence, preferably comprising 30 cytosines; and
f) optionally, a histone stem-loop, preferably comprising the RNA sequence according to SEQ ID NO: 224306.

In a particularly preferred embodiment the mRNA compound comprising an mRNA sequence comprises, preferably in 5'- to 3'-direction:
a) a 5'-CAP structure, preferably m7GpppN;
b) at least one coding region encoding at least one antigenic peptide or protein derived from a protein of an influenza virus or a Rabies virus or a fragment or variant thereof, preferably comprising or consisting of any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Tables 1-5 or Figures 20-24 of PCT/EP2016/075843, SEQ ID NOs: 32013-64024, or SEQ ID NOs: 64025-224084 or of a fragment or variant of any one of these sequences,
c) optionally a 3'-UTR element comprising or consisting of a nucleic acid sequence which is derived from an alpha globin gene, preferably comprising the corresponding RNA sequence of the nucleic acid sequence according to SEQ ID NOs: 224291, 224293, or 224297, preferably according to SEQ ID NO: 224297, or a homolog, a fragment or a variant thereof;
d) optionally, a poly(A) sequence, preferably comprising 64 adenosines;
e) optionally, a poly(C) sequence, preferably comprising 30 cytosines; and
f) optionally, a histone stem-loop, preferably comprising the RNA sequence according to SEQ ID NO: 224306.

According to another particularly preferred embodiment the mRNA compound comprising an mRNA sequence according to the invention comprises, preferably in 5'- to 3'-direction:
a) a 5'-CAP structure, preferably m7GpppN;
b) a 5'-UTR element which comprises or consists of a nucleic acid sequence which is derived from the 5'-UTR of a TOP gene, preferably comprising or consisting of the corresponding RNA sequence of a nucleic acid sequence according to SEQ ID NO: 224287 or SEQ ID NO: 224289 as shown in SEQ ID NO: 224288 or SEQ ID NO: 224290, a homolog, a fragment or a variant thereof;
c) at least one coding region encoding at least one antigenic peptide or protein preferably derived from a protein of an influenza virus or a Rabies virus or a fragment or variant thereof, preferably comprising or consisting of any one of the nucleic acid sequences as disclosed in the sequence listing having a numeric identifier <223> which starts with "derived and/or modified CDS sequence (wt)" or "derived and/or modified CDS sequence (opt1)", "derived and/or modified CDS sequence (opt2)", "derived and/or modified CDS sequence (opt3)", "derived and/or modified CDS sequence (opt4)", or "derived and/or modified CDS sequence (opt5)", or respectively "column B" or "column C" of Tables 1-5 or Figures 20-24 of PCT/EP2016/075843, SEQ ID NOs: 32013-64024, or SEQ ID NOs: 64025-224084 or of a fragment or variant of any one of these sequences,
d) optionally a 3'-UTR element comprising or consisting of a nucleic acid sequence which is derived from a gene providing a stable mRNA, preferably comprising or consisting of the corresponding RNA sequence of a nucleic acid sequence according to SEQ ID NO: 224301 or SEQ ID NO: 224303 as shown in SEQ ID NO: 224302 or SEQ ID NO: 224304 , a homolog, a fragment or a variant thereof;
e) optionally a poly(A) sequence preferably comprising 64 adenosines;
f) optionally a poly(C) sequence, preferably comprising 30 cytosines; and
g) optionally, a histone stem-loop, preferably comprising the RNA sequence according to SEQ ID NO: 224306.

In particularly preferred embodiments the mRNA compound comprising an mRNA sequence according to the invention comprises the following mRNA sequences (or RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the following RNA sequences):
Influenza A HA:
   - mRNA encoding HA protein of influenza A/ Vietnam/1194/2004 (H5N1) according to SEQ ID NOs: 224198-224201, 224203-224210.
   - mRNA encoding HA protein of influenza A/Hong Kong/4801/2014 (H3N2) according to SEQ ID NOs: SEQ ID NOs: 224181-224194.
   - mRNA encoding HA protein of influenza A/Netherlands/602/2009 (H1N1) according to SEQ ID NOs: 224163-224175.
   - mRNA encoding HA protein of influenza A/California/07/2009 (H1N1) according to SEQ ID NOs: 224117-224126, 224129, 224130, 224131, 224132
   - mRNA encoding HA protein of influenza A/Michigan/45/2015 (H1N1)pdm09-like virus according to SEQ ID NOs: 224133-224142-224162.
Influenza B HA:
   - mRNA encoding HA protein of influenza B/Phuket/3037/2013 according to SEQ ID NOs: 224246-224255, 224256, 224257.
   - mRNA encoding HA protein of influenza B/Brisbane/60/2008 (GI: 223950973; FJ766840.1) according to SEQ ID NOs: 224236-224245.
Influenza A NA:
   - mRNA encoding NA protein of influenza A/California/07/2009 (H1N1) according to SEQ ID NOs: 224319-224323.
   - mRNA encoding NA protein of influenza A/Michigan/45/2015 (H1N1)pdm09-like virus according to SEQ ID NOs: 224324-224325.
   - mRNA encoding NA protein of influenza A/Netherlands/602/2009 (H1N1) according to SEQ ID NOs: 224326-224335.
   - mRNA encoding NA protein of influenza A/Hong Kong/4801/2014 (H3N2) according to SEQ ID NOs: 224336-224339.
   - mRNA encoding NA protein of influenza A/ Vietnam/1194/2004 (H5N1) according to SEQ ID NOs: 224342-224343.
   - mRNA encoding NA protein of influenza A/Vietnam/1203/2004 (H5N1) according to SEQ ID NOs: 224344-224345.
Influenza B NA:
   - mRNA encoding NA protein of influenza B/Brisbane/60/2008 (GI: 223950973; FJ766840.1) according to SEQ ID NOs: 224348-224349.
   - mRNA encoding NA protein of influenza B/Phuket/3037/2013 according to SEQ ID NOs: 224350-224351.

Most preferred mRNA sequences include:
An mRNA sequence comprising at least one coding region encoding at least one antigenic peptide or protein derived from hemagglutinin (HA) of an influenza A virus according to SEQ ID NOs: 1-14031 or a fragment or variant thereof.

An mRNA sequence being identical or at least 50%, 60%, 70%, 80%, 85%, 86%, 87%, 88%, 89%, 90%, 91%, 92%, 93%, 94%, 95%, 96%, 97%, 98%, or 99% identical to an RNA sequence according to SEQ ID NOs: 32013-46043, 64025-78055, 224085-224106, 96037-110067, 128049-142079, 160061-174091, 192073-206103 or a fragment or variant thereof.

An mRNA sequence comprising at least one coding region encoding at least one antigenic peptide or protein derived from hemagglutinin (HA) of an influenza B virus according to SEQ ID NOs: 26398-28576 or a fragment or variant thereof.

An mRNA sequence being identical or at least 50%, 60%, 70%, 80%, 85%, 86%, 87%, 88%, 89%, 90%, 91%, 92%, 93%, 94%, 95%, 96%, 97%, 98%, or 99% identical to an RNA sequence according to SEQ ID NOs: 58410-60588, 90422-92600, 224107-224112, 122434-124612, 154446-156624, 186458-188636, 218470-220648 or a fragment or variant thereof.

An mRNA sequence comprising at least one coding region encoding at least one antigenic peptide or protein derived from neuraminidase (NA) of an influenza A virus according to SEQ ID NOs: 14032-26397, 224309, or 224310 or a fragment or variant thereof.

An mRNA sequence being identical or at least 50%, 60%, 70%, 80%, 85%, 86%, 87%, 88%, 89%, 90%, 91%, 92%, 93%, 94%, 95%, 96%, 97%, 98%, or 99% identical to an RNA sequence according to SEQ ID NOs: 110068-122433, 78056-90421, 224113, 224313-224317, 110068-122433, 142080-154445, 174092-186457, 206104-218469 or a fragment or variant thereof.

An mRNA sequence comprising at least one coding region encoding at least one antigenic peptide or protein derived from neuraminidase (NA) of an influenza B virus according to SEQ ID NOs: 28577-30504 or a fragment or variant thereof.

An mRNA sequence being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs: 60589-62516, 92601-94528, 124613-126540, 156625-158552, 188637-190564, 220649-222576 or a fragment or variant thereof.

An mRNA sequence comprising at least one coding region encoding at least one antigenic peptide or protein derived from glycoprotein G (RAV-G, RAVBV-G or RABV-G), nucleoprotein N (RAV-N), phospoprotein P (RAV-P), matrix protein M (RAV-M) or RNA polymerase L (RAV-L) of a Rabies virus or a fragment, variant thereof.

An mRNA sequence comprising at least one coding region encoding at least one antigenic peptide or protein derived from glycoprotein G (RAV-G, RAVBV-G or RABV-G) of a Rabies virus according to SEQ ID NOs: 30505-32012 or a fragment or variant thereof.

An mRNA sequence comprising at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs: 62517-64024, 224270, 224274, 94529-96036, 224271-224273, 126541-128048, 158553-160060, 190565-192072, 222577-224084 or a fragment or variant thereof.

### Signal peptides:

According to another particularly preferred embodiment, the mRNA sequence according to the invention may additionally or alternatively encode a secretory signal peptide. Such signal peptides are sequences, which typically exhibit a length of about 15 to 30 amino acids and are preferably located at the N-terminus of the encoded peptide, without being limited thereto. Signal peptides as defined herein preferably allow the transport of the antigen, antigenic protein or antigenic peptide as encoded by the at least one mRNA sequence into a defined cellular compartiment, preferably the cell surface, the endoplasmic reticulum (ER) or the endosomal-lysosomal compartiment. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201), signal sequences of cytokines or immunoglobulines as defined herein, signal sequences of the invariant chain of immunoglobulines or antibodies as defined herein, signal sequences of Lamp1, Tapasin, Erp57, Calretikulin, Calnexin, and further membrane associated proteins or of proteins associated with the endoplasmic reticulum (ER) or the endosomal-lysosomal compartiment. Most preferably, signal sequences of MHC class I molecule HLA-A*0201 may be used according to the present invention. For example, a signal peptide derived from HLA-A is preferably used in order to promote secretion of the encoded antigen as defined herein or a fragment or variant thereof. More preferably, an HLA-A signal peptide is fused to an encoded antigen as defined herein or to a fragment or variant thereof.

### Production of mRNA:

The mRNA according to the present invention may be prepared using any method known in the art, including synthetic methods such as e.g. solid phase RNA synthesis, as well as in vitro methods, such as RNA in vitro transcription reactions, particularly as described in the examples.

As noted, the mRNA compound according to the invention in encapsulated in or associated with a lipid nanoparticle.

The term "lipid nanoparticle", also referred to as LNP, refers to a particle having at least one dimension on the order of nanometers (e.g., 1-1,000 nm) which includes one or more lipids, for example a lipid of Formula (I), (II) or (III). In some embodiments, such lipid nanoparticles comprise a cationic lipid (e.g., a lipid of Formula (I), (II) or (III)) and one or more excipient selected from neutral lipids, charged lipids, steroids and polymer conjugated lipids (e.g., a pegylated lipid such as a pegylated lipid of formula (IV)). In some embodiments, the mRNA, or a portion thereof, is encapsulated in the lipid portion of the lipid nanoparticle or an aqueous space enveloped by some or all of the lipid portion of the lipid nanoparticle, thereby protecting it from enzymatic degradation or other undesirable effects induced by the mechanisms of the host organism or cells e.g. an adverse immune response. In some embodiments, the mRNA or a portion thereof is associated with the lipid nanoparticles.

In the context of the present invention, lipid nanoparticles are not restricted to any particular morphology, and should be interpreted as to include any morphology generated when a cationic lipid and optionally one or more further lipids are combined, e.g. in an aqueous environment and/or in the presence of a nucleic acid compound. For example, a liposome, a lipid complex, a lipoplex and the like are within the scope of a lipid nanoparticle.

In various embodiments, the lipid nanoparticles have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm, and are substantially non-toxic. In certain embodiments, the mRNA, when present in the lipid nanoparticles, is resistant in aqueous solution to degradation with a nuclease. As used herein, the mean diameter may be represented by the z-average as determined by dynamic light scattering.

An LNP may comprise any lipid capable of forming a particle to which the one or more nucleic acid molecules are attached, or in which the one or more nucleic acid molecules are encapsulated. The term "lipid" refers to a group of organic compounds that are derivatives of fatty acids (e.g., esters) and are generally characterized by being insoluble in water but soluble in many organic solvents. Lipids are usually divided in at least three classes: (1) "simple lipids" which include fats and oils as well as waxes; (2) "compound lipids" which include phospholipids and glycolipids; and (3) "derived lipids" such as steroids.

In one embodiment, the mRNA-comprising LNP comprises one or more cationic lipids as defined herein, and one or more stabilizing lipids. Stabilizing lipids include neutral lipids and pegylated lipids.

As mentioned, the LNP comprises a cationic lipid. The cationic lipid is preferably cationisable, i.e. it becomes protonated as the pH is lowered below the pKa of the ionizable group of the lipid, but is progressively more neutral at higher pH values. When positively charged,, the lipid is then able to associate with negatively charged nucleic acids. In certain embodiments, the cationic lipid comprises a zwitterionic lipid that assumes a positive charge on pH decrease. The LNP may comprise any lipid capable of forming a particle to which the one or more nucleic acid molecules are attached, or in which the one or more nucleic acid molecules are encapsulated.

In certain embodiments, the LNP may comprise any further cationic or cationisable lipid, i.e. any of a number of lipid species which carry a net positive charge at a selective pH, such as physiological pH. Such lipids include, but are not limited to, N,N-dioleyl-N,N-dimethylammonium chloride (DODAC); N-(2,3-dioleyloxy)propyl)-N,N,N-trimethylammonium chloride (DOTMA); N,N-distearyl-N,N-dimethylammonium bromide (DDAB); N-(2,3dioleoyloxy)propyl)-N,N,N-trimethylammonium chloride (DOTAP); 3-(N-(N',N'dimethylaminoethane)-carbamoyl)cholesterol (DC-Chol), N-(1-(2,3-dioleoyloxy)propyl)N-2-(sperminecarboxamido)ethyl)-N,N-dimethylammonium trifluoracetate (DOSPA), dioctadecylamidoglycyl carboxyspermine (DOGS), 1,2-dioleoyl-3-dimethylammonium propane (DODAP), N,N-dimethyl-2,3-dioleoyloxy)propylamine (DODMA), and N-(1,2dimyristyloxyprop-3-yl)-N,N-dimethyl-N-hydroxyethyl ammonium bromide (DMRIE).

Additionally, a number of commercial preparations of cationic lipids are available which can be used in the present invention. These include, for example, LIPOFECTIN^{®} (commercially available cationic liposomes comprising DOTMA and 1,2-dioleoyl-sn-3phosphoethanolamine (DOPE), from GIBCO/BRL, Grand Island, N.Y.); LIPOFECTAMINE^{®} (commercially available cationic liposomes comprising N-(1-(2,3dioleyloxy)propyl)-N-(2-(sperminecarboxamido)ethyl)-N,N-dimethylammonium trifluoroacetate (DOSPA) and (DOPE), from GIBCO/BRL); and TRANSFECTAM^{®} (commercially available cationic lipids comprising dioctadecylamidoglycyl carboxyspermine (DOGS) in ethanol from Promega Corp., Madison, Wis.). The following lipids are cationic and have a positive charge at below physiological pH: DODAP, DODMA, DMDMA, 1,2-dilinoleyloxy-N,N-dimethylaminopropane (DLinDMA), 1,2-dilinolenyloxy-N,N-dimethylaminopropane (DLenDMA).

In one embodiment, the further cationic lipid is an amino lipid. Suitable amino lipids useful in the invention include those described in WO2012/016184, incorporated herein by reference in its entirety. Representative amino lipids include, but are not limited to, 1,2-dilinoleyoxy-3-(dimethylamino)acetoxypropane (DLin-DAC), 1,2-dilinoleyoxy-3morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3dimethylaminopropane (DLin-2-DMAP), 1,2-dilinoleyloxy-3-trimethylaminopropane chloride salt (DLin-TMA.CI), 1,2-dilinoleoyl-3-trimethylaminopropane chloride salt (DLin-TAP.CI), 1,2-dilinoleyloxy-3-(N-methylpiperazino)propane (DLin-MPZ), 3-(N,Ndilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), and 2,2-dilinoleyl-4-dimethylaminomethyl-[1,3]-dioxolane (DLin-K-DMA).

Suitable amino lipids include those having the formula:
wherein R₁ and R₂ are either the same or different and independently optionally substituted C₁₀-C₂₄ alkyl, optionally substituted C₁₀-C₂₄ alkenyl, optionally substituted C₁₀-C₂₄ alkynyl, or optionally substituted C₁₀-C₂₄ acyl;
R₃ and R₄ are either the same or different and independently optionally substituted C₁-C₆ alkyl, optionally substituted C₂-C₆ alkenyl, or optionally substituted C₂-C₆ alkynyl or R₃ and R₄ may join to form an optionally substituted heterocyclic ring of 4 to 6 carbon atoms and 1 or 2 heteroatoms chosen from nitrogen and oxygen;
R₅ is either absent or present and when present is hydrogen or C₁-C₆ alkyl; m, n, and p are either the same or different and independently either 0 or 1 with the proviso that m, n, and p are not simultaneously 0; q is 0, 1, 2, 3, or 4; and
Y and Z are either the same or different and independently O, S, or NH. In one embodiment, R₁ and R₂ are each linoleyl, and the amino lipid is a dilinoleyl amino lipid. In one embodiment, the amino lipid is a dilinoleyl amino lipid.

A representative useful dilinoleyl amino lipid has the formula: wherein n is 0, 1, 2, 3, or 4.

In one embodiment, the cationic lipid is a DLin-K-DMA. In one embodiment, the cationic lipid is DLin-KC2-DMA (DLin-K-DMA above, wherein n is 2).

In one embodiment, the LNP comprises (i) a cationic lipid component of Formula (I): as defined below or a pharmaceutically acceptable salt, tautomer, prodrug or stereoisomer thereof, and (ii) a mRNA compound comprising an mRNA sequence encoding at least one antigenic peptide or protein, wherein the mRNA compound is encapsulated in or associated with said lipid nanoparticle. With respect to the mRNA compound, the mRNA sequence and the antigenic peptide or protein, reference is made to the description of these features including the respective options and preferences above. In one of the preferred embodiments, the mRNA compound does not comprise a nucleoside modification. In another embodiment, it comprises no base modification. In a further embodiment, it does not comprise a 1-methylpseudouridine modification. In a further embodiment the mRNA compound only comprises the natural nucleosides adenine, guanine, cytosine and uracil. Moreover, if the cationic lipid is compound I-6 as defined below, the lipid nanoparticle is not a lipid nanoparticle comprising compound I-6, DSPC, cholesterol and the PEG lipid of formula (IVa) at a ratio of about 50:10:38.5:1.5 that encapsulates unmodified, 1-methylpseudouridine modified or codon-optimized mRNA encoding an influenza PR8 or Cal/7/2009 hemagglutinin or an HIV-1 CD4-independent R3A envelop protein.

With respect to Formula (I):
L¹ and L² are each independently -O(C=O)-, -(C=O)O- or a carbon-carbon double bond;
R^{1a} and R^{1b} are, at each occurrence, independently either (a) H or C₁-C₁₂ alkyl, or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either (a) H or C₁-C₁₂ alkyl, or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either (a) H or C₁-C₁₂ alkyl, or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either (a) H or C₁-C₁₂ alkyl, or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently methyl or cycloalkyl;
R⁷ is, at each occurrence, independently H or C₁-C₁₂ alkyl;
R⁸ and R⁹ are each independently C₁-C₁₂ alkyl; or R⁸ and R⁹, together with the nitrogen atom to which they are attached, form a 5, 6 or 7-membered heterocyclic ring comprising one nitrogen atom;
a and d are each independently an integer from 0 to 24;
b and c are each independently an integer from 1 to 24; and
e is 1 or 2.

In certain embodiments of Formula (I), at least one of R^{1a}, R^{2a}, R^{3a} or R^{4a} is C₁-C₁₂ alkyl, or at least one of L¹ or L² is -O(C=O)- or -(C=O)O-. In other embodiments, R^{1a} and R^{1b} are not isopropyl when a is 6 or n-butyl when a is 8.

In still further embodiments of Formula (I), at least one of R^{1a}, R^{2a}, R^{3a} or R^{4a} is C₁-C₁₂ alkyl, or at least one of L¹ or L² is -O(C=O)- or -(C=O)O-; and
R^{1a} and R^{1b} are not isopropyl when a is 6 or n-butyl when a is 8.

In other embodiments of Formula (I), R⁸ and R⁹ are each independently unsubstituted C₁-C₁₂ alkyl; or R⁸ and R⁹, together with the nitrogen atom to which they are attached, form a 5, 6 or 7-membered heterocyclic ring comprising one nitrogen atom;

In certain embodiments of Formula (I), any one of L¹ or L² may be -O(C=O)- or a carbon-carbon double bond. L¹ and L² may each be -O(C=O)- or may each be a carbon-carbon double bond.

In some embodiments of Formula (I), one of L¹ or L² is -O(C=O)-. In other embodiments, both L¹ and L² are - O(C=O)-.

In some embodiments of Formula (I), one of L¹ or L² is -(C=O)O-. In other embodiments, both L¹ and L² are - (C=O)O-.

In some other embodiments of Formula (I), one of L¹ or L² is a carbon-carbon double bond. In other embodiments, both L¹ and L² are a carbon-carbon double bond.

In still other embodiments of Formula (I), one of L¹ or L² is -O(C=O)- and the other of L¹ or L² is -(C=O)O-. In more embodiments, one of L¹ or L² is -O(C=O)- and the other of L¹ or L² is a carbon-carbon double bond. In yet more embodiments, one of L¹ or L² is -(C=O)O- and the other of L¹ or L² is a carbon-carbon double bond.

It is understood that "carbon-carbon" double bond, as used throughout the specification, refers to one of the following structures: wherein R^{a} and R^{b} are, at each occurrence, independently H or a substituent. For example, in some embodiments R^{a} and R^{b} are, at each occurrence, independently H, C₁-C₁₂ alkyl or cycloalkyl, for example H or C₁-C₁₂ alkyl.

In other embodiments, the lipid compounds of Formula (I) have the following structure (Ia):

In other embodiments, the lipid compounds of Formula (I) have the following structure (Ib):

In yet other embodiments, the lipid compounds of Formula (I) have the following structure (Ic):

In certain embodiments of the lipid compound of Formula (I), a, b, c and d are each independently an integer from 2 to 12 or an integer from 4 to 12. In other embodiments, a, b, c and d are each independently an integer from 8 to 12 or 5 to 9. In some certain embodiments, a is 0. In some embodiments, a is 1. In other embodiments, a is 2. In more embodiments, a is 3. In yet other embodiments, a is 4. In some embodiments, a is 5. In other embodiments, a is 6. In more embodiments, a is 7. In yet other embodiments, a is 8. In some embodiments, a is 9. In other embodiments, a is 10. In more embodiments, a is 11. In yet other embodiments, a is 12. In some embodiments, a is 13. In other embodiments, a is 14. In more embodiments, a is 15. In yet other embodiments, a is 16.

In some other embodiments of Formula (I), b is 1. In other embodiments, b is 2. In more embodiments, b is 3. In yet other embodiments, b is 4. In some embodiments, b is 5. In other embodiments, b is 6. In more embodiments, b is 7. In yet other embodiments, b is 8. In some embodiments, b is 9. In other embodiments, b is 10. In more embodiments, b is 11. In yet other embodiments, b is 12. In some embodiments, b is 13. In other embodiments, b is 14. In more embodiments, b is 15. In yet other embodiments, b is 16.

In some more embodiments of Formula (I), c is 1. In other embodiments, c is 2. In more embodiments, c is 3. In yet other embodiments, c is 4. In some embodiments, c is 5. In other embodiments, c is 6. In more embodiments, c is 7. In yet other embodiments, c is 8. In some embodiments, c is 9. In other embodiments, c is 10. In more embodiments, c is 11. In yet other embodiments, c is 12. In some embodiments, c is 13. In other embodiments, c is 14. In more embodiments, c is 15. In yet other embodiments, c is 16.

In some certain other embodiments of Formula (I), d is 0. In some embodiments, d is 1. In other embodiments, d is 2. In more embodiments, d is 3. In yet other embodiments, d is 4. In some embodiments, d is 5. In other embodiments, d is 6. In more embodiments, d is 7. In yet other embodiments, d is 8. In some embodiments, d is 9. In other embodiments, d is 10. In more embodiments, d is 11. In yet other embodiments, d is 12. In some embodiments, d is 13. In other embodiments, d is 14. In more embodiments, d is 15. In yet other embodiments, d is 16.

In some other various embodiments of Formula (I), a and d are the same. In some other embodiments, b and c are the same. In some other specific embodiments, a and d are the same and b and c are the same.

The sum of a and b and the sum of c and d in Formula (I) are factors which may be varied to obtain a lipid of formula I having the desired properties. In one embodiment, a and b are chosen such that their sum is an integer ranging from 14 to 24. In other embodiments, c and d are chosen such that their sum is an integer ranging from 14 to 24. In further embodiment, the sum of a and b and the sum of c and d are the same. For example, in some embodiments the sum of a and b and the sum of c and d are both the same integer which may range from 14 to 24. In still more embodiments, a. b, c and d are selected such the sum of a and b and the sum of c and d is 12 or greater.

In some embodiments of Formula (I), e is 1. In other embodiments, e is 2.

The substituents at R^{1a}, R^{2a}, R^{3a} and R^{4a} of Formula (I) are not particularly limited. In certain embodiments R^{1a}, R^{2a}, R^{3a} and R^{4a} are H at each occurrence. In certain other embodiments at least one of R^{1a}, R^{2a}, R^{3a} and R^{4a} is C₁-C₁₂ alkyl. In certain other embodiments at least one of R^{1a}, R^{2a}, R^{3a} and R^{4a} is C₁-C₈ alkyl. In certain other embodiments at least one of R^{1a}, R^{2a} R^{3a} and R^{4a} is C₁-C₆ alkyl. In some of the foregoing embodiments, the C₁-C₈ alkyl is methyl, ethyl, n-propyl, iso-propyl, n-butyl, iso-butyl, tert-butyl, n-hexyl or n-octyl.

In certain embodiments of Formula (I), R^{1a}, R^{1b}, R^{4a} and R^{4b} are C₁-C₁₂ alkyl at each occurrence.

In further embodiments of Formula (I), at least one of R^{1b}, R^{2b} R^{3b} and R^{4b} is H or R^{1b}, R^{2b}, R^{3b} and R^{4b} are H at each occurrence.

In certain embodiments of Formula (I), R^{1b} together with the carbon atom to which it is bound is taken together with an adjacent R^{1b} and the carbon atom to which it is bound to form a carbon-carbon double bond. In other embodiments of the foregoing R^{4b} together with the carbon atom to which it is bound is taken together with an adjacent R^{4b} and the carbon atom to which it is bound to form a carbon-carbon double bond.

The substituents at R⁵ and R⁶ of Formula (I) are not particularly limited in the foregoing embodiments. In certain embodiments one or both of R⁵ or R⁶ is methyl. In certain other embodiments one or both of R⁵ or R⁶ is cycloalkyl for example cyclohexyl. In these embodiments the cycloalkyl may be substituted or not substituted. In certain other embodiments the cycloalkyl is substituted with C₁-C₁₂alkyl, for example tert-butyl.

The substituents at R⁷ are not particularly limited in the foregoing embodiments of Formula (I). In certain embodiments at least one R⁷ is H. In some other embodiments, R⁷ is H at each occurrence. In certain other embodiments R⁷ is C₁-C₁₂ alkyl.

In certain other of the foregoing embodiments of Formula (I), one of R⁸ or R⁹ is methyl. In other embodiments, both R⁸ and R⁹ are methyl.

In some different embodiments of Formula (I), R⁸ and R⁹, together with the nitrogen atom to which they are attached, form a 5, 6 or 7-membered heterocyclic ring. In some embodiments of the foregoing, R⁸ and R⁹, together with the nitrogen atom to which they are attached, form a 5-membered heterocyclic ring, for example a pyrrolidinyl ring.

In various different embodiments, the lipid of Formula (I) has one of the structures set forth in Table 7 ("Representative Lipids of Formula (I)") below.

**Table 7: Representative Lipids of Formula (I)**

| **No.** | **Structure** | **Prep. Method** |
|---|---|---|
| I-1 | | B |
| I-2 | | A |
| I-3 | | A |
| I-4 | | B |
| I-5 | | B |
| I-6 | | B |
| I-7 | | A |
| I-8 | | A |
| I-9 | | B |
| I-10 | | A |
| I-11 | | A |
| I-12 | | A |
| I-13 | | A |
| I-14 | | A |
| I-15 | | A |
| I-16 | | A |
| I-17 | | A |
| I-18 | | A |
| I-19 | | A |
| I-20 | | A |
| I-21 | | A |
| I-22 | | A |
| I-23 | | A |
| I-24 | | A |
| I-25 | | A |
| I-26 | | A |
| I-27 | | A |
| I-28 | | A |
| I-29 | | A |
| I-30 | | A |
| I-31 | | C |
| I-32 | | C |
| I-33 | | C |
| I-34 | | B |
| I-35 | | B |
| I-36 | | C |
| I-37 | | C |
| I-38 | | B |
| I-39 | | B |
| I-40 | | B |
| I-41 | | B |

In some embodiments, the LNPs comprise a lipid of Formula (I), a mRNA compound as defined herein and one or more excipient selected from neutral lipids, steroids and pegylated lipids. In some embodiments the lipid of Formula (I) is compound I-5. In some embodiments the lipid of Formula (I) is compound I-6.

In another embodiment, the lipid nanoparticle comprises (i) a cationic lipid with the structure of Formula (II):

as further defined below or a pharmaceutically acceptable salt, tautomer, prodrug or stereoisomer thereof, and (ii) a mRNA compound comprising an mRNA sequence encoding at least one antigenic peptide or protein,
wherein the mRNA compound is encapsulated in or associated with said lipid nanoparticle. With respect to the mRNA compound, the mRNA sequence and the antigenic peptide or protein, reference is made to the description of these features including the respective options and preferences above. In one of the preferred embodiments, the mRNA compound does not comprise a nucleoside modification. In another embodiment, it comprises no base modification. In a further embodiment, it does not comprise a 1-methylpseudouridine modification. In a further embodiment the mRNA compound only comprises the naturally existing nucleosides adenine, guanine, cytosine and uracil.

Formula (II) is further defined in that:
L¹ and L² are each independently -O(C=O)-, -(C=O)O-, -C(=O)-, -O-, -S(O)ₓ-, -S-S-, -C(=O)S-, -SC(=O)-, -NR^{a}C(=O)-, -C(=O)NR^{a}-, -NR^{a}C(=O)NR^{a}, -OC(=O)NR^{a}-, -NR^{a}C(=O)O-, or a direct bond;
G¹ is C₁-C₂ alkylene, -(C=O)- , -O(C=O)-, -SC(=O)-, -NR^{a}C(=O)- or a direct bond;
G² is -C(=O)-, -(C=O)O-, -C(=O)S-, -C(=O)NR^{a} or a direct bond;
G³ is C₁-C₆ alkylene;
R^{a} is H or C₁-C₁₂ alkyl;
R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
R^{3a} and R^{3b} are, at each occurrence, independently either: (a) H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
R⁵ and R⁶ are each independently H or methyl;
R⁷ is C₄-C₂₀ alkyl;
R⁸ and R⁹ are each independently C₁-C₁₂ alkyl; or R⁸ and R⁹, together with the nitrogen atom to which they are attached, form a 5, 6 or 7-membered heterocyclic ring;
a, b, c and d are each independently an integer from 1 to 24; and
x is 0, 1 or 2.

In some embodiments of Formula (II), L¹ and L² are each independently
-O(C=O)-, -(C=O)O- or a direct bond. In other embodiments, G¹ and G² are each independently -(C=O)- or a direct bond. In some different embodiments, L¹ and L² are each independently -O(C=O)-, -(C=O)O- or a direct bond; and G¹ and G² are each independently -(C=O)- or a direct bond.

In some different embodiments of Formula (II), L¹ and L² are each independently -C(=O)-, -O-, -S(O)ₓ-, -S-S-, -C(=O)S-, -SC(=O)-, -NR^{a}-, -NR^{a}C(=O)-, -C(=O)NR^{a}-, -NR^{a}C(=O)NR^{a}, -OC(=O)NR^{a}-, -NR^{a}C(=O)O-, -NR^{a}S(O)ₓNR^{a}-, -NR^{a}S(O)ₓ- or -S(O)ₓNR^{a}-.

In other of the foregoing embodiments of Formula (II), the lipid compound has one of the following structures (IIA) or (IIB):

In some embodiments of Formula (II), the lipid compound has structure (IIA). In other embodiments, the lipid compound has structure (IIB).

In any of the foregoing embodiments of Formula (II), one of L¹ or L² is -O(C=O)-. For example, in some embodiments each of L¹ and L² are -O(C=O)-.

In some different embodiments of Formula (II), one of L¹ or L² is -(C=O)O-. For example, in some embodiments each of L¹ and L² is -(C=O)O-.

In different embodiments of Formula (II), one of L¹ or L² is a direct bond. As used herein, a "direct bond" means the group (e.g., L¹ or L²) is absent. For example, in some embodiments each of L¹ and L² is a direct bond.

In other different embodiments of Formula (II), for at least one occurrence of R^{1a} and R^{1b}, R^{1a} is H or C₁-C₁₂ alkyl, and R^{1b} together with the carbon atom to which it is bound is taken together with an adjacent R^{1b} and the carbon atom to which it is bound to form a carbon-carbon double bond.

In still other different embodiments of Formula (II), for at least one occurrence of R^{4a} and R^{4b}, R^{4a} is H or C₁-C₁₂ alkyl, and R^{4b} together with the carbon atom to which it is bound is taken together with an adjacent R^{4b} and the carbon atom to which it is bound to form a carbon-carbon double bond.

In more embodiments of Formula (II), for at least one occurrence of R^{2a} and R^{2b}, R^{2a} is H or C₁-C₁₂ alkyl, and R^{2b} together with the carbon atom to which it is bound is taken together with an adjacent R^{2b} and the carbon atom to which it is bound to form a carbon-carbon double bond.

In other different embodiments of Formula (II), for at least one occurrence of R^{3a} and R^{3b}, R^{3a} is H or C₁-C₁₂ alkyl, and R^{3b} together with the carbon atom to which it is bound is taken together with an adjacent R^{3b} and the carbon atom to which it is bound to form a carbon-carbon double bond.

In various other embodiments of Formula (II), the lipid compound has one of the following structures (IIC) or (IID): or wherein e, f, g and h are each independently an integer from 1 to 12.

In some embodiments of Formula (II), the lipid compound has structure (IIC). In other embodiments, the lipid compound has structure (IID).

In various embodiments of structures (IIC) or (IID), e, f, g and h are each independently an integer from 4 to 10.

In certain embodiments of Formula (II), a, b, c and d are each independently an integer from 2 to 12 or an integer from 4 to 12. In other embodiments, a, b, c and d are each independently an integer from 8 to 12 or 5 to 9. In some certain embodiments, a is 0. In some embodiments, a is 1. In other embodiments, a is 2. In more embodiments, a is 3. In yet other embodiments, a is 4. In some embodiments, a is 5. In other embodiments, a is 6. In more embodiments, a is 7. In yet other embodiments, a is 8. In some embodiments, a is 9. In other embodiments, a is 10. In more embodiments, a is 11. In yet other embodiments, a is 12. In some embodiments, a is 13. In other embodiments, a is 14. In more embodiments, a is 15. In yet other embodiments, a is 16.

In some embodiments of Formula (II), b is 1. In other embodiments, b is 2. In more embodiments, b is 3. In yet other embodiments, b is 4. In some embodiments, b is 5. In other embodiments, b is 6. In more embodiments, b is 7. In yet other embodiments, b is 8. In some embodiments, b is 9. In other embodiments, b is 10. In more embodiments, b is 11. In yet other embodiments, b is 12. In some embodiments, b is 13. In other embodiments, b is 14. In more embodiments, b is 15. In yet other embodiments, b is 16.

In some embodiments of Formula (II), c is 1. In other embodiments, c is 2. In more embodiments, c is 3. In yet other embodiments, c is 4. In some embodiments, c is 5. In other embodiments, c is 6. In more embodiments, c is 7. In yet other embodiments, c is 8. In some embodiments, c is 9. In other embodiments, c is 10. In more embodiments, c is 11. In yet other embodiments, c is 12. In some embodiments, c is 13. In other embodiments, c is 14. In more embodiments, c is 15. In yet other embodiments, c is 16.

In some certain embodiments of Formula (II), d is 0. In some embodiments, d is 1. In other embodiments, d is 2. In more embodiments, d is 3. In yet other embodiments, d is 4. In some embodiments, d is 5. In other embodiments, d is 6. In more embodiments, d is 7. In yet other embodiments, d is 8. In some embodiments, d is 9. In other embodiments, d is 10. In more embodiments, d is 11. In yet other embodiments, d is 12. In some embodiments, d is 13. In other embodiments, d is 14. In more embodiments, d is 15. In yet other embodiments, d is 16.

In some embodiments of Formula (II), e is 1. In other embodiments, e is 2. In more embodiments, e is 3. In yet other embodiments, e is 4. In some embodiments, e is 5. In other embodiments, e is 6. In more embodiments, e is 7. In yet other embodiments, e is 8. In some embodiments, e is 9. In other embodiments, e is 10. In more embodiments, e is 11. In yet other embodiments, e is 12.

In some embodiments of Formula (II), f is 1. In other embodiments, f is 2. In more embodiments, f is 3. In yet other embodiments, f is 4. In some embodiments, f is 5. In other embodiments, f is 6. In more embodiments, f is 7. In yet other embodiments, f is 8. In some embodiments, f is 9. In other embodiments, f is 10. In more embodiments, f is 11. In yet other embodiments, f is 12.

In some embodiments of Formula (II), g is 1. In other embodiments, g is 2. In more embodiments, g is 3. In yet other embodiments, g is 4. In some embodiments, g is 5. In other embodiments, g is 6. In more embodiments, g is 7. In yet other embodiments, g is 8. In some embodiments, g is 9. In other embodiments, g is 10. In more embodiments, g is 11. In yet other embodiments, g is 12.

In some embodiments of Formula (II), h is 1. In other embodiments, e is 2. In more embodiments, h is 3. In yet other embodiments, h is 4. In some embodiments, e is 5. In other embodiments, h is 6. In more embodiments, h is 7. In yet other embodiments, h is 8. In some embodiments, h is 9. In other embodiments, h is 10. In more embodiments, h is 11. In yet other embodiments, h is 12.

In some other various embodiments of Formula (II), a and d are the same. In some other embodiments, b and c are the same. In some other specific embodiments and a and d are the same and b and c are the same.

The sum of a and b and the sum of c and d of Formula (II) are factors which may be varied to obtain a lipid having the desired properties. In one embodiment, a and b are chosen such that their sum is an integer ranging from 14 to 24. In other embodiments, c and d are chosen such that their sum is an integer ranging from 14 to 24. In further embodiment, the sum of a and b and the sum of c and d are the same. For example, in some embodiments the sum of a and b and the sum of c and d are both the same integer which may range from 14 to 24. In still more embodiments, a. b, c and d are selected such that the sum of a and b and the sum of c and d is 12 or greater.

The substituents at R^{1a}, R^{2a}, R^{3a} and R^{4a} of Formula (II) are not particularly limited. In some embodiments, at least one of R^{1a}, R^{2a}, R^{3a} and R^{4a} is H. In certain embodiments R^{1a}, R^{2a}, R^{3a} and R^{4a} are H at each occurrence. In certain other embodiments at least one of R^{1a}, R^{2a}, R^{3a} and R^{4a} is C₁-C₁₂ alkyl. In certain other embodiments at least one of R^{1a}, R^{2a}, R^{3a} and R^{4a} is C₁-C₈ alkyl. In certain other embodiments at least one of R^{1a}, R^{2a}, R^{3a} and R^{4a} is C₁-C₆ alkyl. In some of the foregoing embodiments, the C₁-C₈ alkyl is methyl, ethyl, n-propyl, iso-propyl, n-butyl, iso-butyl, tert-butyl, n-hexyl or n-octyl.

In certain embodiments of Formula (II), R^{1a}, R^{1b}, R^{4a} and R^{4b} are C₁-C₁₂ alkyl at each occurrence.

In further embodiments of Formula (II), at least one of R^{1b}, R^{2b}, R^{3b} and R^{4b} is H or R^{1b}, R^{2b}, R^{3b} and R^{4b} are H at each occurrence.

In certain embodiments of Formula (II), R^{1b} together with the carbon atom to which it is bound is taken together with an adjacent R^{1b} and the carbon atom to which it is bound to form a carbon-carbon double bond. In other embodiments of the foregoing R^{4b} together with the carbon atom to which it is bound is taken together with an adjacent R^{4b} and the carbon atom to which it is bound to form a carbon-carbon double bond.

The substituents at R⁵ and R⁶ of Formula (II) are not particularly limited in the foregoing embodiments. In certain embodiments one of R⁵ or R⁶ is methyl. In other embodiments each of R⁵ or R⁶ is methyl.

The substituents at R⁷ of Formula (II) are not particularly limited in the foregoing embodiments. In certain embodiments R⁷ is C₆-C₁₆ alkyl. In some other embodiments, R⁷ is C₆-C₉ alkyl. In some of these embodiments, R⁷ is substituted with -(C=O)OR^{b}, -O(C=O)R^{b}, -C(=O)R^{b}, -OR^{b}, -S(O)ₓR^{b}, -S-SR^{b}, -C( =O)SR^{b}, -SC(=O)R^{b}, -NR^{a}R^{b}, -NR^{a}C(=O)R^{b}, -C(=O)NR^{a}R^{b}, -NR^{a}C(=O)NR^{a}R^{b}, -OC(=O)NR^{a}R^{b}, -NR^{a}C(=O)OR^{b}, -NR^{a}S(O)ₓNR^{a}R^{b}, -NR^{a}S(O)ₓR^{b} or -S(O)ₓNR^{a}R^{b}, wherein: R^{a} is H or C₁-C₁₂ alkyl; R^{b} is C₁-C₁₅ alkyl; and x is 0, 1 or 2. For example, in some embodiments R⁷ is substituted with -(C=O)OR^{b} or -O(C=O)R^{b}.

In various of the foregoing embodiments of Formula (II), R^{b} is branched C₁-C₁₅ alkyl. For example, in some embodiments R^{b} has one of the following structures: or

In certain other of the foregoing embodiments of Formula (II), one of R⁸ or R⁹ is methyl. In other embodiments, both R⁸ and R⁹ are methyl.

In some different embodiments of Formula (II), R⁸ and R⁹, together with the nitrogen atom to which they are attached, form a 5, 6 or 7-membered heterocyclic ring. In some embodiments of the foregoing, R⁸ and R⁹, together with the nitrogen atom to which they are attached, form a 5-membered heterocyclic ring, for example a pyrrolidinyl ring. In some different embodiments of the foregoing, R⁸ and R⁹, together with the nitrogen atom to which they are attached, form a 6-membered heterocyclic ring, for example a piperazinyl ring.

In still other embodiments of the foregoing lipids of Formula (II), G³ is C₂-C₄ alkylene, for example C₃ alkylene.

In various different embodiments, the lipid compound has one of the structures set forth in Table 8 ("Representative Lipids of Formula (II)") below.

**Table 8: Representative Lipids of Formula (II)**

| **No.** | **Structure** | **Prep. Method** |
|---|---|---|
| II-1 | | D |
| II-2 | | D |
| II-3 | | D |
| II-4 | | E |
| II-5 | | D |
| II-6 | | D |
| II-7 | | D |
| II-8 | | D |
| II-9 | | D |
| II-10 | | D |
| II-11 | | D |
| II-12 | | D |
| II-13 | | D |
| II-14 | | D |
| II-15 | | D |
| II-16 | | E |
| II-17 | | D |
| II-18 | | D |
| II-19 | | D |
| II-20 | | D |
| II-21 | | D |
| II-22 | | D |
| II-23 | | D |
| II-24 | | D |
| II-25 | | E |
| II-26 | | E |
| II-27 | | E |
| II-28 | | E |
| II-29 | | E |
| II-30 | | E |
| II-31 | | E |
| II-32 | | E |
| II-33 | | E |
| II-34 | | E |
| II-35 | | D |
| II-36 | | D |

In some embodiments, the LNPs comprise a lipid of Formula (II), a mRNA compound as described above and one or more excipient selected from neutral lipids, steroids and pegylated lipids. In some embodiments the lipid of Formula (II) is compound II-9. In some embodiments the lipid of Formula (II) is compound II-10. In some embodiments the lipid of Formula (II) is compound II-11. In some embodiments the lipid of Formula (II) is compound II-12. In some embodiments the lipid of Formula (II) is compound II-32.

In a further embodiment, the LNP comprises (i) a cationic lipid of Formula (III): as further defined below or a pharmaceutically acceptable salt, tautomer, prodrug or stereoisomer thereof, and (ii) a mRNA compound comprising an mRNA sequence encoding at least one antigenic peptide or protein, wherein the mRNA compound is encapsulated in or associated with said lipid nanoparticle. With respect to the mRNA compound, the mRNA sequence and the antigenic peptide or protein, reference is made to the description of these features including the respective options and preferences above. In one of the preferred embodiments, the mRNA compound does not comprise a nucleoside modification. In another embodiment, it comprises no base modification. In a further embodiment, it does not comprise a 1-methylpseudouridine modification. In yet a further embodiment the mRNA compound only comprises the natural nucleosides adenine, guanine, cytosine and uracil.

Formula (III) is further defined in that:
one of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, -NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR^{a}-, -NR^{a}C(=O)NR^{a}-, -OC(=O)NR^{a}- or - NR^{a}C(=O)O- or a direct bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ alkyl;
R¹ and R² are each independently C₆-C₂₄ alkyl or C₆-C₂₄ alkenyl;
R³ is H, OR⁵, CN, -C(=O)OR⁴, -OC(=O)R⁴ or -NR⁵C(=O)R⁴;
R⁴ is C₁-C₁₂ alkyl;
R⁵ is H or C₁-C₆ alkyl; and
x is 0, 1 or 2.

In some of the foregoing embodiments of Formula (III), the lipid has one of the following structures (IIIA) or (IIIB): wherein:
A is a 3 to 8-membered cycloalkyl or cycloalkylene ring;
R⁶ is, at each occurrence, independently H, OH or C₁-C₂₄ alkyl;
n is an integer ranging from 1 to 15.

In some of the foregoing embodiments of Formula (III), the lipid has structure (IIIA), and in other embodiments, the lipid has structure (IIIB).

In other embodiments of Formula (III), the lipid has one of the following structures (IIIC) or (IIID): wherein y and z are each independently integers ranging from 1 to 12.

In any of the foregoing embodiments of Formula (III), one of L¹ or L² is -O(C=O)-. For example, in some embodiments each of L¹ and L² are -O(C=O)-. In some different embodiments of any of the foregoing, L¹ and L² are each independently -(C=O)O- or -O(C=O)-. For example, in some embodiments each of L¹ and L² is -(C=O)O-.

In some different embodiments of Formula (III), the lipid has one of the following structures (IIIE) or (IIIF):

In some of the foregoing embodiments of Formula (III), the lipid has one of the following structures (IIIG), (IIIH), (IIII), or (IIIJ):

In some of the foregoing embodiments of Formula (III), n is an integer ranging from 2 to 12, for example from 2 to 8 or from 2 to 4. For example, in some embodiments, n is 3, 4, 5 or 6. In some embodiments, n is 3. In some embodiments, n is 4. In some embodiments, n is 5. In some embodiments, n is 6.

In some other of the foregoing embodiments of Formula (III), y and z are each independently an integer ranging from 2 to 10. For example, in some embodiments, y and z are each independently an integer ranging from 4 to 9 or from 4 to 6.

In some of the foregoing embodiments of Formula (III), R⁶ is H. In other of the foregoing embodiments, R⁶ is C₁-C₂₄ alkyl. In other embodiments, R⁶ is OH.

In some embodiments of Formula (III), G³ is unsubstituted. In other embodiments, G3 is substituted. In various different embodiments, G³ is linear C₁-C₂₄ alkylene or linear C₁-C₂₄ alkenylene.

In some other foregoing embodiments of Formula (III), R¹ or R², or both, is C₆-C₂₄ alkenyl. For example, in some embodiments, R¹ and R² each, independently have the following structure: wherein:
R^{7a} and R^{7b} are, at each occurrence, independently H or C₁-C₁₂ alkyl; and
a is an integer from 2 to 12,
wherein R^{7a}, R^{7b} and a are each selected such that R¹ and R² each independently comprise from 6 to 20 carbon atoms. For example, in some embodiments a is an integer ranging from 5 to 9 or from 8 to 12.

In some of the foregoing embodiments of Formula (III), at least one occurrence of R^{7a} is H. For example, in some embodiments, R^{7a} is H at each occurrence. In other different embodiments of the foregoing, at least one occurrence of R^{7b} is C₁-C₈ alkyl. For example, in some embodiments, C₁-C₈ alkyl is methyl, ethyl, n-propyl, iso-propyl, n-butyl, iso-butyl, tert-butyl, n-hexyl or n-octyl.

In different embodiments of Formula (III), R¹ or R², or both, has one of the following structures:

In some of the foregoing embodiments of Formula (III), R³ is OH, CN, -C(=O)OR⁴, -OC(=O)R⁴ or -NHC(=O)R⁴. In some embodiments, R⁴ is methyl or ethyl.

In various different embodiments, the cationic lipid of Formula (III) has one of the structures set forth in Table 9 ("Representative Compounds of Formula (III)") below.

**Table 9: Representative Compounds of Formula (III)**

| **No.** | **Structure** | **Prep. Method** |
|---|---|---|
| III-1 | | F |
| III-2 | | F |
| III-3 | | F |
| III-4 | | F |
| III-5 | | F |
| III-6 | | F |
| III-7 | | F |
| III-8 | | F |
| III-9 | | F |
| III-10 | | F |
| III-11 | | F |
| III-12 | | F |
| III-13 | | F |
| III-14 | | F |
| III-15 | | F |
| III-16 | | G |
| III-17 | | G |
| III-18 | | G |
| III-19 | | G |
| III-20 | | G |
| III-21 | | G |
| III-22 | | G |
| III-23 | | G |
| III-24 | | G |
| III-25 | | G |
| III-26 | | G |
| III-27 | | G |
| III-28 | | G |
| III-29 | | G |
| III-30 | | G |
| III-31 | | G |
| III-32 | | G |
| III-33 | | G |
| III-34 | | G |
| III-35 | | G |
| III-36 | | G |

In some embodiments, the LNPs comprise a lipid of Formula (III), a mRNA compound as described herein and one or more excipient selected from neutral lipids, steroids and pegylated lipids. In some embodiments the lipid of Formula (III) is compound III-3. In some embodiments the lipid of Formula (III) is compound III-7.

Within the context of the present invention LNP-III-3 means a lipid nanoparticle as defined herein comprising the cationic lipid compound III-3, according to the tables above. Other lipid nanoparticles are referecend in analogous form.

In certain embodiments, the cationic lipid of Formula (I), (II) or (III) is present in the LNP in an amount from about 30 to about 95 mole percent, relative to the total lipid content of the LNP. If more than one cationic lipid is incorporated within the LNP, such percentages apply to the combined cationic lipids. In one embodiment, the cationic lipid is present in the LNP in an amount from about 30 to about 70 mole percent. In one embodiment, the cationic lipid is present in the LNP in an amount from about 40 to about 60 mole percent, such as about 40, 41, 42, 43, 44, 45, 46, 47, 48, 49, 50, 51, 52, 53, 54, 55, 56, 57, 58, 59 or 60 mole percent, resepctively.

In some embodiments of the invention the LNP comprises a combination or mixture of any the lipids described above.

In one of the preferred embodiments, the lipid nanoparticle comprises a cationic lipid selected from the group of:

In a further embodiment, the invention relates to an mRNA comprising lipid nanoparticle comprising: (i) a PEG lipid with the formula (IV)
wherein R⁸ and R⁹ are each independently a straight or branched, saturated or unsaturated alkyl chain containing from 10 to 30 carbon atoms, wherein the alkyl chain is optionally interrupted by one or more ester bonds;
and w has a mean value ranging from 30 to 60; and
(ii) a mRNA compound comprising an mRNA sequence encoding at least one antigenic peptide or protein; wherein the mRNA compound is encapsulated in or associated with said lipid nanoparticle. With respect to the mRNA compound, the mRNA sequence and the antigenic peptide or protein, reference is made to the description of these features including the respective options and preferences above. In one of the preferred embodiments, the mRNA compound does not comprise a nucleoside modification. In another embodiment, it comprises no base modification. In a further embodiment, it does not comprise a 1-methylpseudouridine modification. Moreover, if the PEG lipid is compound (IVa), the lipid nanoparticle is not a lipid nanoparticle comprising compound I-6, DSPC, cholesterol and the PEG lipid (IVa) at a ratio of about 50:10:38.5:1.5 that encapsulates unmodified, 1-methylpseudouridine modified or codon-optimized mRNA encoding an influenza PR8 or Cal/7/2009 hemagglutinin or an HIV-1 CD4-independent R3A envelop protein.In one of the preferred embodiments, the lipid nanoparticle comprises (i) a cationic lipid according to formula (I), (II), or (III) as defined above, (ii) a mRNA compound comprising an mRNA sequence encoding at least one antigenic peptide or protein as described herein, and (iii) a PEG lipid of formula (IV); wherein the mRNA compound is encapsulated in or associated with said lipid nanoparticle.

The amount of the permanently cationic lipid or lipidoid should also be selected taking the amount of the nucleic acid cargo into account. In one embodiment, these amounts are selected such as to result in an N/P ratio of the nanoparticle(s) or of the composition in the range from about 0.1 to about 20. In this context, the N/P ratio is defined as the mole ratio of the nitrogen atoms ("N") of the basic nitrogen-containing groups of the lipid or lipidoid to the phosphate groups ("P") of the nucleic acid which is used as cargo. The N/P ratio may be calculated on the basis that, for example, 1µg RNA typically contains about 3 nmol phosphate residues, provided that the RNA exhibits a statistical distribution of bases. The "N"-value of the lipid or lipidoid may be calculated on the basis of its molecular weight and the relative content of permanently cationic and - if present - cationisable groups.

Such low N/P ratios are commonly believed to be detrimental to the performance and *in vivo* efficacy of such carrier-cargo complexes, or nucleic-acid loaded nanoparticles. However, the inventors found that such N/P ratios are indeed useful in the context of the present invention, in particular when the local or extravascular administration of the nanoparticles is intended. Here, the respectively nanoparticles have been found to be efficacious and at the same time well-tolerated.

In certain embodiments, the LNP comprises one or more additional lipids which stabilize the formation of particles during their formation.

Suitable stabilizing lipids include neutral lipids and anionic lipids. The term "neutral lipid" refers to any one of a number of lipid species that exist in either an uncharged or neutral zwitterionic form at physiological pH. Representative neutral lipids include diacylphosphatidylcholines, diacylphosphatidylethanolamines, ceramides, sphingomyelins, dihydro sphingomyelins, cephalins, and cerebrosides.

Exemplary neutral lipids include, for example, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearioyl-2-oleoylphosphatidyethanol amine (SOPE), and 1,2-dielaidoyl-sn-glycero-3-phophoethanolamine (transDOPE). In one embodiment, the neutral lipid is 1,2-distearoyl-sn-glycero-3phosphocholine (DSPC).

In some embodiments, the LNPs comprise a neutral lipid selected from DSPC, DPPC, DMPC, DOPC, POPC, DOPE and SM. In various embodiments, the molar ratio of the cationic lipid (e.g., lipid of Formula (I), (II) or (III)) to the neutral lipid ranges from about 2:1 to about 8:1.

In various embodiments, the LNPs further comprise a steroid or steroid analogue. A "steroid" is a compound comprising the following carbon skeleton:

In certain embodiments, the steroid or steroid analogue is cholesterol. In some of these embodiments, the molar ratio of the cationic lipid (e.g., lipid of Formula (I), (II), or (III)) to cholesterol ranges from about 5:1 to 1:1.

The term "anionic lipid" refers to any lipid that is negatively charged at physiological pH. These lipids include phosphatidylglycerol, cardiolipin, diacylphosphatidylserine, diacylphosphatidic acid, Ndodecanoylphosphatidylethanolamines, N-succinylphosphatidylethanolamines, Nglutarylphosphatidylethanolamines, lysylphosphatidylglycerols, palmitoyloleyolphosphatidylglycerol (POPG), and other anionic modifying groups joined to neutral lipids.

In certain embodiments, the LNP comprises glycolipids (e.g., monosialoganglioside GM₁).

In some embodiments, the LNPs comprise a polymer conjugated lipid. The term "polymer conjugated lipid" refers to a molecule comprising both a lipid portion and a polymer portion. An example of a polymer conjugated lipid is a pegylated lipid. The term "pegylated lipid" refers to a molecule comprising both a lipid portion and a polyethylene glycol portion. Pegylated lipids are known in the art and include 1-(monomethoxy-polyethyleneglycol)-2,3-dimyristoylglycerol (PEG-s- DMG) and the like.

In certain embodiments, the LNP comprises an additional, stabilizing-lipid which is a polyethylene glycol-lipid (pegylated lipid). Suitable polyethylene glycollipids include PEG-modified phosphatidylethanolamine, PEG-modified phosphatidic acid, PEG-modified ceramides (e.g., PEG-CerC14 or PEG-CerC20), PEG-modified dialkylamines, PEG-modified diacylglycerols, PEG-modified dialkylglycerols. Representative polyethylene glycollipids include PEG-c-DOMG, PEG-c-DMA, and PEG-s-DMG. In one embodiment, the polyethylene glycol-lipid is N-[(methoxy poly(ethylene glycol)₂₀₀₀)carbamyl]-1,2-dimyristyloxlpropyl-3-amine (PEG-c-DMA). In one embodiment, the polyethylene glycol-lipid is PEG-c-DOMG). In other embodiments, the LNPs comprise a pegylated diacylglycerol (PEG-DAG) such as 1-(monomethoxy-polyethyleneglycol)-2,3-dimyristoylglycerol (PEG-DMG), a pegylated phosphatidylethanoloamine (PEG-PE), a PEG succinate diacylglycerol (PEG-S-DAG) such as 4-O-(2',3'-di(tetradecanoyloxy)propyl-1-O-(ω-methoxy(polyethoxy)ethyl)butanedicate (PEG-S-DMG), a pegylated ceramide (PEG-cer), or a PEG dialkoxypropylcarbamate such as ω-methoxy(polyethoxy)ethyl-N-(2,3di(tetradecanoxy)propyl)carbamate or 2,3-di(tetradecanoxy)propyl-N-(ω-methoxy(polyethoxy)ethyl)carbamate. In various embodiments, the molar ratio of the cationic lipid to the pegylated lipid ranges from about 100:1 to about 25:1.

As mentioned, the mRNA comprising lipid nanoparticle may comprise a pegylated lipid having the structure of formula (IV): or a pharmaceutically acceptable salt, tautomer or stereoisomer thereof, wherein:
R⁸ and R⁹ are each independently a straight or branched, saturated or unsaturated alkyl chain containing from 10 to 30 carbon atoms, wherein the alkyl chain is optionally interrupted by one or more ester bonds; and w has mean value ranging from 30 to 60.

In some of the foregoing embodiments of the pegylated lipid (IV), R⁸ and R⁹ are not both n-octadecyl when w is 42. In some other embodiments, R⁸ and R⁹ are each independently a straight or branched, saturated or unsaturated alkyl chain containing from 10 to 18 carbon atoms. In some embodiments, R⁸ and R⁹ are each independently a straight or branched, saturated or unsaturated alkyl chain containing from 12 to 16 carbon atoms. In some embodiments, R⁸ and R⁹ are each independently a straight or branched, saturated or unsaturated alkyl chain containing 12 carbon atoms. In some embodiments, R⁸ and R⁹ are each independently a straight or branched, saturated or unsaturated alkyl chain containing 14 carbon atoms. In other embodiments, R⁸ and R⁹ are each independently a straight or branched, saturated or unsaturated alkyl chain containing 16 carbon atoms. In still more embodiments, R⁸ and R⁹ are each independently a straight or branched, saturated or unsaturated alkyl chain containing 18 carbon atoms. In still other embodiments, R⁸ is a straight or branched, saturated or unsaturated alkyl chain containing 12 carbon atoms and R⁹ is a straight or branched, saturated or unsaturated alkyl chain containing 14 carbon atoms.

In various embodiments, w spans a range that is selected such that the PEG portion of (IV) has an average molecular weight of about 400 to about 6000 g/mol. In some embodiments, the average w is about 50.

In a preferred embodiment R⁸ and R⁹ are saturated alkyl chains.

In a further preferred embodiment the PEG lipid is of formula (IVa) wherein n has a mean value ranging from 30 to 60, such as about 30±2, 32±2, 34±2, 36±2, 38±2, 40±2, 42±2, 44±2, 46±2, 48±2, 50±2, 52±2, 54±2, 56±2, 58±2, or 60±2. In a most preferred embodiment n is about 49.

In other embodiments, the pegylated lipid has one of the following structures: wherein n is an integer selected such that the average molecular weight of the pegylated lipid is about 2500g/mol, most preferably n is about 49.

In certain embodiments, the PEG lipid is present in the LNP in an amount from about 1 to about 10 mole percent, relative to the total lipid content of the nanoparticle. In one embodiment, the PEG lipid is present in the LNP in an amount from about 1 to about 5 mole percent. In one embodiment, the PEG lipid is present in the LNP in about 1 mole percent or about 1.5 mole percent.

In certain embodiments, the LNP comprises one or more targeting moieties which are capable of targeting the LNP to a cell or cell population. For example, in one embodiment, the targeting moiety is a ligand which directs the LNP to a receptor found on a cell surface.

In certain embodiments, the LNP comprises one or more internalization domains. For example, in one embodiment, the LNP comprises one or more domains which bind to a cell to induce the internalization of the LNP. For example, in one embodiment, the one or more internalization domains bind to a receptor found on a cell surface to induce receptor-mediated uptake of the LNP. In certain embodiments, the LNP is capable of binding a biomolecule in vivo, where the LNP-bound biomolecule can then be recognized by a cell-surface receptor to induce internalization. For example, in one embodiment, the LNP binds systemic ApoE, which leads to the uptake of the LNP and associated cargo.

Other exemplary LNPs and their manufacture are described in the art, for example in U.S. Patent Application Publication No. US20120276209, Semple et al., 2010, Nat Biotechnol., 28(2):172-176; Akinc et al., 2010, Mol Ther., 18(7): 1357-1364; Basha et al., 2011, Mol Ther, 19(12): 2186-2200; Leung et al., 2012, J Phys Chem C Nanomater Interfaces, 116(34): 18440-18450; Lee et al., 2012, Int J Cancer., 131(5): E781-90; Belliveau et al., 2012, Mol Ther nucleic Acids, 1: e37; Jayaraman et al., 2012, Angew Chem Int Ed Engl., 51(34): 8529-8533; Mui et al., 2013, Mol Ther Nucleic Acids. 2, e139; Maier et al., 2013, Mol Ther., 21(8): 1570-1578; and Tam et al., 2013, Nanomedicine, 9(5): 665-74, each of which are incorporated by reference in their entirety.

In preferred embodiments, the lipid nanoparticles have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm, and are substantially non-toxic. As mentioned, the mean diameter may correspond to the z-average as determined by dynamic light scattering.

In another preferred embodiment of the invention the lipid nanoparticles have a hydrodynamic diameter in the range from about 50 nm to about 300 nm, or from about 60 nm to about 250 nm, from about 60 nm to about 150 nm, or from about 60 nm to about 120 nm, respectively.

In certain embodiments, the mRNA, when present in the lipid nanoparticles, is resistant in aqueous solution to degradation with a nuclease.

The total amount of mRNA in the lipid nanoparticles varies and may be defined depending on the mRNA to total lipid w/w ratio. In one embodiment of the invention the invention the mRNA to total lipid ratio is less than 0.06 w/w, preferably between 0.03 and 0.04 w/w.

In some embodiments, the LNPs comprise a lipid of Formula (I), (II) or (III), a mRNA compound as defined above, a neutral lipid, a steroid and a pegylated lipid. In some embodiments the lipid of Formula (I) is compound I-6, or the lipid of formula (III) is compound III-3, the neutral lipid is DSPC, the steroid is cholesterol, and the pegylated lipid is the compound of formula (IVa).

In certain embodiments, the LNP comprises one or more targeting moieties which are capable of targeting the LNP to a cell or cell population. For example, in one embodiment, the targeting moiety is a ligand which directs the LNP to a receptor found on a cell surface.

In certain embodiments, the LNP comprises one or more internalization domains. For example, in one embodiment, the LNP comprises one or more domains which bind to a cell to induce the internalization of the LNP. For example, in one embodiment, the one or more internalization domains bind to a receptor found on a cell surface to induce receptor-mediated uptake of the LNP. In certain embodiments, the LNP is capable of binding a biomolecule in vivo, where the LNP-bound biomolecule can then be recognized by a cell-surface receptor to induce internalization. For example, in one embodiment, the LNP binds systemic ApoE, which leads to the uptake of the LNP and associated cargo.

In particular the invention relates to the following non-limiting specific embodiments.

In a preferred embodiment, the invention relates to a mRNA comprising lipid nanoparticle comprising a cationic lipid according to formula (I), (II) or (II) as defined above and a mRNA compound comprising a mRNA sequence encoding at least one antigenic peptide or protein as defined above, wherein, if the cationic lipid is of formula I-6, the lipid nanoparticle is not a lipid nanoparticle comprising formula I-6, DSPC, cholesterol and a PEG lipid of formula (IVA) at a ratio of about 50:10:38.5:1.5 that encapsulates unmodified, 1-methylpseudouridine modified or codon-optimized mRNA encoding an influenza PR8 or Cal/7/2009 hemagglutinin or an HIV-1 CD4-independent R3A envelop protein.

A further preferred embodiment relates to a mRNA comprising lipid nanoparticle comprising a PEG lipid according to formula (IV) as defined above and a mRNA compound comprising a mRNA sequence encoding at least one antigenic peptide or protein, wherein, if the cationic lipid is of formula I-6, the lipid nanoparticle is not a lipid nanoparticle comprising formula I-6, DSPC, cholesterol and a PEG lipid of formula (IVa) at a ratio of about 50:10:38.5:1.5 that encapsulates unmodified, 1-methylpseudouridine modified or codon-optimized mRNA encoding an influenza PR8 or Cal/7/2009 hemagglutinin or an HIV-1 CD4-independent R3A envelop protein.

In a specific preferred embodiment the invention relates to a mRNA comprising lipid nanoparticle, comprising a cationic lipid according to formula (I), (II) or (III), a PEG-lipid according to formula (IV), a mRNA compound comprising a mRNA sequence encoding at least one antigenic peptide or protein, a steroid and a neutral lipid, wherein preferably, , if the cationic lipid is of formula I-6, the lipid nanoparticle is not a lipid nanoparticle comprising formula I-6, DSPC, cholesterol and a PEG lipid of formula (IVa) at a ratio of about 50:10:38.5:1.5 that encapsulates unmodified, 1-methylpseudouridine modified or codon-optimized mRNA encoding an influenza PR8 or Cal/7/2009 hemagglutinin or an HIV-1 CD4-independent R3A envelop protein, preferably the antigenic peptide or protein is derived from pathogenic antigens, tumour antigens, allergenic antigens or autoimmune self-antigens or a fragment or variant thereof, more preferably the pathogenic antigen is derived from an influenza or rabies virus.

In a further preferred embodiment, the invention relates to a mRNA comprising lipid nanoparticle comprising: a cationic lipid selected from or a PEG lipid with the structure wherein n has a mean value ranging from 30 to 60, preferably about 49, optionally a neutral lipid, preferably 1,2-distearoyl-sn-glycero-3-phosphocholine (DSPC) and optionally a steroid, preferably cholesterol, wherein the molar ratio of the cationic lipid to DSPC is optionally in the range from about 2:1 to 8:1, wherein the molar ratio of the cationic lipid to cholesterol is optionally in the range from about 2:1 to 1:1.

In one preferred embodiment, the invention relates to a mRNA comprising lipid nanoparticle comprising: a cationic lipid with formula (I), (II) or (III) and/or PEG lipid with formula (IV), optionally a neutral lipid, preferably 1,2-distearoyl-sn-glycero-3-phosphocholine (DSPC) and optionally a steroid, preferably cholesterol, wherein the molar ratio of the cationic lipid to DSPC is optionally in the range from about 2:1 to 8:1, wherein the molar ratio of the cationic lipid to cholesterol is optionally in the range from about 2:1 to 1:1, and an mRNA composition comprising an mRNA sequence encoding an antigenic peptide or protein, wherein wherein the mRNA sequence additionally comprises preferably in 5' to 3'-direction, the following elements:
a) a 5'-CAP structure, preferably m7GpppN,
b) at least one coding region encoding at least one antigenic peptide or protein,
c) a poly(A) tail, preferably consisting of 10 to 200, 10 to 100, 40 to 80 or 50 to 70 adenosine nucleotides,
d) optionally a poly(C) tail, preferably consisting of 10 to 200, 10 to 100, 20 to 70, 20 to 60 or 10 to 40 cytosine nucleotides, and
e) optionally a histone stem-loop,
f) and optionally a 3'-UTR element.

In a more preferred embodiment the invention relates to a mRNA comprising lipid nanoparticle comprising: a cationic lipid selected from or
and/or a PEG lipid with the structure
wherein n has a mean value ranging from 30 to 60, preferably about 49,
and a mRNA compound comprising an mRNA sequence encoding an antigenic peptide or protein, wherein preferably wherein the antigenic peptide or protein is derived from pathogenic antigens, tumour antigens, allergenic antigens or autoimmune self-antigens or a fragment or variant thereof, more preferably the antigen is derived from an influenza or rabies virus,
optionally a neutral lipid, preferably 1,2-distearoyl-sn-glycero-3-phosphocholine (DSPC) and optionally a steroid, preferably cholesterol, wherein the molar ratio of the cationic lipid to DSPC is optionally in the range from about 2:1 to 8:1, wherein the molar ratio of the cationic lipid to cholesterol is optionally in the range from about 2:1 to 1:1, wherein wherein the mRNA sequence optionally comprises
   a) a 5'-CAP structure, and/or
   b) a poly(A) sequence, and/or
   c) a poly (C) sequence.

In a more preferred embodiment the mRNA sequence comprises a coding region encoding the at least one antigenic peptide or protein, wherein the mRNA sequence comprises a sequence modification selected from a G/C content modification, a codon modification, a codon optimization or a C-optimization of the sequence.

In a specific preferred embodiment, the invention relates to a mRNA comprising lipid nanoparticle comprising: a cationic lipid selected from or
optionally a PEG lipid with the structure
wherein n has a mean value ranging from 30 to 60, preferably about 49,
and a mRNA compound, comprising an mRNA sequence
optionally a neutral lipid, preferably 1,2-distearoyl-sn-glycero-3-phosphocholine (DSPC) and
optionally a steroid, preferably cholesterol, wherein the molar ratio of the cationic lipid to DSPC is optionally in the range from about 2:1 to 8:1, wherein the molar ratio of the cationic lipid to cholesterol is optionally in the range from about 2:1 to 1:1,
wherein the mRNA sequence additionally comprises preferably in 5' to 3'-direction, the following elements:
   a) a 5'-CAP structure, preferably m7GpppN,
   b) at least one coding region encoding at least one antigenic peptide or protein, preferably the antigenic peptide or protein is derived from pathogenic antigens, tumour antigens, allergenic antigens or autoimmune self-antigens or a fragment or variant thereof, more preferably the pathogenic antigen is derived from an influenza or rabies virus;
   c) a poly(A) tail, preferably consisting of 10 to 200, 10 to 100, 40 to 80 or 50 to 70 adenosine nucleotides,
   d) optionally a poly(C) tail, preferably consisting of 10 to 200, 10 to 100, 20 to 70, 20 to 60 or 10 to 40 cytosine nucleotides, and
   e) optionally a histone stem-loop,
   f) and optionally a 3'-UTR element.

In a particular preferred embodiment, the (pharmaceutical) composition or the vaccine according to the invention comprising mRNA comprises lipid nanoparticles, which have a molar ratio of approximately 50:10:38.5:1.5, preferably 47.5:10:40.8:1.7 or more preferably 47.4:10:40.9:1.7 (i.e. proportion (mol%) of cationic lipid, DSPC, cholesterol and PEG-lipid; solubilized in ethanol).

In particular preferred embodiments the lipid nanoparticle is a mRNA comprising lipid nanoparticle as defined above, wherein preferably the antigenic peptide or protein is derived from hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, or polymerase basic protein 2 (PB2) of an influenza virus or a fragment or variant thereof. More preferably the antigenic peptide or protein is derived from hemagglutinin (HA) or neuraminidase (NA) of an influenza virus or a fragment or variant thereof. Even more preferably the antigenic peptide or protein is at least one full-length protein of hemagglutinin (HA) and/or at least one full-length protein of neuraminidase (NA) of an influenza virus or a variant thereof. In a further preferred embodiment the influenza virus is selected from an influenza A, B or C virus. In a particularly preferred embodiment the influenza A virus is selected from an influenza virus characterized by a hemagglutinin (HA) selected from the group consisting of H1, H2, H3, H4, H5, H6, H7, H8, H9, H10, H11, H12, H13, H14, H15, H16, H17 and H18 and/or the influenza A virus is selected from an influenza virus characterized by a neuraminidase (NA) selected from the group consisting of N1, N2, N3, N4, N5, N6, N7, N8, N9, N10, and N11. Preferably, the influenza A virus is selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, and H10N7, preferably from H1N1, H3N2, H5N1. Most preferably, the mRNA sequence comprises at least one coding region encoding at least one antigenic peptide or protein derived from hemagglutinin (HA) of an influenza virus or a fragment or variant thereof and at least one antigenic peptide or protein derived from neuraminidase (NA) of an influenza virus or a fragment or variant thereof. In a specifically preferred embodiment the mRNA sequence comprises at least one coding region encoding at least one antigenic peptide or protein derived from hemagglutinin (HA) and/or at least one antigenic peptide or protein derived from neuraminidase (NA) of an influenza A virus selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, and H10N7, preferably from H1N1, H3N2, H5N1 or a fragment or variant thereof.

The invention further relates to a method of preparing said lipid nanoparticles comprising the steps of:
(i) providing a cationic lipid of formula (I)
   as defined above or a pharmaceutically acceptable salt, tautomer, prodrug or stereoisomer thereof, and/or of formula (II)
   as defined above or a pharmaceutically acceptable salt, tautomer, prodrug or stereoisomer thereof, and/or of formula III:
   as defined above or a pharmaceutically acceptable salt, tautomer, prodrug or stereoisomer thereof; and/or
   b) a PEG lipid with the formula (IV): as defined above;
   c) at least one mRNA compound comprising an mRNA sequence encoding at least one antigenic peptide or protein; and
   d) optionally a steroid; and
   e) optionally a neutral lipid;
(ii) solubilizing the cationic lipid and/or the PEG lipid and optionally the neutral lipid and/or the steroid or a steroid derivative in ethanol;
(iii) mixing the ethanolic lipid solution with an aqueous solution comprising the mRNA polynucleotide
(iv) removing the ethanol to form lipid nanoparticles encapsulating or associating with the mRNA polynucleotide; and optionally
(v) separating or purifying the lipid nanoparticles.

The ethanol may be removed by any suitable method which does not negatively affect the lipids or the forming lipid nanoparticles. In one embodiment of the invention the ethanol is removed by dialysis. In an alternative embodiment the ethanol is removed by diafiltration.

Separation and optinal purification of the lipid nanoparticles might also be performed by any suitable method. Preferably the lipid nanoparticles are filtrated, more preferably the lipid nanoparticles are separated or purified by filtration through a sterile filter.

The invention further relates to a pharmaceutical composition comprising at least one lipid nanoparticle according to the present invention. The lipid nanoparticle might comprise an mRNA compound comprising a sequence encoding at least one antigenic peptide or protein as defined herein.

In one embodiment of the invention the mRNA sequence encondes one antigenic peptide or protein. In an alternative embodiment of the invention the mRNA sequence encodes more than one antigenic peptide or protein.

In one embodiment of the invention, the pharmaceutical composition comprises a lipid nanoparticle according to the invention, wherein the lipid nanoparticle comprises more than one mRNA compounds, which each comprise a different mRNA sequence encoding an antigenic peptide or protein.

In an alternative embodiment of the invention the pharmaceutical composition comprises a second lipid nanoparticle, wherein the mRNA compound comprised by the second lipid nanoparticle is different from the mRNA compound comprised by the first lipid nanoparticle.

In a further aspect, the present invention concerns a composition comprising mRNA comprising lipid nanoparticles wherein the mRNA comprises an mRNA sequence comprising at least one coding region as defined herein and a pharmaceutically acceptable carrier. The composition according to the invention is preferably provided as a pharmaceutical composition or as a vaccine.

According to a preferred embodiment, the (pharmaceutical) composition or the vaccine according to the invention comprises mRNA comprising lipid nanoparticles comprising at least one mRNA comprising at least one mRNA sequence as defined above, wherein the at least one coding region of the at least one mRNA sequence encodes at least one antigenic peptide or protein preferably derived from a protein of an influenza virus or Rabies virus, preferably any one of the hemagglutinin (HA) or neuraminidase (NA) proteins or glycoproteins, as disclosed in the sequence listing of the present invention or respectively in Tables 1-5 or Figures 20-24 of PCT/EP2016/075843 or a fragment or variant of any one of these proteins.

Preferably, the (pharmaceutical) composition or the vaccine according to the invention comprises mRNA comprising lipid nanoparticles comprising at least one mRNA comprising at least one mRNA sequence as defined above, wherein the at least one coding sequence of the at least one mRNA sequence comprises or consists of a nucleic acid sequence encoding at least one antigenic peptide or protein preferably derived from a protein of an influenza virus, preferably any one of the hemagglutinin (HA) or neuraminidase (NA) proteins, as defined in the sequence listing or respectively in Tables 1-4 or Figures 20-23 of PCT/EP2016/075843, or a fragment or variant thereof, wherein the protein derived from a protein of an influenza virus preferably comprises or consists of any one of the amino acid sequences defined in the sequence listing or respectively in Tables 1-4 or Figures 20-23 of PCT/EP2016/075843, preferably SEQ ID NOs: 1-30504 of the sequence listing or respectively in Tables 1-4 or Figures 20-23 of PCT/EP2016/075843, or a fragment or variant of any one of these sequences. Alternatively, the antigenic peptide or protein is derived from a Rabies virus, preferably from glycoprotein of a Rabies virus, preferably comprising or consisting of any one of the amino acid sequences disclosed in the sequence listing, or respectively in Table 5 or Figures 24 of PCT/EP2016/075843, preferably SEQ ID NOs: 30505-32012 of the sequence listing, or a fragment or variant of any one of these sequences.

Preferably, the (pharmaceutical) composition or the vaccine according to the invention comprises mRNA comprising lipid nanoparticles comprising at least one mRNA comprising at least one mRNA sequence as defined above, wherein the at least one coding sequence of the mRNA sequence comprises or consists of a nucleic acid sequence encoding at least one antigenic peptide or protein derived from a protein of an influenza virus or Rabies virus, or a fragment or variant thereof, wherein the antigenic peptide or protein derived from a protein of an influenza virus or Rabies virus preferably comprises or consists of an amino acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the amino acid sequences disclosed in the sequence listing, preferably SEQ ID NOs: 1-32012, or respectively "column A" of Tables 1-5 or Figures 20-24 of PCT/EP2016/075843, or a fragment or variant of any one of these sequences.

More preferably, the (pharmaceutical) composition or the vaccine according to the invention comprises mRNA comprising lipid nanoparticles comprising at least one mRNA comprising at least one mRNA sequence as defined above, wherein the at least one coding sequence of the at least one mRNA sequence comprises or consists of a nucleic acid sequence encoding at least one antigenic peptide or protein derived from a protein of an influenza virus or Rabies virus, or a fragment or variant thereof, wherein the antigenic peptide or protein derived from a protein of an influenza virus or Rabies virus preferably comprises or consists of an amino acid sequence having a sequence identity of at least 80% with any one of the amino acid sequences disclosed in the sequence listing, preferably in SEQ ID NOs: 1-32012, or respectively "column A" of Tables 1-5 or Figures 20-24 of PCT/EP2016/075843, or a fragment or variant of any one of these sequences.

In preferred embodiments, the (pharmaceutical) composition or the vaccine according to the invention comprises mRNA comprising lipid nanoparticles comprising at least one mRNA comprising at least one mRNA sequence as defined above, wherein the at least one coding sequence of the at least one mRNA sequence comprises or consists of any one of the nucleic acid sequences disclosed in the sequence listing, preferably SEQ ID NOs: 32013-64024 or SEQ ID NOs: 64025-224084 or columns "B" or "C" of Tables 1-5 or Figures 20-24 of PCT/EP2016/075843, or a fragment or variant of any one of these sequences.

According to another embodiment, the (pharmaceutical) composition or the vaccine according to the invention comprises mRNA comprising lipid nanoparticles comprising at least one mRNA comprising at least one mRNA sequence as defined above, wherein the at least one coding sequence of the at least one mRNA sequence comprises or consists of a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the nucleic acid sequences disclosed in the sequence listing, preferably SEQ ID NOs: 32013-64024 or SEQ ID NOs: 64025-224084, or respectively "column B" or "column C" of Tables 1-5 or Figures 20-24 of PCT/EP2016/075843, or a fragment or variant of any one of these sequences.

According to a particularly preferred embodiment, the (pharmaceutical) composition or the vaccine according to the invention comprises mRNA comprising lipid nanoparticles comprising at least one mRNA comprising at least one mRNA sequence as defined above, wherein the at least one coding sequence of the at least one mRNA sequence comprises or consists of a nucleic acid sequence having a sequence identity of at least 80% with any one of the nucleic acid sequences disclosed in the sequence listing, preferably in the SEQ ID NOs: 32013-64024 or SEQ ID NOs: 64025-224084, or respectively "column B" or "column C" of Tables 1-5 or Figures 20-24 of PCT/EP2016/075843 , or a fragment or variant of any one of these sequences.

More preferably, the (pharmaceutical) composition or the vaccine according to the invention comprises mRNA comprising lipid nanoparticles comprising at least one mRNA comprising at least one mRNA sequence as defined above, wherein the at least one coding sequence of the at least one mRNA sequence comprises or consists of any one of the nucleic acid sequences disclosed in the sequence listing, or respectively "column C" of Tables 1-5 or Figures 20-24 of PCT/EP2016/075843, or SEQ ID NOs: 64025-224084, or a fragment or variant of any one of these sequences.

In the context of the present invention, the (pharmaceutical) composition or vaccine may comprise mRNA comprising lipid nanoparticles comprising mRNA encoding one or more of the antigenic peptides or proteins as defined herein, preferably derived from a protein of an influenza virus or Rabies virus as defined herein or a fragment or variant thereof.

The (pharmaceutical) composition or vaccine according to the invention may thus comprise mRNA comprising lipid nanoparticles comprising at least one mRNA comprising at least one mRNA sequence comprising at least one coding region, encoding at least one antigenic peptide or protein , preferably derived from a protein of an influenza virus or Rabies virus or a fragment or variant thereof, wherein the at least one coding region of the at least one mRNA sequence encodes one specific antigenic peptide or protein e.g. derived from a protein of an influenza virus defined herein or a fragment or a variant thereof.

Alternatively, the (pharmaceutical) composition or vaccine of the present invention may comprise mRNA comprising lipid nanoparticles comprising at least one mRNA compound comprising at least one mRNA sequence according to the invention, wherein the at least one mRNA sequence encodes at least two, three, four, five, six, seven, eight, nine, ten, eleven or twelve distinct antigenic peptides or proteins e.g. derived from a protein of an influenza virus as defined herein or a fragment or variant thereof.

In this context it is particularly preferred that the at least one mRNA compound comprised in the (pharmaceutical) composition or vaccine is a bi- or multicistronic mRNA as defined herein, which encodes the at least two, three, four, five, six, seven, eight, nine, ten, eleven or twelve distinct antigenic peptides or proteins e.g. derived from a protein of an influenza virus. Mixtures between these embodiments are also envisaged, such as compositions comprising more than one mRNA sequence, wherein at least one mRNA sequence may be monocistronic, while at least one other mRNA sequence may be bi- or multicistronic.

The (pharmaceutical) composition or vaccine according to the present invention, preferably the at least one coding sequence of the mRNA sequence comprised therein, may thus comprise any combination of the nucleic acid sequences as defined herein.

Preferably, the (pharmaceutical) composition or vaccine comprises mRNA comprising lipid nanoparticle comprising a plurality or more than one of the mRNA sequences according to the invention, wherein each mRNA sequence comprises at least one coding region encoding at least one antigenic peptide or protein derived from a protein of an influenza virus or a fragment or variant thereof.

In a particularly preferred embodiment the composition comprises at least 2, 3, 4, 5, 6, 7, 6, 9, 10, 11, 12, 13, 14, 15, 16, 17, 18, 19, 20, 25, 30, 35, 40, 45, 50, 60, 70, 80, or 100 different mRNA sequences each encoding at least one antigenic peptide or protein preferably derived from a protein of an influenza virus or a fragment or variant thereof as defined above, preferably derived from hemagglutinin (HA) or neuraminidase (NA) of an influenza virus or a fragment or variant thereof.

In a most preferred embodiment the composition comprises 4 different mRNA sequences each encoding at least one antigenic peptide or protein preferably derived from a protein of an influenza virus or a fragment or variant thereof as defined above, preferably derived from hemagglutinin (HA) or neuraminidase (NA) of an influenza virus or a fragment or variant thereof.

In this context it is particularly preferred that each mRNA sequence encodes at least one different antigenic peptide or protein derived from proteins of the same pathogen, e.g. influenza virus, wherein it is particularly preferred that the antigenic peptide or protein is derived from different proteins of the same pathogen, e.g. influenza virus. Preferably the composition comprises at least two mRNA sequences, wherein at least one mRNA sequence encodes at least one antigenic peptide or protein derived from hemagglutinin (HA) of the influenza virus and at least one mRNA sequence encodes at least one antigenic peptide or protein derived from neuraminidase (NA) of the same influenza virus.

In another preferred embodiment each mRNA sequence encodes at least one different antigenic peptide or protein derived from proteins of different pathogens, e.g. influenza viruses. Preferably each mRNA sequence encodes at least one antigenic peptide or protein derived from hemagglutinin (HA) and/or neuraminidase (NA) of different influenza viruses.

Preferably, the (pharmaceutical) composition or vaccine according to the present invention comprises a plurality of mRNA sequences each encoding at least one antigenic peptide or protein derived from hemagglutinin (HA) and/or neuraminidase (NA) of an influenza virus, wherein at least one antigenic peptide or protein derived from hemagglutinin (HA) and/or neuraminidase (NA) of 2, 3, 4, 5, 6, 7, 6, 9, 10, 11, 12, 13, 14, 15, 16, 17, 18, 19, 20, 25, 30, 35, 40, 45, 50, 60, 70, 80, or 100 different influenza viruses are encoded by the plurality of mRNA sequences.

In this context it is particularly preferred that the (pharmaceutical) composition or vaccine comprises at least one mRNA comprising lipid nanoparticle comprising a mRNA compound comprising a mRNA sequence encoding at least one antigenic peptide or protein derived from a protein of influenza A virus H1, preferably hemagglutinin (HA) and/or neuraminidase (NA), at least one mRNA sequence encoding at least one antigenic peptide or protein derived from a protein of influenza A virus H3, preferably hemagglutinin (HA) and/or neuraminidase (NA), at least one mRNA sequence encoding at least one antigenic peptide or protein derived from a protein of influenza A virus H5, preferably hemagglutinin (HA) and/or neuraminidase (NA), and optionally at least one mRNA sequence encoding at least one antigenic peptide or protein derived from a protein of influenza A virus H7, preferably hemagglutinin (HA) and/or neuraminidase (NA), and/or optionally at least one mRNA sequence encoding at least one antigenic peptide or protein derived from a protein of influenza A virus H9, preferably hemagglutinin (HA) and/or neuraminidase (NA).

Preferably, the (pharmaceutical) composition or vaccine comprises at least one mRNA comprising lipid nanoparticle comprising a mRNA compound comprising a mRNA sequence encoding at least one antigenic peptide or protein derived from hemagglutinin (HA) and/or at least one mRNA sequence encoding at least one antigenic peptide or protein derived from neuraminidase (NA) of influenza A virus H1, at least one mRNA sequence encoding at least one antigenic peptide or protein derived from hemagglutinin (HA) and/or at least one mRNA sequence encoding at least one antigenic peptide or protein derived from neuraminidase (NA) of influenza A virus H3, at least one mRNA sequence encoding at least one antigenic peptide or protein derived from hemagglutinin (HA) and/or at least one mRNA sequence encoding at least one antigenic peptide or protein derived from neuraminidase (NA) of influenza A virus H5, and optionally at least one mRNA sequence encoding at least one antigenic peptide or protein derived from preferably hemagglutinin (HA) and/or at least one mRNA sequence encoding at least one antigenic peptide or protein derived from neuraminidase (NA) of influenza A virus H7, and/or optionally at least one mRNA sequence encoding at least one antigenic peptide or protein derived from, preferably hemagglutinin (HA) and/or at least one mRNA sequence encoding at least one antigenic peptide or protein derived from neuraminidase (NA) of influenza A virus H9.

In a specific embodiment the (pharmaceutical) composition or vaccine comprises at least one mRNA comprising lipid nanoparticle comprising an mRNA compound comprising a mRNA sequence encoding at least one antigenic peptide or protein derived from hemagglutinin (HA) and/or at least one mRNA sequence encoding at least one antigenic peptide or protein derived from neuraminidase (NA) of influenza A virus H1N1, at least one mRNA sequence encoding at least one antigenic peptide or protein derived from hemagglutinin (HA) and/or at least one mRNA sequence encoding at least one antigenic peptide or protein derived from neuraminidase (NA) of influenza A virus H3N2, at least one mRNA sequence encoding at least one antigenic peptide or protein derived from hemagglutinin (HA) and/or at least one mRNA sequence encoding at least one antigenic peptide or protein derived from neuraminidase (NA) of influenza A virus H5N1.

Additionally, the (pharmaceutical) composition or vaccine preferably further comprises at least one mRNA comprising lipid nanoparticle comprising a mRNA compound comprising a mRNA sequence encoding at least one antigenic peptide or protein derived from hemagglutinin (HA) and/or at least one mRNA sequence encoding at least one antigenic peptide or protein derived from neuraminidase (NA) of at least one influenza B virus, encapsulated or associated with mRNA comprising lipid nanoparticles according to the invention.

In this context it is particularly preferred that the (pharmaceutical) composition or vaccine comprises mRNA comprising lipid nanoparticles comprising mRNA comprising a plurality of mRNA sequences encoding at least one antigenic peptide or protein derived from hemagglutinin (HA) and/or at least one antigenic peptide or protein derived from neuraminidase (NA) of influenza
- A/Netherlands/602/2009 and/or A/California/7/2009,
- A/Hong Kong/4801/2014,
- B/Brisbane/60/2008, and
- A/Vietnam/1203/2004;
   or of influenza
- A/California/07/2009 (H1N1),
- A/Hong Kong/4801/2014 (H3N2),
- B/Brisbane/60/2008,
- B/Phuket/3073/2013,
- and optionally A/Michigan/45/2015 (H1N1)pdm09-like virus.

In a more preferred embodiment the pharmaceutical composition or vaccine comprises at least 4 different mRNA sequences derived from influenza virus antigens as defined above encapsulated or associated with mRNA comprising lipid nanoparticles according to the invention.

Preferably in this context the mRNA sequence(s) comprises or consists of the following RNA sequences of Table 11 ("preferred RNA sequences"):

**Table 11: preferred RNA sequences**

| **Strain** / **Organism** | **HA** | **NA** |
|---|---|---|
| A/Netherlands/602/2009 | SEQ ID NO: 224163 | SEQ ID NO: 224326 |
| A/California/7/2009 | SEQ ID NO: 224117 | SEQ ID NO: 224318 |
| A/Hong Kong/4801/2014 | SEQ ID NO: 224181 | SEQ ID NO: 224336 |
| A/Vietnam/1203/2004 or A/Vietnam/1194/2004 | SEQ ID NO: 224198 | SEQ ID NO: 224342 or SEQ ID NO: 224344 |
| B/Brisbane/60/2008 | SEQ ID NO: 224236 | SEQ ID NO: 224348 |
| B/Phuket/3073/2013 | SEQ ID NO: 224246 | SEQ ID NO: 224350 |
| A/Michigan/45/2015 (H1N1)pdm09-like virus | SEQ ID NO: 224133 | SEQ ID NO: 224324 |

In a specifically preferred embodiment the pharmaceutical composition or vaccine is a tetravalent influenza vaccine, comprising lipid nanoparticles, which comprise mRNA compounds as defined above.

Particularly preferred in this context is the combination of mRNAs encoding the following protein sequences (f.e. for preparing a tetravalent cocktail):
- HA protein of influenza A/Hong Kong/4801/2014 (H3N2) (preferably selected from the group consisting of SEQ ID NOs: 13853, 13854, 13855 and 13856); and/or
- HA protein of influenza A/California/07/2009 (H1N1) (preferably selected from the group consisting of SEQ ID NOs: 13836, 13837, 13838, 13839, 13840, 13841, 13842, 13843, and 13844); and/or
- HA protein of influenza B/Phuket/3037/2013 (EPI540671) (preferably selected from the group consisting of SEQ ID NOs: 28530, 28531 and 28532); and/or
- HA protein of influenza B/Brisbane/60/2008 (preferably selected from the group consisting of SEQ ID NOs: 28524, 28525, 28526, 28527, 28528, and 28529).

Further particularly preferred in this context is the combination of mRNAs encoding the following protein sequences (f.e. for preparing a triavalent cocktail):
- NA protein of influenza A/Hong Kong/4801/2014 (H3N2) (preferably selected from the group consisting of SEQ ID NOs: 26251, 26252, 26253, and 26254); and/or
- NA protein of influenza A/California/7/2009 (H1N1)pdm09 (preferably selected from the group consisting of SEQ ID NOs: 26238, 26239, 26240, 26241, 26242, and 26243); and/or
- NA protein of influenza B/Brisbane/60/2008 (preferably selected from the group consisting of SEQ ID NOs: 30455, 30456, 30457, 30458, 30459, and 30460).

Even further particularly preferred in this context is the combination of mRNAs encoding the following protein sequences (f.e. for preparing a tetravalent cocktail):
- HA protein of influenza A/Netherlands/602/2009 (H1N1) (preferably selected from the group consisting of SEQ ID NOs: 13848, 13849, and 13850); and/or
- HA protein of influenza A/Hong Kong/4801/2014 (H3N2) (preferably selected from the group consisting of SEQ ID NOs: 13853, 13854, 13855, and 13856); and/or
- HA protein of influenza B/Brisbane/60/2008 (preferably selected from the group consisting of SEQ ID NOs: 28524, 28525, 28526, 28527, 28528 and 28529); and/or
- HA protein of influenza A/Vietnam/1194/2004 (H5N1) (preferably selected from the group consisting of SEQ ID NOs: 13859 and 13860).

Also particularly preferred in this context is the combination of mRNAs encoding the following protein sequences (f.e. for preparing a septavalent cocktail):
- HA protein of influenza A/Hong Kong/4801/2014 (H3N2) (preferably selected from the group consisting of SEQ ID NOs: 13853, 13854, 13855, and 13856); and/or
- HA protein of influenza A/California/07/2009 (H1N1) (preferably selected from the group consisting of SEQ ID NOs: 13836, 13837, 13838, 13839, 13840, 13841, 13842, 13843, and 13844); and/or
- HA protein of influenza B/Phuket/3037/2013 (EPI540671) (preferably selected from the group consisting of SEQ ID NOs: 28530, 28531, and 28532); and/or
- HA protein of influenza B/Brisbane/60/2008 (preferably selected from the group consisting of SEQ ID NOs: 28524, 28525, 28526, 28527, 28528, and 28529); and/or
- NA protein of influenza A/Hong Kong/4801/2014 (H3N2) (preferably selected from the group consisting of SEQ ID NOs: 26251, 26252, 26253, and 26254); and/or
- NA protein of influenza A/California/7/2009 (H1N1)pdm09 (preferably selected from the group consisting of SEQ ID NOs: 26238, 26239, 26240, 26241, 26242 and 26243); and/or
- NA protein of influenza B/Brisbane/60/2008 (preferably selected from the group consisting of SEQ ID NOs: 30455, 30456, 30457, 30458, 30459, and 30460).

The composition according to the invention might also comprise suitable pharmaceutically acceptable adjuvants and excipients. In preferred embodiments the adjuvant is preferably added in order to enhance the immunostimulatory properties of the composition. In this context, an adjuvant may be understood as any compound, which is suitable to support administration and delivery of the composition according to the invention. Furthermore, such an adjuvant may, without being bound thereto, initiate or increase an immune response of the innate immune system, i.e. a non-specific immune response. In other words, when administered, the composition according to the invention typically initiates an adaptive immune response due to an antigen as defined herein or a fragment or variant thereof, which is encoded by the at least one coding sequence of the inventive mRNA contained in the composition of the present invention. Additionally, the composition according to the invention may generate an (supportive) innate immune response due to addition of an adjuvant as defined herein to the composition according to the invention.

Such an adjuvant may be selected from any adjuvant known to a skilled person and suitable for the present case, i.e. supporting the induction of an immune response in a mammal. Preferably, the adjuvant may be selected from the group consisting of, without being limited thereto, TDM, MDP, muramyl dipeptide, pluronics, alum solution, aluminium hydroxide, ADJUMERTM (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%), Tween 80 (0.2%), Pluronic L121 (1.25%), phosphate-buffered saline, pH 7.4); AVRIDINETM (propanediamine); BAY R1005TM ((N-(2-deoxy-2-L-leucylamino-b-D-glucopyranosyl)-N-octadecyl-dodecanoyl-amide hydroacetate); CALCITRIOLTM (1-alpha,25-dihydroxy-vitamin D3); calcium phosphate gel; CAPTM (calcium phosphate nanoparticles); cholera holotoxin, cholera-toxin-A1-protein-A-D-fragment fusion protein, sub-unit B of the cholera toxin; CRL 1005 (block copolymer P1205); cytokine-containing liposomes; DDA (dimethyldioctadecylammonium bromide); DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant; Freund's incomplete adjuvant; gamma inulin; Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c]quinoline-4-amine); ImmTherTM (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7; interleukin-12; ISCOMSTM; ISCOPREP 7.0.3.TM; liposomes; LOXORIBINETM (7-allyl-8-oxoguanosine); LT oral adjuvant (E.coli labile enterotoxin-protoxin); microspheres and microparticles of any composition; MF59TM; (squalene-water emulsion); MONTANIDE ISA 51TM (purified incomplete Freund's adjuvant); MONTANIDE ISA 720TM (metabolisable oil adjuvant); MPLTM (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDETM (Nac-Mur-L-Ala-D-Gln-OCH3); MURAPALMITINETM and D-MURAPALMITINETM (Nac-Mur-L-Thr-D-isoGIn-sn-glyceroldipalmitoyl); NAGO (neuraminidase-galactose oxidase); nanospheres or nanoparticles of any composition; NISVs (non-ionic surfactant vesicles); PLEURANTM (β-glucan); PLGA, PGA and PLA (homo- and co-polymers of lactic acid and glycolic acid; microspheres/nanospheres); PLURONIC L121TM; PMMA (polymethyl methacrylate); PODDSTM (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULONTM (QS-21); Quil-A (Quil-A saponin); S-28463 (4-amino-otec-dimethyl-2-ethoxymethyl-1H-imidazo[4,5 c]quinoline-1-ethanol); SAF-1TM ("Syntex adjuvant formulation"); Sendai proteoliposomes and Sendai-containing lipid matrices; Span-85 (sorbitan trioleate); Specol (emulsion of Marcol 52, Span 85 and Tween 85); squalene or Robane^{®} (2,6,10,15,19,23-hexamethyltetracosan and 2,6,10,15,19,23-hexamethyl-2,6,10,14,18,22-tetracosahexane); stearyltyrosine (octadecyltyrosine hydrochloride); Theramid^{®} (N-acetylglucosaminyl-N-acetylmuramyl-L-Ala-D-isoGlu-L-Ala-dipalmitoxypropylamide); Theronyl-MDP (TermurtideTM or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor, LNFPIII/Lewis X, antimicrobial peptides, UC-1V150, RSV fusion protein, cdiGMP; and adjuvants suitable as antagonists including CGRP neuropeptide.

Particularly preferred, an adjuvant may be selected from adjuvants, which support induction of a Th1-immune response or maturation of naïve T-cells, such as GM-CSF, IL-12, IFNy, any immunostimulatory nucleic acid as defined above, preferably an immunostimulatory RNA, CpG DNA, etc.

In a further preferred embodiment it is also possible that the inventive composition contains besides the antigen-providing mRNA further components which are selected from the group comprising: further antigens (e.g. in the form of a peptide or protein) or further antigen-encoding nucleic acids; a further immunotherapeutic agent; one or more auxiliary substances; or any further compound, which is known to be immunostimulating due to its binding affinity (as ligands) to human Toll-like receptors; and/or an adjuvant nucleic acid, preferably an immunostimulatory RNA (isRNA).

The composition of the present invention can additionally contain one or more auxiliary substances in order to increase its immunogenicity or immunostimulatory capacity, if desired. A synergistic action of the mRNA as defined herein and of an auxiliary substance, which may be optionally contained in the inventive composition, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

Suitable adjuvants may furthermore be selected from nucleic acids having the formula GIXmGn, wherein: G is guanosine, uracil or an analogue of guanosine or uracil; X is guanosine, uracil, adenosine, thymidine, cytosine or an analogue of the above-mentioned nucleotides; I is an integer from 1 to 40, wherein when I = 1 G is guanosine or an analogue thereof, when I > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof, or formula: (NuGlXmGnNv)a, wherein: G is guanosine (guanine), uridine (uracil) or an analogue of guanosine (guanine) or uridine (uracil), preferably guanosine (guanine) or an analogue thereof; X is guanosine (guanine), uridine (uracil), adenosine (adenine), thymidine (thymine), cytidine (cytosine), or an analogue of these nucleotides (nucleosides), preferably uridine (uracil) or an analogue thereof; N is a nucleic acid sequence having a length of about 4 to 50, preferably of about 4 to 40, more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides); a is an integer from 1 to 20, preferably from 1 to 15, most preferably from 1 to 10; I is an integer from 1 to 40, wherein when I = 1, G is guanosine (guanine) or an analogue thereof, when I > 1, at least 50% of these nucleotides (nucleosides) are guanosine (guanine) or an analogue thereof; m is an integer and is at least 3; wherein when m = 3, X is uridine (uracil) or an analogue thereof, and when m > 3, at least 3 successive uridines (uracils) or analogues of uridine (uracil) occur; n is an integer from 1 to 40, wherein when n = 1, G is guanosine (guanine) or an analogue thereof, when n > 1, at least 50% of these nucleotides (nucleosides) are guanosine (guanine) or an analogue thereof; u,v may be independently from each other an integer from 0 to 50, preferably wherein when u = 0, v ≥ 1, or when v = 0, u ≥ 1; wherein the nucleic acid molecule of formula (NuGlXmGnNv)a has a length of at least 50 nucleotides, preferably of at least 100 nucleotides, more preferably of at least 150 nucleotides, even more preferably of at least 200 nucleotides and most preferably of at least 250 nucleotides.

Other suitable adjuvants may furthermore be selected from nucleic acids having the formula: ClXmCn, wherein: C is cytosine, uracil or an analogue of cytosine or uracil; X is guanosine, uracil, adenosine, thymidine, cytosine or an analogue of the above-mentioned nucleotides; I is an integer from 1 to 40, wherein when I = 1 C is cytosine or an analogue thereof, when I > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof.

In this context the disclosure of WO2008/014979 and WO2009/095226 is also incorporated herein by reference.

In a further aspect, the present invention provides a vaccine, which is based on the mRNA comprising lipid nanoparticles according to the invention comprising at least one mRNA compound comprising a mRNA sequence comprising coding region as defined herein. The vaccine according to the invention is preferably a (pharmaceutical) composition as defined herein.

Accordingly, the vaccine according to the invention is based on the same components as the (pharmaceutical) composition described herein. Insofar, it may be referred to the description of the (pharmaceutical) composition as provided herein. Preferably, the vaccine according to the invention comprises at least one mRNA comprising lipid nanoparticles comprising at least one mRNA sequence as defined herein and a pharmaceutically acceptable carrier. In embodiments, where the vaccine comprises more than one mRNA sequence (such as a plurality of RNA sequences according to the invention, wherein each preferably encodes a distinct antigenic peptide or protein) encapsulated in mRNA comprising lipid nanoparticles, the vaccine may be provided in physically separate form and may be administered by separate administration steps. The vaccine according to the invention may correspond to the (pharmaceutical) composition as described herein, especially where the mRNA sequences are provided by one single composition. However, the inventive vaccine may also be provided physically separated. For instance, in embodiments, wherein the vaccine comprises more than one mRNA sequences/species encapsulated in mRNA comprising lipid nanoparticles as defined herein, these RNA species may be provided such that, for example, two, three, four, five or six separate compositions, which may contain at least one mRNA species/sequence each (e.g. three distinct mRNA species/sequences), each encoding distinct antigenic peptides or proteins, are provided, which may or may not be combined. Also, the inventive vaccine may be a combination of at least two distinct compositions, each composition comprising at least one mRNA encoding at least one of the antigenic peptides or proteins defined herein. Alternatively, the vaccine may be provided as a combination of at least one mRNA, preferably at least two, three, four, five, six or more mRNAs, each encoding one of the antigenic peptides or proteins defined herein. The vaccine may be combined to provide one single composition prior to its use or it may be used such that more than one administration is required to administer the distinct mRNA sequences/species encoding any of the antigenic peptides or proteins encapsulated in mRNA comprising lipid nanoparticles as defined herein. If the vaccine contains at least one mRNA comprising lipid nanoparticles, typically comprising at least two mRNA sequences, encoding the antigen combinations defined herein, it may e.g. be administered by one single administration (combining all mRNA species/sequences), by at least two separate administrations. Accordingly; any combination of mono-, bi- or multicistronic mRNAs encoding the at least one antigenic peptide or protein or any combination of antigens as defined herein (and optionally further antigens), provided as separate entities (containing one mRNA species) or as combined entity (containing more than one mRNA species), is understood as a vaccine according to the present invention. According to a particularly preferred embodiment of the inventive vaccine, the at least one antigen, preferably a combination as defined herein of at least two, three, four, five, six or more antigens encoded by the inventive composition as a whole, is provided as an individual (monocistronic) mRNA, which is administered separately.

As with the (pharmaceutical) composition according to the present invention, the entities of the vaccine may be provided in liquid and or in dry (e.g. lyophilized) form. They may contain further components, in particular further components allowing for its pharmaceutical use. The vaccine or the (pharmaceutical) composition may, e.g., additionally contain a pharmaceutically acceptable carrier and/or further auxiliary substances and additives and/or adjuvants.

The vaccine or (pharmaceutical) composition typically comprises a safe and effective amount of the mRNA compound according to the invention as defined herein, encoding an antigenic peptide or protein as defined herein or a fragment or variant thereof or a combination of antigens, encapsulate within and/or associated with the lipid nanoparticles. As used herein, "safe and effective amount" means an amount of the mRNA that is sufficient to significantly induce a positive modification of cancer or a disease or disorder related to cancer. At the same time, however, a "safe and effective amount" is small enough to avoid serious side-effects, that is to say to permit a sensible relationship between advantage and risk. The determination of these limits typically lies within the scope of sensible medical judgment. In relation to the vaccine or (pharmaceutical) composition of the present invention, the expression "safe and effective amount" preferably means an amount of the mRNA (and thus of the encoded antigen) that is suitable for stimulating the adaptive immune system in such a manner that no excessive or damaging immune reactions are achieved but, preferably, also no such immune reactions below a measurable level. Such a "safe and effective amount" of the mRNA of the (pharmaceutical) composition or vaccine as defined herein may furthermore be selected in dependence of the type of mRNA, e.g. monocistronic, bi- or even multicistronic mRNA, since a bi- or even multicistronic mRNA may lead to a significantly higher expression of the encoded antigen(s) than the use of an equal amount of a monocistronic mRNA. A "safe and effective amount" of the mRNA of the (pharmaceutical) composition or vaccine as defined above will furthermore vary in connection with the particular condition to be treated and also with the age and physical condition of the patient to be treated, the severity of the condition, the duration of the treatment, the nature of the accompanying therapy, of the particular pharmaceutically acceptable carrier used, and similar factors, within the knowledge and experience of the accompanying doctor. The vaccine or composition according to the invention can be used according to the invention for human and also for veterinary medical purposes, as a pharmaceutical composition or as a vaccine.

In a preferred embodiment, the mRNA comprising lipid nanoparticle of the (pharmaceutical) composition, vaccine or kit of parts according to the invention is provided in lyophilized form. Preferably, the lyophilized mRNA comprising lipid nanparticles are reconstituted in a suitable buffer, advantageously based on an aqueous carrier, prior to administration, e.g. Ringer-Lactate solution, Ringer solution, a phosphate buffer solution. In a preferred embodiment, the (pharmaceutical) composition, the vaccine or the kit of parts according to the invention contains at least one, two, three, four, five, six or more mRNA compounds, which may be provided as a single species of lipid nanoparticles, or separately for each LNP species, optionally in lyophilized form (optionally together with at least one further additive) and which are preferably reconstituted separately in a suitable buffer (such as Ringer-Lactate solution) prior to their use so as to allow individual administration of each of the (monocistronic) mRNAs.

The vaccine or (pharmaceutical) composition according to the invention may typically contain a pharmaceutically acceptable carrier. The expression "pharmaceutically acceptable carrier" as used herein preferably includes the liquid or non-liquid basis of the inventive vaccine. If the inventive vaccine is provided in liquid form, the carrier will be water, typically pyrogen-free water; isotonic saline or buffered (aqueous) solutions, e.g phosphate, citrate etc. buffered solutions. Particularly for injection of the inventive vaccine, water or preferably a buffer, more preferably an aqueous buffer, may be used, containing a sodium salt, preferably at least 50mM of a sodium salt, a calcium salt, preferably at least 0,01mM of a calcium salt, and optionally a potassium salt, preferably at least 3mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, NaI, NaBr, Na2CO3, NaHCO3, Na2SO4, examples of the optional potassium salts include e.g. KCl, KI, KBr, K2CO3, KHCO3, K2SO4, and examples of calcium salts include e.g. CaCl2, CaI2, CaBr2, CaCO3, CaSO4, Ca(OH)2. Furthermore, organic anions of the aforementioned cations may be contained in the buffer. According to a more preferred embodiment, the buffer suitable for injection purposes as defined above, may contain salts selected from sodium chloride (NaCl), calcium chloride (CaCl2) and optionally potassium chloride (KCl), wherein further anions may be present additional to the chlorides. CaCl2 can also be replaced by another salt like KCl. Typically, the salts in the injection buffer are present in a concentration of at least 50mM sodium chloride (NaCl), at least 3mM potassium chloride (KCl) and at least 0,01mM calcium chloride (CaCl2). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. in "in vivo" methods occurring liquids such as blood, lymph, cytosolic liquids, or other body liquids, or e.g. liquids, which may be used as reference media in "in vitro" methods, such as common buffers or liquids. Such common buffers or liquids are known to a skilled person.

However, one or more compatible solid or liquid fillers or diluents or encapsulating compounds may be used as well, which are suitable for administration to a person. The term "compatible" as used herein means that the constituents of the inventive vaccine are capable of being mixed with the mRNA according to the invention as defined herein, in such a manner that no interaction occurs, which would substantially reduce the pharmaceutical effectiveness of the inventive vaccine under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose, trehalose and sucrose; starches, such as, for example, corn starch or potato starch; dextrose; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

The choice of a pharmaceutically acceptable carrier is determined, in principle, by the manner, in which the pharmaceutical composition or vaccine according to the invention is administered. The composition or vaccine can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, and sublingual injections. More preferably, composition or vaccines according to the present invention may be administered by an intradermal, subcutaneous, or intramuscular route, preferably by injection, which may be needle-free and/or needle injection. Compositions/vaccines are therefore preferably formulated in liquid or solid form. The suitable amount of the vaccine or composition according to the invention to be administered can be determined by routine experiments, e.g. by using animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to a physiologically tolerable pH, such as about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive composition or vaccine is to be administered perorally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention, and can be made without difficulty by a person skilled in the art.

The inventive vaccine or composition can additionally contain one or more auxiliary substances in order to further increase the immunogenicity. A synergistic action of the mRNA contained in the inventive composition and of an auxiliary substance, which may be optionally be co-formulated (or separately formulated) with the inventive vaccine or composition as described above, is preferably achieved thereby. Depending on the various types of auxiliary substances, various mechanisms may play a role in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS, which allow an immune response produced by the immune-stimulating adjuvant according to the invention to be enhanced and/or influenced in a targeted manner. Particularly preferred auxiliary substances are cytokines, such as monokines, lymphokines, interleukins or chemokines, that - additional to induction of the adaptive immune response by the encoded at least one antigen - promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, INF-alpha, IFN-beta, INF-gamma, GM-CSF, G-CSF, M-CSF, LT-beta or TNF-alpha, growth factors, such as hGH. Preferably, such immunogenicity increasing agents or compounds are provided separately (not co-formulated with the inventive vaccine or composition) and administered individually.

Further additives which may be included in the inventive vaccine or composition are emulsifiers, such as, for example, Tween; wetting agents, such as, for example, sodium lauryl sulfate; colouring agents; taste-imparting agents, pharmaceutical carriers; tablet-forming agents; stabilizers; antioxidants; preservatives.

The inventive vaccine or composition can also additionally contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an inventive vaccine or composition in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

According to another aspect of the present invention, the present invention also provides a kit, in particular a kit of parts, comprising the mRNA compound comprising mRNA sequence as defined herein and at least one lipid according to formula (I), (II), (III) or (IV) as defined above. In a further embodiment the kit comprises a lipid nanoparticle as defined above or the (pharmaceutical) composition comprising a lipid nanoparticle as defined above, and/or the vaccine according to the invention, optionally a liquid vehicle for solubilising and optionally technical instructions with information on the administration and dosage of the mRNA comprising lipid nanoparticles, the composition and/or the vaccine. The technical instructions may contain information about administration and dosage of the mRNA comprising lipid nanoparticles, the composition and/or the vaccine. Such kits, preferably kits of parts, may be applied e.g. for any of the above mentioned applications or uses, preferably for the use of the lipid nanoparticle according to the invention (for the preparation of an inventive medicament, preferably a vaccine) for the treatment or prophylaxis of influenza virus infections or diseases or disorders related thereto. The kits may also be applied for the use of the lipid nanoparticle, the composition or the vaccine as defined herein (for the preparation of an inventive vaccine) for the treatment or prophylaxis of influenza virus infections or diseases or disorders related thereto, wherein the lipid nanoparticle, the composition and/or the vaccine may be capable of inducing or enhancing an immune response in a mammal as defined above. Such kits may further be applied for the use of the lipid nanoparticle, the composition or the vaccine as defined herein (for the preparation of an inventive vaccine) for modulating, preferably for eliciting, e.g. to induce or enhance, an immune response in a mammal as defined above, and preferably for supporting treatment or prophylaxis of influenza virus infections or diseases or disorders related thereto. Kits of parts, as a special form of kits, may contain one or more identical or different compositions and/or one or more identical or different vaccines as described herein in different parts of the kit. Kits of parts may also contain an (e.g. one) composition, an (e.g. one) vaccine and/or the mRNA comprising lipid nanoparticles according to the invention in different parts of the kit, e.g. each part of the kit containing an mRNA comprising lipid nanoparticles as defined herein, preferably encoding a distinct antigen. Preferably, the kit or the kit of parts contains as a part a vehicle for solubilising the mRNA according to the invention, the vehicle optionally being Ringer-lactate solution. Any of the above kits may be used in a treatment or prophylaxis as defined above.

In another embodiment of this aspect, the kit according to the present invention may additionally contain at least one adjuvant. In a further embodiment, the kit according to the present invention may additionally contain at least one further pharmaceutically active component, preferably a therapeutic compound suitable for treatment and/or prophylaxis of cancer or a related disorder. Moreover, in another embodiment, the kit may additionally contain parts and/or devices necessary or suitable for the administration of the composition or the vaccine according to the invention, including needles, applicators, patches, injection-devices.

In a further aspect the invention relates to the use of the mRNA comprising lipid nanoparticles or the pharmaceutical composition as a medicament. In an alternative embodiment the present invention relates to the use of the pharmaceutical composition or the mRNA comprising lipid in the manufacture of a medicament. In particular said medicament is for therapeutically or prophylactically raising an immune response of a subject in need thereof.

In a preferred embodiment the medicament is for prevention or treatment of cancer or tumour diseases, infectious diseases, allergies, or autoimmune diseases or disorders related thereto.

In particular the medicament is for the treatment of a subject, preferably a vertebrate. In a preferred embodiment the subject is a mammal, preferably selected from the group comprising goat, cattle, swine, dog, cat, donkey, monkey, ape, a rodent such as a mouse, hamster, rabbit and, particularly, human.

In a particular preferred embodiment the medicament is a vaccine, preferably a tumor, influenza or rabies vaccine. In a specific embodiment the medicament is a rabies vaccine used in rabies treatment.

The medicament might be administered in any suitable way. Preferably the medicament is for parenteral administration, in particular injection.

The invention further relates to a method for raising an immune response in a subject in need thereof, comprising administering to the subject a lipid nanoparticle as defined above or a pharmaceutical composition as defined above.

In a further aspect the invention relates to a method for prevention or treatment of cancer or tumour diseases, infectious diseases, allergies, or autoimmune diseases or disorders related thereto in a subject in need thereof, comprising administering to the subject a lipid nanoparticle as defined above or a pharmaceutical composition as defined above.

According to one aspect of the present invention, the mRNA comprising lipid nanoparticles, the (pharmaceutical) composition or the vaccine may be used according to the invention (for the preparation of a medicament) for the treatment or prophylaxis of cancer or tumour diseases, infectious diseases, allergies, or autoimmune diseases or disorders related thereto. In this context particularly preferred is the treatment or prophylaxis of Influenza virus or Rabies virus infections.

Furthermore, also included in the present inventions are methods of treating or preventing cancer or tumour diseases, infectious diseases, allergies, or autoimmune diseases or disorders related thereto, preferably as defined herein, by administering to a subject in need thereof a pharmaceutically effective amount of the mRNA comprising lipid nanoparticles, the (pharmaceutical) composition or the vaccine according to the invention. Such a method typically comprises an optional first step of preparing the mRNA comprising lipid nanoparticles, the composition or the vaccine of the present invention, and a second step, comprising administering (a pharmaceutically effective amount of) said composition or vaccine to a patient/subject in need thereof. A subject in need thereof will typically be a mammal. In the context of the present invention, the mammal is preferably selected from the group comprising, without being limited thereto, e.g. goat, cattle, swine, dog, cat, donkey, monkey, ape, a rodent such as a mouse, hamster, rabbit and, particularly, human. In some embodiments of the invention, the subject is a bird, preferably a chicken.

In this context, preferably included in the present invention are methods of treating or preventing influenza virus or Rabies virus infections or disorders related thereto.

The invention also relates to the use of the mRNA comprising lipid nanoparticles, the composition or the vaccine according to the invention, preferably for eliciting an immune response in a mammal, preferably for the treatment or prophylaxis of cancer or tumour diseases, infectious diseases, allergies, or autoimmune diseases or disorders related thereto, preferably of influenza virus or Rabies virus infections or a related condition as defined herein.

The present invention furthermore comprises the use of the mRNA comprising lipid nanoparticles, the (pharmaceutical) composition or the vaccine according to the invention as defined herein for modulating, preferably for inducing or enhancing, an immune response in a mammal as defined herein, more preferably for preventing and/or treating influenza virus infections, or of diseases or disorders related thereto. In this context, support of the treatment or prophylaxis of influenza virus infections may be any combination of a conventional influenza therapy method such as therapy with antivirals such as neuraminidase inhibitors (e.g. oseltamivir and zanamivir) and M2 protein inhibitors (e.g. adamantane derivatives), and a therapy using the RNA or the pharmaceutical composition as defined herein. Support of the treatment or prophylaxis of influenza virus infections may be also envisaged in any of the other embodiments defined herein. Accordingly, any use of the mRNA comprising lipid nanoparticles, the (pharmaceutical) composition or the vaccine according to the invention in co-therapy with any other approach, preferably one or more of the above therapeutic approaches, in particular in combination with antivirals is within the scope of the present invention.

For administration, preferably any of the administration routes may be used as defined herein. In particular, an administration route is used, which is suitable for treating or preventing an influenza virus infection as defined herein or diseases or disorders related thereto, by inducing or enhancing an adaptive immune response on the basis of an antigen encoded by the mRNA comprising lipid nanoparticles according to the invention. Administration of the composition and/or the vaccine according to the invention may then occur prior, concurrent and/or subsequent to administering another composition and/or vaccine as defined herein, which may - in addition - contain another mRNA comprising lipid nanoparticle or combination of mRNA comprising lipid nanoparticles encoding a different antigen or combination of antigens, wherein each antigen encoded by the mRNA sequence according to the invention is preferably suitable for the treatment or prophylaxis of influenza virus infections and diseases or disorders related thereto. In this context, a treatment as defined herein may also comprise the modulation of a disease associated to influenza virus infection and of diseases or disorders related thereto.

According to a preferred embodiment of this aspect of the invention, the (pharmaceutical) composition or the vaccine according to the invention is administered by injection. Any suitable injection technique known in the art may be employed. Preferably, the inventive composition is administered by injection, preferably by needle-less injection, for example by jet-injection.

In one embodiment, the inventive composition comprises at least one, two, three, four, five, six, seven, eight, nine, ten, eleven, twelve or more mRNAs as defined herein, each of which is preferably injected separately, preferably by needle-less injection. Alternatively, the inventive composition comprises at least one, two, three, four, five, six, seven, eight, nine, ten, eleven, twelve or more mRNAs, wherein the at least one, two, three, four, five, six, seven, eight, nine, ten, eleven, twelve or more mRNAs are administered, preferably by injection as defined herein, as a mixture.

In a further aspect the invention relates to a method of immunization of a subject against an antigen or a combination of antigens.

The immunization protocol for the immunization of a subject against an antigen or a combination of at least two, three, four, five, six, seven, eight, nine, ten, eleven, twelve or more antigens as defined herein typically comprises a series of single doses or dosages of the (pharmaceutical) composition or the vaccine according to the invention. A single dosage, as used herein, refers to the initial/first dose, a second dose or any further doses, respectively, which are preferably administered in order to "boost" the immune reaction. In this context, each single dosage preferably comprises the administration of the same antigen or the same combination of antigens as defined herein, wherein the interval between the administration of two single dosages can vary from at least one day, preferably 2, 3, 4, 5, 6 or 7 days, to at least one week, preferably 2, 3, 4, 5, 6, 7 or 8 weeks. The intervals between single dosages may be constant or vary over the course of the immunization protocol, e.g. the intervals may be shorter in the beginning and longer towards the end of the protocol. Depending on the total number of single dosages and the interval between single dosages, the immunization protocol may extend over a period of time, which preferably lasts at least one week, more preferably several weeks (e.g. 2, 3, 4, 5, 6, 7, 8, 9, 10, 11 or 12 weeks), even more preferably several months (e.g. 3, 4, 5, 6, 7, 8, 9, 10, 11, 12, 18 or 24 months). Each single dosage preferably encompasses the administration of an antigen, preferably of a combination of at least two, three, four, five, six, seven, eight, nine, ten, eleven, twelve or more antigens as defined herein and may therefore involve at least one, preferably 1, 2, 3, 4, 5, 6, 7, 8, 9, 10, 11 or 12 injections. In some cases, the composition or the vaccine according to the invention is administered as a single dosage typically in one injection. In the case, where the vaccine according to the invention comprises separate mRNA formulations encoding distinct antigens as defined herein, the minimum number of injections carried out during the administration of a single dosage corresponds to the number of separate components of the vaccine. In certain embodiments, the administration of a single dosage may encompass more than one injection for each component of the vaccine (e.g. a specific mRNA formulation comprising an mRNA encoding, for instance, one antigenic peptide or protein as defined herein). For example, parts of the total volume of an individual component of the vaccine may be injected into different body parts, thus involving more than one injection. In a more specific example, a single dosage of a vaccine comprising four separate mRNA formulations, each of which is administered in two different body parts, comprises eight injections. Typically, a single dosage comprises all injections required to administer all components of the vaccine, wherein a single component may be involve more than one injection as outlined above. In the case, where the administration of a single dosage of the vaccine according to the invention encompasses more than one injection, the injection are carried out essentially simultaneously or concurrently, i.e. typically in a time-staggered fashion within the time-frame that is required for the practitioner to carry out the single injection steps, one after the other. The administration of a single dosage therefore preferably extends over a time period of several minutes, e.g. 2, 3, 4, 5, 10, 15, 30 or 60 minutes.

Administration of the mRNA comprising lipid nanoparticles as defined herein, the (pharmaceutical) composition or the vaccine according to the invention may be carried out in a time staggered treatment. A time staggered treatment may be e.g. administration of the mRNA comprising lipid nanoparticles, the composition or the vaccine prior, concurrent and/or subsequent to a conventional therapy of influenza virus infections or diseases or disorders related thereto, e.g. by administration of the mRNA comprising lipid nanoparticles, the composition or the vaccine prior, concurrent and/or subsequent to a therapy or an administration of a therapeutic suitable for the treatment or prophylaxis of influenza virus infections or diseases or disorders related thereto. Such time staggered treatment may be carried out using e.g. a kit, preferably a kit of parts as defined herein.

Time staggered treatment may additionally or alternatively also comprise an administration of the mRNA comprising lipid nanoparticles as defined herein, the (pharmaceutical) composition or the vaccine according to the invention in a form, wherein the mRNA encoding an antigenic peptide or protein as defined herein or a fragment or variant thereof, preferably forming part of the composition or the vaccine, is administered parallel, prior or subsequent to another mRNA comprising lipid nanoparticles as defined above, preferably forming part of the same inventive composition or vaccine. Preferably, the administration (of all mRNA comprising lipid nanoparticles) occurs within an hour, more preferably within 30 minutes, even more preferably within 15, 10, 5, 4, 3, or 2 minutes or even within 1 minute. Such time staggered treatment may be carried out using e.g. a kit, preferably a kit of parts as defined herein.

In a preferred embodiment, the pharmaceutical composition or the vaccine of the present invention is administered repeatedly, wherein each administration preferably comprises individual administration of the at least one mRNA comprising lipid nanoparticles of the inventive composition or vaccine. At each time point of administration, the at least one mRNA may be administered more than once (e.g. 2 or 3 times). In a particularly preferred embodiment of the invention, at least two, three, four, five, six or more mRNA sequences (each encoding a distinct one of the antigens as defined herein) encapsulated or associated with mRNA comprising lipid nanoparticles as defined above, wherein the mRNA sequences are part of mRNA compounds of the same or different lipid nanoparticles, are administered at each time point, wherein each mRNA is administered twice by injection, distributed over the four limbs.

The following Reaction Schemes illustrate methods to make lipids of Formula (I), (II) or (III).

Embodiments of the lipid of Formula (I) (e.g., compound A-5) can be prepared according to General Reaction Scheme 1 ("Method A"), wherein R is a saturated or unsaturated C₁-C₂₄ alkyl or saturated or unsaturated cycloalkyl, m is 0 or 1 and n is an integer from 1 to 24. Referring to General Reaction Scheme 1, compounds of structure A-1 can be purchased from commercial sources or prepared according to methods familiar to one of ordinary skill in the art. A mixture of A-1, A-2 and DMAP is treated with DCC to give the bromide A-3. A mixture of the bromide A-3, a base (e.g., N,N-diisopropylethylamine) and the N,N-dimethyldiamine A-4 is heated at a temperature and time sufficient to produce A-5 after any necessarily workup and or purification step.

Other embodiments of the compound of Formula (I) (e.g., compound B-5) can be prepared according to General Reaction Scheme 2 ("Method B"), wherein R is a saturated or unsaturated C₁-C₂₄ alkyl or saturated or unsaturated cycloalkyl, m is 0 or 1 and n is an integer from 1 to 24. As shown in General Reaction Scheme 2, compounds of structure B-1 can be purchased from commercial sources or prepared according to methods familiar to one of ordinary skill in the art. A solution of B-1 (1 equivalent) is treated with acid chloride B-2 (1 equivalent) and a base (e.g., triethylamine). The crude product is treated with an oxidizing agent (e.g., pyridinum chlorochromate) and intermediate product B-3 is recovered. A solution of crude B-3, an acid (e.g., acetic acid), and N,N-dimethylaminoamine B-4 is then treated with a reducing agent (e.g., sodium triacetoxyborohydride) to obtain B-5 after any necessary work up and/or purification.

It should be noted that although starting materials A-1 and B-1 are depicted above as including only saturated methylene carbons, starting materials which include carbon-carbon double bonds may also be employed for preparation of compounds which include carbon-carbon double bonds.

Different embodiments of the lipid of Formula (I) (e.g., compound C-7 or C9) can be prepared according to General Reaction Scheme 3 ("Method C"), wherein R is a saturated or unsaturated C₁-C₂₄ alkyl or saturated or unsaturated cycloalkyl, m is 0 or 1 and n is an integer from 1 to 24. Referring to General Reaction Scheme 3, compounds of structure C-1 can be purchased from commercial sources or prepared according to methods familiar to one of ordinary skill in the art.

Embodiments of the compound of Formula (II) (e.g., compounds D-5 and D-7) can be prepared according to General Reaction Scheme 4 ("Method D"), wherein R^{1a}, R^{1b}, R^{2a}, R^{2b}, R^{3a}, R^{3b}, R^{4a} R^{4b} R⁵, R⁶, R⁸, R⁹, L¹, L², G¹, G², G³, a, b, c and d are as defined herein, and R^{7'} represents R⁷ or a C₃-C₁₉ alkyl. Referring to General Reaction Scheme 1, compounds of structure D-1 and D-2 can be purchased from commercial sources or prepared according to methods familiar to one of ordinary skill in the art. A solution of D-1 and D-2 is treated with a reducing agent (e.g., sodium triacetoxyborohydride) to obtain D-3 after any necessary work up. A solution of D-3 and a base (e.g. trimethylamine, DMAP) is treated with acyl chloride D-4 (or carboxylic acid and DCC) to obtain D-5 after any necessary work up and/or purification. D-5 can be reduced with LiAlH4 D-6 to give D-7 after any necessary work up and/or purification.

Embodiments of the lipid of Formula (II) (e.g., compound E-5) can be prepared according to General Reaction Scheme 5 ("Method E"), wherein R^{1a}, R^{1b}, R^{2a}, R^{2b} R^{3a}, R^{3b} , R^{4a}, R^{4b} R⁵, R⁶, R⁷, R⁸, R⁹, L¹, L², G³, a, b, c and d are as defined herein. Referring to General Reaction Scheme 2, compounds of structure E-1 and E-2 can be purchased from commercial sources or prepared according to methods familiar to one of ordinary skill in the art. A mixture of E-1 (in excess), E-2 and a base (e.g., potassium carbonate) is heated to obtain E-3 after any necessary work up. A solution of E-3 and a base (e.g. trimethylamine, DMAP) is treated with acyl chloride E-4 (or carboxylic acid and DCC) to obtain E-5 after any necessary work up and/or purification.

Other embodiments of the compound of Formula (II) (e.g., F-9) are prepared according to General Reaction Scheme 6 (Method F). As illustrated in General Reaction Scheme 6, an appropriately protected ketone (F-1) is reacted under reductive amination conditions with amine F-2 to yield F-3. Acylation of F-3 with acid chloride F-4 yields acylated product F-5. Removal of the alcohol protecting group on F-5 followed by reaction with F-7 and/or F-8 and appropriate activating reagent (e.g., DCC) yields the desired compound F-9.

General Reaction Scheme 7 provides an exemplary method (Method G) for preparation of Lipids of Formula (III). G¹, G³, R¹ and R³ in General Reaction Scheme 7 are as defined herein for Formula (III), and G1' refers to a one-carbon shorter homologue of G1. Compounds of structure G-1 are purchased or prepared according to methods known in the art. Reaction of G-1 with diol G-2 under appropriate condensation conditions (e.g., DCC) yields ester/alcohol G-3, which can then be oxidized (e.g., PCC) to aldehyde G-4. Reaction of G-4 with amine G-5 under reductive amination conditions yields a lipid of Formula (III).

It should be noted that various alternative strategies for preparation of lipids of Formula (III) are available to those of ordinary skill in the art. For example, other lipids of Formula (III) wherein L¹ and L² are other than ester can be prepared according to analogous methods using the appropriate starting material. Further, General Reaction Scheme 6 depicts preparation of lipids of Formula (III), wherein G¹ and G² are the same; however, this is not a required aspect of the invention and modifications to the above reaction scheme are possible to yield compounds wherein G¹ and G² are different.

It will be appreciated by those skilled in the art that in the process described herein the functional groups of intermediate compounds may need to be protected by suitable protecting groups. Such functional groups include hydroxy, amino, mercapto and carboxylic acid. Suitable protecting groups for hydroxy include trialkylsilyl or diarylalkylsilyl (for example, t-butyldimethylsilyl, t-butyldiphenylsilyl or trimethylsilyl), tetrahydropyranyl, benzyl, and the like. Suitable protecting groups for amino, amidino and guanidino include t-butoxycarbonyl, benzyloxycarbonyl, and the like. Suitable protecting groups for mercapto include -C(O)-R" (where R" is alkyl, aryl or arylalkyl), p-methoxybenzyl, trityl and the like. Suitable protecting groups for carboxylic acid include alkyl, aryl or arylalkyl esters. Protecting groups may be added or removed in accordance with standard techniques, which are known to one skilled in the art and as described herein. The use of protecting groups is described in detail in Green, T.W. and P.G.M. Wutz, Protective Groups in Organic Synthesis (1999), 3rd Ed., Wiley. As one of skill in the art would appreciate, the protecting group may also be a polymer resin such as a Wang resin, Rink resin or a 2-chlorotrityl-chloride resin.

### Preparation of Lipid Nanoparticle Compositions:

LNPs were prepared as follows. Cationic lipid, DSPC, cholesterol and PEG-lipid were solubilized in ethanol. Lipid nanoparticles (LNP) were prepared at a total lipid to mRNA weight ratio of approximately 10:1 to 30:1. Briefly, the mRNA was diluted to 0.05 to 0.2mg/mL in 10 to 50mM citrate buffer, pH 4. Syringe pumps were used to mix the ethanolic lipid solution with the mRNA aqueous solution at a ratio of about 1:5 to 1:3 (vol/vol) with total flow rates above 15 ml/min. The ethanol was then removed and the external buffer replaced with PBS by dialysis. Finally, the lipid nanoparticles were filtered through a 0.2 µm pore sterile filter. Lipid nanoparticle particle size was 70-90 nm diameter as determined by quasi-elastic light scattering using a Malvern Zetasizer Nano (Malvern, UK).

### Items

Item 1. A lipid nanoparticle comprising
(i) a cationic lipid with the formula I: or a pharmaceutically acceptable salt, tautomer, prodrug or stereoisomer thereof, wherein:
   L1 and L2 are each independently -O(C=O)-, -(C=O)O- or a carbon-carbon double bond;
   R1a and R1b are, at each occurrence, independently either (a) H or C1-C12 alkyl, or (b) R1a is H or C1-C12 alkyl, and R1b together with the carbon atom to which it is bound is taken together with an adjacent R1b and the carbon atom to which it is bound to form a carbon-carbon double bond;
   R2a and R2b are, at each occurrence, independently either (a) H or C1-C12 alkyl, or (b) R2a is H or C1-C12 alkyl, and R2b together with the carbon atom to which it is bound is taken together with an adjacent R2b and the carbon atom to which it is bound to form a carbon-carbon double bond;
   R3a and R3b are, at each occurrence, independently either (a) H or C1-C12 alkyl, or (b) R3a is H or C1-C12 alkyl, and R3b together with the carbon atom to which it is bound is taken together with an adjacent R3b and the carbon atom to which it is bound to form a carbon-carbon double bond;
   R4a and R4b are, at each occurrence, independently either (a) H or C1-C12 alkyl, or (b) R4a is H or C1-C12 alkyl, and R4b together with the carbon atom to which it is bound is taken together with an adjacent R4b and the carbon atom to which it is bound to form a carbon-carbon double bond;
   R5 and R6 are each independently methyl or cycloalkyl;
   R7 is, at each occurrence, independently H or C1-C12 alkyl;
   R8 and R9 are each independently C1-C12 alkyl; or R8 and R9, together with the nitrogen atom to which they are attached, form a 5, 6 or 7-membered heterocyclic ring comprising one nitrogen atom;
   a and d are each independently an integer from 0 to 24;
   b and c are each independently an integer from 1 to 24; and
   e is 1 or 2;
(ii) a mRNA compound comprising an mRNA sequence encoding at least one antigenic peptide or protein,

wherein the mRNA compound optionally does not comprise a nucleoside modification, in particular not a base modification,
wherein the mRNA compound is encapsulated in or associated with said lipid nanoparticle.

Item 2. A lipid nanoparticle comprising
(i) a cationic lipid with the formula II: or a pharmaceutically acceptable salt, tautomer, prodrug or stereoisomer thereof, wherein:
   L1 and L2 are each independently O(C=O) , (C=O)O , C(=O) , O , S(O)x , S S, C(=O)S , SC(=O) , NRaC(=O) , C(=O)NRa , NRaC(=O)NRa, OC(=O)NRa , NRaC(=O)O , or a direct bond; G1 is C1-C2 alkylene, -(C=O) , -O(C=O) , SC(=O) , -NRaC(=O)- or a direct bond G2 is -C(=O) , (C=O)O-, C(=O)S-, C(=O)NRa or a direct bond;
   G3 is C1-C6 alkylene;
   Ra is H or C1-C12 alkyl;
   R1a and R1b are, at each occurrence, independently either: (a) H or C1-C12 alkyl; or (b) R1a is H or C1-C12 alkyl, and R1b together with the carbon atom to which it is bound is taken together with an adjacent R1b and the carbon atom to which it is bound to form a carbon-carbon double bond;
   R2a and R2b are, at each occurrence, independently either: (a) H or C1-C12 alkyl; or (b) R2a is H or C1-C12 alkyl, and R2b together with the carbon atom to which it is bound is taken together with an adjacent R2b and the carbon atom to which it is bound to form a carbon-carbon double bond;
   R3a and R3b are, at each occurrence, independently either: (a) H or C1-C12 alkyl; or (b) R3a is H or C1-C12 alkyl, and R3b together with the carbon atom to which it is bound is taken together with an adjacent R3b and the carbon atom to which it is bound to form a carbon-carbon double bond;
   R4a and R4b are, at each occurrence, independently either: (a) H or C1-C12 alkyl; or (b) R4a is H or C1-C12 alkyl, and R4b together with the carbon atom to which it is bound is taken together with an adjacent R4b and the carbon atom to which it is bound to form a carbon-carbon double bond;
   R5 and R6 are each independently H or methyl;
   R7 is C4-C20 alkyl;
   R8 and R9 are each independently C1-C12 alkyl; or R8 and R9, together with the nitrogen atom to which they are attached, form a 5, 6 or 7-membered heterocyclic ring;
   a, b, c and d are each independently an integer from 1 to 24; and
   x is 0, 1 or 2;
(ii) a mRNA compound comprising an mRNA sequence encoding at least one antigenic peptide or protein,

wherein the mRNA compound optionally does not comprise a nucleoside modification, in particular not a base modification;
wherein the mRNA compound is encapsulated in or associated with said lipid nanoparticle.

Item 3. A lipid nanoparticle comprising
(i) a cationic lipid with the formula III: or a pharmaceutically acceptable salt, tautomer, prodrug or stereoisomer thereof, wherein:
   L1 or L2 is each independently -O(C=O)-, -(C=O)O-, -C(=O)-, -O-, -S(O)x-, -S-S-, C(=O)S-, SC(=O)-, - NRaC(=O)-, -C(=O)NRa-, NRaC(=O)NRa , -OC(=O)NRa- or NRaC(=O)O-, preferably L1 or L2 is -O(C=O)- or - (C=O)O-;
   G1 and G2 are each independently unsubstituted C1-C12 alkylene or C1-C12 alkenylene;
   G3 is C1-C24 alkylene, C1-C24 alkenylene, C3-C8 cycloalkylene, or C3-C8 cycloalkenylene;
   Ra is H or C1-C12 alkyl;
   R1 and R2 are each independently C6-C24 alkyl or C6-C24 alkenyl;
   R3 is H, OR5, CN, C(=O)OR4, OC(=O)R4 or -NR5C(=O)R4;
   R4 is C1-C12 alkyl;
   R5 is H or C1-C6 alkyl; and
   x is 0, 1 or 2;
(ii) a mRNA compound comprising an mRNA sequence encoding at least one antigenic peptide or protein,

wherein the mRNA compound optionally does not comprise a nucleoside modification, in particular not a base modification;
wherein the mRNA compound is encapsulated in or associated with said lipid nanoparticle.

Item 4. A lipid nanoparticle comprising:
(i) a PEG lipid with the formula (IV)
   wherein R8 and R9 are each independently a straight or branched, saturated or unsaturated alkyl chain containing from 10 to 30 carbon atoms, wherein the alkyl chain is optionally interrupted by one or more ester bonds;
   and w has a mean value ranging from 30 to 60; and
(ii) a mRNA compound comprising an mRNA sequence encoding at least one antigenic peptide or protein,

wherein the mRNA compound optionally does not comprise a nucleoside modification, in particular not a base modification;
wherein the mRNA compound is encapsulated in or associated with said lipid nanoparticle.

Item 5. The lipid nanoparticle according to any one of items 1to 3, additionally comprising
(iii) a PEG lipid with the formula (IV):
wherein R8 and R9 are each independently a straight or branched, saturated or unsaturated alkyl chain containing from 10 to 30 carbon atoms, wherein the alkyl chain is optionally interrupted by one or more ester bonds;
and w has a mean value ranging from 30 to 60.

Item 6. The lipid nanoparticle according to item 3 or 5, wherein the cationic lipid is a compound of formula III, and wherein:
L1 and L2 are each independently -O(C=O)- or (C=O)-O-;
G3 is C1-C24 alkylene or C1-C24 alkenylene; and
R3 is H or OR5.

Item 7. The lipid nanoparticle according to any one of items 3, 5 or 6, wherein the cationic lipid is a compound of formula III, and wherein:
L1 and L2 are each independently -O(C=O)- or (C=O)-O-;
R1 and R2 each independently have one of the following structures:

Item 8. The lipid nanoparticle according to any one of items 3 or 5 to 7, wherein the cationic lipid is a compound of formula III, and wherein R3 is OH.

Item 9. The lipid nanoparticle according to any one of items 1 to 3 or 5 to 8, wherein the cationic lipid is selected from structures I-1 to I-41, II-1 to II-34 or III-1 to III-36, or Table 7: Representative Lipids of Formula (I), Table 8: Representative Lipids of Formula (II), or Table 9: Representative Lipids of Formula (III).

Item 10. The lipid nanoparticle according to any one of items 1 to 3 or 5 to 9, wherein the cationic lipid is selected from: or

Item 11. The lipid nanoparticle according to any one of items 4 to 10, wherein in the PEG lipid R8 and R9 are saturated alkyl chains.

Item 12. The lipid nanoparticle according to item 11, wherein the PEG lipid is wherein n has a mean value ranging from 30 to 60.

Item 13. The lipid nanoparticle according to any one of items 1 to 12, wherein the mRNA compound comprises at least one chemical modification, and wherein the mRNA compound preferably does not comprise a nucleoside modification, wherein said nucleoside modification is optionally a base modification, and wherein said base modification is optionally a 1-methylpseudouridine modification.

Item 14. The lipid nanoparticle according to item 13, wherein the chemical modification is selected from the group comprising sugar modifications, backbone modifications and lipid modifications.

Item 15. The lipid nanoparticle according to any one of items 1 to 14, wherein the mRNA sequence is an artificial mRNA sequence.

Item 16. The lipid nanoparticle according to any one of items 1 to 15 wherein the coding region of the mRNA sequence encoding the at least one antigenic peptide or protein comprises a sequence modification.

Item 17. The lipid nanoparticle according to item 16, wherein the sequence modification is selected from a G/C content modification, a codon modification, a codon optimization or a C-optimization of the sequence.

Item 18. The lipid nanoparticle according to item 17, wherein the G/C content of the coding region of the mRNA sequence is increased compared to the G/C content of the corresponding coding sequence of the wild-type mRNA, or wherein the C content of the coding region of the mRNA sequence is increased compared to the C content of the corresponding coding sequence of the wild-type mRNA, or wherein the codon usage in the coding region of the mRNA sequence is adapted to the human codon usage, or wherein the codon adaptation index (CAI) is increased or maximised in the coding region of the mRNA sequence, wherein the encoded amino acid sequence of the mRNA sequence is preferably not being modified compared to the encoded amino acid sequence of the wild-type mRNA.

Item 19. The lipid nanoparticle according to any one of items 1 to 18, wherein the mRNA sequence additionally comprises
a) a 5'-CAP structure, and/or
b) a poly(A) sequence, and/or
c) a poly (C) sequence.

Item 20. The lipid nanoparticle according to item 19, wherein the mRNA sequence comprises a poly(A) sequence, wherein the poly(A) sequence comprises a sequence of about 25 to about 400 adenosine nucleotides, preferably a sequence of about 50 to about 400 adenosine nucleotides, more preferably a sequence of about 50 to about 300 adenosine nucleotides, even more preferably a sequence of about 50 to about 250 adenosine nucleotides, most preferably a sequence of about 60 to about 250 adenosine nucleotides.

Item 21. The lipid nanoparticle according to any one of items 1 to 19, wherein the mRNA sequence additionally comprises at least one histone stem loop.

Item 22. The lipid nanoparticle according to item 21, wherein the at least one histone stem-loop comprises a nucleic acid sequence according to the following formulae (V) or (VI): wherein
stem1 or stem2 bordering elements N1-6 is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
stem1 [N0-2GN3-5] is reverse complementary or partially reverse complementary with element stem2, and is a consecutive sequence between of 5 to 7 nucleotides;
wherein N0-2 is a consecutive sequence of 0 to 2, preferably of 0 to 1, more preferably of 1 N, wherein each N is independently from another selected from a nucleotide selected from A, U, T, G and C or a nucleotide analogue thereof;
wherein N3-5 is a consecutive sequence of 3 to 5, preferably of 4 to 5, more preferably of 4 N, wherein each N is independently from another selected from a nucleotide selected from A, U, T, G and C or a nucleotide analogue thereof, and
loop sequence [N0-4(U/T)N0-4] is located between elements stem1 and stem2, and is a consecutive sequence of 3 to 5 nucleotides, more preferably of 4 nucleotides;
wherein each N0-4 is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
wherein U/T represents uridine, or optionally thymidine;
stem2 [N3-5CN0-2] is reverse complementary or partially reverse complementary with element stem1, and is a consecutive sequence between of 5 to 7 nucleotides;
wherein N3-5 is a consecutive sequence of 3 to 5, preferably of 4 to 5, more preferably of 4 N, wherein each N is independently from another selected from a nucleotide selected from A, U, T, G and C or a nucleotide analogue thereof;
wherein N0-2 is a consecutive sequence of 0 to 2, preferably of 0 to 1, more preferably of 1 N, wherein each N is independently from another selected from a nucleotide selected from A, U, T, G and C or a nucleotide analogue thereof; and
wherein C is cytidine or an analogue thereof, and may be optionally replaced by a guanosine or an analogue thereof provided that its complementary nucleotide guanosine in stem1 is replaced by cytidine;

Item 23. The lipid nanoparticle according to item 21 or 22, wherein the at least one histone stem loop comprises a nucleic acid sequence according to SEQ ID NO: 224305 and/or most preferably an RNA sequence according to SEQ ID NO: 224306.

Item 24. The lipid nanoparticle according to any one of items 19 to 23, wherein the mRNA sequence comprises a poly(A) sequence, preferably comprising 10 to 200, 10 to 100, 40 to 80 or 50 to 70 adenosine nucleotides, and/or a poly(C) sequence, preferably comprising 10 to 200, 10 to 100, 20 to 70, 20 to 60 or 10 to 40 cytosine nucleotides.

Item 25. The lipid nanoparticle according to any one of items 1 to 24, wherein the mRNA sequence comprises, preferably in 5' to 3' direction, the following elements:
a) a 5'-CAP structure, preferably m7GpppN,
b) at least one coding region encoding at least one antigenic peptide or protein,
c) a poly(A) tail, preferably consisting of 10 to 200, 10 to 100, 40 to 80 or 50 to 70 adenosine nucleotides,
d) optionally a poly(C) tail, preferably consisting of 10 to 200, 10 to 100, 20 to 70, 20 to 60 or 10 to 40 cytosine nucleotides, and
e) optionally a histone stem-loop, preferably comprising the RNA sequence according to SEQ ID NO: 224306

Item 26. The lipid nanoparticle according to any one of items 1 to 25, wherein the mRNA sequence comprises a 3'-UTR element.

Item 27. The lipid nanoparticle according to item 26, wherein the at least one 3'-UTR element comprises or consists of a nucleic acid sequence which is derived from a 3'-UTR of a gene providing a stable mRNA or from a homolog, a fragment or a variant thereof.

Item 28. The lipid nanoparticle according to item 26 or 27, wherein the 3'-UTR element comprises a nucleic acid sequence derived from a 3'-UTR of an a-globin gene, preferably comprising the corresponding RNA sequence of the nucleic acid sequence according to SEQ ID NO: 224297, a homolog, a fragment, or a variant thereof.

Item 29. The lipid nanoparticle according to any one of items 26 to 28, wherein the at least one 3'-UTR element comprises a nucleic acid sequence, which is derived from the 3'-UTR of a vertebrate albumin gene or from a variant thereof, preferably from the 3'-UTR of a mammalian albumin gene or from a variant thereof, more preferably from the 3'-UTR of a human albumin gene or from a variant thereof, even more preferably from the 3' UTR of the human albumin gene according to GenBank Accession number NM_000477.5, or from a fragment or variant thereof.

Item 30. The lipid nanoparticle according to any one of items 26 to 29, wherein the 3-'UTR element is derived from a nucleic acid sequence according to SEQ ID NO: 224301 or 224303, preferably from a corresponding RNA sequence, or a homolog, a fragment or a variant thereof.

Item 31. The lipid nanoparticle according to any one of items 1 to 30, wherein the mRNA sequence comprises, preferably in 5' to 3' direction, the following elements:
a) a 5'-CAP structure, preferably m7GpppN,
b) at least one coding region encoding at least one antigenic peptide or protein,
c) a 3'-UTR element comprising or consisting of a nucleic acid sequence which is derived from an alpha globin gene, preferably comprising the corresponding RNA sequence of the nucleic acid sequence according to SEQ ID NO. 224297, a homolog, a fragment or a variant thereof;
d) a poly(A) tail, preferably consisting of 10 to 200, 10 to 100, 40 to 80 or 50 to 70 adenosine nucleotides,
e) optionally a poly(C) tail, preferably consisting of 10 to 200, 10 to 100, 20 to 70, 20 to 60 or 10 to 40 cytosine nucleotides, and
f) optionally a histone stem-loop, preferably comprising the RNA sequence according to SEQ ID NO: 224306.

Item 32. The lipid nanoparticle according to any one of items 1 to 31, wherein the mRNA sequence comprises a 5'-UTR element.

Item 33. The lipid nanoparticle according to item 32, wherein the 5'-UTR element comprises or consists of a nucleic acid sequence which is derived from the 5'-UTR of a TOP gene preferably from a corresponding RNA sequence, a homolog, a fragment, or a variant thereof, preferably lacking the 5'TOP motif.

Item 34. The lipid nanoparticle according to item 33, wherein the 5'-UTR element comprises or consists of a nucleic acid sequence which is derived from a 5'-UTR of a TOP gene encoding a ribosomal protein, preferably from a corresponding RNA sequence or from a homolog, a fragment or a variant thereof, preferably lacking the 5'TOP motif.

Item 35. The lipid nanoparticle according to item 34, wherein the 5'-UTR element comprises or consists of a nucleic acid sequence which is derived from a 5'-UTR of a TOP gene encoding a ribosomal Large protein (RPL) or from a homolog, a fragment or variant thereof, preferably lacking the 5'TOP motif and more preferably comprising or consisting of a corresponding RNA sequence of the nucleic acid sequence according to SEQ ID NO. 224287.

Item 36. The lipid nanoparticle according to item 35, wherein the 5'-UTR element which is derived from a 5'-UTR of a TOP gene comprises or consists of a corresponding RNA sequence of a nucleic acid sequence according to SEQ ID NO. 224287 or 224289.

Item 37. The lipid nanoparticle according to any one of items 1 to 36, wherein the mRNA sequence comprises, preferably in 5' to 3' direction, the following elements:
a) a 5'-CAP structure, preferably m7GpppN,
b) a 5'-UTR element which comprises or consists of a nucleic acid sequence which is derived from the 5'-UTR of a TOP gene, preferably comprising or consisting of the corresponding RNA sequence of a nucleic acid sequence according to SEQ ID NO. 224287 or 224289, a homolog, a fragment or a variant thereof;
c) at least one coding region encoding at least one antigenic peptide or protein;
d) a 3'-UTR element comprising or consisting of a nucleic acid sequence which is derived from an albumin gene, preferably comprising the corresponding RNA sequence of the nucleic acid sequence according to SEQ ID NO. 224303, a homolog, a fragment or a variant thereof;
e) a poly(A) tail, preferably consisting of 10 to 200, 10 to 100, 40 to 80 or 50 to 70 adenosine nucleotides,
f) optionally a poly(C) tail, preferably consisting of 10 to 200, 10 to 100, 20 to 70, 20 to 60 or 10 to 40 cytosine nucleotides, and
g) optionally a histone stem-loop, preferably comprising the RNA sequence according to SEQ ID NO: 224306.

Item 38. The lipid nanoparticle according to any one of items 1 to 37, wherein the antigenic peptide or protein is derived from pathogenic antigens, tumour antigens, allergenic antigens or autoimmune self-antigens or a fragment or variant thereof.

Item 39. The lipid nanoparticle according to item 38, wherein the pathogenic antigen is derived from an influenza or rabies virus.

Item 40. The lipid nanoparticle according item 39, wherein the antigenic peptide or protein is derived from hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, or polymerase basic protein 2 (PB2) of an influenza virus or a fragment or variant thereof.

Item 41. The lipid nanoparticle according to items 40, wherein the antigenic peptide or protein is derived from hemagglutinin (HA) or neuraminidase (NA) of an influenza virus or a fragment or variant thereof.

Item 42. The lipid nanoparticle according to item 41, wherein the antigenic peptide or protein is at least one full-length protein of hemagglutinin (HA) and/or at least one full-length protein of neuraminidase (NA) of an influenza virus or a variant thereof.

Item 43. The lipid nanoparticle according to any one of items 39 to 41, wherein the influenza virus is selected from an influenza A, B or C virus.

Item 44. The lipid nanoparticle according to item 43, wherein the influenza A virus is selected from an influenza virus characterized by a hemagglutinin (HA) selected from the group consisting of H1, H2, H3, H4, H5, H6, H7, H8, H9, H10, H11, H12, H13, H14, H15, H16, H17 and H18.

Item 45. The lipid nanoparticle according to item 43 or 44, wherein the influenza A virus is selected from an influenza virus characterized by a neuraminidase (NA) selected from the group consisting of N1, N2, N3, N4, N5, N6, N7, N8, N9, N10, and N11.

Item 46. The lipid nanoparticle according to any one of items 43 to 45, wherein the influenza A virus is selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, and H10N7, preferably from H1N1, H3N2, H5N1.

Item 47. The lipid nanoparticle according to any one of items 39 to 46, wherein the mRNA sequence comprises at least one coding region encoding at least one antigenic peptide or protein derived from hemagglutinin (HA) of an influenza virus or a fragment or variant thereof and at least one antigenic peptide or protein derived from neuraminidase (NA) of an influenza virus or a fragment or variant thereof.

Item 48. The lipid nanoparticle according to any one of items 39 to 47, wherein the mRNA sequence comprises at least one coding region encoding at least one antigenic peptide or protein derived from hemagglutinin (HA) and/or at least one antigenic peptide or protein derived from neuraminidase (NA) of an influenza A virus selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, and H10N7, preferably from H1N1, H3N2, H5N1 or a fragment or variant thereof.

Item 49. The lipid nanoparticle according to any of items 39 to 48, wherein the mRNA sequence comprises at least one coding region encoding at least one antigenic peptide or protein derived from hemagglutinin (HA) of an influenza A virus according to SEQ ID NOs: 1-14031 or a fragment or variant thereof.

Item 50. The lipid nanoparticle according to any one of items 39 to 49, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs. 32013-46043, 64025-78055, 96037-110067, 128049-142079, 160061-174091, 192073-206103 or a fragment or variant thereof.

Item 51. The lipid nanoparticle according to any one of items 39 to 50, wherein the mRNA sequence comprises at least one coding region encoding at least one antigenic peptide or protein derived from hemagglutinin (HA) of an influenza B virus according to SEQ ID NOs. 26398-28576 or a fragment or variant thereof.

Item 52. The lipid nanoparticle according to any of items 39 to 51, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs. 58410-60588, 90422-92600, 122434-124612, 154446-156624, 186458-188636, 218470-220648 or a fragment or variant thereof.

Item 53. The lipid nanoparticle according to any one of items 39 to 52, wherein the mRNA sequence comprises at least one coding region encoding at least one antigenic peptide or protein derived from neuraminidase (NA) of an influenza A virus according to SEQ ID NOs. 14032-26397 or a fragment or variant thereof.

Item 54. The lipid nanoparticle according to any one of items 39 to 53, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs. 46044-58409, 78056-90421, 110068-122433, 142080-154445, 174092-186457, 206104-218469 or a fragment or variant thereof.

Item 55. The lipid nanoparticle according to any one of items 39 to 54, wherein the mRNA sequence comprises at least one coding region encoding at least one antigenic peptide or protein derived from neuraminidase (NA) of an influenza B virus according to SEQ ID NOs. 28577-30504 or a fragment or variant thereof.

Item 56. The lipid nanoparticle according to any of items 39 to 55, wherein the mRNA sequence comprises at least one RNA sequence or a mixture of RNA sequences selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs. 60589-62516, 92601-94528, 124613-126540, 156625-158552, 188637-190564, 220649-222576 or a fragment or variant thereof.

Item 57. The lipid nanoparticle according to any of items 39 to 56, wherein the mRNA sequence comprises at least one coding region encoding at least one antigenic peptide or protein derived from glycoprotein G (RAV-G, RAVBV-G or RABV-G), nucleoprotein N (RAV-N), phospoprotein P (RAV-P), matrix protein M (RAV-M) or RNA polymerase L (RAV-L) of a Rabies virus or a fragment, variant thereof.

Item 58. The lipid nanoparticle according to any of items 39 to 57, wherein the mRNA sequence comprises at least one coding region encoding at least one antigenic peptide or protein derived from glycoprotein G (RAV-G, RAVBV-G or RABV-G) of a Rabies virus according to SEQ ID NOs. 30505-32012 or a fragment or variant thereof.

Item 59. The lipid nanoparticle according to any of items 39 to 58, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs. 62517-64024, 94529-96036, 126541-128048, 158553-160060, 190565-192072, 222577-224084 or a fragment or variant thereof.

Item 60. The lipid nanoparticle according to any one of items 1 to 59, wherein the mRNA compound comprises at least two mRNA sequences, wherein the at least two mRNA sequences encode two different antigenic peptides and/or proteins.

Item 61. The lipid nanoparticle according to any one of items 1 to 59, comprising at least two different mRNA compounds, each compound comprising a mRNA sequence which encodes at least one antigenic peptide or protein.

Item 62. The lipid nanoparticle according to any one of items 1 to 61, additionally comprising:
(iv) a neutral lipid; and/or
(v) a steroid or steroid analogue.

Item 63. The lipid nanoparticle according to item 62, wherein the neutral lipid is selected from the group comprising distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1carboxylate (DOPE-mal), dipalmitoyl phosphatidyl ethanolamine (DPPE), dimyristoylphosphoethanolamine (DMPE), distearoyl-phosphatidylethanolamine (DSPE), 16-0-monomethyl PE, 16-O-dimethyl PE, 18-1-trans PE, 1-stearioyl-2-oleoylphosphatidyethanol amine (SOPE), and 1,2-dielaidoyl-sn-glycero-3-phophoethanolamine (transDOPE).

Item 64. The lipid nanoparticle according to item 63 wherein the neutral lipid is 1,2-distearoyl-sn-glycero-3-phosphocholine (DSPC), and wherein the molar ratio of the cationic lipid to DSPC is optionally in the range from about 2 : 1 to 8 : 1.

Item 65. The lipid nanoparticle according to any one of items 62 to 64, wherein the steroid is cholesterol, and wherein the molar ratio of the cationic lipid to cholesterol is optionally in the range from about 2 : 1 to 1 : 1

Item 66. A method for the preparation of a lipid nanoparticle according to any one of items 5 to 67, comprising the steps of:
(i) providing
   a) a cationic lipid of formula (I)
      as defined above or a pharmaceutically acceptable salt, tautomer, prodrug or stereoisomer thereof, and/or
      of formula (II)
      as defined abova or a pharmaceutically acceptable salt, tautomer, prodrug or stereoisomer thereof, and/or
      of formula III:
      as defined above or a pharmaceutically acceptable salt, tautomer, prodrug or stereoisomer thereof; and/or
   b) a PEG lipid with the formula (IV): as defined above;
   c) at least one mRNA compound comprising an mRNA sequence encoding at least one antigenic peptide or protein, wherein the mRNA compound optionally does not comprise a nucleoside modification, in particular not a base modification; and
   d) optionally a steroid; and
   e) optionally a neutral lipid;
(ii) solubilizing the cationic lipid and/or the PEG lipid and optionally the neutral lipid and/or the steroid or a steroid derivative in ethanol;
(iii) mixing the ethanolic lipid solution with an aqueous solution comprising the mRNA polynucleotide
(iv) removing the ethanol; and optionally
(v) separating or purifying the lipid nanoparticles.

Item 67. The method according to item 66, wherein in step (iv) the ethanol is removed by dialysis or diafiltration.

Item 68. The method according to item 66 or 67, wherein in step (v) the lipid nanoparticles are purified by filtration, preferably by filtration through a sterile filter.

Item 69. A pharmaceutical composition comprising at least one lipid nanoparticle according to any one of items 1 to 65.

Item 70. The pharmaceutical composition according to item 69, comprising at least a first and a second lipid nanoparticle according to any one of items 1 to 65, wherein the mRNA compound comprised by the second lipid nanoparticle is different from the mRNA compound comprised by the first lipid nanoparticle.

Item 71. The pharmaceutical composition according to item 69 or 70, additionally comprising a pharmaceutically acceptable adjuvant or excipient.

Item 72. A lipid nanoparticle according to any one of items 1 to 65 or a pharmaceutical composition according to any one of items 69 to 71 for use as a medicament.

Item 73. The lipid nanoparticle or the pharmaceutical composition for use according to item 72, wherein the medicament is for therapeutically or prophylactically raising an immune response of a subject in need thereof.

Item 74. The lipid nanoparticle or the pharmaceutical composition for use according to item 72 or 73, wherein the medicament is for prevention or treatment of cancer or tumour diseases, infectious diseases, allergies, or autoimmune diseases or disorders related thereto.

Item 75. The lipid nanoparticle or the pharmaceutical composition for use according to any one of items 72 to 74, wherein the medicament is a vaccine.

Item 76. The lipid nanoparticle or the pharmaceutical composition for use according to item 75, wherein the medicament is a tumor, influenza or rabies vaccine.

Item 77. The lipid nanoparticle or the pharmaceutical composition for use according to item 76, wherein the medicament is a rabies vaccine used in rabies treatment.

Item 78. The lipid nanoparticle or the pharmaceutical composition for use according to any one of items 72 to 77, wherein the subject is a vertebrate, preferably a mammal.

Item 79. The lipid nanoparticle or the pharmaceutical composition for use according to any one of items 72 to 78, wherein the medicament is for parenteral administration and wherein parenteral administration comprises injection.

Item 80. The lipid nanoparticle or the pharmaceutical composition for use according to any one of items 78 to 79, wherein the subject is a bird, preferably chicken, or a mammal, preferably selected from the group comprising goat, cattle, swine, dog, cat, donkey, monkey, ape, a rodent such as a mouse, hamster, rabbit and, particularly, human.

Item 81. A method for raising an immune response in a subject in need thereof, comprising administering to the subject a lipid nanoparticle according to any one of items 1 to 65 or a pharmaceutical composition according to any of the items 69 to 72.

Item 82. A method for prevention or treatment of cancer or tumour diseases, infectious diseases, allergies, or autoimmune diseases or disorders related thereto in a subject in need thereof, comprising administering to the subject a lipid nanoparticle according to any one of items 1 to 65 or a pharmaceutical composition according to any of the items 69 to 72.

### Figures

The figures shown in the following are merely illustrative and shall describe the present invention in a further way. These figures shall not be construed to limit the present invention thereto.
Figure 1A-1B: show bioluminescence imaging results of mice after intraperitoneal Luciferin injection after 24h (Figure 1A) and after 48h (Figure 1B). LNP formulation of mRNA led to a significantly increased and dose-dependent protein expression after intramuscular application, compared to the same amount of unformulated mRNA.
Figures 2A-2G: show PpLuc expression measured 48h after i.m injection in organ lysates. Luciferase Relative Light Units (RLU) from individual organs were reported for each group (Figure 2A = brain, Figure 2B = heart, Figure 2C = kidney, Figure 2D = liver, Figure 2E = lung, Figure 2F = muscle, Figure 2G = spleen).
Figures 3A-3B: show HI titers 21 days after prime vaccination with three different LNP-formulated HA-mRNA (Figure 3A) and 14 days after boost vaccination (Figure 3B). The dashed line indicates the conventionally defined protective HI titer of 1:40.
Figures 4A-4D: show results of ELISA assays 21 days after prime vaccination and 14 days after boost vaccination with three different LNP-formulated HA-mRNA and 14 days after boost vaccination (IgG1 subtypes day 21 post-prime are shown in Figure 4A, IgG1 subtypes day 14 post-boost are shown in Figure 4B, IgG2a subtypes day 21 post-prime are shown in Figure 4C, IgG2a subtypes day 14 post-boost are shown in Figure 4D).
Figures 5A-5C: show T cell assay results after induction of antigen-specific T cells using intracellular cytokine staining. Figure 5A shows IFNy/TNFa producing CD4 T cells, Figure 5B shows IFNy/TNFa producing CD8 T cells and Figure 5C shows IFNy/CD107+ producing CD8 T cells.
Figures 6A-6B: show HI titers 21 days after prime vaccination with three different LNP-formulated HA-mRNA (Figure 6A) and 14 days after boost vaccination (Figure 6B). The dashed line indicates the conventionally defined protective HI titer of 1:40.
Figures 7A-7D: show results of ELISA assays 21 days after prime vaccination and 14 days after boost vaccination with three different LNP-formulated HA-mRNA and 14 days after boost vaccination (IgG1 subtypes day 21 post-prime are shown in Figure 7A, IgG1 subtypes day 14 post-boost are shown in Figure 7B, IgG2a subtypes day 21 post-prime are shown in Figure 7C, IgG2a subtypes day 14 post-boost are shown in Figure 7D).
Figures 8A-8C: show T cell assay results after induction of antigen-specific T cells using intracellular cytokine staining. Figure 8A shows IFNy/TNFa producing CD4 T cells, Figure 5B shows IFNy/TNFa producing CD8 T cells and Figure 8C shows IFNy/CD107+ producing CD8 T cells.
Figures 9A-9B: show HI titers 21 days after prime vaccination with three different LNP-formulated HA-mRNA (Figure 6A) and 14 days after boost vaccination (Figure 6B) using only 1µg LNP-formulated HA-mRNA. The dashed line indicates the conventionally defined protective HI titer of 1:40.
Figures 10A-10D: show results of ELISA assays 21 days after prime vaccination and 14 days after boost vaccination with three different LNP-formulated HA-mRNA and 14 days after boost vaccination (IgG1 subtypes day 21 post-prime are shown in Figure 10A, IgG1 subtypes day 14 post-boost are shown in Figure 10B, IgG2a subtypes day 21 post-prime are shown in Figure 10C, IgG2a subtypes day 14 post-boost are shown in Figure 10D).
Figure 11: shows T cell immune responses measured by IFNy production using Elispot.
Figure 12: shows results of an antibody titer analysis after intramuscular vaccination of NHP with LNP-III-3-formulated HA-encoding mRNA.
Figure 13: shows rabies virus neutralizing titers (VNTs) after intramuscular vaccination of NHPs with LNP-III-3-formulated RABV-G-encoding mRNA.
Figure 14: shows HI-Titer analysis after vaccination with LNP-III-3-formulated HA-mRNA.
Figures 15A-15D: shows the results of a cytokine analysis after vaccination with LNP-III-3-formulated mRNA (Figure 15A = IFNy, Figure 15B = IL-6, Figure 15C = IL-8, Figure 15D = TNF).
Figures 16A-16B: show rabies virus neutralizing titers (VNTs) after intramuscular vaccination of mice with LNP-III-3-formulated RABV-G-encoding mRNA (Figure 16A = VNT day 21; Figure 16B = VNT day 35).
Figures 17A-17C: show the results of a T cell assays of mice immunized twice with 5µg, 1µg and 0.5µg LNP-formulated mRNA on day 35 (Figure 17A shows IFNy/TNFa producing CD4 T cells, Figure 17B shows IFNy/TNFa producing CD8 T cells and Figure 17C shows IFNy/CD107+ producing CD8 T cells). Negative DMSO control is indicated by a dashed line in the graphs.
Figures 18A-18D: show the results of a liver damage analysis, i.e. determination of AST levels on day 1 and 21 (Figures 18A-18B) and ALT levels on day 1 and 21 (Figures 18C-18D).
Figures 19A-19D: show the detection of an HA-specific immune response (B-cell immune response) by detecting IgG2a antibodies directed against the particular influenza virus, i.e. Influenza A/California/7/2009 (H1N1; Figure 19A), Influenza A/Hong Kong/4801/2014 (H3N2; Figure 19B), Influenza B/Brisbane/60/2008 (B; Figure 19C) and Influenza A/Vietnam/1203/2004 (H5N1; Figure 19D).
Figure 20: shows exemplary hemagglutinin (HA) proteins of influenza A virus (see section "Preferred sequences of the present invention"; Legend: First column: Protein or Nucleic Acid Accession No. (GenBank); second column (A): Protein Sequence wild type SEQ ID NO:; third column (B): Nucleotide Sequence wild type SEQ ID NO:; fourth column (C): Optimized Nucleotide Sequence SEQ ID NO:)
Figure 21: shows exemplary hemagglutinin (HA) proteins of influenza B virus (see section "Preferred sequences of the present invention"; Legend: First column: Protein or Nucleic Acid Accession No. (GenBank); second column (A): Protein Sequence wild type SEQ ID NO:; third column (B): Nucleotide Sequence wild type SEQ ID NO:; fourth column (C): Optimized Nucleotide Sequence SEQ ID NO:)
Figure 22: shows exemplary neuraminidase (NA) proteins of influenza A virus (see section "Preferred sequences of the present invention"; Legend: First column: Protein or Nucleic Acid Accession No. (GenBank); second column (A): Protein Sequence wild type SEQ ID NO:; third column (B): Nucleotide Sequence wild type SEQ ID NO:; fourth column (C): Optimized Nucleotide Sequence SEQ ID NO:)
Fiaure 23: shows exemplary neuraminidase (NA) proteins of influenza B virus (see section "Preferred sequences of the present invention"; Legend: First column: Protein or Nucleic Acid Accession No. (GenBank); second column (A): Protein Sequence wild type SEQ ID NO:; third column (B): Nucleotide Sequence wild type SEQ ID NO:; fourth column (C): Optimized Nucleotide Sequence SEQ ID NO:)
Figure 24: shows exemplary glycoproteins of Rabies virus (see section "Preferred sequences of the present invention"; Legend: First column: Protein or Nucleic Acid Accession No. (GenBank); second column (A): Protein Sequence wild type SEQ ID NO:; third column (B): Nucleotide Sequence wild type SEQ ID NO:; fourth column (C): Optimized Nucleotide Sequence SEQ ID NO:)
Figure 25: shows the presence of total IgG1 and IgG2 antibodies specific for Influenza H1N1 (A/California/7/2009) of mice vaccinated with a combination of four mRNAs coding for different Influenza antigens (C-F) compared to controls injected with RiLa (A) or Influsplit (B). For each setting (A-F) three different time points are shown (d21, d35, and d49). Vaccination scheme, see Table A. A detailed description of the experiment is provided in Example 12.
Figure 26: shows HI titers specific for influenza H1N1 (A/California/7/2009) of mice vaccinated with a combination of four mRNAs coding for different Influenza antigens (C-F) compared to controls injected with RiLa (A) or Influsplit (B). For each setting (A-F) three different time points are shown (d21, d35, and d49). Vaccination scheme, see Table A. A detailed description of the experiment is provided in Example 12.
Figure 27: shows the presence of total IgG1 and IgG2 antibodies specific for Influenza H3N2 (A/HongKong/4801/2014) of mice vaccinated with a combination of four mRNAs coding for different Influenza antigens (C-F) compared to controls injected with RiLa (A) or Influsplit (B). For each setting (A-F) three different time points are shown (d21, d35, and d49). Vaccination scheme, see Table A. A detailed description of the experiment is provided in Example 12.
Figure 28: shows HI titers specific for influenza H3N2 (A/HongKong/4801/2014) of mice vaccinated with a combination of four mRNAs coding for different Influenza antigens (C-F) compared to controls injected with RiLa (A) or Influsplit (B). For each setting (A-F) three different time points are shown (d21, d35, and d49). Vaccination scheme, see Table A. A detailed description of the experiment is provided in Example 12.
Figure 29: shows the presence of total IgG1 and IgG2 antibodies specific for Influenza B (B/Brisbane/60/2008) of mice vaccinated with a combination of four mRNAs coding for different Influenza antigens (C-F) compared to controls injected with RiLa (A) or Influsplit (B). For each setting (A-F) three different time points are shown (d21, d35, and d49). Vaccination scheme, see Table A. A detailed description of the experiment is provided in Example 12.
Figure 30: shows the presence of total IgG1 and IgG2 antibodies specific for Influenza B (B/Phuket/3073/2013) of mice vaccinated with a combination of four mRNAs coding for different Influenza antigens (C-F) compared to controls injected with RiLa (A) or Influsplit (B). For each setting (A-F) three different time points are shown (d21, d35, and d49). Vaccination scheme, see Table A. A detailed description of the experiment is provided in Example 12.
Figure 31: shows that vaccination of mice with a combination of four mRNAs coding for different Influenza antigens (C-F) induced CD4+ T-cell responses against H1N1 (A/California/7/2009), H3N2 (A/HongKong/4801/2014), influenza B (B/Brisbane/60/2008), influenza B (B/Phuket/3073/2013) (1-4 respectively). As a control, cells were stimulated with buffer (5). As further controls, mice were injected Rila (A) or Influsplit (B). Vaccination scheme, see Table A. A detailed description of the experiment is provided in Example 12.
Figure 32: shows that vaccination of mice with a combination of four mRNAs coding for different Influenza antigens (C-F) induced CD8+ T-cell responses against H1N1 (A/California/7/2009) (1). As a control, cells were stimulated with buffer (5). As further controls, mice were injected Rila (A) or Influsplit (B). Vaccination scheme, see Table A. A detailed description of the experiment is provided in Example 12.
Figure 33: shows the presence of total IgG1 and IgG2 antibodies specific for Influenza H1N1 (A/California/7/2009) of mice vaccinated with a combination of four mRNAs coding for different Influenza antigens (C-E) compared to controls injected with RiLa (A) or Fluarix (B). For each setting (A-E), three different time points are shown (d21, d35, and d49). Vaccination scheme, see Table B. A detailed description of the experiment is provided in Example 13.
Figure 34: shows HI titers specific for influenza H1N1 (A/California/7/2009) of mice vaccinated with a combination of four mRNAs coding for different Influenza antigens (C-E) compared to controls injected with RiLa (A) or Fluarix (B). For each setting (A-E), three different time points are shown (d21, d35, and d49). Vaccination scheme, see Table B. A detailed description of the experiment is provided in Example 13.
Figure 35: shows the presence of total IgG1 and IgG2 antibodies specific for Influenza H3N2 (A/HongKong/4801/2014) of mice vaccinated with a combination of four mRNAs coding for different Influenza antigens (C-E) compared to controls injected with RiLa (A) or Fluarix (B). For each setting (A-E) three different time points are shown (d21, d35, and d49). Vaccination scheme, see Table B. A detailed description of the experiment is provided in Example 13.
Figure 36: shows HI titers specific for influenza H3N2 (A/HongKong/4801/2014) of mice vaccinated with a combination of four mRNAs coding for different Influenza antigens (C-E) compared to controls injected with RiLa (A) or Fluarix (B). For each vaccination setting (A-E) three different time points are shown (d21, d35, and d49). Vaccination scheme, see Table B. A detailed description of the experiment is provided in Example 13.
Figure 37: shows the presence of total IgG1 and IgG2 antibodies specific for Influenza B (B/Brisbane/60/2008) of mice vaccinated with a combination of four mRNAs coding for different Influenza antigens (C-E) compared to controls injected with RiLa (A) or Fluarix (B). For each setting (A-E) three different time points are shown (d21, d35, and d49). Vaccination scheme, see Table B. A detailed description of the experiment is provided in Example 13.
Figure 38: shows the presence of total IgG1 and IgG2 antibodies specific for Influenza H5N1 (A/Vietnam/1203/2004) of mice vaccinated with a combination of four mRNAs coding for different Influenza antigens (C-E) compared to controls injected with RiLa (A) or Fluarix (B). For each setting (A-E) three different time points are shown (d21, d35, and d49). Vaccination scheme, see Table B. A detailed description of the experiment is provided in Example 13.
Figure 39: shows that vaccination of mice with a combination of four mRNAs coding for different Influenza antigens (C-E) induced CD4+ T-cell responses against H1N1 (A/California/7/2009), H3N2 (A/HongKong/4801/2014), influenza B (B/Brisbane/60/2008), H5N1 (A/Vietnam/1203/2004) (1-4 respectively). As a control, cells were stimulated with buffer (5). As further controls, mice were injected Rila (A) or Influsplit (B). Vaccination scheme, see Table B. A detailed description of the experiment is provided in Example 13.
Figure 40: shows that vaccination of mice with a combination of four mRNAs coding for different Influenza antigens (C-F) induced CD8+ T-cell responses against H1N1 (A/California/7/2009), H5N1 (A/Vietnam/1203/2004) (1 and 2). As a control, cells were stimulated with buffer (3). As further controls, mice were injected Rila (A) or Influsplit (B). Vaccination scheme, see Table B. A detailed description of the experiment is provided in Example 13.
Figure 41: shows the presence of binding influenza N1 (A/California/7/2009) neuraminidase specific antibodies in and ELLA titers (50% inhibition titers) in serum samples of mice that were vaccinated with LNP formulated mRNA coding for Influenza N1 (A/California/7/2009) neuraminidase (C and D) compared to a control injected with RiLa (A) or Influsplit^{®} (B). Vaccination scheme, see Table C. A detailed description of the experiment is provided in Example 14.
Figure 42: shows that vaccination of mice with mRNAs coding for Influenza N1 (A/California/7/2009) neuraminidase induced CD4+ T-cell responses against neuraminidase (C and D). As controls, mice were injected with RiLa (A) or Influsplit^{®} (B). Vaccination scheme, see Table C. A detailed description of the experiment is provided in Example 14.
Figure 43: shows that vaccination of mice with mRNAs coding for Influenza N1 (A/California/7/2009) neuraminidase induced CD8+ T-cell responses against Neuraminidase (C and D). As controls, mice were injected with and RiLa (A) or Influsplit^{®} (B). Vaccination scheme, see Table C. A detailed description of the experiment is provided in Example 14.
Figure 44: shows that LNP formulated mRNAs coding for RAVBV-G induces a pro-inflammatory environment. Cytokine levels in muscle for four different timepoints (4h, 14h, 24h, and 96h) are shown (TNF, IL6). Arrow indicates the data series for mice vaccinated with LNP-formulated mRNA. Values represent mean of 6 samples with SD. A detailed description of the experiment is provided in Example 15.
Figure 45: shows that LNP formulated mRNAs coding for RAVBV-G induces a pro-inflammatory environment. Cytokine levels in dLNs for four different timepoints (4h, 14h, 24h, and 96h) are shown (TNF, IL6). Arrow indicates the data series for mice vaccinated with LNP-formulated mRNA. Values represent mean of 6 samples with SD. A detailed description of the experiment is provided in Example 15.
Figure 46: shows no systemic release of TNF following i.m. vaccination with LNP formulated mRNAs coding for RAVBV-G. Cytokine levels in serum for four different timepoints (4h, 14h, 24h, and 96h) are shown (TNF, IL6). Arrow indicates the data series for mice vaccinated with LNP-formulated mRNA. Values represent mean of 6 samples with SD. A detailed description of the experiment is provided in Example 15.
Figure 47: shows that LNP formulated mRNAs coding for RAVBV-G induces a pro-inflammatory environment. Chemokine levels in muscle for four different timepoints (4h, 14h, 24h, and 96h) are shown (MIP-1beta, CXCL9). Arrow indicates the data series for mice vaccinated with LNP-formulated mRNA. Values represent mean of 6 samples with SD. A detailed description of the experiment is provided in Example 15.
Figure 48: shows that LNP formulated mRNAs coding for RAVBV-G induces a pro-inflammatory environment. Chemokine levels in dLNs for four different timepoints (4h, 14h, 24h, and 96h) are shown (MIP-1beta, CXCL9). Arrow indicates the data series for mice vaccinated with LNP-formulated mRNA. Values represent mean of 6 samples with SD. A detailed description of the experiment is provided in Example 15.
Figure 49: shows that LNP formulated mRNAs coding for RAVBV-G induces a pro-inflammatory environment. Chemokine levels in serum for four different timepoints (4h, 14h, 24h, and 96h) are shown (MIP-1beta, CXCL9). Arrow indicates the data series for mice vaccinated with LNP-formulated mRNA. Values represent mean of 6 samples with SD. A detailed description of the experiment is provided in Example 15.
Figure 50A-50B: shows that LNP formulated F*mRNAs induces an increase in number and activation of both innate and adaptive immune cells within the dLNs. In Figure 50A, number of CD4+ T cells, NK cells, CD11b+ Gr1+ cells (monocytes and granolocytes) and total cells in dLNs for three different timepoints (4h, 24h, and 48h) are shown. In Figure 50B frequencies of CD4+ T cells, CD8+ T cells, B cells and NK cells expressing the activation marker CD69 for three different timepoints (4h, 24h, and 48h) are shown. Arrow indicates the data series for mice injected with LNP-formulated mRNA. Values represent mean of 6 samples with SD. A detailed description of the experiment is provided in Example 15.
Figure 51: shows that vaccination of monkeys with LNP formulated mRNAs coding for RABV-G induces rabies virus neutralizing titers (VNTs) after single i.m. vaccination. Two mRNA concentrations are shown (1µg and 10µg) before vaccination ("pre dose") and after the first vaccination ("post prime"). Dashed lines indicate the conventionally defined protective titers for VNTs. A detailed description of the experiment is provided in Example 16.
Figure 52: shows the kinetic of VNTs after vaccination (prime vaccination at day 0, boost vaccination at day 28) of monkeys with LNP formulated mRNAs coding for RABV-G. Dashed lines indicate the conventionally defined protective titers for VNTs. A detailed description of the experiment is provided in Example 16.
Figure 53: Two mRNA concentrations are shown (1µg and 10µg) before boost ("pre recall") and after the boost vaccination ("post recall"). Dashed lines indicate the conventionally defined protective titers for VNTs. A detailed description of the experiment is provided in Example 16.
Figure 54: LNP formulated RAVBV-G mRNA vaccines induce stronger functional immune responses against rabies than a licensed vaccine in NHPs. Rabies VNTs in the sera of NHPs (*n* = 2 male, 2 female per group) vaccinated with LNP formulated RABV-G mRNA at days 0 and 28, or with the inactivated rabies virus vaccine Rabipur^{®} at days 0 and 28 (one boost). Dashed lines indicate the conventionally defined protective titers for VNTs. A detailed description of the experiment is provided in Example 16.
Figure 55: LNP formulated RAVBV-G mRNA vaccines induce RABV-G specific CD4+ T cells in monkeys. Shown are frequencies of RABV-G-specific IFNγ⁺/IL-2⁺ CD4⁺ cells in the blood 7 days after the last vaccination (day 35). PBMCs were either stimulated with an overlapping peptide library covering the RABV-G protein (RABV-G peptides) or unstimulated (media) and analyzed by ICS. A detailed description of the experiment is provided in Example 16.
Figure 56: LNP formulated RAVBV-G mRNA vaccines induce RABV-G specific CD8+ T cells in monkeys. Shown are frequencies of RABV-G-specific IFNy+/GrzB+ CD8 T cells in the blood 7 days after the last vaccination (day 35). PBMCs were either stimulated with an overlapping peptide library covering the RABV-G protein (RABV-G peptides) or unstimulated (media) and analyzed by ICS. A detailed description of the experiment is provided in Example 16.
Figure 57: shows that vaccination of monkeys with LNP formulated mRNAs coding for influenza HA antigens of the H1N1 or H3N2 strains induces functional antibodies after single i.m. vaccination. HI titers before vaccination ("pre dose") and after the first vaccination ("post prime") are shown. Dashed lines indicate the conventionally defined protective titers for HI. A detailed description of the experiment is provided in Example 17.
Figure 58: shows the kinetic of HI titers (mean with SEM) after vaccination (prime vaccination at day 0, boost vaccination at day 28) of monkeys with LNP formulated mRNAs coding for H1N1 HA. Timecourse of HI titers is shown for up to 544 days. Dashed lines indicate the conventionally defined protective titers for HI. Triangles: Datapoints for monkeys vaccinated with 10µg; Rectangles: Datapoints for monkeys vaccinated with 1µg. A detailed description of the experiment is provided in Example 17.
Figure 59: LNP formulated H3N2-HA mRNA vaccine induced stronger functional immune responses against influenza than a licensed vaccine in NHPs. Shown are H3N2-HI titers in sera of NHPs vaccinated at days 0 and 28 with LNP formulated H3N2-HA mRNA or the adjuvanted vaccine Fluad^{®}. Dashed lines indicate the conventionally defined protective titers for HI. A detailed description of the experiment is provided in Example 17.
Figure 60: LNP formulated H3N2-HA mRNA vaccines induce H3N2 specific CD4+ T cells in monkeys. PBMCs were either stimulated with an overlapping peptide library covering the H3N2-HA protein (H3N2-HA peptides) or unstimulated (media) and analyzed by ICS. Figure 60A: Shown are frequencies of H3N2-HA-specific IFNy+/IL-2+ CD4+ cells in the blood 7 days after the last vaccination (day 35). Figure 60B: Shown are frequencies of H3N2-HA-specific TNFa+/IL-2+ CD4+ cells in the blood 7 days after the last vaccination (day 35). A detailed description of the experiment is provided in Example 17.
Figures 61A-61B: show rabies virus neutralizing titers (VNTs) after intramuscular vaccination of mice with LNP-III-3-formulated RABV-G-encoding mRNA (Figure 60A = VNT day 21; Figure 60B = VNT day 35). A detailed description of the experiment is provided in Example 7.
Figures 62A-62B: show rabies virus neutralizing titers (VNTs) after intramuscular vaccination of mice with LNP-III-3-formulated RABV-G-encoding mRNA (Figure 60A = VNT day 21; Figure 60B = VNT day 35) applying LNPs which were stored at 5°C for three month. A detailed description of the experiment is provided in Example 20.
Figures 63A-63D: shows that vaccination of ferrets with LNP formulated tetravalent mRNA vaccine induces functional antibodies. Data shown for indicated groups (group A-D), measured on day 0, day 21, day 35 and day 49 respectively. Figures 63A: HI titers for HA A/California/07/09; Figures 63A: HI titers for HA A/HongKong/4801/2014; Figure 63C: HI titers for HA B/Brisbane/60/2008; Figure 63D: MN titers for HA B/Phuket/3073/2013. Positive control (Group D, Fluad) is not shown as Fluad does not contain HA B/Phuket. A detailed description of the experiment is provided in Example 22.
Figure 64: shows that vaccination of mice with LNP formulated trivalent NA mRNA vaccine induces binding influenza NA antibodies. ELLA (50% inhibition titer) are shown. Figure 64A shows ELLA titers for influenza N1 A/California/7/2009 for indicated groups; Figure 64B shows shows ELLA titers for influenza N1 A/HongKong/4801/2014 for indicated groups; Figure 64C shows ELLA titers for NA B/Brisbane/60/2008 for indicated groups. 1=mRNA vaccine, 2=control; 3=buffer control. A detailed description of the experiment is provided in Example 24.
Figure 65: shows the presence of total IgG1 and IgG2a antibodies specific for HA of H1N1 A/California/7/2009 of mice vaccinated with LNP formulated septavalent HA/NA mRNA vaccine. For each setting (group 1-6) three different time points are shown (d21, d35, and d49) (ELISA). A detailed description of the experiment is provided in Example 25.
Figure 66: shows the presence of total IgG1 and IgG2a antibodies specific for HA of H3N2 A/Hong Kong/4801/2014 of mice vaccinated with LNP formulated septavalent HA/NA mRNA vaccine. For each setting (group 1-6) three different time points are shown (d21, d35, and d49) (ELISA). A detailed description of the experiment is provided in Example 25.
Figure 67: shows the presence of total IgG1 and IgG2a antibodies specific for HA of B/Brisbane/60/2008 of mice vaccinated with septavalent HA/NA mRNA vaccine (ELISA). For each setting (group 1-6) three different time points are shown (d21, d35, and d49). A detailed description of the experiment is provided in Example 25.
Figure 68: shows the presence of total IgG1 and IgG2a antibodies for HA of B/Phuket/3073/2013 of mice vaccinated with LNP formulated septavalent HA/NA mRNA vaccine (ELISA). For each setting (group 1- group 6) three different time points are shown (d21, d35, and d49). A detailed description of the experiment is provided in Example 25.
Figure 69: shows the presence of specific antibodies for NA of H1N1A/California/7/2009 of mice vaccinated with LNP formulated septavalent HA/NA mRNA vaccine (ELISA). For each setting (group 1- group 6) three different time points are shown (d21, d35, and d49). Figure 69A: specific antibodies for NA of H1N1A/California/7/2009; Figure 69B: specific antibodies for NA of H3N2 A/Hong Kong/4801/2014; Figure 69C: specific antibodies for NA of B/Brisbane/60/2008. A detailed description of the experiment is provided in Example 25.
Figure 70: shows that vaccination of mice with LNP formulated septavalent HA/NA mRNA vaccine induces functional antibodies. Data shown for indicated groups (group 1- group 6), measured on day 49. Figures 63A: HI titers for HA A/California/07/09; Figures 63A: HI titers for HA A/HongKong/4801/2014. A detailed description of the experiment is provided in Example 25.
Figure 71: shows that LNP formulated GP (Ebola) mRNA vaccine induce strong IgG1 and IgG2a antibody responses in mice. A detailed description of the experiment is provided in Example 26.
Figure 72: shows that LNP formulated mRNA vaccine induce strong and durable HI-titers when administered subcutaneously. A detailed description of the experiment is provided in Example 27.
Figure 73: shows a comparison of Ova-specific CD8 positive T cells in the blood on day 7 after vaccination with 1µg LNP formulated Ova mRNA (Component A) and 32µg protamine formulated Ova mRNA (Component B). 1µg LNP formulated Ova mRNA (Component A) induces higher levels of circulating antigen-specific CD8 positive T cells after intradermal application. A detailed description of the experiment is provided in Example 28.
Figure 74: shows a comparison of Ova-specific CD8 positive T cells in the blood on day 21 after boosting vaccinated animals with 1µg of LNP-formulated OVA mRNA (Component A) and 32µg protamine formulated Ova mRNA (Component B). 1µg of LNP-formulated OVA mRNA (Component A) induces boostable levels of circulating antigen-specific CD8 positive T cells after intradermal application. A detailed description of the experiment is provided in Example 28.
Figure 75: shows a comparison of multifunctional Ova-specific CD8 positive T cells in the blood after vaccination with 1µg LNP formulated Ova mRNA (Component A) and 32µg protamine formulated Ova mRNA (Component B). 1µg LNP formulated Ova mRNA vaccine (Component A) induces high levels of multifunctional CD8 positive T cells after intradermal application. A detailed description of the experiment is provided in Example 28.
Figure 76: shows a comparison of OVA-specific IgG2c titers after vaccination with 1µg LNP formulated OvamRNA (Component A) and 32µg protamine formulated Ova-mRNA (Component B). 1µg LNP formulated OvamRNA vaccine (Component A) leads to increased OVA-specific IgG2c titers after intradermal application. A detailed description of the experiment is provided in Example 28.
Figure 77: shows a comparison of median tumor growth of tumor-bearing mice after vaccination with 1µg LNP formulated Ova mRNA (Component A) and an irrelevant LNP formulated PpLuc mRNA (Component B). 1µg LNP formulated Ova mRNA (Component A) strongly decreased the median tumor volume compared to the other treatment with an irrelevant mRNA (Component B). A detailed description of the experiment is provided in Example 29.
Figure 78: shows a comparison of the overall survival of tumor challenged mice after vaccination with 1µg LNP formulated Ova mRNA (Component A) and an irrelevant LNP formulated PpLuc mRNA (Component B). 1µg LNP formulated Ova mRNA (Component A) strongly increased the survival of tumor challenged mice compared to the other treatments (Component B and Buffer). A detailed description of the experiment is provided in Example 29.
Figure 79: shows a comparison of median tumor growth of tumor-bearing mice after vaccination with 1µg LNP formulated Trp2 mRNA (Component A) and an irrelevant LNP formulated PpLuc mRNA (Component B) in combination with two checkpoint inhibitors anti-PD1 and anti-CTLA4. 1µg LNP formulated Trp2 mRNA (Component A) strongly decreased the median tumor volume compared to the other treatment with an irrelevant mRNA (Component B) in combination with checkpoint inhibitors or a control antibody. A detailed description of the experiment is provided in Example 30.
Figure 80: shows a comparison of the survival of tumor challenged mice after vaccination with 1µg LNP formulated Trp2 mRNA (Component A) and an irrelevant LNP formulated PpLuc mRNA (Component B) in combination with two checkpoint inhibitors anti-PD1 and anti-CTLA4. 1µg LNP formulated Trp2 mRNA (Component A) increased the survival of tumor challenged mice compared to the other treatments (Component B in combination with checkpoint inhibitors or a control antibody). A detailed description of the experiment is provided in Example 30.

### Examples

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the present invention and practice the claimed methods. The following working examples therefore, specifically point out the preferred embodiments of the present invention, and are not to be construed as limiting in any way the remainder of the disclosure.

### Example 1: Preparation of mRNA Constructs

For the present examples, DNA sequences encoding different proteins were prepared and used for subsequent RNA in vitro transcription reactions. The DNA sequences encoding the proteins were prepared by modifying the wild type encoding DNA sequence by introducing a GC-optimized sequence for stabilization. Sequences were introduced into a derived pUC19 vector. For further stabilization and/or increased translation UTR elements were introduced 5'- and/or 3' of the coding region.

The following mRNA constructs were used in the examples:
Photinus pyralis luciferase:
   - 5'-TOP-UTR derived from 32L4 ribosomal protein - GC-enriched coding sequence encoding PpLuc - 3'-UTR derived from albumin gene - a stretch of 64 adenosines - a stretch of 30 cytosines - a histone stem-loop sequence (SEQ ID NO: 224286).
Influenza hemagglutinin (HA):
   - 5'-TOP-UTR derived from 32L4 ribosomal protein - GC-enriched coding sequence encoding HA of Influenza A/California/07/2009 (H1N1) - 3'-UTR derived from albumin gene - a stretch of 64 adenosines - a stretch of 30 cytosines - a histone stem-loop sequence (SEQ ID NO: 224118)
   - GC-enriched coding sequence encoding HA of Influenza A/California/07/2009 (H1N1) - 3'-UTR derived from human alpha globin (muag) - a stretch of 64 adenosines - a stretch of 30 cytosines - a histone stem-loop sequence (SEQ ID NO: 224117)
   - GC-enriched coding sequence encoding HA of Influenza A/Hong Kong/4801/2014 (H3N2) - 3'-UTR derived from human alpha globin (muag) - a stretch of 64 adenosines - a stretch of 30 cytosines - a histone stem-loop sequence (SEQ ID NO: 224181)
   - GC-enriched coding sequence encoding HA of Influenza B/Brisbane/60/2008 - 3'-UTR derived from human alpha globin (muag) - a stretch of 64 adenosines - a stretch of 30 cytosines - a histone stem-loop sequence (SEQ ID NO: 224236).
   - GC-enriched coding sequence encoding HA of Influenza A/Vietnam/1203/2004 (H5N1) - 3'-UTR derived from human alpha globin (muag) - a stretch of 64 adenosines - a stretch of 30 cytosines - a histone stem-loop sequence (SEQ ID NO: 224200).
   - GC-enriched coding sequence encoding HA of Influenza A/Netherlands/602/2009 (H1N1) - 3'-UTR derived from human alpha globin (muag) - a stretch of 64 adenosines - a stretch of 30 cytosines - a histone stem-loop sequence (SEQ ID NO: 224166).
   - GC-enriched coding sequence encoding HA of Influenza B/Brisbane/60/2008 - 3'-UTR derived from human alpha globin (muag) - a stretch of 64 adenosines - a stretch of 30 cytosines - a histone stem-loop sequence (SEQ ID NO: 224236).
   - GC-enriched coding sequence encoding HA of Influenza B/Phuket/3073/2013 - 3'-UTR derived from human alpha globin (muag) - a stretch of 64 adenosines - a stretch of 30 cytosines - a histone stem-loop sequence (SEQ ID NO: 224246).
Influenza neuraminidase (NA):
   - GC-enriched coding sequence encoding NA of Influenza A/California/07/2009 (H1N1) - 3'-UTR derived from human alpha globin (muag) - a stretch of 64 adenosines - a stretch of 30 cytosines - a histone stem-loop sequence (SEQ ID NO: 224318).
   - GC-enriched coding sequence encoding NA of Influenza A/Hong Kong/4801/2014 (H3N2) - 3'-UTR derived from human alpha globin (muag) - a stretch of 64 adenosines - a stretch of 30 cytosines - a histone stem-loop sequence (SEQ ID NO: 224336).
   - GC-enriched coding sequence encoding NA of Influenza B/Brisbane/60/2008 - 3'-UTR derived from human alpha globin (muag) - a stretch of 64 adenosines - a stretch of 30 cytosines - a histone stem-loop sequence (SEQ ID NO: 224348).
Rabies:
   - RABV-G A: GC-enriched coding sequence encoding glycoprotein (RABV-G) of the Pasteur strain (GenBank accession number: AAA47218.1) - 3'-UTR derived from human alpha globin (muag) - a stretch of 64 adenosines - a stretch of 30 cytosines - a histone stem-loop sequence (SEQ ID NO: 224276)
   - RABV-G B: 5'-TOP-UTR derived from 32L4 ribosomal protein - GC-enriched coding sequence encoding glycoprotein (RABV-G) of the Pasteur strain (GenBank accession number: AAA47218.1) - 3'-UTR derived from albumin gene - a stretch of 64 adenosines - a stretch of 30 cytosines - a histone stem-loop sequence (SEQ ID NO: 224280).
Ebola:
   - GP of Ebola virus: GC-enriched coding sequence encoding glycoprotein of ZEBOV Sierra Leone 2014; 5'-TOP-UTR derived from 32L4 ribosomal protein - 3'-UTR derived from albumin gene (SEQ ID NO: 224362).

The obtained plasmid DNA constructs were transformed and propagated in bacteria (Escherichia coli) using common protocols known in the art. Subsequently, the DNA plasmids are enzymatically linearized using EcoRI and transcribed in vitro using DNA dependent T7 RNA polymerase in vitro run-off transcription in the presence of a nucleotide mixture and CAP analog (m7GpppG) under suitable buffer conditions. The obtained mRNAs were purified using PureMessenger^{®} (CureVac, Tübingen, Germany; WO2008/077592 A1) and were used for further experimentation. NHP were administered with RABV-G A as protamine formulation and RABV-G B as LNP formulation.Compositions comprising more than one mRNA encoding different Influenza proteins/antigens may also be produced according to procedures as disclosed in the PCT application PCT/EP2016/082487.

### Example LNP Formulation

Lipid nanoparticles, cationic lipids and polymer conjugated lipids (PEG-lipid) were prepared and tested according to the general procedures described in PCT Pub. Nos. WO 2015/199952, WO 2017/004143 and WO 2017/075531, the full disclosures of which are incorporated herein by reference. Lipid nanoparticle (LNP)-formulated mRNA was prepared using an ionizable amino lipid (cationic lipid), phospholipid, cholesterol and a PEGylated lipid. LNPs were prepared as follows. Cationic lipid, DSPC, cholesterol and PEG-lipid were solubilized in ethanol at a molar ratio of approximately 50:10:38.5:1.5 or 47.5:10:40.8:1.7. LNPs for the Examples included, for example, cationic lipid compound III-3 and the foregoing components. Lipid nanoparticles (LNP) comprising compound III-3 were prepared at a ratio of mRNA to Total Lipid of 0.03-0.04 w/w. Briefly, the mRNA was diluted to 0.05 to 0.2mg/mL in 10 to 50mM citrate buffer, pH 4. Syringe pumps were used to mix the ethanolic lipid solution with the mRNA aqueous solution at a ratio of about 1:5 to 1:3 (vol/vol) with total flow rates above 15ml/min. The ethanol was then removed and the external buffer replaced with PBS by dialysis. Finally, the lipid nanoparticles were filtered through a 0.2µm pore sterile filter. Lipid nanoparticle particle diameter size was 60-90 nm as determined by quasi-elastic light scattering using a Malvern Zetasizer Nano (Malvern, UK). For other cationic lipid compounds mentioned in the present specification, the formulation process is similar.

### Example 2: Ppluc expression after i.m. application of LNP-formulated mRNA

Expression of luciferase (Ppluc) in BALB/c mice was determined 24h and 48h after intramuscular injection (i.m.) into the M. tibialis.

Therefore, 0.1µg, 1µg and 10µg mRNA coding for Ppluc were LNP-formulated to yield the respective LNP-formulation according to Table I. As a control served unformulated Ppluc mRNA (10µg and 1µg). At time point 0h, four mice per group were transfected with Ppluc mRNA in accordance with the scheme shown in table I.

**Table I (Example 2): Transfection scheme**

| **Group** | **Treatment** | **mRNA dose [µg]** | **Route (Volume)** | **Mice #** |
|---|---|---|---|---|
| A | LNP-II-9-formulated Ppluc mRNA | 10 | i.m. (25µl) | 4 |
| B | LNP-II-9-formulated Ppluc mRNA | 1 | i.m. (25µl) | 4 |
| C | LNP-II-9-formulated Ppluc mRNA | 0.1 | i.m. (25µl) | 4 |
| D | LNP-II-10-formulated Ppluc mRNA | 10 | i.m. (25µl) | 4 |
| E | LNP-II-10-formulated Ppluc mRNA | 1 | i.m. (25µl) | 4 |
| F | LNP-II-10-formulated Ppluc mRNA | 0.1 | i.m. (25µl) | 4 |
| G | LNP-III-3-formulated Ppluc mRNA | 10 | i.m. (25µl) | 4 |
| H | LNP-III-3-formulated Ppluc mRNA | 1 | i.m. (25µl) | 4 |
| I | LNP-III-3-formulated Ppluc mRNA | 0.1 | i.m. (25µl) | 4 |
| J | unformulated Ppluc mRNA | 10 | i.m. (25µl) | 4 |
| K | unformulated Ppluc mRNA | 1 | i.m. (25µl) | 5 |

After 24h and 48h, in vivo bioluminescence imaging of mice was performed with an IVIS Lumina II Imaging System 10 minutes after intraperitoneal Luciferin injection, using an exposure time of 60s. Bioluminescence values were quantified by measuring photon flux (photons/second) in the region of interest. After 48 hours, mice were sacrificed and muscle, lung, liver, spleen, brain, kidney and heart were collected, shock frozen in dry ice and stored at -80°C for further analysis. Organ samples were lysed for 3 Minutes at full speed in a tissue lyser (Qiagen, Hilden, Germany). Afterwards 800µl of lysis buffer were added and the resulting solutions were subjected another 6 minutes at full speed in the tissue lyser. After 10 minutes centrifugation at 13500 rpm at 4°C the supernatants were mixed with luciferin buffer (25mM glycylglycin, 15mM MgSO4, 5mM ATP, 62.5µM luciferin) and luminescence was detected using a Chameleon plate reader (Hidex).

### Results:

As can be seen in Figure 1A and Figure 1B, LNP formulation of mRNA led to a significantly increased protein expression after intramuscular application, compared to the same amount of unformulated mRNA after 24h (Figure 1A) and 48h(Figure 1B). A dose-dependent expression of Ppluc was observed.

As shown in Figures 2A-2G, PpLuc expression was measured 48h after i.m injection in organ lysates. Luciferase Relative Light Units (RLU) from individual organs were reported for each group. As expected, strong luciferase expression was observed at the injection site. Notably, only at the highest dose of 10µg LNP-formulated mRNA, luciferase expression was detected in the spleen and to a minor degree in liver, kidney and lung (Figure 2A = brain, Figure 2B = heart, Figure 2C = kidney, Figure 2D = liver, Figure 2E = lung, Figure 2F = muscle, Figure 2G = spleen).

### Example 3: Immunogenicity after intramuscular (i.m.) application of LNP-formulated mRNA

LNP formulated HA-mRNA was used for testing the immunogenicity after intramuscular (i.m.) application.

Specifically, a GC-enriched H1N1 (Netherlands 2009)-HA mRNA sequence as LNP formulation was applied as described above.

For vaccination, 8 BALB/c mice were intramuscularly injected into the M. tibialis of both legs (25µl per leg) according to the vaccination scheme shown in Table II. As apparent, 10µg mRNA encoding Influenza HA was LNP-formulated (as described above) to yield the respective LNP-formulation for vaccination; unformulated mRNA (10µg) served as a control.

**Table II (Example 3): Vaccination scheme**

| **Group** | **Treatment** | **RNA dose** | **Route (Volume)** | **Mice #** |
|---|---|---|---|---|
| A | LNP-II-9-formulated HA mRNA | 10µg | i.m. 25µl per leg | 8 |
| B | LNP-II-10-formulated HA mRNA | 10µg | i.m. 25µl per leg | 8 |
| C | LNP-III-3-formulated HA mRNA | 10µg | i.m. 25µl per leg | 8 |
| D | unformulated HA mRNA | 10µg | i.m. 25µl per leg | 8 |
| E | RiLa buffer | - | i.m. 25µl per leg | 8 |

On day 0, a prime vaccination was administered. Animals vaccinated with buffer served as negative control. On day 21, a blood sample was collected from the retrobulbar sinus and a boost vaccination was administered. After 35 days, the mice were sacrificed and blood and organ samples (spleen) were collected for further analysis. Splenocytes were isolated at day 35 and stimulated with an HA peptide library for T cell analysis.

For immunogenicity assays, Hemagglutination inhibition (HI) titers were analyzed in the sera 3 weeks after prime and 2 weeks after boost. Frequencies of activated, HA-specific, multifunctional CD4+ and CD8+ T cells (IFN-y+/TNF+) were measured by intracellular staining and flow cytometry. ELISA was applied for determining antibody titers.

### Hemagglutination inhibition assay:

Hemagglutination inhibition (HI) assays were used for analyzing functional anti-HA antibody titers. Mouse sera were heat inactivated (56°C, 30min), incubated with kaolin (Carl Roth, Germany) and pre-adsorbed to chicken red blood cells (CRBC; Lohmann Tierzucht, Germany). 50µl of 2-fold dilutions of pre-treated sera were incubated for 45min with 4 hemagglutination units (HAU) of inactivated Influenza A/California/7/2009 (H1N1) virus (NIBSC, UK) and 50µl 0.5% CRBC was added. HI titers were determined by the reciprocal of the highest dilution of the serum able to inhibit hemagglutination.

### ELISA:

Detection of an antigen-specific immune response (B-cell immune response) was carried out by detecting influenza specific IgG1 and IgG2a antibodies. Therefore, blood samples were taken from the vaccinated mice 21 days post prime and 14 days post boost and sera were prepared. MaxiSorb plates (Nalgene Nunc International) were coated with the inactivated virus. After blocking with 1xPBS containing 0.05% Tween-20 and 1% BSA the plates were incubated with diluted mouse serum (as indicated). Subsequently a biotin-coupled secondary antibody (anti-mouse-IgG1 and IgG2a, Pharmingen) was added. After washing, the plate was incubated with horseradish peroxidase-streptavidin and subsequently the conversion of the ABTS substrate (2,2'-azino-bis(3-ethyl-benzthiazoline-6-sulfonic acid) was measured.

### Intracellular cytokine staining:

Induction of antigen-specific T cells was determined 14 days after boost using intracellular cytokine staining (ICS). Splenocytes from vaccinated and control mice were isolated and stimulated with an HA peptide library (PepMix^{™} Influenza A (HA /California (H1N1)), JPT) and anti-CD28 antibody (BD Biosciences) for 6 hours at 37°C in the presence of the GolgiPlug containing protein transport inhibitor Brefeldin A (BD Biosciences). After stimulation, cells were washed and stained for intracellular cytokines using the Cytofix/Cytoperm reagent (BD Biosciences) according to the manufacturer's instructions. The following antibodies were used for staining: CD8-APC H7 (1:100), CD4-BD Horizon V450 (1:200) (BD Biosciences), Thy1.2-FITC (1:300), TNFa-PE (1:100), IFN-γ-APC (1:100) (eBioscience), and incubated with FcyR-block diluted 1:100. Aqua Dye was used to distinguish live/dead cells (Invitrogen). Cells were acquired using a Canto II flow cytometer (BD Biosciences) and flow cytometry data were analyzed using FlowJo software (Tree Star).

### Results:

### Results Hemagglutination inhibition (HI) assays:

LNP-formulated HA-mRNA led to very high HI titers already after prime vaccination as apparent from Figure 3A, i.e. all 3 different LNPs comprising HA mRNA at 10µg dose induced HI titers above the threshold of 1:40 21 days after prime vaccination. In contrast unformulated mRNA did not reach the threshold of 1:40. HI titers even further increased after boost vaccination as apparent from Figure 3B (14 days after boost vaccination). The dashed line indicates the conventionally defined protective HI titer of 1:40.

### Results ELISA assays:

LNP-formulated HA-mRNA induced significantly higher functional antibody titers, both IgG1 and IgG2a subtypes, when compared to unformulated mRNA already after a single i.m. injection as apparent from Figures 4A-4D 21 days after prime vaccination and 14 days after boost vaccination.

### Results T cell assays:

As apparent from Figures 5A-5C, it was observed that LNP-formulated HA-mRNA significantly induced higher levels of antigen-specific multifunctional CD4+ T cells and CD8+ T cells compared to unformulated mRNA. The medium control as expected never generated a response.

### Example 4: Immunogenicity after intradermal (i.d.) application of LNP-formulated mRNA

LNP formulated HA-mRNA was used for testing the immunogenicity after intradermal (i.d.) application. Specifically, a LNP-formulated GC-enriched H1N1 (Netherlands 2009)-HA mRNA sequence was applied as described above.

Therefore, 8 mice per group were each vaccinated intradermally according to the vaccination scheme shown in Table III. As apparent, 10µg HA-mRNA was LNP-formulated (as described above) to yield the respective LNP-formulation for the vaccination; unformulated HA mRNA (10µg) served as a control.

**Table III (Example 4): Vaccination scheme**

| **Group** | **Treatment** | **RNA dose** | **Route (Volume)** | **Mice #** |
|---|---|---|---|---|
| A | LNP-II-9-formulated HA mRNA | 10µg | i.d. (25µl) | 8 |
| B | LNP-II-10-formulated HA-mRNA | 10µg | i.d. (25µl) | 8 |
| C | LNP-III-3-formulated HA-mRNA | 10µg | i.d. (25pl) | 8 |
| D | unformulated HA-mRNA | 10µg | i.d. (25µl) | 8 |
| E | RiLa buffer | - | i.d. (25µl) | 8 |

At time point 0 days, a prime vaccination was administered to 8 mice per group. On day 21, a blood sample was collected and a boost vaccination was administered. After 35 days, the mice were sacrificed and blood and organ samples (spleen) were collected for further analysis.

For immunogenicity assays, the HI titer was measured. ELISA was applied for determining antibody titers. Further, CD4 T cell immune response (IFNy/TNFa producing CD4 T cells) and CD8 T cell immune response (IFNy/TNFa producing CD8 T cells and CD107+ IFNy producing CD8 T cells) was assessed 14 days after boost vaccination. Induction of antigen-specific T cells was determined using intracellular cytokine staining (ICS). Assays were performed as described above.

### Results:

### Results Hemagglutination inhibition (HI) assays:

LNP-formulated HA-mRNA led to very high HI titers already after prime vaccination compared to unformulated mRNA as apparent from Figure 6A. HI titers even further increased after boost vaccination as apparent from Figure 6B.

### Results ELISA assays:

LNP-formulated HA-mRNA induced significantly higher functional antibody titers, both IgG1 and IgG2a subtypes, when compared to unformulated mRNA already after a single i.d. injection as apparent from Figures 7A-7D.

### Results T cell assays:

As apparent from Figures 8A-8C, it was observed that LNP-formulated HA-mRNA significantly induced higher levels of antigen-specific multifunctional CD4+ T and CD8+ T cells compared to unformulated mRNA. The medium control as expected never generated a response.

### Example 5: Immunogenicity after intramuscular (i.m.) application of 1µg LNP-formulated mRNA

In the following example, the amount of 1µg HA-mRNA (H1N1 (Netherlands 2009)-HA mRNA sequence LNP formulation, was used for testing the immunogenicity after intramuscular (i.m.) application as described above. Therefore, 8 BALB/c mice per group were each vaccinated intramuscularly in one leg according to the vaccination scheme shown in Table IV. As apparent, 1µg HA-mRNA was LNP-formulated (as described above) to yield the respective LNP-formulation for the vaccination; unformulated HA mRNA (10µg) served as a control.

**Table IV (Example 5): Vaccination scheme**

| **Group** | **Treatment** | **RNA dose** | **Route (Volume)** | **Mice #** |
|---|---|---|---|---|
| A | LNP-III-3-formulated HA-mRNA | 1µg | i.m. (25µl) | 8 |
| B | unformulated HA-mRNA | 10µg | i.m. (25µl) | 8 |
| C | Rila buffer | - | i.m. (25µl) | 8 |

At time point 0 days, a prime vaccination was administered to 8 mice per group. On day 21, a blood sample was collected and a boost vaccination was administered. After 35 days, the mice were sacrificed and blood and organ samples (spleen) were collected for further analysis.

For immunogenicity assays, the HI titer was measured. ELISA was applied for determining antibody titers. Further, frozen splenocytes were stimulated with a HA overlapping peptide library and T cell immune responses were assessed by measuring IFNy production using Elispot.

### Results:

### Results Immunogenicity assays:

1µg HA-mRNA, LNP-formulated, induced HI titers above the threshold of 1:40 already after prime vaccination as apparent from Figure 9A. HI titers even further increased after boost vaccination as apparent from Figure 9B.

### Results ELISA assays:

1µg HA-mRNA, LNP-formulated, induced significantly higher functional antibody titers, both IgG1 and IgG2a subtypes, when compared to non-formulated mRNA already after a single i.m. injection (see Figures 10A-10D).

### Results T cell assays:

1µg HA-mRNA, LNP-formulated, led to increased IFNy production compared to non-formulated mRNA. The medium control as expected never generated a response (see Figure 11).

### Example 6: HA-mRNA and RABV-G mRNA vaccination of monkeys

The rabies mRNA vaccine encoded the glycoprotein (RABV-G) of the Pasteur strain (GenBank accession number: AAA47218.1). Optimized mRNA constructs (RABV-G A and B) were used, which contain identical ORFs but different UTRs. The protamine-formulated RABV-G mRNA vaccines contained RABV-G mRNA A. The unformulated or LNP-formulated RABV-G mRNA vaccines contained RABV-G mRNA B (as described above) if not otherwise indicated.

### Protamine RNA Formulation

For the preparation of protamine complexed mRNA ("RNActive^{®} formulation"), the obtained antigen mRNA constructs were complexed with protamine prior to use in in vivo vaccination experiments. The mRNA complexation consists of a mixture of 50% free mRNA and 50% mRNA complexed with protamine at a weight ratio of 2:1. First, mRNA was complexed with protamine by addition of protamine-Ringer's lactate solution to mRNA. After incubation for 10 minutes, when the complexes were stably generated, free mRNA was added, and the final concentration of the vaccine was adjusted with Ringer's lactate solution.

LNP formulated HA-mRNA and RABV-G mRNA vaccines as prepared in the previous example were used for vaccination. Four nulliparous and nonpregnant cynomolgus monkeys (Macaca fascicularis) were vaccinated by intramuscular injection at days 1 and 29 with LNP-III-3-formulated HA-mRNA or RABV-G mRNA (1µg or 10µg, mRNA as described above) or protamine-formulated HA-mRNA or RABV-G mRNA (240µg) into the biceps femoris muscle (500µl). As negative control buffer was injected. Serum samples were collected from the femoral vein on days 0, 29 and 50.

Detection of an antigen-specific immune response was carried out on days 0, 29 and 50.

### Hemagglutination inhibition assay:

Hemagglutination inhibition (HI) assays were used for analyzing functional anti-HA antibody titers. Non-human primate (NHP) sera were incubated with receptor destroying enzyme (RDEII, Denka Seiken, Japan) at 37°C overnight, inactivated (56°C, 60min) and incubated with kaolin. 50µl of 2-fold dilutions of pre-treated sera were incubated for 45min with 4 hemagglutination units (HAU) of inactivated Influenza A/California/7/2009 (H1N1) virus (NIBSC, UK) and 50µl 0.5% CRBC was added. HI titers were determined by the reciprocal of the highest dilution of the serum able to inhibit hemagglutination.

### Antibody analysis:

HA-specific IgG titers in NHP sera were measured by ELISA using inactivated A/California/7/2009 (H1N1) virus for coating (1µg/ml) and anti-human total IgG-HRP (ImmunoResearch) as detection antibody. Anti-rabies virus neutralizing titers (VNTs) in serum were analyzed by the Eurovir^{®} Hygiene-Labor GmbH, Germany, using the FAVN test (Fluorescent Antibody Virus Neutralization test) and the Standard Challenge Virus CVS-11 according to WHO protocol.

### Cytokine analysis:

Blood samples of NHPs were collected on days 1 and 29 for HA immunization and day 1 for RABV-G immunization from all NHPs prior to administration and 6 and 24 hours after dosing to determine inflammation biomarkers (G-CSF, IFNγ, IL-1β, IL-2, IL 4, IL-5, IL-6, IL-8 and TNF). Plasma was analyzed using the Luminex-based PRCYTOMAG-40K kit (MD MILLIPORE).

### Body temperature:

Body temperature of NHPs was determined by rectal measurement right before and at 0.5, 2, 6 and 24 hours after each dose.

### Results:

As a general result, a single intramuscular administration of a 1µg mRNA dose, LNP-III-3-formulated, surprisingly induced protective antibody titers in all vaccinated non-human primates in the two indications rabies and influenza.

### Results antibody titer analysis:

The results from the neutralizing antibody titer analysis are shown in Figure 12. The intramuscular vaccination of NHP with LNP-III-3-formulated HA-encoding mRNA surprisingly led to strong induction of neutralizing antibodies in a dose-dependent manner, already after prime vaccination with LNP-III-3-formulated HA-mRNA.

### Results rabies virus neutralizing titers (VNTs):

As shown in Figure 13 intramuscular vaccination of NHPs with LNP-III-3-formulated RABV-G-encoding mRNA led to a strong induction of neutralizing antibodies in a dose-dependent manner surprisingly already after prime vaccination with only 1µg LNP-III-3-formulated RABV-G-mRNA showing a VNT of >1 IU/ml (median value) after prime-vaccination and approximately 50 IU/ml (median value) after boost-vaccination (VNTs equal to or greater than the WHO-specified antibody titer of 0.5 IU/ml considered as a correlate of protection). Single vaccination with 10µg LNP-III-3-formulated RABV-G-mRNA induced a median antibody titer of 17 IU/ml. After a second vaccination titers reached a median of 419 UI/ml.

### Results HI-Titer:

LNP-III-3-formulated HA-mRNA led to very high HI titers already after prime vaccination as apparent from Figure 14. In detail, vaccination with 10µg LNP-III-3-formulated HA-mRNA induced in all animals HI titers at or above the HI titer of 1:40, which is considered protective in humans. Again, HI titers even further increased after second (boost) vaccination as apparent from Figure 14.

Animals which received 1µg of LNP-III-3-formulated HA-mRNA required a second vaccination to reach the protective titer in 3 of 4 animals, while none of the animals receiving 240µg protamine-formulated HA-mRNA exhibited detectable HI titers.

### Results cytokine analysis:

Systemic cytokine concentrations after vaccination with LNP-III-3-formulated mRNA stayed below detection level for IL-1β, IL 4, IL-5, IL-6, IFN-γ and TNF or did not increase for IL-2, IL-8 and G-CSF (Figures 15A-15D). Importantly, lower concentrations of the pro-inflammatory cytokines TNF, IFN-γ and IL-6 were induced by LNP-formulations compared to protamine-formulations, which are generally well tolerated in humans.

Results body temperature-results and other results:
Intramuscular application of LNP-III-3-formulated mRNA in non-human primates showed no impact on body temperature or the body weight [data not shown]. Injection sites showed only slight erythema and/or edema in 1 of 4 animals in each group receiving the LNP-III-3-formulated HA-mRNA, which resolved 24 to 96 hours after injection and no injection site reactions after vaccination with the RABV-G-mRNA formulations [data not shown]. In summary, the LNP-III-3-formulated mRNA was well tolerated by NHPs.

### Example 7: Immunogenicity after intramuscular (i.m.) application of low doses LNP-formulated RABV-G MRNA

The amount of 0.5µg LNP-formulated RABV-G-mRNA was used for testing the immunogenicity after intramuscular (i.m.) application. The sequence which was used is shown in SEQ ID NO: 224276.

Therefore, BALB/c mice were each vaccinated according to the vaccination scheme shown in Table Va intramuscularly in one leg. Unformulated RABV-G mRNA (40µg) served as a control.

**Table Va (Example 7): Vaccination scheme**

| **Strain sex** | **Mice #** | **Treatment RNA/mouse** | **Route, Volume** | **Immunisation schedule** | **Retrobular bleeding** |
|---|---|---|---|---|---|
| BALB/c Female | 10 | 5µg LNP-III-3 formulated RABV-G mRNA | i.m. 1x25µl | d0, d21 | d1, d21, d35 |
| BALB/c Female | 10 | 1µg LNP-III-3 formulated RABV-G mRNA | i.m. 1x25µl | d0, d21 | d1, d21, d35 |
| BALB/c Female | 10 | 0.5µg LNP-III-3 formulated RABV-G mRNA | i.m. 1x25µl | d0, d21 | d1, d21, d35 |
| BALB/c Female | 10 | 0.1µg LNP-III-3 formulated RABV-G mRNA | i.m. 1x25µl | d0, d21 | d1, d21, d35 |
| BALB/c Female | 10 | 0.05µg LNP-III-3 formulated RABV-G mRNA | i.m. 1x25µl | d0, d21 | d1, d21, d35 |
| BALB/c Female | 10 | 0.01µg LNP-III-3 formulated RABV-G mRNA | i.m. 1x25µl | d0, d21 | d1, d21, d35 |
| BALB/c Female | 10 | 40µg unformulated RABV-G mRNA | i.m. 1x25µl | d0, d21 | d1, d21, d35 |
| BALB/c Female | 6 | PBS | i.m. 2x25µl | d0, d21 | d1, d21, d35 |
| BALB/c Female | 6 | 32µg RNActive (protamine formulation) | i.d. 2x50µl | d0, d21 | d1, d21, d35 |

At time point 0 days, a prime vaccination was administered to 8 mice per group and a blood samples was taken one day later. On day 21, a blood sample was collected and a boost vaccination was administered. Prime and boost vaccinations were performed in different legs, i.e. left and right leg, respectively. After 35 days, the mice were sacrificed and blood and organ samples (spleen and liver) were collected for further analysis.

For immunogenicity assays, the VNT was measured as described before, i.e. anti-rabies virus neutralizing titers (VNTs) in serum were analyzed by the Eurovir^{®} Hygiene-Labor GmbH, Germany, using the FAVN test (Fluorescent Antibody Virus Neutralization test) and the Standard Challenge Virus CVS-11 according to WHO protocol.

CD4 T cell immune response (IFNy/TNFa producing CD4 T cells) and CD8 T cell immune response (IFNy/TNFa producing CD8 T cells and CD107+ IFNy producing CD8 T cells) were assessed. Assays were performed as described before. Histology of liver specimens from animals vaccinated with 5µg LNP or buffer controls was examined. Serum levels of ALT/AST were examined in serum samples taken d1 and d21. AST/ALT and liver were analyzed by mfd Diagnostics GmbH, Germany.

### Results:

Results rabies virus neutralizing titers (VNTs):
As shown in Figures 16A-16B, intramuscular vaccination of mice with LNP-III-3-formulated RABV-G-encoding mRNA led to a strong induction of neutralizing antibodies in a dose-dependent manner surprisingly already after prime vaccination already with 0.5µg LNP-III-3-formulated RABV-G-mRNA. WHO standard of 0.5 IU/ml is indicated by a dashed line in the graphs. Accordingly, doses of 5µg, 1µg and surprisingly also 0.5µg LNP-III-3-formulated RABV-G mRNA induced responses that were significantly higher compared to 40µg unformulated mRNA after prime vaccination (Figure 16A). No VNTs were induced after prime vaccination with 0.05µg and 0.01µg LNP-III-3-formulated mRNA. 0.1µg LNP-III-3-formulated mRNA induced VNTs after prime vaccination that were equal or above the WHO standard in 5 out of 10 animals. The VNTs even increased after boost vaccination (Figure 16B).

As VNT-levels of Figures 16A/16B appeared to be even higher but were limited through experimental VNT detection (the diluted samples still appeared to be in saturation), a similar experiment was performed and new VNT analyses were generated (see vaccination scheme shown in Table Vb). PBS buffer served as a control.

**Table Vb (Example 7): Vaccination scheme**

| **Strain sex** | **Mice #** | **Treatment RNA/ mouse** | **Route, Volume** | **Immunisation schedule** | **Retrobular bleeding** |
|---|---|---|---|---|---|
| BALB/c Female | 14 | 0.1µg LNP-III-3 formulated RABV-G mRNA | i.m. 1x25µl | d0, d21 | d21, d35 |
| BALB/c Female | 14 | 0.3µg LNP-III-3 formulated RABV-G mRNA | i.m. 1x25µl | d0, d21 | d21, d35 |
| BALB/c Female | 14 | 0.9µg LNP-III-3 formulated RABV-G mRNA | i.m. 1x25µl | d0, d21 | d21, d35 |
| BALB/c Female | 6 | PBS | i.m. 2x25µl | d0, d21 | d21, d35 |

As proven in Figures 61A-61B, intramuscular vaccination of mice with LNP-III-3-formulated RABV-G-encoding mRNA led to a very strong induction of neutralizing antibodies in a dose-dependent manner surprisingly already after prime vaccination already with 0.1µg, 0.3µg and 0.9µg LNP-III-3-formulated RABV-G-mRNA. WHO standard of 0.5 IU/ml is indicated by a dashed line in the graphs. Accordingly, doses of 0.1µg, 0.3µg and 0.9µg LNP-III-3-formulated RABV-G mRNA induced responses that were significantly higher compared to previous experiments with unformulated mRNA after prime vaccination. The VNTs even increased after boost vaccination (Figure 60B; data displays median - tested with Mann-Whitney).

### Results:

### Results T cell assays:

T cell responses were observed in mice immunized twice with 5µg, 1µg and 0.5µg as apparent from Figures 17A-17C. Accordingly, already 0.5µg mRNA, LNP-formulated, significantly induced high levels of antigen-specific multifunctional CD4+ T and CD8+ T cells compared to non-formulated mRNA. The negative DMSO control as expected never generated a response, as indicated by the dashed line in the graphs.

### Results Liver damage analysis:

Histology of RABV-G LNP 5µg mRNA and buffer administered control animals was performed. No pathological findings observed in any of the tested animals. AST levels were increased on day 1 post vaccination with the highest dose of LNPs (5µg) but decreased over time and returned to background levels on day 21 (see Figures 18A-18D). Dashed lines indicate the standard murine AST/ALT levels. Only some animals had elevated AST levels on day 21, in all other animals AST/ALT levels were without pathological findings.

### Example 8: Vaccination experiment with a combination of mRNAs encoding HA of four different Influenza viruses

For vaccination 9 mice per group were intramuscularly injected twice with a composition comprising LNP formulated mRNA encoding HA of 4 different influenza virus strains: A/California/7/2009 (H1N1), A/Hong Kong/4801/2014 (H3N2), B/Brisbane/60/2008 (B) and A/Vietnam/1203/2004 (H5N1). Therefore, the four mRNAs were mixed in a ratio of 1:1:1:1 and then formulated as described above. As control, Fluarix Quadrivalent 2015-2016 was injected, a split virus vaccine comprising 4 different inactivated influenza virus strains (A/California/7/2009, A/Switzerland/9715293/2013, B/Phuket/3073/2013, and B/Brisbane/60/2008) indicated for active immunization for the prevention of disease caused by influenza A subtype viruses and type B viruses. As negative control Ringer lactate buffer was injected.

Detection of an HA-specific immune response (B-cell immune response) was carried out by detecting IgG2a antibodies directed against the particular influenza virus. Therefore, blood samples were taken from the vaccinated mice three weeks after vaccination and sera were prepared. MaxiSorb plates (Nalgene Nunc International) were coated with the particular recombinant HA protein. After blocking with 1xPBS containing 0.05% Tween-20 and 1% BSA the plates were incubated with diluted mice serum (as indicated). Subsequently a biotin-coupled secondary antibody (anti-mouse-IgG2a, Pharmingen) was added. After washing, the plate was incubated with horseradish peroxidase-streptavidin, followed by addition of the Amplex UltraRed Reagent (Invitrogen) and subsequent quantification of the fluorescent product.

### Results:

The results shown in Figures 19A-19D demonstrate that HA-specific IgG antibodies directed against the different influenza viruses could be detected after single intramuscular vaccination with the LNP based vaccine comprising the four different mRNAs each encoding an HA antigen of a different influenza virus (Influenza A/California/7/2009 (H1N1; Figure 19A), Influenza A/Hong Kong/4801/2014 (H3N2; Figure 19B), Influenza B/Brisbane/60/2008 (B, Figure 19C) and Influenza A/Vietnam/1203/2004 (H5N1, Figure 19D).

These data proof that mRNA encoded antigens e.g. of different influenza viruses can be combined in one composition/vaccine.

In contrast to Fluarix, the LNP-III-3 formulated mRNA multivalent vaccine induced IgG2a antibody responses in naïve animals against all subtypes and the low dose of mRNA was immunogenic in mice surprisingly after single intramuscular injection.

### Example 9: Vaccination experiment with a combination of mRNAs encoding HA and NA of different influenza viruses

For vaccination 8 mice per group are intramuscularly injected with a composition comprising LNP formulated mRNA encoding HA of 4 different influenza virus strains: A/California/7/2009 (H1N1) and/or A/Netherlands/602/2009 (H1N1), A/Hong Kong/4801/2014 (H3N2), B/Brisbane/60/2008 and A/Vietnam/1203/2004 (H5N1) and NA of 3 different influenza virus strains: A/California/7/2009 (H1N1), A/Hong Kong/4801/2014 (H3N2), and B/Brisbane/60/2008.

In another vaccination experiment, 8 mice per group are intramuscularly injected with a composition comprising mRNA sequences encoding HA of InfluenzaA/California/7/2009 (H1N1) and/or A/Netherlands/602/2009 (H1N1), A/Hong Kong/4801/2014 (H3N2), B/Brisbane/60/2008 and B/Phuket/3073/2013 and NA of 3 different influenza virus strains: A/California/7/2009 (H1N1), A/Hong Kong/4801/2014 (H3N2), and B/Brisbane/60/2008.

As control, Influvac^{®} Tetravalent 2016-2017 is injected, a split virus vaccine comprising 4 different inactivated influenza virus strains (A/California/7/2009, A/Hong Kong/4801/2014, B/Phuket/3073/2013, and B/Brisbane/60/2008) indicated for active immunization for the prevention of disease caused by influenza A subtype viruses and type B viruses. As negative control Ringer lactate buffer is injected.

Detection of an HA-specific immune response (B-cell immune response) is carried out by detecting IgG2a antibodies directed against the particular influenza virus as described above.

NA-specific immune responses (B-cell immune response) directed against the particular influenza virus are determined using NA inhibition assay (NAI).

### Example 10: Synthesis of compound I-3

A solution of 6-(2'-hexyldecanoyloxy)hexan-1-al (2.4g), acetic acid (0.33g) and 4-aminobutan-1-ol (0.23g) in methylene chloride (20mL) was treated with sodium triacetoxyborohydride (1.3g) for two hours. The solution was washed with aqueous sodium bicarbonate solution. The organic phase was dried over anhydrous magnesium sulfate, filtered and the solvent removed. The residue was passed down a silica gel column using a methanol/methylene chloride (0-8/100-92%) gradient, yielding compound 3 as a colorless oil (0.4g).

### Example 11: Synthesis of a representative PEG lipid

Pegylated lipid IVa ("PEG-DMA") was prepared wherein n approximates the center of the range of ethylene oxide repeating units in the pegylated lipid.

### Synthesis of IVa-1 and IVa-2:

To a solution of myristic acid (6g, 26mmol) in toluene (50mL) was added oxalyl chloride (39mmol, 1.5 equivalents, 5g) at RT. Accordingly, 39 mmol of oxalyl chloride is 1.5 molar equivalents relative to the starting material myristic acid (26 mmoles x 1.5 = 39 mmoles). After the resulting mixture was heated at 70°C for 2h, the mixture was concentrated. The residue was taken up in toluene and concentrated again. The residual oil was added via a syringe to a concentrated ammonia solution (20mL) at 10°C. The reaction mixture was filtered and washed with water. The white solid was dried in vacuo. The desired product was obtained as a white solid (3.47g, 15mmol, 58.7%).

### Synthesis of IVa-3:

To suspension of 20-2 (3.47g, 15mmol) in THF (70 mL) was added in portions of lithium aluminium hydride (1.14g, 30mmol) at RT during 30min period of time. Then the mixture was heated to reflux gently (oil bath at 65°C) overnight. The mixture was cooled to 5°C and sodium sulphate 9 hydrate was added. The mixture was stirred for 2h, filtered through a layer of celite, washed with 15% of MeOH in DCM (200mL). The filtrate and washings were combined and concentrated. The residual solid was dried in vacuo. The desired product was obtained as a white solid (2.86g 13.4mmol, 89.5%).

### Synthesis of IVa-4:

To a solution of myristic acid (3.86g, 16.9mmol) in benzene (40mL) and DMF (1 drop) was added oxalyl chloride (25.35mmol, 1.5 equivalents, 3.22g) at RT. Accordingly, 25.35 mmol of oxalyl chloride is 1.5 molar equivalents relative to the starting material myristic acid (16.9mmol x 1.5 = 25.35mmol). The mixture was stirred at RT for 1.5h. Heated at 60°C for 30min. The mixture was concentrated. The residue was taken up in toluene and concentrated again. The residual oil (light yellow) was taken in 20mL of benzene and added via syringe to a solution of 20-3 (2.86g 13.4mmol) and triethylamine (3.53mL, 1.5 equivalents) in benzene (40mL) at 10°C. Accordingly, 3.53 mL of triethylamine is 1.5 molar equivalents with respect to the 13.4 mmol of 20-3 i.e. a 50% molar excess of this reagent compared to the starting material for this step. After addition, the resulting mixture was stirred at RT overnight. The reaction mixture was diluted with water and was adjusted to pH 6-7 with 20% H2SO4. The mixture was filtered and washed with water. A pale solid was obtained. The crude product was recrystallized from methanol. This gave the desired product as an off-white solid (5.65g, 13mmol, 100%).

### Synthesis of IVa-5:

To suspension of 20-4 (5.65g, 13mmol) in THF (60 mL) was added in portions lithium aluminium hydride (0.99g, 26mmol) at RT during 30min period of time. Then the mixture was heated to reflux gently overnight. The mixture was cooled to 0°C and sodium sulphate 9 hydrate. The mixture was stirred for 2h, then filtered through a pad of celite and silica gel and washed with ether first. The filtrate turned cloudy and precipitation formed. Filtration gave a white solid. The solid was recrystallized from MeOH and a colorless crystalline solid (2.43g).

The pad of celite and silica gel was then washed 5% of MeOH in DCM (400mL) and then 10% of MeOH in DCM with 1% of triethylamine (300mL). The fractions containing the desired product were combined and concentrated. A white solid was obtained. The solid was recrystalized from MeOH and a colorless crystalline solid (0.79g). The above two solids (2.43g and 0.79g) were combined and dried in vacuo (3.20g, 60%). 1HNMR (CDCl3 at 7.27ppm) δ: 2.58 (t-like, 7.2Hz, 4H), 1.52-1.44 (m, 4H), 1.33-1.24 (m, 44H), 0.89 (t-like, 6.6 Hz, 6H), 2.1-1.3 (very broad, 1H).

### Synthesis of Iva:

To a solution of 20-5 (7mmol, 2.87g) and triethylamine (30mmol, 4.18mL) in DCM (100mL) was added a solution of mPEG-NHS (from NOF, 5.0mmol, 9.97g, PEG MW approx. 2,000, n = about 45) in DCM (120mL,). After 24h the reaction solution was washed with water (300mL). The aqueous phase was extracted twice with DCM (100mL x 2). DCM extracts were combined, washed with brine (100mL). The organic phase was dried over sodium sulfate, filtered, concentrated partially. The concentrated solution (ca 300mL) was cooled at approximately15 C. Filtration gave a white solid (1.030g, the unreacted starting amine). To the filtration was added Et3N (1.6mmol, 0.222mL, 4 equivalents) and acetic anhydride (1.6mmol, 164mg). The mixture was stirred at RT for 3h and then concentrated to a solid. The residual solid was purified by column chromatography on silica gel (0-8% methanol in DCM). This gave the desired product as a white solid (9.211g). 1HNMR (CDCl3 at 7.27ppm) δ: 4.19 (s, 2H), 3.83-3.45 (m, 180200H), 3.38 (s, 3H), 3.28 (t-like, 7.6 Hz, 2H, CH2N), 3.18 (t-like, 7.8 Hz, 2H, CH2N), 1.89 (s, 6.6 H, water), 1.58-1.48 (m, 4H), 1.36-1.21 (m, 48-50H), 0.88 (t-like, 6.6 Hz, 6H).

### Example 12: Vaccination experiment with a combination of LNP-III-3 formulated mRNAs encoding HA of different influenza viruses:

Female BALB/c mice were immunized intramuscularly (i.m.) with LNP-III-3 formulated mRNA vaccine compositions with doses, application routes and vaccination schedules as indicated in Table A (mRNA sequences according to Example 1). As a negative control, one group of mice was injected with buffer (ringer lactate, Rila). As a positive control, one group of mice was vaccinated with an approved Influenza vaccine (Influsplit^{®} tetra 2016/2017; A/California/07/2009, A/Hong Kong/4801/2014, B/Brisbane/60/2008, B/Phuket/3073/2013). All animals were injected with the respective composition on day 0 and day 21. Blood samples were collected on day 21, 35, and 49 for the determination of binding antibody titers (using ELISA) and blocking antibody titers (using a HI assay). T cell responses were analyzed by intracellular cytokine staining (ICS) using splenocytes isolated on day 49. Detailed descriptions of the performed experiments are provided below.

**Table A: Immunization regimen (Example 12)**

| **Group** | **No. of mice** | **Treatment groups (control and mRNA compositions)** | **Dose** / **formulation** | **Treatment** |
|---|---|---|---|---|
| A | 6 | Rila buffer | - | i.m., 2x 25µl |
| B | 6 | Influsplit^{®} | 1/10 human dose | i.m., 2x 25µl |
| C | 9 | H1N1 A/Netherlands/602/2009 | 1µg (0.25µg each) | i.m., |
| | | H3N2 A/Hong Kong/4801/2014 | LNP-III-3 formulated | 1x 25µl |
| | | HA B/Brisbane/60/2008 | | |
| | | HA B/Phuket/3073/2013 | | |
| D | 9 | H1N1 A/Netherlands/602/2009 | 4µg (1µg each) | i.m., |
| | | H3N2 A/Hong Kong/4801/2014 | LNP-III-3 formulated | 1x 25µl |
| | | HA B/Brisbane/60/2008 | | |
| | | HA B/Phuket/3073/2013 | | |
| E | 9 | H1N1 A/California/07/2009 | 1µg (0.25µg each) | i.m., |
| | | H3N2 A/Hong Kong/4801/2014 | LNP-III-3 formulated | 1x 25µl |
| | | HA B/Brisbane/60/2008 | | |
| | | HA B/Phuket/3073/2013 | | |
| F | 9 | H1N1 A/California/07/2009 | 4µg (1µg each) | i.m., |
| | | H3N2 A/Hong Kong/4801/2014 | LNP-III-3 formulated | 1x 25µl |
| | | HA B/Brisbane/60/2008 | | |
| | | HA B/Phuket/3073/2013 | | |

### 12.1. Determination of anti HA protein specific IgG1 and IgG2a antibodies by ELISA:

ELISA assay was performed essentially as commonly known in the art, or as described above. ELISA was performed for each antigen comprised in the mRNA vaccine composition (as indicated in Table A). Results are shown in Figure 25 (H1N1 (A/California/7/2009)), Figure 27 (H3N2 (A/HongKong/4801/2014)), Figure 29 (Influenza B (B/Brisbane/60/2008)) and Figure 30 (Influenza B (B/Phuket/3073/2013)).

### 12.2. Hemagglutination inhibition assay (HI):

In a 96-well plate, the obtained sera were mixed with HA H1N1 antigen (A/California/07/2009 (H1N1); NIBSC) or HA H3N2 antigen and red blood cells (4% erythrocytes; Lohmann Tierzucht). In the presence of HA neutralizing antibodies, an inhibition of hemagglutination of erythrocytes can be observed. The lowest level of titered serum that resulted in a visible inhibition of hemagglutination was the assay result. The results are shown in Figure 26 ((H1N1 (A/California/7/2009)) and Figure 28 (H3N2 (A/HongKong/4801/2014)).

### 12.3. Detection of T-cell responses:

Splenocytes from vaccinated mice were isolated according to a standard protocol known in the art. Briefly, isolated spleens were grinded through a cell strainer and washed in PBS/1%FBS followed by red blood cell lysis. After an extensive washing step with PBS/1%FBS splenocytes were seeded into 96-well plates (2x10⁶ cells per well). The cells were stimulated with a pool of overlapping 15mer peptides of H1N1 (A/California/07/2009) for determining CD8+ T-cell responses or they were stimulated with recombinant HA protein for determining CD4+ T-cell responses. After stimulation, cells were washed and stained for intracellular cytokines using the Cytofix/Cytoperm reagent (BD Biosciences) according to the manufacturer's instructions. The following antibodies were used for staining: CD3-FITC (1:100), CD8-PE-Cy7 (1:200), TNF-PE (1:100), IFNγ-APC (1:100) (eBioscience), CD4-BD Horizon V450 (1:200) (BD Biosciences) and incubated with Fcy-block diluted 1:100. Aqua Dye was used to distinguish live/dead cells (Invitrogen). Cells were acquired using a Canto II flow cytometer (Beckton Dickinson). Flow cytometry data was analyzed using FlowJo software package (Tree Star, Inc.). Results for CD4+ T-cells are shown in Figure 31; the results for CD8+ T-cells are shown in Figure 32.

### Results:

The data shows that IgG1 and IgG2a antibodies could be detected after vaccination with the LNP formulated mRNA combination vaccines. Notably, for all mRNA encoded antigens comprised in the respective combination, specific IgG1 and IgG2a antibodies could be detected demonstrating that all mRNAs comprised in the respective compositions are translated into protein and trigger a humoral immune response in mice as shown in Figure 25 (H1N1 (A/California/7/2009)), Figure 27 (H3N2 (A/HongKong/4801/2014)), Figure 29 (Influenza B (B/Brisbane/60/2008)) and Figure 30 (Influenza B (B/Phuket/3073/2013)). Compared to mice vaccinated with the approved Influsplit^{®} vaccine, the responses were stronger or at least equally strong for all tested antigens.

Functional neutralizing antibodies were demonstrated for H1N1 (A/California/7/2009) and H3N2 (A/HongKong/4801/2014) (see Figures 26 and 28). Compared to mice vaccinated with the approved Influsplit^{®} vaccine, the induction of functional neutralizing antibodies was more pronounced and more durable for mRNA combination vaccines. Notably, the tested mRNA combination vaccine often reached HI titers >40 already after one immunization.

Figure 31 shows that the tested influenza mRNA combinations stimulated robust CD4+ IFNy/TNF-a T-cell responses in spleen of immunized mice for all antigens.

Figure 32 shows that the tested influenza mRNA combinations stimulated robust CD8+ IFN-y/TNF-a T-cell responses in spleen of immunized mice as shown for H1N1 (A/California/07/2009), whereas, notably, the approved Influsplit^{®} vaccine did not induce CD8+ T-cell responses.

Overall, the data demonstrates that LNP formulated mRNA based combination vaccines for HA antigens derived from different influenza viruses (A types and B types) induce strong and durable humoral immune responses and T-cell mediated cellular immune responses.

Example 13: Vaccination experiment with a combination of LNP-III-3 formulated mRNAs encoding HA of different influenza viruses:

Female BALB/c mice were immunized intramuscularly (i.m.) with LNP-III-3 formulated mRNA vaccine compositions with doses, application routes and vaccination schedules as indicated in Table B (mRNA Sequences according to Example 1). As a negative control, one group of mice was injected with buffer (ringer lactate, Rila). ). As a positive control, one group of mice was vaccinated with an approved Influenza vaccine (Fluarix^{®}; A/California/07/2009, A/Switzerland/9715293/2013, B/Brisbane/60/2008, B/Phuket/3073/2013). All animals were vaccinated on day 0 and day 21. Blood samples were collected on day 21, 35, and 49 for the determination of binding antibody titers (using ELISA), blocking antibody titers (using a HI assay) and the determination of virus neutralizing titers (VNTs). T cell responses were analyzed by intracellular cytokine staining (ICS) using splenocytes isolated on day 49. Detailed descriptions of the performed experiments are provided below.

**Table B: Immunization regimen (Example 13)**

| **Group** | **No. of mice** | **Treatment groups (control / mRNA compositions)** | **Dose** / **formulation** | **Treatment** |
|---|---|---|---|---|
| A | 6 | Rila buffer | - | i.m., 2x 25µl |
| B | 6 | Fluarix^{®} | 1/10 human dose | i.m., 2x 25µl |
| C | 9 | H1N1 A/Netherlands/602/2009 | 0.25µg (0.06µg each) | i.m., |
| | | H3N2 A/Hong Kong/4801/2014 | LNP-III-3 formulated | 1x 25µl |
| | | HA B/Brisbane/60/2008 | | |
| | | H5N1 A/Vietnam/1203/2004 | | |
| D | 9 | H1N1 A/Netherlands/602/2009 | 1µg (0.25µg each) | i.m., |
| | | H3N2 A/Hong Kong/4801/2014 | LNP-III-3 formulated | 1x 25µl |
| | | HA B/Brisbane/60/2008 | | |
| | | H5N1 A/Vietnam/1203/2004 | | |
| E | 9 | H1N1 A/Netherlands/602/2009 | 4µg (1µg each) | i.m., |
| | | H3N2 A/Hong Kong/4801/2014 | LNP-III-3 formulated | 1x 25µl |
| | | HA B/Brisbane/60/2008 | | |
| | | H5N1 A/Vietnam/1203/2004 | | |

### 13.1. Determination of anti HA protein specific IgG1 and IgG2a antibodies by ELISA:

ELISA assay was performed essentially as commonly known in the art, or as described above. ELISA was performed for each antigen comprised in the mRNA vaccine composition (as indicated in Table B). Results are shown in Figure 33 (H1N1 (A/California/7/2009)), Figure 35 (H3N2 (A/HongKong/4801/2014)), Figure 37 (Influenza B (B/Brisbane/60/2008)), and Figure 38(H5N1 (A/Vietnam/1203/2004)).

### 13.2. Hemagglutination inhibition assay (HI) and virus neutralizing assay:

HI-assay was performed as described above. The results are shown in Figure 34 ((H1N1 (A/California/7/2009)) and Figure 36 (H3N2 (A/HongKong/4801/2014)).

### 13.3. Detection of T-cell responses:

Splenocytes from vaccinated mice were isolated according to a standard protocol known in the art. ICS experiment was performed essentially as described in Example 12. Results for CD4+ T-cells are shown in Figure 39; the results for CD8+ T-cells are shown in Figure 40.

### Results:

The data shows that IgG1 and IgG2a antibodies could be detected after vaccination with the LNP formulated mRNA combination vaccines. Notably, for all mRNA encoded antigens comprised in the respective combination, specific IgG1 and IgG2a antibodies could be detected demonstrating that all mRNAs comprised in the respective compositions are translated into protein and trigger a humoral immune response in mice as shown in Figure 33 (H1N1 (A/California/7/2009)), Figure 35 (H3N2 (A/HongKong/4801/2014)), Figure 37 (Influenza B (B/Brisbane/60/2008)) and Figure 38 H5N1 (A/Vietnam/1203/2004)). Compared to mice vaccinated with the approved Fluarix^{®} vaccine, the responses were often stronger or at least equally strong for all tested antigens, even for the lowest mRNA vaccine dose tested.

Functional neutralizing antibodies were demonstrated for H1N1 (A/California/7/2009) and H3N2 (A/HongKong/4801/2014) (see Figure 34 and Figure 36). Compared to mice vaccinated with the approved Fluarix^{®} vaccine, the induction of functional neutralizing antibodies was more pronounced and more durable for mRNA combination vaccines. Notably, the tested mRNA combination vaccine reached HI titers >40 already after one immunization for the highest tested dose.

Figure 39 shows that the tested influenza mRNA combinations stimulated robust CD4+ IFNy/TNF-a T-cell responses in spleen of immunized mice for all antigens, with higher responses as observed for Fluarix^{®}.

Figure 40 shows that the tested influenza mRNA combinations stimulated robust CD8+ IFN-y/TNF-a T-cell responses in spleen of immunized mice as shown for H1N1 (A/California/07/2009) and H5N1 (A/Vietnam/1203/2004), whereas, notably, the approved Fluarix^{®} vaccine did not induce CD8+ T-cell responses.

Overall, the data demonstrates that LNP formulated mRNA based combination vaccines for HA antigens derived from different influenza viruses (A types and B types) induce strong and durable humoral immune responses and T-cell mediated cellular immune responses.

### Example 14: Vaccination experiment with LNP-III-3 formulated mRNA encoding Neuraminidase NA1 of influenza virus:

Female BALB/c mice were immunized intramuscularly (i.m.) with LNP-III-3 formulated mRNA vaccine compositions with doses, application routes and vaccination schedules as indicated in Table C (mRNA Sequences according to Example 1). As a negative control, one group of mice was injected with buffer (ringer lactate, Rila). As positive control, one group of mice was injected i.m. with Influsplit Tetra^{®} 2016/2017 (A/California/7/2009 (H1N1); A/Hong Kong/4801/2014 (H3N2); B/Brisbane/60/2008; B/Phuket/3073/2013). All animals were vaccinated on day 0 and day 21. Blood samples were collected on day 21, 35, and 49 for determination of immune responses. T cell responses were analyzed by intracellular cytokine staining (ICS) using splenocytes isolated on day 49.

**Table C: Immunization regimen (Example 14)**

| **Setup** | **No. of mice** | **Treatment groups (control / mRNA compositions)** | **Dose** / **formulation** | **Route, Volume** |
|---|---|---|---|---|
| A | 6 | Rila | - | i.m., 1x 25µl |
| B | 6 | Influsplit Tetra^{®} 2016/2017 | 1/10 of human dose | i.m., 2x 25µl |
| C | 6 | NA1 A/California/07/2009 | 1µg; LNP-III-3 formulated | i.m., 1x 25µl |
| D | 6 | NA1 A/California/07/2009 | 2.5µg; LNP-III-3 formulated | i.m., 1x 25µl |

### 14.1. Determination of immune responses for N1 NA (A/California/7/2009):

Functional NA-specific antibodies were analyzed using an enzyme-linked lectin assay (ELLA), essentially performed as previously described in the art. ELLA was performed in 96 well plates coated with a large glycoprotein substrate fetuin. NA cleaves terminal sialic acids from fetuin, exposing the penultimate sugar, galactose. Peanut agglutinin (PNA) is a lectin with specificity for galactose and therefore the extent of desialylation can be quantified using a PNA-horseradish peroxidase conjugate, followed by addition of a chromogenic peroxidase substrate. The optical density that is measured is proportional to NA activity. To measure functional NA inhibiting (NI) antibody titers, serial dilutions of sera were incubated on fetuin-coated plates with A/California/7/2009(H1N1) virus (pre-treated with Triton-X-100). The reciprocal of the highest serum dilution that results in ≥50% inhibition of NA activity is designated as the NI antibody titer. The result is shown in Figure 41.

To determine T-cell responses, an ICS experiment was performed, essentially as outlined above. Cells were stimulated with NA specific peptide mixture and CD8+ T-cell responses and CD4+ T-cell responses were determined. The results are shown in Figure 42 (CD4+) and Figure 43 (CD4+).

### Results:

As shown in Figures 41, strong and specific functional immune responses could be detected in mice vaccinated with LNP formulated mRNA coding for NA1 A/California/2009, whereas only weak responses could be determined for mice injected with Influsplit^{®} vaccine.

As shown in Figures 42 and 43, the tested NA1 mRNA vaccine stimulated robust CD4+ and CD8+ T-cell responses in spleen of immunized mice as shown, whereas, notably, the approved Influsplit^{®} vaccine did not induce CD8+ T-cell responses.

### Example 15: Vaccination experiment with LNP-III-3 formulated mRNAs encoding rabies virus antigen in mice and evaluation of pro-inflammatory environment and injection of LNP-III-3 formulated fluorescently labeled mRNA (F*-mRNA) in mice and analysis of composition and activaction status of immune cells in draining lymph-nodes (dLNs).

F*-mRNA corresponds to a fluorescently labeled PpLuc mRNA (60%-UTP-40%-5-Aminoallyl-UTP) which cannot be translated into a protein due to the labeling (SEQ ID NO: 224286).

The activation of the innate immune system is required to mount efficacious adaptive immune responses after vaccination. Therefore, the inventive LNP-III-3-formulated mRNA according to the invention was tested for their potency to (transiently) induce pro-inflammatory cytokines and chemokines after i.m. administration of LNP-formulated RABV-G mRNA in mice.

BALB/c mice (n=6/group) were vaccinated i.m. with 10µg non-formulated (mRNA) or LNP-formulated RABV-G-mRNA (mRNA/LNP), or with buffer control. Muscle tissues, dLNs and serum samples were isolated 4h, 14h, 24h and 96h after i.m. application and cytokine or chemokine content was measured in protein lysates and sera by Cytometric Bead Array (CBA). The results are shown in Figures 44-49.

To elucidate whether the pro-inflammatory environment translates into activation and changes in the composition of immune cells, the number and activation status of leukocytes in the dLNs was analyzed. To ensure that any observed effect was independent of the mRNA-encoded protein, a fluorescently labeled mRNA that cannot be translated (F*-mRNA) was used. BALB/c mice (n=3/group) were injected i.m. in both legs with 10µg non-formulated (F*mRNA) or LNP-formulated F*mRNA (F*mRNA/LNP), or with buffer. Right and left dLNs were isolated 4h, 24h and 48h after i.m. application and analyzed separately by flow cytometry. Numbers of each cell population (50A) and frequency of activated immune cells (50B) in the dLNs are shown in Figures 50A/B.

### Results:

As shown in Figures 44-46, LNP-formulated RABV-G mRNA induces a pronounced but transient release of the pro-inflammatory cytokines TNF and IL-6 with peak concentrations at 14h after injection. Importantly for the safety of the approach, the cytokine release was local and no systemic release of TNF (in serum) was observed. Transiently, 10-fold lower IL-6 concentrations were detected in the serum compared to the injection site, which returned to baseline at 96h after injection (see Figure 46).

As shown in Figures 47-49, LNP-formulated RABV-G mRNA induces a pronounced but transient release of pro-inflammatory chemokines. Among the strongly upregulated chemokines were MIP-1β, which plays a pivotal role in the chemotaxis of macrophages, monocytes and NK cells, and CXCL-9, which recruits T cells, NK cells and NKT cells to the site of inflammation. Moreover, there was a transient elevation in the concentrations of MCP-1, MIP-1a, and CXCL1, which attract a variety of immune cells such as monocytes, macrophages, dendritic cells and neutrophils.. We also observed a transient increase in serum concentrations of the chemokines described above, but to a much lower extent compared to those detected at the injection site or in the dLNs.

As shown in Figures 50A and 50B, intramuscular injection of the LNP-formulated F*-mRNA induced a strong increase in cellularity, which was absent after injection of unformulated F*-mRNA. The strongest elevation in cell numbers was observed 24 h after injection, except for NK cells which increased over time. CD11b+ Gr1+ cells, consisting mainly of monocytes and granulocytes, accounted for the largest increase in leukocytes. The increase in cellularity in dLNs was accompanied by a strong activation of both adaptive and innate immune cells, which peaked at 24 hours after injection, when more than 90% of the T and B cells expressed the activation marker CD69.

Taken together, these results suggest that i.m. injection of LNP-formulated mRNA vaccines induces a broad but transient local immunostimulatory milieu, which is relevant for the induction of strong adaptive immune responses.

### Example 16: Vaccination experiment with LNP-III-3 formulated mRNAs encoding rabies virus antigen in non-human primates:

LNP-III-3 formulated RABV-G mRNA vaccines as prepared in the previous example were used for vaccination.

Studies with cynomolgus monkeys (Macaca fascicularis) were conducted at Envigo CRS, S.A.U., Santa Perpètua de Mogoda, Spain. Animals were of Vietnamese origin, bred in captivity, nulliparous and not pregnant. Animals had at treatment start an age of 2.5 to 3.5 years and a body weight of 2.2-3.3 kg. NHPs were vaccinated i.m. at days 0 and 28 into the biceps femoris muscle with a single dose of 500µl. Vaccination with the licensed human rabies vaccine Rabipur^{®} (Novartis) was performed i.m. in NHPs with the full human dose according to the pre-exposure prophylaxis schedule on days 0, 7, and 28 or on a reduced schedule on days 0 and 28. VNTs of vaccinated monkeys were analyzed as described above. T-cell responses (CD 4+ and CD8+) were analyzed as described above. The results are shown in Figures 51-55.

### Results:

Figure 51 shows that already a single i.m. immunization with 1µg LNP-formulated RABV-G-mRNA induced robust VNTs at or above the protective titer of 0.5 IU/ml in all animals at day 28 after prime vaccination. The observed immunogenicity was dose dependent with a 10-fold higher mRNA dose yielding 10-fold higher VNTs.

Figure 52 shows that the observed primary responses could be boosted with a second vaccination with RABV-G-mRNA at day 28 was performed resulting in a 20-fold increase in VNTs. Monitoring of the antibody titers for six months demonstrated that after initial decline titers stabilized at a protective level of about 40 IU/ml for the 10µg mRNA dose and about 4 IU/ml for the 1µg mRNA dose.

Figure 53 shows the existence of B cell memory. To demonstrate the existence of B cell memory five months after completed vaccination a third vaccination (recall vaccination) was performed and VNTs were measured five days later. In both dose groups a very rapid 10-fold increase in VNTs was observed, demonstrating the induction of a strong recall-response by the mRNA vaccine.

Figure 54 shows that the LNP-formulated RABV-G-mRNA vaccine induced protective neutralizing antibody titers above 0.5 IU/ml after a single vaccination, which were comparable or higher compared to a full human dose of the licensed rabies vaccine Rabipur^{®}. Four weeks after a single vaccination mean VNTs measured for mRNA vaccinated monkeys were dramatically increased compared to Rabipur^{®} vaccinated monkeys. Boost vaccination at day 28 further increased VNT levels reaching up to 1000 IU/ml for mRNA vaccinated monkeys outperforming Rabipur^{®} induced VNTs by a factor of 10. These data suggest that vaccination with two injections of the LNP-formulated RABV-G-mRNA vaccine is sufficient to induce protection against rabies virus infections. For the 100µg dose of LNP-formulated RABV-G-mRNA, even a single administration may be sufficient to induce protective and sustained antibody titers. This is a particular advantage over the state-of-the-art rabies vaccine Rabipur^{®} that has to be applied three times.

Figures 55 and 56 shows that the inventive LNP-formulated RABV-G mRNA vaccine induced specific cellular responses after vaccination, effects that were not observed in in Rabipur^{®}-vaccinated animals. RABV-G-specific IFN-y+/IL-2+ CD4+ T cells (Figure 55) were observed for both mRNA vaccine doses, whereas RABV-G-specific IFN-y+/GrzB+ CD8+ T (Figure 56) cells were detected in animals receiving the 100µg dose. Notably, only minor cellular responses were observed in monkeys that received Rabipur^{®}.

### Example 17: Vaccination experiment with LNP-III-3 formulated mRNAs encoding influenza H1N1 or influenza H3N2 HA antigens in non-human primates:

LNP-III-3 formulated HA-mRNA Influenza A/California/7/2009 (H1N1) or HA-mRNA Influenza A/Hong Kong/4801/2014 (H3N2) vaccine as described in the previous example were used for vaccination.

Studies with cynomolgus monkeys (Macaca fascicularis) were conducted at Envigo CRS, S.A.U., Santa Perpètua de Mogoda, Spain. Animals were of Vietnamese origin, bred in captivity, nulliparous and not pregnant. Animals had at treatment start an age of 2.5 to 3.5 years and a body weight of 2.2-3.3 kg. NHPs were vaccinated with 10µg of either the H1N1-HA or the H3N2-HA vaccine and measured functional antibodies against the respective viruses by hemagglutination inhibition (HI) assays. As control, a group of animals was vaccinated with a full human dose of Fluad^{®}.

Naïve NHPs were vaccinated i.m. with 10µg of either the H1N1-HA or the H3N2-HA vaccine at days 0 and 28 into the biceps femoris muscle with a single dose of 500µl. Functional antibodies against the respective viruses were measured by hemagglutination inhibition (HI) assays. The results are shown in Figures 57-59. T-cell responses were analyzed as described above. The results are shown in Figures 60A-B.

### Results:

Figure 57 shows that already a single i.m. immunization with 10µg LNP-formulated H1N1-HA mRNA or H3N2-HA mRNA induced HI titers at or above the titer of 1:40 in all animals at day 28 after prime vaccination which is considered to be protective in human.

Figure 58 shows that the observed primary responses could be boosted with a second vaccination with the respective H1N1-HA mRNA at day 28 resulting in a strong increase of the measured HI titers. Monitoring of the antibody titers for more than 12 months (544 days) demonstrated that after initial decline titers stabilized at a protective H1N1 HI-titer of about 640. Importantly, all vaccinated animals maintained HI titers clearly above the protective limit until the end of the observation period one year after prime vaccination, confirming the remarkable longevity of the H1N1 HA antigen specific humoral immune response.

Figure 59 shows that the LNP-formulated H3N2-HA mRNA vaccine induce protective H3N2-HI titers after a single vaccination with responses comparable to the potent flu vaccine Fluad^{®} (season 2016/17; contains the surface antigens HA and neuraminidase of the influenza strains H1N1, H3N2 and B/Brisbane, as well as the adjuvant MF59C.1). A single dose of the LNP formulated mRNA vaccine was sufficient to induce protective H3N2-HI titers, comparable to titers induced by a full human dose of Fluad^{®}. A second dose further increased the HI titers, with a much stronger effect for the LNP formulated H3N2-HA mRNA vaccine.

Figure 60 shows that the inventive LNP-formulated H3N2-HA mRNA vaccine induced specific IFN-y+/IL-2+ CD4+ T-cell responses and TNFa+/IL-2+ CD4+ T-cell responses after vaccination, effects that were not observed in Fluad^{®}-vaccinated animals.

### Example 18: Vaccination experiment with a combination of mRNAs encoding different influenza antigens in non-human primates

Non-human primates (NHPs) are immunized (6 animals per group) with LNP-III-3 formulated mRNA vaccines with doses, application routes and vaccination schedules as indicated in Table D (mRNA Sequences preferably according to Example 1). As vaccines, an mRNA composition comprising four HA antigens is used ("tetravalent HA") or an mRNA composition comprising seven HA+NA antigens (four HA, three NA; heptavalent or "septavalent HA+NA") is used. All animals are vaccinated on day 0 and day 28. Blood samples are collected on day 0, 14, 28, 56, 77, and 84 for determination of antibody responses. T cell responses are analyzed by intracellular cytokine staining (ICS) using isolated splenocytes. Analysis of immune responses performed essentially as described above (ELLA, HI assay, ELISA, ICS, VNTs).

**Table D: Immunization regimen**

| **Treatment** | **Dose / formulation** | **Route, Volume** |
|---|---|---|
| HA A/California/7/2009 H1N1 | 40µg* | i.m., 500µl |
| HA A/Hong Kong/4801/2014 H3N2 | LNP-III-3 | |
| HA B/Brisbane/60/2008 | | |
| HA B/Phuket/3073/2013 | | |
| "Tetravalent HA" | | |
| HA A/California/7/2009 H1N1 | 200µg* | i.m., 500µl |
| HA A/Hong Kong/4801/2014 H3N2 | LNP-III-3 | |
| HA B/Brisbane/60/2008 | | |
| HA B/Phuket/3073/2013 | | |
| "Tetravalent HA" | | |
| NA1 A/California/07/2009 | 70µg* | i.m., 500µl |
| NA2 A/Hong Kong/4801/2014 | LNP-III-3 | |
| NA B/Brisbane/60/2008) | | |
| HA A/California/7/2009 H1N1 | | |
| HA A/Hong Kong/4801/2014 H3N2 | | |
| HA B/Brisbane/60/2008 | | |
| HA B/Phuket/3073/2013 | | |
| "Septavalent HA+NA" | | |
| NA1 A/California/07/2009 | 350µg* | i.m., 500µl |
| NA2 A/Hong Kong/4801/2014 | LNP-III-3 | |
| NA B/Brisbane/60/2008) | | |
| HA A/California/7/2009 H1N1 | | |
| HA A/Hong Kong/4801/2014 H3N2 | | |
| HA B/Brisbane/60/2008 | | |
| HA B/Phuket/3073/2013 | | |
| "Septavalent HA+NA" | | |
| Licensed vaccines | 1 human dose | i.m., 500µl |

| | | |
|---|---|---|
| * Each mRNA represented equally in the composition, i.e. 4x10µg, 4x50µg, 7x10µg, or 7x50µg | | |

### Example 19: Clinical development of an LNP-III-3 formulated influenza mRNA vaccine

To demonstrate safety and efficiency of the Influenza mRNA vaccine composition, a randomized, double blind, placebo-controlled clinical trial (phase I) is initiated.

For clinical development, GMP-grade RNA is produced using an established GMP process, implementing various quality controls on DNA level and RNA level as described in detail in WO 2016/180430 A1.

In the clinical trial, human volunteers (adult subjects, 18-45 years of age) are intramuscularly (i.m.) injected for at least two times with an mRNA composition comprising one mRNA coding for one influenza antigen as specified herein ("monovalent", H3N2 A/Hong Kong/4801/2014), or with an mRNA composition comprising four HA influenza antigens as specified herein ("tetravalent HA"), or with an mRNA composition comprising four HA and three NA influenza antigens as specified herein("septavalent HA+NA") or with an mRNA composition comprising multiple HA and multiple NA influenza antigens as specified herein ("multivalent HA+NA") . In addition, a group of elderly volunteers is treated (elderly adults >65 years of age). The design of the studies is indicated in Tables E-H below.

**Table E: Clinical design of a tetravalent HA influenza study**

| **Group** | **Treatment** | **Total mRNA per dose (µg)** | **Formulation** / **Route** | **Clinical dose volume (ml)** | **No. human of adult subjects** |
|---|---|---|---|---|---|
| 1 | Control (saline) | 0 | - | 0.5 | 30 |
| 2 | tetravalent HA | 20* | LNP-III-3 (i.m.) | 0.5 | 30 |
| 3 | tetravalent HA | 40* | LNP-III-3 (i.m.) | 0.5 | 30 |
| 4 | tetravalent HA | 80* | LNP-III-3 (i.m.) | 0.5 | 30 |
| 5 | Licensed vaccine control | - | i.m. | 0.5 | 30 |
| 6 elderly | mRNA vaccine | 40 or 80* | LNP-III-3 (i.m.) | 0.5 | 30 |

| | | | | | |
|---|---|---|---|---|---|
| * each mRNA represented equally in the composition | | | | | |

**Table F: Clinical design of a monovalent influenza study (H3N2)**

| **Group** | **Treatment** | **Total mRNA per dose (µg)** | **Formulation / Route** | **Clinical dose volume (mL)** | **No. human of adult subjects** |
|---|---|---|---|---|---|
| 1 | Control (saline) | 0* | i.m. | 0.5 | 30 |
| 2 | monovalent HA | 20* | LNP-III-3 (i.m.) | 0.5 | 30 |
| 3 | monovalent HA | 40* | LNP-III-3 (i.m.) | 0.5 | 30 |
| 4 | monovalent HA | 80* | LNP-III-3 (i.m.) | 0.5 | 30 |
| 5 | Licensed vaccine control | - | i.m. | 0.5 | 30 |
| 6 elderly | monovalent HA | 40 or 80* | LNP-III-3 (i.m.) | 0.5 | 30 |

| | | | | | |
|---|---|---|---|---|---|
| * each mRNA represented equally in the composition | | | | | |

**Table G: Clinical design of a heptavalent/septavalent HA+NA influenza study**

| **Group** | **Treatment** | **Total mRNA per dose (µg)** | **Formulation / Route** | **Clinical dose volume (ml)** | **No. human of adult subjects** |
|---|---|---|---|---|---|
| 1 | Control (saline) | 0 | i.m. | 0.5 | 30 |
| 2 | septavalent HA+NA | 20* | LNP-III-3 (i.m.) | 0.5 | 30 |
| 3 | septavalent HA+NA | 40* | LNP-III-3 (i.m.) | 0.5 | 30 |
| 4 | septavalent HA+NA | 80* | LNP-III-3 (i.m.) | 0.5 | 30 |
| 5 | Licensed vaccine control | - | i.m. | 0.5 | 30 |
| 6 elderly | septavalent HA+NA | 40 or 80* | LNP-III-3 (i.m.) | 0.5 | 30 |

| | | | | | |
|---|---|---|---|---|---|
| * each mRNA represented equally in the composition | | | | | |

**Table H: Clinical design of a multivalent HA+NA influenza study**

| **Group** | **Treatment** | **Total mRNA per dose (µg)** | **Formulation / Route** | **Clinical dose volume (ml)** | **No. human of adult subjects** |
|---|---|---|---|---|---|
| 1 | Control (saline) | 0 | i.m. | 0.5 | 30 |
| 2 | Multivalent HA+NA | 20* | LNP-III-3 (i.m.) | 0.5 | 30 |
| 3 | Multivalent HA+NA | 40* | LNP-III-3 (i.m.) | 0.5 | 30 |
| 4 | Multivalent HA+NA | 80* | LNP-III-3 (i.m.) | 0.5 | 30 |
| 5 | Licensed vaccine control | - | i.m. | 0.5 | 30 |
| 6 elderly | Multivalent HA+NA | 40 or 80* | LNP-III-3 (i.m.) | 0.5 | 30 |

| | | | | | |
|---|---|---|---|---|---|
| * each mRNA represented equally in the composition | | | | | |

In order to assess the safety profile of the Influenza vaccine compositions according to the invention, subjects are monitored after administration (vital signs, vaccination site tolerability assessments, hematologic analysis).

The efficacy of the immunization is analysed by determination of HI-titers and ELLA assay. Blood samples are collected on day 0 as baseline and after completed vaccination. Sera are analyzed for functional antibodies (HI assay, ELLA, VNTs (FAVN test)). In addition, a RFFIT assay is performed to analyze the presence of early VNTs using the rapid fluorescent foci inhibition test using the cell culture-adapted challenge virus strain CVS 11 as recommended by the World Organization for Animal Health. In brief, heat-inactivated sera are tested in serial two-fold dilutions for their potential to neutralize a 100 tissue culture infectious dose 50% of CVS. Sera dilutions are incubated with virus for about 70min at 37°C (in a water-jacket incubator with 5% CO2). 30,000 BHK-21 cells are added per well. Infected cell cultures are incubated for 22 hours at 37°C and 5% CO2. Cells are fixed using 80% acetone/20% PBS at -20°C for 10min and stained using FITC-conjugated anti-rabies globulin. Plates are washed twice using PBS and excess of PBS is removed. Cell cultures are scored positive or negative for the presence of rabies virus detected by FITC-positive signals. Negative scored cells in sera treated wells represent neutralization of rabies virus. Each RFFIT test includes WHO or OIE standard serum (positive reference serum), which serves as reference for standardisation of the assay. Neutralization activity of test sera is calculated with reference to the standard serum provided by the WHO and displayed as International Units/ml (IU/ml).

Furthermore, a subset of healthy subjects is challenged with live Influenza virus or placebo by oral administration. Subjects are followed post-challenge for symptoms of Influenza associated illness, infection and immune responses.

### Example 20: Stability of LNPs (LNP-III-3) stored at 5°C for 3 months

To compare immunogenicity and reactogenicity of LNP-III-3 formulated RABV-G mRNA stored at 5°C for 3 months, immunogenicity was assessed by determining humoral responses, including functional antibodies and cellular immune responses two weeks post boost vaccination (see Table J).

RABV-G-specific antibody titers were determined by Virus Neutralization Assay as described above.

**Table J: Immunization protocol (of Example 20):**

| **Strain sex** | **Mice #** | **Treatment RNA/mouse** | **Route, Volume** | **Immunisation schedule** | **Retrobular bleeding** |
|---|---|---|---|---|---|
| BALB/c Female | 8 | 0.9µg LNP-III-3 formulated RABV-G mRNA (LNP-batch freshly prepared) | i.m. 1x25µl | d0, d21 | d0, d21, d35 |
| BALB/c Female | 8 | 0.9µg LNP-III-3 formulated RABV-G mRNA (LNP-batch stored at 5°C for three month) | i.m. 1x25µl | d0, d21 | d0, d21, d35 |
| BALB/c Female | 8 | 0.3µg LNP-III-3 formulated RABV-G mRNA (LNP-batch freshly prepared) | i.m. 1x25µl | d0, d21 | d0, d21, d35 |
| BALB/c Female | 8 | 0.9µg LNP-III-3 formulated RABV-G mRNA (LNP-batch stored at 5°C for three month) | i.m. 1x25µl | d0, d21 | d0, d21, d35 |
| BALB/c Female | 6 | PBS | i.m. 2x25µl | d0, d21 | d0, d21, d35 |

As apparent from Figures 62A and 62B, surprisingly the stability of LNPs was not negatively influenced after storage at 5°C for three months, i.e. such LNPs only showed minor effects in vivo and were sufficient to generate very high VNTs after prime and after boost vaccination.

### Example 21: Toxicity analysis of LNPs (LNP-III-3)

The aim of this example was to evaluate the toxicity of the inventive LNPs (LNP-III-3). To this end, several *in vivo* toxicity studies were carried out in different animal models (e.g. mice, rats, or rabbits) with different mRNA doses (f.e. 1µg, 10µg, 40µg, 100µg, or 200µg). The results showed that the inventive LNPs showed no significant toxicity in vivo, evidenced by analysis of local reactions, pain, food consumption, body weight, organ weight, clinical chemistry (i.e. no adverse test substance related changes observed) and hematology (i.e. no adverse test substance related changes observed). Only minor local reactions like erythemas and edemas, i.e. normal reactions to vaccines which usually occur within 1-3 days, were found in a minority of the animals vaccinated with e.g. 10µg and 100µg.

### Example 22: Vaccination experiment with a combination of LNP-III-3 formulated mRNAs encoding HA of different influenza viruses in ferrets:

Ferrets (*Mustela putorius furo;* 6-12 months old) were immunized intramuscularly (i.m.) with LNP-III-3 formulated mRNA vaccine compositions comprising mRNA constructs encoding H1N1 A/California/07/2009, H3N2 A/Hong Kong/4801/2014, HA B/Brisbane/60/2008, and HA B/Phuket/3073/2013, herein referred to as "tetravalent mRNA vaccine"( see Table K). Respective mRNA sequences according to **Example 1.** As a positive control, one group of ferrets was vaccinated with an approved Influenza vaccine (Fluad^{®} tetra 2016/2017). All animals were injected with the respective composition on day 0 and day 21. Blood samples were collected on day 0, 21, 35, and 49 for the determination of blocking antibody titers for each encoded antigen (using a HI assay as described above and MN assay). The results are shown in **Figure 63****.**

**Table K: Immunization regimen (Example 22)**

| **Group** | **No. of ferrets** | **Treatment groups** | **Dose** | **Treatment** |
|---|---|---|---|---|
| A | 3 | tetravalent mRNA vaccine | 160µg (40µg each mRNA) | i.m., 1x 250µl |
| B | 3 | tetravalent mRNA vaccine | 40µg (10µg each mRNA) | i.m., 1x 250µl |
| C | 3 | tetravalent mRNA vaccine | 10µg (2.5µg each mRNA) | i.m., 1x 250µl |
| D | 6 | Fluarix^{®} | full human dose | i.m., 2x 250µl |

### Results:

As shown in **Figure 63****,** blocking antibody titers were detected for each encoding antigen. **Figure 63A and B** show that for H1N1 and H3N2 a clear dose response was observable and that protective HI titers (>40) were induced after the second vaccination for all tested groups. Notably, for the 160µg group and the 80µg group protective HI titers were already achived after prime vaccination. **Figure 63C and D** shows that protective HI titers were also detectable for B/Brisbane for the 160µg concentration. MN titers were observed for B/Phuket (Figure 63D).

Overall, the results demonstrate that the herein used tetravalent mRNA vaccine induces functional antibody responses for all four antigens. Moreover, the antibody responses were comparable to those observed for the approved vaccine Fluad, showing the enormous potential of the inventive LNP-formulated vaccine.

### Example 23: Challenge vaccination experiment with a combination of LNP-III-3 formulated mRNAs encoding HA of different influenza viruses in ferrets:

Ferrets (*Mustela putorius furo; 6* - 12 months old) are immunized intramuscularly (i.m.) with LNP-III-3 formulated tetravalent mRNA vaccine of **Example 22.** As a positive control, groups are vaccinated withFluad^{®} tetra 2016/2017. As negative control, groups are injected with placebo. For each group, 6 animals are treated ("immunization phase" in **Table L).**

To simulate a past season infection with influenza virus, some groups are also infected prior to the vaccination experiment with influenza virus. Group 5-8 are infected with H3N2 A/Fukui/20/2004 virus and group 13-16 were infected with B/Massachusetts/2/2012 Yamagata lineage (see "prime phase" in **Table L)**

After immunization, ferrets are challenged intratracheal with influenza virus. Group 5-8 were challenged with HA A/Netherlands/602/2009 virus and group 13-16 were challenged with B/Brisbane/60/2008 Victoria lineage (see "challenge phase" in **Table L).** Day 1-3 post virus challenge ferrets are analysed for virus load (nose, throat, swabs) and health parameters (fever, body weight). 4 days after virus challenge, animals are euthanized and analysed for immune responses (HI titers, IgG, Mn etc).

**Table L: Experimental procedure (Example 23)**

| | **prime phase** | **Vaccination shedule** | | **Challenge phase** | |
|---|---|---|---|---|---|
| 1 | No treatment | placebo | day 49 | | |
| 2 | | Fluad | day 49 | | |
| 3 | | tetravalent mRNA vaccine | day 49 | | |
| 4 | | | day 28 | | |
| | | | day 49 | H1N1 influenza A | Nose, Throat, Swabs, Body weight |
| 5 | H3N2 | placebo | day 49 | | |
| 6 | day 21 | Fluad | day 49 | | |
| 7 | | tetravalent mRNA vaccine | day 49 | | |
| 8 | H3N2 | | day 28 | | |
| | day 0 | | day 49 | | |
| 9 | No treatment | placebo | day 49 | | |
| 10 | | Fluad | day 49 | | |
| 11 | | tetravalent mRNA vaccine | day 49 | | |
| 12 | | | day 28 | | |
| | | | day 49 | B Brisbane | Nose, Throat, Swabs, Body weight |
| 13 | B Massachusetts | placebo | day 49 | | |
| 14 | day 21 | Fluad | day 49 | | |
| 15 | | tetravalent mRNA vaccine | day 49 | | |
| 16 | B Massachusetts | | day 28 | | |
| | day 0 | | day 49 | | |

### Example 24: Vaccination experiment with LNP-III-3 formulated mRNAs encodina three different NA antigens (Trivalent NA mRNA vaccine)

As exemplarily shown in **Example 14,** LNP-III-3 formulated mRNA encoding neuraminidase induces strong and effective immune responses. In the present example, a trivalent mRNA composition comprising mRNA encoding NA of Influenza A/California/07/2009 (H1N1), mRNA encoding NA Influenza A/Hong Kong/4801/2014 (H3N2) and mRNA encoding encoding NA of Influenza B/Brisbane/60/2008 was vaccinated (herein referred to as "Trivalent NA mRNA vaccine"). Respective mRNA sequences according to **Example 1**

Female BALB/c mice were injected at day 0 and day 21 with a trivalent NA mRNA vaccine, Influsplit Tetra (2016/2017) as positive control or a buffer control (RiLa) according to a regimen as provided in **Table M** below. Serum samples were taken for the determination of specific antibody titers (ELLA assay, performed according to **Example 14).** Results of the ELLA assay are shown in **Figure 64****.**

**Table M: Immunization regimen (Example 24):**

| **Group** | **No. of mice** | **Treatment groups** | **Dose** | **Treatment** |
|---|---|---|---|---|
| 1 | 13 | trivalent NA mRNA vaccine LNP-III-3 formulated | 7.5µg (2.5µg each mRNA) | i.m., 1x 25µl |
| 2 | 6 | Influsplit Tetra 2016/2017 | Full human dose | i.m., 1x 25µl |
| 3 | 3 | RiLa buffer | | i.m., 1x 25µl |

### Results:

As shown in **Figures 64****,** strong and specific functional immune responses could be detected in mice vaccinated with LNP formulated mRNA coding for three different NA antigens (trivalent NA mRNA vaccine). Compared to the responses detected after vaccination with a full human dose Influsplit Tetra, the responses obtained with the inventive trivalent LNP-III-3 formulated NA mRNA vaccine were more pronounced. The results show that for each NA antigen strong functional immune responses were induced after vaccination with a LNP-III-3 formulated trivalent NA mRNA vaccine.

### Example 25: Vaccination experiment with LNP-III-3 formulated mRNAs encoding three different NA antigens and four different HA antigens (Septavalent mRNA vaccine)

As exemplarily shown in the Examples above, LNP-III-3 formulated tetravalent HA mRNA vaccines and trivalent NA mRNA vaccines induce strong and effective immune responses for each encoded antigen. In the present example, an LNP-III-3 formulated mRNA composition encoding three different NA antigens (mRNA encoding NA of Influenza A/California/07/2009 (H1N1), mRNA encoding NA of Influenza A/Hong Kong/4801/2014 (H3N2) and mRNA encoding encoding NA of Influenza B/Brisbane/60/2008 was vaccinated) and four different HA antigens (mRNA encoding HA of Influenza A/California/07/2009 (H1N1), mRNA encodingHA of Influenza A/Hong Kong/4801/2014 (H3N2) and mRNA encoding HA of Influenza B/Brisbane/60/2008 and mRNA encoding encoding HA of Influenza B) was vaccinated (herein referred to as "septavalent HA/NA mRNA vaccine"). Respective mRNA sequences according to **Example 1.**

Female BALB/c mice were injected i.m. at day 0 and day 21 with LNP-III-3 formulated tetravalent HA mRNA vaccine, LNP-III-3 formulated trivalent NA mRNA vaccine, or LNP-III-3 formulated septavalent HA/NA mRNA vaccine. As positive control, one group of mice was injected with Influsplit Tetra^{®} 2016/2017. As negative control, one group of mice was injected with RiLa buffer. Serum samples for the analysis of immune responses (HI titer, ELISA) were collected at day 21, 35, 49 (assays performed as described above). Splenocytes collected at day 49 (ICS). Experimental details provided in **Table N.** ELISA and HI-titer results are shown in **Figures 65-70****.**

**Table N: Immunization regimen (Example 25)**

| **Group** | **No. of mice** | **Treatment groups** | **Dose** | **Treatment** |
|---|---|---|---|---|
| 1 | 8 | Tetravalent HA mRNA vaccine | 4µg (1µg each) | i.m., 1x 25µl |
| 2 | 8 | Trivalent NA mRNA vaccine | 3µg (1µg each) | i.m., 1x 25µl |
| 3 | 8 | septavalent HA/NA mRNA vaccine | 7µg (1µg each) | i.m., 1x 25µl |
| 4 | 8 | septavalent HA/NA mRNA vaccine | 7µg (0.5µg each) | |
| 5 | 6 | Influsplit Tetra | 1/10 human dose | i.m., 2x 25µl |
| 6 | 6 | RiLa | --- | i.m., 1x 25µl |

### Results:

The results show that the LNP-III-3 formulated septavalent HA/NA mRNA vaccine induces strong and effective immune responses in vaccinated mice.

**Figure 65****-****Figure 68****:** shows the presence of total IgG1 and IgG2a antibodies for each of the four HA antigens. Of note: No differences were detected between the immune responses detected in mice vaccinated with tetravalent HA mRNA vaccine, showing that the addition of further mRNA constructs encoding NA antigens did not reduce the effectiveness of the septavalent mRNA vaccine.

**Figure 69****:** shows the presence of specific antibodies for each of the three NA antigens. Of note: No differences were detected between the immune responses detected in mice vaccinated with trivalent NA mRNA vaccine, showing that the addition of further mRNA constructs encoding HA antigens did not reduce the effectiveness of the septavalent mRNA vaccine.

**Figure 70****:** shows that vaccination of mice with LNP formulated septavalent HA/NA mRNA vaccine induces functional antibodies.

### Example 26: Vaccination experiment with LNP-III-3 formulated mRNAs encoding Ebola GP

In the present example, the inventive mRNA LNP-III-3 formulation was compared with an established mRNA vaccine format (Protamine formulation; see Example 6) using mRNA encoding glycoprotein GP of Ebola virus (ZEBOV GP Sierra Leone 2014).

Protamine formulation of mRNA encoding GP of Ebola virus (SEQ ID NO: 224362) as described in Example 6. LNP formulation of mRNA encoding GP of Ebola virus (SEQ ID NO: 224362) as described above.

Immunogenicity of Ebola GP mRNA vaccine in mice:
Female BALB/c mice were injected at day 0 and day 21 with a LNP-III-3 formulated mRNA encoding GP of Ebola virus (RNA ID "R3875"; intramuscular (i.m.)) or protamine formulated mRNA encoding GP of Ebola virus (RNA ID "R3875"; intradermal (i.d.)). As negative control, one group of mice was injected with RiLa buffer. Serum samples for the analysis of IgG endpoint titers (ELISA) were collected at day 35. ELISA was performed using recombinant ZEBOV Mayinga GP protein (lacking the transmamebrane domain) for coating. ELISA results are shown in **Figure 71****.** The outline of the vaccination experimental is provided in **Table O.**

**Table O: Immunization regimen (Example 26)**

| **Group** | **No. of mice** | **Treatment groups** | **Dose** | **Treatment** |
|---|---|---|---|---|
| 1 | 8 | GP Ebola, Protamine formulated | 80µg | i.d., 1x 50µl |
| 2 | 8 | GP Ebola; LNP formulated | 5µg | i.m., 1x 25µl |
| 3 | 8 | GP Ebola; LNP formulated | 7µg (1µg each) | i.m., 1x 25µl |
| 4 | 8 | RiLa | 7µg (0.5µg each) | i.d., 1x 50µl |

### Results:

**Figure 71** shows that LNP-III-3 formulated mRNA encoding GP of Ebola virus induce strong humoral immune responses in mice (i.m. application). Compared with the established protamine mRNA vaccine format (i.d. application), the mRNA amount needed for similar immune response could be dramatically reduced. Moreover, LNP-III-3 formulated mRNA vaccines can be applied intramuscularily which is an important feature for prophylactic vaccines (easy and fails-safe intramuscular application of the vaccine in e.g. human subjects).

### Example 27: Vaccination experiment with LNP-III-3 formulated mRNAs encoding H3N2 administered subcutaneously

As described in the previous examples, LNP-III-3 formulated mRNA vaccines induce strong and effective immune responses when administered intramuscularily or intradermally (e.g. see Example 4). To evaluate the effectivity of the inventive vaccine format for other suitable administration routes, sub-cutaneous injection was tested.

Non-human primates *(Macaca fascicularis)* were injected with LNP-III-3 formulated mRNA encoding Influenza A/Hong Kong/4801/2014 H3N2 (mRNA sequences according to Example 1). Three goups were vaccinated subcutaneously (sc) with different vaccine doses (10µg, 50µg, 100µg) and one control group was vaccinated intramuscularily. HI titers at day 0, day 28, day 49 and day 70 were determined as described herein. Results of the experiment are shown in **Figure 72****.** The outline of the vaccination experimental is provided in **Table P.**

**Table P: Immunization regimen (Example 27)**

| **Group** | **No. of NHP** | **Treatment groups** | **Dose and route** | **Treatment** |
|---|---|---|---|---|
| 1 | 8 | LNP-III-3 formulated H3N2 | 10µg, subcutaneous | Day 0, day 28 |
| 2 | 8 | LNP-III-3 formulated H3N2 | 50µg, subcutaneous | Day 0, day 28 |
| 3 | 8 | LNP-III-3 formulated H3N2 | 100µg, subcutaneous | Day 0, day 28 |
| 4 | 8 | LNP-III-3 formulated H3N2 | 100µg, i.m. | Day 0, day 28 |

Results: As shown in **Figure 72****,** the inventive LNP-III-3 formulated mRNA vaccine format induces strong protective antibody titers against the encoded antigen when administered sub-cutaneously. Notably, the HI titers were comparable to those achieved through i.m. administration. Moreover, protective titers were already achieved after one administration (day 28). Stable protective titers were detected for s.c. vaccinated animals for 50µg and 100µg. Summarizing the above, the results demonstrate that the inventive LNP-III-3 formulated mRNA vaccine format is suitable for intradermal, intramuscular and also sub-cutaneous administration.

### Example 28: Vaccination experiment with LNP-III-3 formulated OVA mRNA vaccine

For vaccination 9 mice (C57 BL/6) per group were intradermally injected 3 times within 3 weeks with 1µg LNP formulated Ova mRNA (Component A) and 32µg protamine formulated Ova mRNA (Component B), as negative control RiLa was injected (see **Table Q).**

Levels of circulating antigen-specific CD8 positive T cells were measured with OVA-specific dextramers (bind to antigen specific T cell receptors of CD8 positive cells) at day 7 and 21. 1µg LNP formulated Ova mRNA (Component A) vaccine induces high and boostable levels of circulating antigen-specific CD8 positive T cells after intradermal application **(see** **Figure 73** and **Figure 74****).**

**Table Q: Components, treatment and RNA dilution (Example 28)**

| **Component** | **Treatment** | **RNA dose** | **Route (Volume)** | **Mice #** |
|---|---|---|---|---|
| A | OVA mRNA formulated in LNP | 1µg | i.d (25µl)/1 site of injection | 9 |
| B | Ova mRNA formulated with protamine | 32µg | i.d (100µl)/4 sites of injection (state of the art) | 9 |
| C | Rila buffer | - | i.d (25µl)/1 site of injection | 3 |

Levels of multifunctional antigen-specific CD8 positive T cells (IFNy/TNFa) were measured by intracellular cytokine staining (ICS). Therefore splenocytes were isolated from the vaccinated mice one week after the last vaccination and CD8 positive T cells were stimulated with OVA specific peptides (SIINFEKL and TEWTSSNVMEERKIKV) **(see** **Figure 75). Figure 75** shows that 1µg LNP formulated Ova mRNA vaccine (Component A) induces high levels of multifunctional CD8 positive T cells after intradermal application compare to protamine formulated Ova mRNA (Component B).

Detection of B-cell immune responses was carried out by detecting OVA-specific IgG2c titers. Therefore, serum samples were taken from the vaccinated mice one week after the last vaccination and analyzed by ELISA. 1µg of LNP-formulated OVA-mRNA vaccine leads to increased OVA-specific IgG2c titers after intradermal application compare to protamine formulated Ova mRNA (see **Figure 76****).**

The results demonstrate that the inventive LNP-III-3 formulated mRNA vaccine format is suitable to induce anti-tumor responses *in vivo* and therefore useable for vaccination against tumor.

### Example 29: Tumor challenge experiment with LNP-III-3 formulated OVA mRNA vaccine

C57BL/6 mice were injected subcutaneously (s.c.) with 3x105 E.G7-OVA cells per mouse (in a volume of 100µl PBS) on the right flank on day 0 of the experiment. Intradermal (i.d.) therapy started at day 4 and continued twice a week for three weeks. Mice were injected with 1µg OVA mRNA and an irrelevant PpLuc mRNA formulated in LNPs. To control for anti-tumor effects due to injection procedure, mice were injected with buffer (RiLa).

The results of the experiment are shown in **Figure 77** and **Figure 78****,** wherein Figure 77 shows the effect of the inventive composition on tumor growth and Figure 78 shows the effect of the inventive composition on survival.

Tumor growth was monitored by measuring the tumor size in three dimensions using a calliper. Tumor volume was calculated according to the following formula: $volume \left({mm}^{3}\right) = \frac{length \left(mm\right) \times π \times {width}^{2} \left({mm}^{2}\right)}{6}$

The results in **Figure 77** show that the inventive Component A (LNP formulated Ova mRNA) strongly decreased the median tumor volume compared to the other treatment with an irrelevant mRNA (Component B). In addition, the results in **Figure 78** show that the inventive Component A (LNP formulated Ova mRNA) strongly increased the survival of tumor challenged mice compared to the other treatments (Component B and Buffer).

**Table R: Components, treatment and RNA dilution (Example 29)**

| **Component** | **Treatment** | **RNA dose** | **Route (Volume)** | **Schedule** | **Mice #** |
|---|---|---|---|---|---|
| A | OVA mRNA formulated in LNP | 1µg | i.d (25µl)/ 1 site of injection | 2 x week | 10 |
| B | PpLuc mRNA formulated in LNP | 1µg | i.d (25µl)/ 1 site of injection | 2 x week | 10 |
| C | RiLa | - | i.d (25µl)/1 site of injection | 2 x week | 6 |

The results demonstrate that the inventive LNP-III-3 formulated mRNA vaccine format is suitable for tumor vaccination.

### Example 30: Vaccination experiment with LNP-III-3 formulated endoaene tumor associated antigens mRNA vaccine in combination with checkpoint inhibitor

C57BL/6 mice were injected subcutaneously (s.c.) with 1x10⁵ B16.F10 cells (murine melanoma cell line) per mouse on the right flank on day 0 of the experiment. At day 6 after tumor challenge, mice were vaccinated intradermal with LNP formulated Trp2 mRNA (Component A) and irrelevant LNP formulated PpLuc mRNA (Component B). In addition immune checkpoint inhibitors anti-PD1 (Clone: RMP1-14) and anti-CTLA4 (clone: 9H10) (both BioXCell) were administered intraperitoneal (i.p.), median tumor size and survival rates were analyzed. To exclude an anti-tumor effect due to the checkpoint inhibitors, mice were injected with component B and a control antibody (rat IgG2a, BioXCell).

Tumor-bearing mice treated with vaccine against mTrp2 and checkpoint inhibitors anti-PD1 and anti-CTLA4 show delayed tumor growth and increased survival compared to other treatments with irrelevant mRNA (Component B) in combination with checkpoint inhibitors anti-PD1 and anti-CTLA4 or a control antibody (see Figure 79 and 80).

**Table S: Components, treatment and RNA/Antibody dilution**

| | **Treatment** | **RNA dose (i.d) / volume** | **Antibody (i.p)** | **Schedule** | **Mice #** |
|---|---|---|---|---|---|
| A | mTrp2 mRNA formulated in LNP | 1µg / 25µl | Anti-PD1 + anti-CTLA4 | 2 x week | 10 |
| B | PpLuc mRNA formulated in LNP | 1µg / 25µl | Isotype | 2 x week | 10 |
| C | PpLuc mRNA formulated in LNP | 1µg / 25µl | Anti-PD1 + anti-CTLA4 | 2 x week | 10 |

The results demonstrate that the inventive LNP-III-3 formulated mRNA vaccine format is suitable for the combination with checkpoint inhibitors for anti-tumor therapy.

A set of embodiments of the present invention relates to:
1. A lipid nanoparticle comprising
(i) a cationic lipid with the formula III: or a pharmaceutically acceptable salt, tautomer, prodrug or stereoisomer thereof, wherein:
   L¹ or L² is each independently -O(C=O)-, -(C=O)O-, -C(=O)-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}- or -NR^{a}C(=O)O-, preferably L¹ or L² is -O(C=O)- or -(C=O)O-;
   G¹ and G² are each independently unsubstituted C₁-C₁₂ alkylene or C₁-C₁₂ alkenylene;
   G³ is C₁-C₂₄ alkylene, C₁-C₂₄ alkenylene, C₃-C₈ cycloalkylene, or C₃-C₈ cycloalkenylene;
   R^{a} is H or C₁-C₁₂ alkyl;
   R¹ and R² are each independently C₆-C₂₄ alkyl or C₆-C₂₄ alkenyl;
   R³ is H, OR⁵, CN, -C(=O)OR⁴, -OC(=O)R⁴ or -NR⁵C(=O)R⁴;
   R⁴ is C₁-C₁₂ alkyl;
   R⁵ is H or C₁-C₆ alkyl; and
   x is 0, 1 or 2;
(ii) a mRNA compound comprising an mRNA sequence encoding at least one antigenic peptide or protein,

wherein the mRNA compound optionally does not comprise a nucleoside modification, in particular not a base modification;
wherein the mRNA compound is encapsulated in or associated with said lipid nanoparticle.
2. A lipid nanoparticle comprising:
(i) a PEG lipid with the formula (IV)
   wherein R⁸ and R⁹ are each independently a straight or branched, saturated or unsaturated alkyl chain containing from 10 to 30 carbon atoms, wherein the alkyl chain is optionally interrupted by one or more ester bonds;
   and w has a mean value ranging from 30 to 60; and
(ii) a mRNA compound comprising an mRNA sequence encoding at least one antigenic peptide or protein,

wherein the mRNA compound optionally does not comprise a nucleoside modification, in particular not a base modification;
wherein the mRNA compound is encapsulated in or associated with said lipid nanoparticle.
3. A lipid nanoparticle comprising
(i) a cationic lipid with the formula I: or a pharmaceutically acceptable salt, tautomer, prodrug or stereoisomer thereof, wherein:
   L¹ and L² are each independently -O(C=O)-, -(C=O)O- or a carbon-carbon double bond;
   R^{1a} and R^{1b} are, at each occurrence, independently either (a) H or C₁-C₁₂ alkyl, or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
   R^{2a} and R^{2b} are, at each occurrence, independently either (a) H or C₁-C₁₂ alkyl, or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
   R^{3a} and R^{3b} are, at each occurrence, independently either (a) H or C₁-C₁₂ alkyl, or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
   R^{4a} and R^{4b} are, at each occurrence, independently either (a) H or C₁-C₁₂ alkyl, or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
   R⁵ and R⁶ are each independently methyl or cycloalkyl;
   R⁷ is, at each occurrence, independently H or C₁-C₁₂ alkyl;
   R⁸ and R⁹ are each independently C₁-C₁₂ alkyl; or R⁸ and R⁹, together with the nitrogen atom to which they are attached, form a 5, 6 or 7-membered heterocyclic ring comprising one nitrogen atom;
   a and d are each independently an integer from 0 to 24;
   b and c are each independently an integer from 1 to 24; and
   e is 1 or 2;
and (ii) a mRNA compound comprising an mRNA sequence encoding at least one antigenic peptide or protein,

wherein the mRNA compound optionally does not comprise a nucleoside modification, in particular not a base modification,
wherein the mRNA compound is encapsulated in or associated with said lipid nanoparticle.
4. A lipid nanoparticle comprising
(i) a cationic lipid with the formula II: or a pharmaceutically acceptable salt, tautomer, prodrug or stereoisomer thereof, wherein:
   L¹ and L² are each independently -O(C=O)-, -(C=O)O-, -C(=O)-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}-, -NR^{a}C(=O)O-, or a direct bond;
   G¹ is C₁-C₂ alkylene, -(C=O)- , -O(C=O)-, -SC(=O)-, -NR^{a}C(=O)- or a direct bond
   G² is -C(=O)- , -(C=O)O-, -C(=O)S-, -C(=O)NR^{a}- or a direct bond;
   G³ is C₁-C₆ alkylene;
   R^{a} is H or C₁-C₁₂ alkyl;
   R^{1a} and R^{1b} are, at each occurrence, independently either: (a) H or C₁-C₁₂ alkyl; or (b) R^{1a} is H or C₁-C₁₂ alkyl, and R^{1b} together with the carbon atom to which it is bound is taken together with an adjacent R^{1b} and the carbon atom to which it is bound to form a carbon-carbon double bond;
   R^{2a} and R^{2b} are, at each occurrence, independently either: (a) H or C₁-C₁₂ alkyl; or (b) R^{2a} is H or C₁-C₁₂ alkyl, and R^{2b} together with the carbon atom to which it is bound is taken together with an adjacent R^{2b} and the carbon atom to which it is bound to form a carbon-carbon double bond;
   R^{3a} and R^{3b} are, at each occurrence, independently either: (a) H or C₁-C₁₂ alkyl; or (b) R^{3a} is H or C₁-C₁₂ alkyl, and R^{3b} together with the carbon atom to which it is bound is taken together with an adjacent R^{3b} and the carbon atom to which it is bound to form a carbon-carbon double bond;
   R^{4a} and R^{4b} are, at each occurrence, independently either: (a) H or C₁-C₁₂ alkyl; or (b) R^{4a} is H or C₁-C₁₂ alkyl, and R^{4b} together with the carbon atom to which it is bound is taken together with an adjacent R^{4b} and the carbon atom to which it is bound to form a carbon-carbon double bond;
   R⁵ and R⁶ are each independently H or methyl;
   R⁷ is C₄-C₂₀ alkyl;
   R⁸ and R⁹ are each independently C₁-C₁₂ alkyl; or R⁸ and R⁹, together with the nitrogen atom to which they are attached, form a 5, 6 or 7-membered heterocyclic ring;
   a, b, c and d are each independently an integer from 1 to 24; and
   x is 0, 1 or 2;
and (ii) a mRNA compound comprising an mRNA sequence encoding at least one antigenic peptide or protein,

wherein the mRNA compound optionally does not comprise a nucleoside modification, in particular not a base modification;
wherein the mRNA compound is encapsulated in or associated with said lipid nanoparticle.
5. The lipid nanoparticle according to any one of embodiments 1, 3 or 4, additionally comprising (iii) a PEG lipid with the formula (IV):
wherein R⁸ and R⁹ are each independently a straight or branched, saturated or unsaturated alkyl chain containing from 10 to 30 carbon atoms, wherein the alkyl chain is optionally interrupted by one or more ester bonds;
and w has a mean value ranging from 30 to 60.
6. The lipid nanoparticle according to any one of the preceeding embodiments, wherein the cationic lipid is a compound of formula III, and wherein:
L¹ and L² are each independently -O(C=O)- or (C=O)-O-;
G³ is C₁-C₂₄ alkylene or C₁₋C₂₄ alkenylene; and
R³ is H or OR⁵.
7. The lipid nanoparticle according to any one of the preceeding embodiments, wherein the cationic lipid is a compound of formula III, and wherein:
L¹ and L² are each independently -O(C=O)- or (C=O)-O-; and
R¹ and R² each independently have one of the following structures:
8. The lipid nanoparticle according to any one of the preceeding embodiments, wherein the cationic lipid is a compound of formula III, and wherein R³ is OH.
9. The lipid nanoparticle according to any one of the preceeding embodiments, wherein the cationic lipid is selected from structures I-1 to I-41, II-1 to II-34 or III-1 to III-36:

| **No.** | **Structure** |
|---|---|
| I-1 | |
| I-2 | |
| I-3 | |
| I-4 | |
| I-5 | |
| I-6 | |
| I-7 | |
| I-8 | |
| I-9 | |
| I-10 | |
| I-11 | |
| I-12 | |
| I-13 | |
| I-14 | |
| I-15 | |
| I-16 | |
| I-17 | |
| I-18 | |
| I-19 | |
| I-20 | |
| I-21 | |
| I-22 | |
| I-23 | |
| I-24 | |
| I-25 | |
| I-26 | |
| I-27 | |
| I-28 | |
| I-29 | |
| I-30 | |
| I-31 | |
| I-32 | |
| I-33 | |
| I-34 | |
| I-35 | |
| I-36 | |
| I-37 | |
| I-38 | |
| I-39 | |
| I-40 | |
| I-41 | |

or

| **No.** | **Structure** |
|---|---|
| II-1 | |
| II-2 | |
| II-3 | |
| II-4 | |
| II-5 | |
| II-6 | |
| II-7 | |
| II-8 | |
| II-9 | |
| II-10 | |
| II-11 | |
| II-1 | |
| II-1 | |
| II-1 | |
| II-1 | |
| II-1 | |
| II-1 | |
| II-1 | |
| II-19 | |
| II-20 | |
| II-21 | |
| II-22 | |
| II-23 | |
| II-24 | |
| II-25 | |
| II-26 | |
| II-27 | |
| II-28 | |
| II-29 | |
| II-30 | |
| II-31 | |
| II-32 | |
| II-33 | |
| II-34 | |
| II-35 | |
| II-36 | |

or

| **No.** | **Structure** |
|---|---|
| III-1 | |
| III-2 | |
| III-3 | |
| III-4 | |
| III-5 | |
| III-6 | |
| III-7 | |
| III-8 | |
| III-9 | |
| III-10 | |
| III-11 | |
| III-12 | |
| III-13 | |
| III-14 | |
| III-15 | |
| III-16 | |
| III-17 | |
| III-18 | |
| III-19 | |
| III-20 | |
| III-21 | |
| III-22 | |
| III-23 | |
| III-24 | |
| III-25 | |
| III-26 | |
| III-27 | |
| III-28 | |
| III-29 | |
| III-30 | |
| III-31 | |
| III-32 | |
| III-33 | |
| III-34 | |
| III-35 | |
| III-36 | |

10. The lipid nanoparticle according to any one of the preceeding embodiments, wherein the cationic lipid is selected from: and 11. The lipid nanoparticle according to any one of the preceeding embodiments, wherein in the PEG lipid R⁸ and R⁹ are saturated alkyl chains.
12. The lipid nanoparticle according to embodiment 11, wherein the PEG lipid is wherein n has a mean value ranging from 30 to 60.
13. The lipid nanoparticle according to any one of embodiments 1 to 12, wherein the mRNA compound comprises at least one chemical modification, and wherein the mRNA compound preferably does not comprise a nucleoside modification, wherein said nucleoside modification is optionally a base modification, and wherein said base modification is optionally a 1-methylpseudouridine modification.
14. The lipid nanoparticle according to embodiment 13, wherein the chemical modification is selected from the group comprising sugar modifications, backbone modifications and lipid modifications.
15. The lipid nanoparticle according to any one of embodiments 1 to 14, wherein the mRNA sequence is an artificial mRNA sequence.
16. The lipid nanoparticle according to any one of embodiments 1 to 15 wherein the coding region of the mRNA sequence encoding the at least one antigenic peptide or protein comprises a sequence modification.
17. The lipid nanoparticle according to embodiment 16, wherein the sequence modification is selected from a G/C content modification, a codon modification, a codon optimization or a C-optimization of the sequence.
18. The lipid nanoparticle according to embodiment 17, wherein the G/C content of the coding region of the mRNA sequence is increased compared to the G/C content of the corresponding coding sequence of the wild type mRNA, or wherein the C content of the coding region of the mRNA sequence is increased compared to the C content of the corresponding coding sequence of the wild type mRNA, or wherein the codon usage in the coding region of the mRNA sequence is adapted to the human codon usage, or wherein the codon adaptation index (CAI) is increased or maximised in the coding region of the mRNA sequence, wherein the encoded amino acid sequence of the mRNA sequence is preferably not being modified compared to the encoded amino acid sequence of the wild type mRNA.
19. The lipid nanoparticle according to any one of embodiments 1 to 18, wherein the mRNA sequence additionally comprises
a) a 5'-CAP structure, and/or
b) a poly(A) sequence, and/or
c) a poly (C) sequence.
20. The lipid nanoparticle according to embodiment 19, wherein the mRNA sequence comprises a poly(A) sequence, wherein the poly(A) sequence comprises a sequence of about 25 to about 400 adenosine nucleotides, preferably a sequence of about 50 to about 400 adenosine nucleotides, more preferably a sequence of about 50 to about 300 adenosine nucleotides, even more preferably a sequence of about 50 to about 250 adenosine nucleotides, most preferably a sequence of about 60 to about 250 adenosine nucleotides.
21. The lipid nanoparticle according to any one of embodiments 1 to 19, wherein the mRNA sequence additionally comprises at least one histone stem loop.
22. The lipid nanoparticle according to embodiment 21, wherein the at least one histone stem-loop comprises a nucleic acid sequence according to the following formulae (V) or (VI): wherein
stem1 or stem2 bordering elements N1-6 is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
stem1 [N₀₋₂GN₃₋₅]is reverse complementary or partially reverse complementary with element stem2, and is a consecutive sequence between of 5 to 7 nucleotides;
wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
loop sequence [N₀₋₄(U/T)N₀₋₄]is located between elements stem1 and stem2, and is a consecutive sequence of 3 to 5 nucleotides, more preferably of 4 nucleotides;
wherein each N₀₋₄ is independent from another a consecutive sequence of 0 to 4, preferably of 1 to 3, more preferably of 1 to 2 N, wherein each N is independently from another selected from a nucleotide selected from A, U, T, G and C or a nucleotide analogue thereof; and
wherein U/T represents uridine, or optionally thymidine;
stem2 [N₃₋₅CN₀₋₂] is reverse complementary or partially reverse complementary with element stem1, and is a consecutive sequence between of 5 to 7 nucleotides;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof; and
wherein C is cytidine or an analogue thereof, and may be optionally replaced by a guanosine or an analogue thereof provided that its complementary nucleotide guanosine in stem1 is replaced by cytidine;
23. The lipid nanoparticle according to embodiment 21 or 22, wherein the at least one histone stem loop comprises a nucleic acid sequence according to SEQ ID NO: 224305 and/or most preferably an RNA sequence according to SEQ ID NO: 224306.
24. The lipid nanoparticle according to any one of embodiments 19 to 23, wherein the mRNA sequence comprises a poly(A) sequence, preferably comprising 10 to 200, 10 to 100, 40 to 80 or 50 to 70 adenosine nucleotides, and/or a poly(C) sequence, preferably comprising 10 to 200, 10 to 100, 20 to 70, 20 to 60 or 10 to 40 cytosine nucleotides.
25. The lipid nanoparticle according to any one of embodiments 1 to 24, wherein the mRNA sequence comprises, preferably in 5' to 3'-direction, the following elements:
a) a 5'-CAP structure, preferably m7GpppN,
b) at least one coding region encoding at least one antigenic peptide or protein,
c) a poly(A) tail, preferably consisting of 10 to 200, 10 to 100, 40 to 80 or 50 to 70 adenosine nucleotides,
d) optionally a poly(C) tail, preferably consisting of 10 to 200, 10 to 100, 20 to 70, 20 to 60 or 10 to 40 cytosine nucleotides, and
e) optionally a histone stem-loop, preferably comprising the RNA sequence according to SEQ ID NO: 224306.
26. The lipid nanoparticle according to any one of embodiments 1 to 25, wherein the mRNA sequence comprises a 3'-UTR element.
27. The lipid nanoparticle according to embodiment 26, wherein the at least one 3'-UTR element comprises or consists of a nucleic acid sequence which is derived from a 3'-UTR of a gene providing a stable mRNA or from a homolog, a fragment or a variant thereof.
28. The lipid nanoparticle according to embodiment 26 or 27, wherein the 3'-UTR element comprises a nucleic acid sequence derived from a 3'-UTR of an α-globin gene, preferably comprising the corresponding RNA sequence of the nucleic acid sequence according to SEQ ID NO: 224297, a homolog, a fragment, or a variant thereof.
29. The lipid nanoparticle according to any one of embodiments 26 to 28, wherein the at least one 3'-UTR element comprises a nucleic acid sequence, which is derived from the 3'-UTR of a vertebrate albumin gene or from a variant thereof, preferably from the 3'-UTR of a mammalian albumin gene or from a variant thereof, more preferably from the 3'-UTR of a human albumin gene or from a variant thereof, even more preferably from the 3'-UTR of the human albumin gene according to GenBank Accession number NM_000477.5, or from a fragment or variant thereof.
30. The lipid nanoparticle according to any one of embodiments 26 to 29, wherein the 3'-UTR element is derived from a nucleic acid sequence according to SEQ ID NO: 224301 or 224303, preferably from a corresponding RNA sequence, or a homolog, a fragment or a variant thereof.
31. The lipid nanoparticle according to any one of embodiments 1 to 30, wherein the mRNA sequence comprises, preferably in 5' to 3'-direction, the following elements:
a) a 5'-CAP structure, preferably m7GpppN,
b) at least one coding region encoding at least one antigenic peptide or protein,
c) a 3'-UTR element comprising or consisting of a nucleic acid sequence which is derived from an alpha globin gene, preferably comprising the corresponding RNA sequence of the nucleic acid sequence according to SEQ ID NO: 224297, a homolog, a fragment or a variant thereof;
d) a poly(A) tail, preferably consisting of 10 to 200, 10 to 100, 40 to 80 or 50 to 70 adenosine nucleotides,
e) optionally a poly(C) tail, preferably consisting of 10 to 200, 10 to 100, 20 to 70, 20 to 60 or 10 to 40 cytosine nucleotides, and
f) optionally a histone stem-loop, preferably comprising the RNA sequence according to SEQ ID NO: 224306.
32. The lipid nanoparticle according to any one of embodiments 1 to 31, wherein the mRNA sequence comprises a 5'-UTR element.
33. The lipid nanoparticle according to embodiment 32, wherein the 5'-UTR element comprises or consists of a nucleic acid sequence which is derived from the 5'-UTR of a TOP gene preferably from a corresponding RNA sequence, a homolog, a fragment, or a variant thereof, preferably lacking the 5'-TOP motif.
34. The lipid nanoparticle according to embodiment 33, wherein the 5'-UTR element comprises or consists of a nucleic acid sequence which is derived from a 5'-UTR of a TOP gene encoding a ribosomal protein, preferably from a corresponding RNA sequence or from a homolog, a fragment or a variant thereof, preferably lacking the 5'TOP motif.
35. The lipid nanoparticle according to embodiment 34, wherein the 5'-UTR element comprises or consists of a nucleic acid sequence which is derived from a 5'-UTR of a TOP gene encoding a ribosomal Large protein (RPL) or from a homolog, a fragment or variant thereof, preferably lacking the 5'-TOP motif and more preferably comprising or consisting of a corresponding RNA sequence of the nucleic acid sequence according to SEQ ID NO: 224287.
36. The lipid nanoparticle according to embodiment 35, wherein the 5'-UTR element which is derived from a 5'-UTR of a TOP gene comprises or consists of a corresponding RNA sequence of a nucleic acid sequence according to SEQ ID NO: 224287 or 224289.
37. The lipid nanoparticle according to any one of embodiments 1 to 36, wherein the mRNA sequence comprises, preferably in 5' to 3'-direction, the following elements:
a) a 5'-CAP structure, preferably m7GpppN,
b) a 5'-UTR element which comprises or consists of a nucleic acid sequence which is derived from the 5'-UTR of a TOP gene, preferably comprising or consisting of the corresponding RNA sequence of a nucleic acid sequence according to SEQ ID NO: 224287 or 224289, a homolog, a fragment or a variant thereof;
c) at least one coding region encoding at least one antigenic peptide or protein;
d) a 3'-UTR element comprising or consisting of a nucleic acid sequence which is derived from an albumin gene, preferably comprising the corresponding RNA sequence of the nucleic acid sequence according to SEQ ID NO: 224303, a homolog, a fragment or a variant thereof;
e) a poly(A) tail, preferably consisting of 10 to 200, 10 to 100, 40 to 80 or 50 to 70 adenosine nucleotides,
f) optionally a poly(C) tail, preferably consisting of 10 to 200, 10 to 100, 20 to 70, 20 to 60 or 10 to 40 cytosine nucleotides, and
g) optionally a histone stem-loop, preferably comprising the RNA sequence according to SEQ ID NO: 224306.
38. The lipid nanoparticle according to any one of embodiments 1 to 37, wherein the antigenic peptide or protein is derived from pathogenic antigens, tumour antigens, allergenic antigens or autoimmune self-antigens or a fragment or variant thereof.
39. The lipid nanoparticle according to embodiment 38, wherein the pathogenic antigen is derived from an influenza or rabies virus.
40. The lipid nanoparticle according embodiment 39, wherein the antigenic peptide or protein is derived from hemagglutinin (HA), neuraminidase (NA), nucleoprotein (NP), matrix protein 1 (M1), matrix protein 2 (M2), non-structural protein 1 (NS1), non-structural protein 2 (NS2), nuclear export protein (NEP), polymerase acidic protein (PA), polymerase basic protein PB1, PB1-F2, or polymerase basic protein 2 (PB2) of an influenza virus or a fragment or variant thereof.
41. The lipid nanoparticle according to embodiments 40, wherein the antigenic peptide or protein is derived from hemagglutinin (HA) or neuraminidase (NA) of an influenza virus or a fragment or variant thereof.
42. The lipid nanoparticle according to embodiment 41, wherein the antigenic peptide or protein is at least one full-length protein of hemagglutinin (HA) and/or at least one full-length protein of neuraminidase (NA) of an influenza virus or a variant thereof.
43. The lipid nanoparticle according to any one of embodiments 39 to 41, wherein the influenza virus is selected from an influenza A, B or C virus.
44. The lipid nanoparticle according to embodiment 43, wherein the influenza A virus is selected from an influenza virus characterized by a hemagglutinin (HA) selected from the group consisting of H1, H2, H3, H4, H5, H6, H7, H8, H9, H10, H11, H12, H13, H14, H15, H16, H17 and H18.
45. The lipid nanoparticle according to embodiment 43 or 44, wherein the influenza A virus is selected from an influenza virus characterized by a neuraminidase (NA) selected from the group consisting of N1, N2, N3, N4, N5, N6, N7, N8, N9, N10, and N11.
46. The lipid nanoparticle according to any one of embodiments 43 to 45, wherein the influenza A virus is selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, and H10N7, preferably from H1N1, H3N2, H5N1.
47. The lipid nanoparticle according to any one of embodiments 39 to 46, wherein the mRNA sequence comprises at least one coding region encoding at least one antigenic peptide or protein derived from hemagglutinin (HA) of an influenza virus or a fragment or variant thereof and at least one antigenic peptide or protein derived from neuraminidase (NA) of an influenza virus or a fragment or variant thereof.
48. The lipid nanoparticle according to any one of embodiments 39 to 47, wherein the mRNA sequence comprises at least one coding region encoding at least one antigenic peptide or protein derived from hemagglutinin (HA) and/or at least one antigenic peptide or protein derived from neuraminidase (NA) of an influenza A virus selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, and H10N7, preferably from H1N1, H3N2, H5N1 or a fragment or variant thereof.
49. The lipid nanoparticle according to any of embodiments 39 to 48, wherein the mRNA sequence comprises at least one coding region encoding at least one antigenic peptide or protein derived from hemagglutinin (HA) of an influenza A virus according to SEQ ID NOs: 1-14031 or SEQ ID NO: 224309 or 224310 or a fragment or variant thereof.
50. The lipid nanoparticle according to any one of embodiments 39 to 49, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs: 32013-46043, 64025-78055, 224085-224106, 96037-110067, 128049-142079, 160061-174091, 192073-206103 or a fragment or variant thereof.
51. The lipid nanoparticle according to any one of embodiments 39 to 50, wherein the mRNA sequence comprises at least one coding region encoding at least one antigenic peptide or protein derived from hemagglutinin (HA) of an influenza B virus according to SEQ ID NOs: 26398-28576 or a fragment or variant thereof.
52. The lipid nanoparticle according to any of embodiments 39 to 51, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs: 58410-60588, 90422-92600, 224107-224112, 122434-124612, 154446-156624, 186458-188636, 218470-220648 or a fragment or variant thereof.
53. The lipid nanoparticle according to any one of embodiments 39 to 52, wherein the mRNA sequence comprises at least one coding region encoding at least one antigenic peptide or protein derived from neuraminidase (NA) of an influenza A virus according to SEQ ID NOs: 14032-26397, 224309, or 224310 or a fragment or variant thereof.
54. The lipid nanoparticle according to any one of embodiments 39 to 53, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs: 46044-58409, 224311, 224312, 78056-90421, 224113, 224313-224317, 110068-122433, 142080-154445, 174092-186457, 206104-218469 or a fragment or variant thereof.
55. The lipid nanoparticle according to any one of embodiments 39 to 54, wherein the mRNA sequence comprises at least one coding region encoding at least one antigenic peptide or protein derived from neuraminidase (NA) of an influenza B virus according to SEQ ID NOs: 28577-30504 or a fragment or variant thereof.
56. The lipid nanoparticle according to any of embodiments 39 to 55, wherein the mRNA sequence comprises at least one RNA sequence or a mixture of RNA sequences selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs: 60589-62516, 92601-94528, 124613-126540, 156625-158552, 188637-190564, 220649-222576 or a fragment or variant thereof.
57. The lipid nanoparticle according to any of embodiments 39 to 56, wherein the mRNA sequence comprises at least one coding region encoding at least one antigenic peptide or protein derived from glycoprotein G (RAV-G, RAVBV-G or RABV-G), nucleoprotein N (RAV-N), phospoprotein P (RAV-P), matrix protein M (RAV-M) or RNA polymerase L (RAV-L) of a Rabies virus or a fragment, variant thereof.
58. The lipid nanoparticle according to any of embodiments 39 to 57, wherein the mRNA sequence comprises at least one coding region encoding at least one antigenic peptide or protein derived from glycoprotein G (RAV-G, RAVBV-G or RABV-G) of a Rabies virus according to SEQ ID NOs: 30505-32012 or a fragment or variant thereof.
59. The lipid nanoparticle according to any of embodiments 39 to 58, wherein the mRNA sequence comprises at least one RNA sequence selected from RNA sequences being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according to SEQ ID NOs: 62517-64024, 224270, 224274, 94529-96036, 224271-224273, 126541-128048, 158553-160060, 190565-192072, 222577-224084 or a fragment or variant thereof.
60. The lipid nanoparticle according to any one of the preceding embodiments, wherein the mRNA sequence comprises an RNA sequence being identical or at least 50%, 60%, 70%, 80%, 85%, 86%, 87%, 88%, 89%, 90%, 91%, 92%, 93%, 94%, 95%, 96%, 97%, 98%, or 99% identical to the RNA sequences according SEQ ID NOs:224085-224106, 224114-224235, 224085-224106, 224114-224235, 224107-224112, 224236-224263, 224107-224112, 224107-224112, 224236-224263, 224113, 224264-224266, 224309-224347, 224309, 224310, 224311, 224312, 224113, 224313-224317, 224113, 224311-224317, 224264-224266, 224318-224347, 224348-224357, 224348-224357, 224270-224284, 224270, 224271-224273, 224270-224273, 224274-224284, 224358-224362, 224358, 224359, 224360, 224359, 224360, 224361, 224362, 224363, 224364, preferably at least one coding region according to SEQ ID NO: 224114-224235, 224236-224263, 224264-224266, 224318-224347, 224348-224357, 224274-224284, 224361, 224362, 224363, 224364, most preferably at least one coding region according to SEQ ID NO: 224118, SEQ ID NO: 224117, SEQ ID NO: 224181, SEQ ID NO: 224236, SEQ ID NO: 224200, SEQ ID NO: 224166, SEQ ID NO: 224236, SEQ ID NO: 224246, SEQ ID NO: 224318, SEQ ID NO: 224336, SEQ ID NO: 224348, SEQ ID NO: 224276, SEQ ID NO: 224280, or a fragment or variant thereof.
61. The lipid nanoparticle according to any one of embodiments 1 to 60, wherein the mRNA compound comprises at least two mRNA sequences, wherein the at least two mRNA sequences encode two different antigenic peptides and/or proteins.
62. The lipid nanoparticle according to any one of embodiments 1 to 61, comprising at least two different mRNA compounds, each compound comprising a mRNA sequence which encodes at least one antigenic peptide or protein.
63. The lipid nanoparticle according to any one of embodiments 1 to 62, additionally comprising:
(iv) a neutral lipid; and/or
(v) a steroid or steroid analogue.
64. The lipid nanoparticle according to any one of embodiments 1 to embodiment 63, wherein the neutral lipid is selected from the group comprising distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl-phosphatidylethanolamine (POPE) and dioleoyl-phosphatidylethanolamine 4-(N-maleimidomethyl)-cyclohexane-1carboxylate (DOPE-mal), dipalmitoyl phosphatidyl ethanolamine (DPPE), dimyristoylphosphoethanolamine (DMPE), distearoyl-phosphatidylethanolamine (DSPE), 16-0-monomethyl PE, 16-O-dimethyl PE, 18-1-trans PE, 1-stearioyl-2-oleoylphosphatidyethanol amine (SOPE), and 1,2-dielaidoyl-sn-glycero-3-phophoethanolamine (transDOPE).
65. The lipid nanoparticle according to any one of embodiments 1 to embodiment 64 wherein the neutral lipid is 1,2-distearoyl-sn-glycero-3-phosphocholine (DSPC), and wherein the molar ratio of the cationic lipid to DSPC is optionally in the range from about 2:1 to 8:1.
66. The lipid nanoparticle according to any one of embodiments 1 to 65, wherein the steroid is cholesterol, and wherein the molar ratio of the cationic lipid to cholesterol is optionally in the range from about 2:1 to 1:1
67. A method for the preparation of a lipid nanoparticle according to any one of embodiments 1 to 66, comprising the steps of:
(i) providing
   a) a cationic lipid of formula (I)
      as defined above or a pharmaceutically acceptable salt, tautomer, prodrug or stereoisomer thereof, and/or
      of formula (II)
      as defined abova or a pharmaceutically acceptable salt, tautomer, prodrug or stereoisomer thereof, and/or
      of formula III:
      as defined above or a pharmaceutically acceptable salt, tautomer, prodrug or stereoisomer thereof; and/or
   b) a PEG lipid with the formula (IV): as defined above;
   c) at least one mRNA compound comprising an mRNA sequence encoding at least one antigenic peptide or protein, wherein the mRNA compound optionally does not comprise a nucleoside modification, in particular not a base modification; and
   d) optionally a steroid; and
   e) optionally a neutral lipid;
(ii) solubilizing the cationic lipid and/or the PEG lipid and optionally the neutral lipid and/or the steroid or a steroid derivative in ethanol;
(iii) mixing the ethanolic lipid solution with an aqueous solution comprising the mRNA polynucleotide
(iv) removing the ethanol; and optionally
(v) separating or purifying the lipid nanoparticles.
68. The method according to embodiment 67, wherein in step (iv) the ethanol is removed by dialysis or diafiltration.
69. The method according to embodiment 67 or 68, wherein in step (v) the lipid nanoparticles are purified by filtration, preferably by filtration through a sterile filter.
70. A pharmaceutical composition comprising at least one lipid nanoparticle according to any one of embodiments 1 to 66.
71. The pharmaceutical composition according to embodiment 70, comprising at least a first and a second lipid nanoparticle according to any one of embodiments 1 to 66, wherein the mRNA compound comprised by the second lipid nanoparticle is different from the mRNA compound comprised by the first lipid nanoparticle.
72. The pharmaceutical composition according to embodiment 70 or 71, additionally comprising a pharmaceutically acceptable adjuvant or excipient.
73. A lipid nanoparticle according to any one of embodiments 1 to 66 or a pharmaceutical composition according to any one of embodiments 70 to 72 for use as a medicament.
74. The lipid nanoparticle or the pharmaceutical composition for use according to embodiment 73, wherein the medicament is for therapeutically or prophylactically raising an immune response of a subject in need thereof.
75. The lipid nanoparticle or the pharmaceutical composition for use according to embodiment 73 or 74, wherein the medicament is for prevention or treatment of cancer or tumour diseases, infectious diseases, allergies, or autoimmune diseases or disorders related thereto.
76. The lipid nanoparticle or the pharmaceutical composition for use according to any one of embodiments 73 to 75, wherein the medicament is a vaccine.
77. The lipid nanoparticle or the pharmaceutical composition for use according to any one of embodiments 73 to 76, wherein the medicament is a tumor, influenza or rabies vaccine.
78. The lipid nanoparticle or the pharmaceutical composition for use according any one of embodiments 73 to 77, wherein the medicament is a rabies vaccine used in rabies treatment.
79. The lipid nanoparticle or the pharmaceutical composition for use according to any one of embodiments 73 to 78, wherein the subject is a vertebrate, preferably a mammal.
80. The lipid nanoparticle or the pharmaceutical composition for use according to any one of embodiments 73 to 79, wherein the medicament is for parenteral administration and wherein parenteral administration comprises injection.
81. The lipid nanoparticle or the pharmaceutical composition for use according to any one of embodiments 73 to 80, wherein the subject is a bird, preferably chicken, or a mammal, preferably selected from the group comprising goat, cattle, swine, dog, cat, donkey, monkey, ape, a rodent such as a mouse, hamster, rabbit and, particularly, human.
82. A method for raising an immune response in a subject in need thereof, comprising administering to the subject a lipid nanoparticle according to any one of embodiments 1 to 66 or a pharmaceutical composition according to any of the embodiments 70 to 72.
83. A method for prevention or treatment of cancer or tumour diseases, infectious diseases, allergies, or autoimmune diseases or disorders related thereto in a subject in need thereof, comprising administering to the subject a lipid nanoparticle according to any one of embodiments 1 to 66 or a pharmaceutical composition according to any of the embodiments 70 to 72.
84. The lipid nanoparticle according to any one of the preceeding embodiments, whereby the lipid nanoparticle has a molar ratio (mol%) of 47.4:10:40.9:1.7 (cationic lipid, DSPC, cholesterol and PEG-lipid).
85. The lipid nanoparticle according to any one of the preceeding embodiments, whereby the lipid nanoparticle comprises:
(i) an mRNA encoding a HA protein of influenza A/Hong Kong/4801/2014 (H3N2) (preferably selected from the group consisting of SEQ ID NOs: 13853, 13854, 13855 and 13856); and/or
(ii) an mRNA encoding a HA protein of influenza A/California/07/2009 (H1N1) (preferably selected from the group consisting of SEQ ID NOs: 13836, 13837, 13838, 13839, 13840, 13841, 13842, 13843, and 13844); and/or
(iii) an mRNA encoding a HA protein of influenza B/Phuket/3037/2013 (EPI540671) (preferably selected from the group consisting of SEQ ID NOs: 28530, 28531 and 28532); and/or
(iv) an mRNA encoding a HA protein of influenza B/Brisbane/60/2008 (preferably selected from the group consisting of SEQ ID NOs: 28524, 28525, 28526, 28527, 28528, and 28529).
86. The lipid nanoparticle according to any one of the preceeding embodiments, whereby the lipid nanoparticle comprises:
(i) an mRNA encoding a NA protein of influenza A/Hong Kong/4801/2014 (H3N2) (preferably selected from the group consisting of SEQ ID NOs: 26251, 26252, 26253, and 26254); and/or
(ii) an mRNA encoding a NA protein of influenza A/California/7/2009 (H1N1)pdm09 (preferably selected from the group consisting of SEQ ID NOs: 26238, 26239, 26240, 26241, 26242, and 26243); and/or
(iii) an mRNA encoding a NA protein of influenza B/Brisbane/60/2008 (preferably selected from the group consisting of SEQ ID NOs: 30455, 30456, 30457, 30458, 30459, and 30460).
87. The lipid nanoparticle according to any one of the preceeding embodiments, whereby the lipid nanoparticle comprises:
(i) an mRNA encoding a HA protein of influenza A/Netherlands/602/2009 (H1N1) (preferably selected from the group consisting of SEQ ID NOs: 13848, 13849, and 13850); and/or
(ii) an mRNA encoding a HA protein of influenza A/Hong Kong/4801/2014 (H3N2) (preferably selected from the group consisting of SEQ ID NOs: 13853, 13854, 13855, and 13856); and/or
(iii) an mRNA encoding a HA protein of influenza B/Brisbane/60/2008 (preferably selected from the group consisting of SEQ ID NOs: 28524, 28525, 28526, 28527, 28528 and 28529); and/or
(iv) an mRNA encoding a HA protein of influenza A/Vietnam/1194/2004 (H5N1) (preferably selected from the group consisting of SEQ ID NOs: 13859 and 13860).
88. The lipid nanoparticle according to any one of the preceeding embodiments, whereby the lipid nanoparticle comprises:
(i) an mRNA encoding a HA protein of influenza A/Hong Kong/4801/2014 (H3N2) (preferably selected from the group consisting of SEQ ID NOs: 13853, 13854, 13855, and 13856); and/or
(ii) an mRNA encoding a HA protein of influenza A/California/07/2009 (H1N1) (preferably selected from the group consisting of SEQ ID NOs: 13836, 13837, 13838, 13839, 13840, 13841, 13842, 13843, and 13844); and/or
(iii) an mRNA encoding a HA protein of influenza B/Phuket/3037/2013 (EPI540671) (preferably selected from the group consisting of SEQ ID NOs: 28530, 28531, and 28532); and/or
(iv) an mRNA encoding a HA protein of influenza B/Brisbane/60/2008 (preferably selected from the group consisting of SEQ ID NOs: 28524, 28525, 28526, 28527, 28528, and 28529); and/or
(v) an mRNA encoding a NA protein of influenza A/Hong Kong/4801/2014 (H3N2) (preferably selected from the group consisting of SEQ ID NOs: 26251, 26252, 26253, and 26254); and/or
(vi) an mRNA encoding a NA protein of influenza A/California/7/2009 (H1N1)pdm09 (preferably selected from the group consisting of SEQ ID NOs: 26238, 26239, 26240, 26241, 26242 and 26243); and/or
(vii) an mRNA encoding a NA protein of influenza B/Brisbane/60/2008 (preferably selected from the group consisting of SEQ ID NOs: 30455, 30456, 30457, 30458, 30459, and 30460).

## Claims

1. A pharmaceutical composition comprising
(a) at least one lipid nanoparticle comprising:
(i) a cationic lipid with the formula III: or a pharmaceutically acceptable salt, tautomer or stereoisomer thereof, wherein:
L¹ and L² are each independently -O(C=O)- or (C=O)-O-;
G¹ and G² are each independently unsubstituted C₁-C₁₂ alkylene or C₁-C₁₂ alkenylene;
G³ is C₁-C₂₄ alkylene or C₁-C₂₄ alkenylene;
R¹ and R² are each independently C₆-C₂₄ alkyl or C₆-C₂₄ alkenyl;
R³ is H or OR⁵; and
R⁵ is H or C₁-C₆ alkyl;
and
(ii) a polyethylene glycol-lipid (pegylated lipid);
(iii) a neutral lipid; and
(iv) a steroid or steroid analogue;
wherein the neutral lipid is 1,2-distearoyl-sn-glycero-3-phosphocholine (DSPC), and wherein the molar ratio of the cationic lipid to DSPC is in the range from about 2:1 to 8:1; and wherein the steroid is cholesterol, and wherein the molar ratio of the cationic lipid to cholesterol is in the range from about 2:1 to 1:1; and wherein the molar ratio of the cationic lipid to the pegylated lipid ranges from about 100:1 to about 25:1; and
(b) a mRNA comprising at least one mRNA sequence encoding at least one antigenic peptide or protein derived from hemagglutinin (HA) and/or neuraminidase (NA) of an influenza virus, preferably wherein the influenza virus is selected from an influenza A, B or C virus, more preferably wherein the virus is an influenza A virus selected from the group consisting of H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, H10N8, and H10N7, even more preferably selected from the group consisting of H1N1, H3N2, and H5N1,
wherein the at least one mRNA sequence additionally comprises at least one of the following structural elements:
i. a heterologous 5'-UTR element, preferably comprising a nucleic acid sequence which is derived from the 5'-UTR of a TOP gene, more preferably comprising or consisting of the corresponding RNA sequence of a nucleic acid sequence according to SEQ ID NO:224287 or SEQ ID NO:224288; or
ii. a heterologous 3'-UTR element, preferably derived from a 3'-UTR of an α-or β-globin gene, more preferably a vertebrate α-or β-globin gene, even more preferably a mammalian α-or β-globin gene, most preferably a human α-or β globin gene according to SEQ ID NO:224291, 224293, 224295, or 224297, preferably according to SEQ ID NO:224297.

2. The pharmaceutical composition according to claim 1, wherein R³ is OH.

3. The pharmaceutical composition according to any one of claim 1 to claim 2, wherein the pegylated lipid is pegylated diacylglycerol (PEG-DAG), pegylated phosphatidylethanoloamine (PEG-PE), PEG succinate diacylglycerol (PEG-S-DAG), or PEG dialkoxypropylcarbamate.

4. The pharmaceutical composition according to any one of claim 1 to claim 2, wherein the pegylated lipid has the structure of formula (IV): or a pharmaceutically acceptable salt, tautomer or stereoisomer thereof, wherein:
R⁸ and R⁹ are each independently a straight or branched, saturated or unsaturated alkyl chain containing from 10 to 30 carbon atoms, wherein the alkyl chain is optionally interrupted by one or more ester bonds; and w has mean value ranging from 30 to 60.

5. The pharmaceutical composition according to any one of claim 1 to claim 4, wherein the cationic lipid is present in the lipid nanoparticle in an amount from about 40 to about 60 mole percent and wherein the pegylated lipid is present in the lipid nanoparticle in an amount from about 1 to about 10 mole percent, relative to the total lipid content of the lipid nanoparticle.

6. The pharmaceutical composition according to any one of claim 1 to claim 5, wherein the mRNA sequence comprises at least one coding region encoding an mRNA encoding a HA protein of influenza A/Hong Kong/4801/2014 (H3N2), preferably at least one antigenic peptide or protein derived from hemagglutinin (HA) of an influenza A virus having a sequence identity of at least 80% with any one of SEQ ID NO:1-14031, preferably selected from the group consisting of SEQ ID NO:13853, 13854, 13855, and 13856, or an mRNA encoding HA protein of influenza A/Hong Kong/4801/2014 (H3N2) selected from any one of SEQ ID NO:224181 to 224194.

7. The pharmaceutical composition according to any one of claim 1 to claim 5 comprising a plurality of mRNA sequences each encoding at least one antigenic peptide or protein derived from hemagglutinin (HA) and/or neuraminidase (NA) of an influenza virus, wherein at least one antigenic peptide or protein derived from hemagglutinin (HA) and/or neuraminidase (NA) of 2, 3, 4, 5, 6, 7, 8, 9, 10, 11, 12, 13, 14, 15, 16, 17, 18, 19, 20, 25, 30, 35, 40, 45, 50, 60, 70, 80, or 100 different influenza viruses are encoded by the plurality of mRNA sequences.

8. The pharmaceutical composition according to any one of claim 1 to claim 7, wherein at least one mRNA sequence is selected from RNA sequences being at least 80% identical to any one of SEQ ID NO:32013-46043, 64025-78055, 96037-110067, 128049-142079, 160061-174091, or 192073-206103.

9. The pharmaceutical composition according to any one of claim 1 to claim 7, wherein at least one mRNA sequence is selected from RNA sequences being at least 80% identical to any one of SEQ ID NO:64025-78055, 224085-224106, 192073-206103.

10. The pharmaceutical composition according to any one of claim 1 to claim 7, wherein at least one mRNA sequence is selected from RNA sequences being at least 80% identical to any one of SEQ ID NO:90422-92600, 224107-224112, 218470-220648.

11. The pharmaceutical composition according to any one of claim 1 to claim 7, wherein at least one mRNA sequence is selected from RNA sequences being at least 80% identical to any one of SEQ ID NO:78056-90421, 224113, 224313-224317, 206104-218469.

12. The pharmaceutical composition according to any one of claim 1 to claim 7, wherein at least one mRNA sequence is selected from RNA sequences being at least 80% identical to any one of SEQ ID NO:92601-94528, 220649-222576.

13. The pharmaceutical composition according to any one of claim 1 to claim 5, wherein at least one mRNA sequence additionally comprises in 5' to 3' direction, the following elements:
(a) a 5'-CAP structure, preferably m7GpppN;
(b) a 5'-UTR element which comprises or consists of a nucleic acid sequence which is derived from the 5'-UTR of a TOP gene, more preferably comprising or consisting of the corresponding RNA sequence of a nucleic acid sequence according to SEQ ID NO:224287, as shown in SEQ ID NO:224288;
(c) a poly(A) tail, preferably consisting of 10 to 200, 10 to 100, 40 to 80 or 50 to 70 adenosine nucleotides.

14. The pharmaceutical composition according to any one of claim 1 to claim 7, wherein at least one mRNA sequence additionally comprises in 5' to 3' direction, the following elements:
(a) a 5'-CAP structure, preferably m7GpppN;
(b) a 3'-UTR element which is derived from a 3'-UTR of an α-or β-globin gene, preferably a vertebrate α-or β-globin gene, more preferably a mammalian α-or β-globin gene, most preferably a human α-or β globin gene according to SEQ ID NO:224291, 224293, 224295, or 224297, preferably according to SEQ ID NO:224297; and
(c) a poly(A) tail, preferably consisting of 10 to 200, 10 to 100, 40 to 80 or 50 to 70 adenosine nucleotides.

15. The pharmaceutical composition according to any one of claim 1 to claim 7 comprising a mRNA comprising a mRNA sequence encoding at least one antigenic peptide or protein derived from hemagglutinin (HA) and/or at least one mRNA sequence encoding at least one antigenic peptide or protein derived from neuraminidase (NA) of influenza A virus H1N1, at least one mRNA sequence encoding at least one antigenic peptide or protein derived from hemagglutinin (HA) and/or at least one mRNA sequence encoding at least one antigenic peptide or protein derived from neuraminidase (NA) of influenza A virus H3N2, at least one mRNA sequence encoding at least one antigenic peptide or protein derived from hemagglutinin (HA) and/or at least one mRNA sequence encoding at least one antigenic peptide or protein derived from neuraminidase (NA) of influenza A virus H5N1.
